# EUROPÄISCHE PATENTSCHRIFT

(11) **EP 2 125 789 B1**
(45) Veröffentlichungstag und Bekanntmachung des Hinweises auf die Patenterteilung: **16.03.2011**
(21) Anmeldenummer: 07856587.6
(22) Anmeldetag: 12.12.2007
(51) Int. Cl.: C07D 403/04, A01N 43/54

(54) **PYRIMIDINYLPYRAZOLE ALS INSEKTIZIDE UND PARASITIZIDE WIRKSTOFFE**
PYRIMIDINYL PYRAZOLES AS INSECTICIDES AND PARASITICIDE ACTIVE AGENTS
PYRIMIDINYLPYRAZOLES EN TANT QUE SUBSTANCES ACTIVES INSECTICIDES ET PARASITICIDES

(30) Priorität: 20.12.2006 DE 102006060230; 20.01.2007 DE 102007003036
(43) Veröffentlichungstag der Anmeldung: 02.12.2009
(60) Teilanmeldung: 10173541.3
(73) Patentinhaber: Bayer CropScience AG, 40789 Monheim (DE)
(72) Erfinder: FRACKENPOHL, Jens, 60322 Frankfurt (DE); GEBAUER, Olaf, 51377 Leverkusen (DE); CEREZO-GALVEZ, Silvia, 40674 Langenfeld (DE); ES-SAYED, Mazen, 40764 Langenfeld (DE); GÖRGENS, Ulrich, 40882 Ratingen (DE); FRANKEN, Eva-Maria, 69009 Lyon (FR); MALSAM, Olga, 51503 Rösrath (DE); SCHNATTERER, Stefan, 65795 Hattersheim (DE); ARNOLD, Christian, 40764 Langenfeld (DE); LÜMMEN, Peter, 65510 Idstein (DE); SCHWARZ, Hans-Georg, 40764 Langenfeld (DE); HENSE, Achim, 51379 Leverkusen (DE); WERNER, Stefan, 40789 Monheim am Rhein (DE); MAECHLING, Simon, 50670 Köln (DE)
(86) Internationale Anmeldenummer: PCT/EP2007/010839
(87) Internationale Veröffentlichungsnummer: WO 2008/077483

(56) Entgegenhaltungen:
- WO-A-02/068413
- AGGARWAL, RANJANA ET AL: "Synthesis and antibacterial activity of some new 1-heteroaryl-5-amino-3H/methyl-4-phenylpyr azoles" BIOORGANIC & MEDICINAL CHEMISTRY, Bd. 14, Nr. 6, 21. November 2005 (2005-11-21), Seiten 1785-1791, XP002474805 in der Anmeldung erwähnt

## Beschreibung

Die vorliegende Erfindung betrifft Pyrimidinylpyrazole und ihre Verwendung als Insektizide und/oder Parasitizide sowie Verfahren zu ihrer Herstellung und Mittel, die solche Arylpyrazole enthalten.

US 5,201,938 beschreibt Arylpyrazole als Zwischenprodukte in der Synthese von Herbiziden. Eine eigene Wirkung der Arylpyrazole (weder als Herbizide noch mit einer anderen biologischen Wirkung) wird dort weder offenbart noch nahegelegt. Auch in WO 93/19054 werden Arylpyrazole offenbart. Auch dort werden sie als Zwischenprodukte, diesmal in der Synthese von Fungiziden benutzt. Eine eigene Wirkung wird auch hier nicht offenbart.

In WO 02/68413 werden insektizid wirksame Pyrimidinylpyrazole offenbart, die allerdings einen 4-Pyrirnidinyl-Rest enthalten. Einen Hinweis auf die Wirksamkeit von erfindungsgemäßen Pyrimidinylpyrazolen enthält diese Veröffentlichung nicht.

WO 95/22530 offenbart N-Pyrazolylaniline und N-Pyrazolylaminopyridine, für deren Herstellung als Zwischenprodukte ebenfalls Pyrimidinylpyrazole benutzt werden, die generisch die in der vorliegenden Anmeldung offenbarten Verbindungen umfassen. Es werden jedoch keine unter die Ansprüche der vorliegenden Anmeldung fallenden Verbindungen explizit offenbart. Auch eine biologische Wirkung wird den dort offenbarten Verbindungen nicht zugeschrieben.

Aggerwal et al. (Bioorganic & Medicinal Chemistry, 14 (2006), 1785-1791) beschreiben antibakteriell wirksame Phenylpyrimidinylpyrazol-2-yle, die allerdings nur unsubstituiertes Phenyl und 4,6-dimethyliertes Pyrimidinyl enthalten. Andere Substitutionsmuster werden genausowenig offenbart oder nahegelegt wie eine andere biologische Wirkung.

Gegenstand der vorliegenden Erfindung sind demnach neue Pyrimidinylpyrazole der Formel (I) 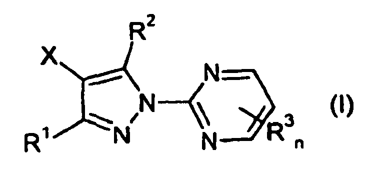 in welcher
- X: für Phenyl, 2-Pyridyl, 3-Pyridyl oder 2-Pyrimidinyl steht, jeweils substituiert durch einen oder mehreren Substituenten ausgewählt aus Halogen, Alkyl, Haloalkyl, Alkoxy, Alkoxyalkyl, Alkoxyalkoxy, Cycloalkyl, Alkenoxy, Alkinoxy, Benzyloxy, Cycloalkylalkoxy, Haloalkoxy, Haloalkoxyalkyl, Alkylsulfanyl, Haloalkylsulfanyl, Alkylsulfinyl, Haloalkylsulfinyl, Alkylsulfonyl, Haloalkylsulfonyl, Cyano, Alkylcarbonyl, Alkoxycarbonyl, Alkoxycarbonylalkyl, Carboxyl, Carboxamid, Dialkylcarboxamid, Trialkylsilyl, Nitro, Amino, Alkylamino, Dialkylamino, Alkylsulfonylamino, Dialkylsulfonylamino, -CH=NO-H, -CH=NO-Alkyl, -CH=NO- Halolkyl, -C(CH₃)=NO-H, -C(CH₃)=NO-Alkyl, -C(CH₃)=NO-Haloalkyl, Formyl, Benzyl (gegebenfalls wiederum substituiert mit einem oder mehreren Halogenatomen), Phenoxy oder Pyrid-2-yloxy (die beiden letzteren gegebenenfalls substituiert mit einem oder mehreren ausgewählt aus Alkyl, Haloge und/oder Haloalkyl) und Phenyl (gegebenenfalls substituiert mit einem oder mehreren Halogenatomen), wobei vicinale Alkyl-, Haloalkyl, Alkoxy- und/oder Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkylanteil gegebenenfalls mit einem oder mehreren Fluoratomen substituiert sein kann,
- R¹: für Alkyl (gegebenenfalls ein- oder zweifach substituiert mit Substituenten unabhängig voneinander ausgewählt aus Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, Hydroxy, Cycloalkyl, Phenyl (wiederum gegebenenfalls substituiert mit einem oder mehreren Halogenatomen) und Heterocyclyl), Cycloalkyl (gegebenenfalls substituiert mit einem oder mehreren Haloalkylgruppen und/oder Halogenatomen), Haloalkyl (gegebenenfalls substituiert mit Alkoxy), Cyano, Formyl, -CH=NO-H, -CH=NO-Alkyl, -CH=NO-Haloalkyl, -C(CH₃)=NO-H, -C(CH₃)=NO- Alkyl, -C(CH₃)=NO-Halolkyl oder Benzyl (gegebenenfalls einfach oder mehrfach substituiert mit einem oder mehreren Substituenten unabhängig voneinander ausgewählt aus Halogen, Alkyl, Haloalkyl und Alkoxy) steht,
- R²: für Amino steht und
- R²: weiterhin für Amino steht, welches einfach substituiert ist mit Alkyl, Alkylcarbonyl, Cycloalkyl, Cycloalkylalkyl, Alkenyl, Alkoxycarbonyl, Alkenyloxycarbonyl, Alkinyloxycarbonyl, Alkoxycarbonylalkyl, Alkoxycarbonylcarbonyl, Benzyl oder Phenylcarbonyl (wobei der Phenylring im Benzyl und Phenylcarbonl wiederum gegebenenfalls substituiert ist mit einem oder mehreren Halogenatomen) und
- R²: weiterhin für Amino steht welches disubstituiert ist mit zwei Substituenten unabhängig voneinander ausgewählt aus Benzyl, Alkyl, Alkoxycarbonyl, Alkenyloxycarbonyl, Alkinyloxycarbonyl und Alkylcarbonyl, und
- R²: weiterhin für Piperidin, Pyrrolidin oder Morpholin steht, welche gegebenenfalls einfach oder zweifach substituiert sind mit Alkyl und
- R²: weiterhin für Halogen steht,
- R³: für Halogen, Alkyl, Haloalkyl, Alkoxy oder Dialkylamino steht und
- n: für 0 oder 1 steht,
die Verbindungen der allgemeinen Formel (I) außerdem N-Oxide und Salze umfassen.

Schließlich wurde gefunden, dass die erfindungsgemäßen Verbindungen der Formel (I) sehr gute insektizide und parasitizide Eigenschaften besitzen und sich im Pflanzenschutz, in der Veterinärhygiene und im Materialschutz zur Bekämpfung unerwünschter Schädlinge, wie Insekten, Endo- oder Ektoparasiten, verwenden lassen.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Pyrimidinylparyzole sind durch die Formel (I) allgemein definiert. Bevorzugte, besonders bevorzugte und ganz besonders bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- X: steht bevorzugt für Phenyl, 2-Pyridyl, 3-Pyridyl oder 2-Pyrimidinyl, jeweils substituiert durch einen oder mehreren Substituenten ausgewählt aus Fluor, Chlor, Brom, Jod, C₁- C₆-Alkyl, C₁-C₆-Haloalkyl, C₁-C₆-Alkoxy-C₁-C₆-alkyl, C₁-C₆-Alkoxy, C₁-C₆- Haloalkoxy, C₁-C₆-Haloalkoxy-C₁-C₆-alkyl, C₁-C₆-Alkylsulfanyl, Halo-C₁-C₆. alkylsulfanyl, C₁-C₆-Alkylsulfnyl, Halo-C₁-C₆-alkylsulfinyl, C₁-C₆-Alkylsulfonyl, Halo-C₁-C₆-alkylsulfonyl, Cyano, C₁-C₆-Alkylcarbonyl. C₁-C₆-Alkoxycarbonyl, C₁- C₆-Alkoxycarbonyl-C₁-C₆-alkyl, Nitro, Amino, C₁-C₆-Alkylamino, Di(C₁-C₆- alkyl)amino, C₁-C₆-Alkylsulfonylamino, Di(C₁-C₆-alkyl)sulfonylamino, -CH=NO-H, -CH=NO-C₁-C₆-Alkyl, -CH=NO-Halo-C₁-C₆-Alkyl, -C(CH₃)=NO-H, -C(CH₃)NO- C₁-C₆-Alkyl, -C(CH₃)=NO-Halo-C₁-C₆-alkyl, Formyl, Benzyl (gegebenfalls wiederum substituiert mit einem oder mehreren Halogenatomen), Phenoxy oder Pyrid- 2-yloxy (die beiden letzteren gegebenenfalls substituiert mit einem oder mehreren Halogenatomen und/oder Halo-C₁-C₆-alkylgruppen) und Phenyl (gegebenenfalls substituiert mit einem oder mehreren Halogenatomen), wobei vicinale Alkyl-, Haloalkyl, Alkoxy- und/oder Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkylanteil gegebenenfalls mit einem oder mehreren Fluoratomen substituiert sein kann.
- X: steht besonders bevorzugt für Phenyl, 2-Pyridyl, 3-Pyridyl oder 2-Pyrimidinyl, jeweils substituiert durch einen oder mehreren Substituenten ausgewählt aus Fluor, Chlor, Brom, Jod, C₁-C₄-Alkyl, C₁-C₄-Haloalkyl, C₁-C₄-Alkoxy-C₁-C₄-alkyl, C₁-C₄- Alkoxy, C₁-C₄-Haloalkoxy, C₁-C₄-Haloalkoxy-C₁-C₄-alkyl, C₁-C₄-Alkylsulfanyl, Halo-C₁-C₄-alkylsulfanyl, C₁-C₄-Alkylsulfinyl, Halo-C₁₋C₄₋alkylsulfinyl, C₁-C₄- Alkylsulfonyl, Halo-C₁-C₄-alkylsulfonyl, Cyano, C₁-C₄-Alkylcarbonyl, C₁-C₄- Alkoxycarbonyl, C₁-C₄-Alkoxycarbonyl-C₁-C₄-alkyl, Nitro, Amino, C₁-C₄- Alkylamino, Di(C1₋C4-ₐlkyl)amino, C₁-C₄-Alkylsulfonylamino, Di(C₁-C₄- alkyl)sulfonylamino, -CH=NO-H, -CH=NO-C₁-C₄-Alkyl, -CH=NO-Halo-C₁-C₄-Alkyl, -C(CH₃)=NO-H, -C(CH₃)=NO-C₁-C₄-Alkyl, -C(CH₃)=NO-Halo-C₁-C₄-alkyl, Formyl, Benzyl (gegebenfalls wiederum substituiert mit einem oder mehreren Halogenatomen), Phenoxy oder Pyrid-2-yloxy (die beiden letzteren gegebenenfalls substituiert mit einem oder mehreren Halogenatomen und/oder Halo-C₁-C₄- alkylgruppen) und Phenyl (gegebenenfalls substituiert mit einem oder mehreren Halogenatomen), wobei vicinale Alkyl-, Haloalkyl, Alkoxy- und/oder Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkylanteil gegebenenfalls mit einem oder mehreren Fluoratomen substituiert sein kann.
- X: steht ganz besonders bevorzugt für Phenyl, 2-Pyridyl, 3-Pyridyl oder 2-Pyrimidinyl, jeweils substituiert durch einen oder mehreren Substituenten ausgewählt aus Fluor, Chlor, Brom, Jod, Methyl, Ethyl, iso-Propyl, tert.-Butyl, -(CH₂)₄-, -O-C₂H₄-0-, Difluormethyl, Trifluormethyl, C₁-C₄-Alkoxy-C₁-C₄-alkyl, Methoxy, Ethoxy, iso- Propyloxy, n-Propyloxy, Trifluormethoxy, 1,1,2,3,3,3-Hexafluorpropyloxy, Difluorchlormethoxy, Difluormethoxy, 1,1,2-Trifluor-2-Chlor-ethoxy, 2,2,2- Trifluorethoxy, Methylsulfanyl, Methylsulfinyl, Methylsulfonyl, Trifluonmethylsulfanyl, Cyano, Nitro, Formyl, Amino, Dimethylamino, Diethylamino, Di(n-propyl)amino, Di(i-propyl)amino, Methylamino, Ethylamino, n-Propylamino, i- Propylamino, Methylsulfonylamino, Ethylsulfonylamino, n-Propylsulfonylamino, i- Propylsulfonylamino, -CH=NO-H, -CH=NO-CH₃, -CH=NO-C₂H₅, -CH=NO- CH(CH₃)₂, -CH=NO-CH₂CH₂CH₃, -C(CH₃)=NO-H, -C(CH₃)=NO-CH₃, -C(CH₃)=NO-C₂H₅, -C(CH₃)=NO-CH(CH₃)₂, -C(CH₃)=NO-CH₂CH₂CH₃, Benzyl, Phenoxy oder Pyrid-2-yloxy (die beiden letzteren gegebenenfalls substituiert mit einem oder mehreren Substituenten ausgewählt aus Chlor und Trifluormethyl) und Phenyl (gegebenenfalls substituiert mit Chlor).
- X: steht insbesondere bevorzugt für Phenyl, 2-Pyridyl, 3-Pyridyl oder 2-Pyrimidinyl, jeweils substituiert durch einen oder mehreren Substituenten ausgewählt aus Fluor, Chlor, Brom, Jod, Methyl, Ethyl, iso-Propyl, tert.-Butyl, -(CH₂)₄- , -O-C₂H₄-O-, Trifluormethyl, C₁-C₄-Alkoxy-C₁-C₄-alkyl, Methoxy, Ethoxy, iso-Propyloxy, n- Propyloxy, Trifluormethoxy, 1,1,2,3.3,3-Hexafluorpropyloxy, Difluorchlormethoxy, Difluormethoxy, 1,1,2-Trifluor-2-Chlor-ethoxy, 2,2,2-Trifluorethoxy, Methylsulfanyl, Methylsulfinyl, Methylsulfonyl, Trifluormethylsulfanyl, Cyano, Benzyl, Phenoxy oder Pyrid-2-yloxy (die beiden letzteren gegebenenfalls substituiert mit einem oder mehreren Substituenten ausgewählt aus Chlor und Trifluormethyl) und Phenyl (gegebenenfalls substituiert mit Chlor).
- R¹: steht bevorzugt für C₁-C₆-Alkyl (gegebenenfalls ein- oder zweifach substituiert mit Substituenten unabhängig voneinander ausgewählt aus C₁-C₆-Alkoxy, Halo-C₁₋C₆- alkoxy, C₁-C₆-Alkylsulfanyl, Halo-C₁-C₆-alkylsulfanyl, C₁-C₆-Alkylsulfinyl. Halo- C₁-C₆-alkylsulfinyl, C₁-C₆-Alkylsulfonyl, Halo-C₁-C₆-alkylsulfonyl, C₁₋C₆- Alkylcarbonyl, C₁₋C₆-Alkoxycarbonyl, Hydroxy, C₃-C₆-Cycloalkyl, Phenyl und Heterocyclyl), C₃-C₆-Cycloalkyl (gegebenenfalls substituiert mit einem oder mehreren Halogenatomen), Halo-C₁-C₆-alkyl (gegebenenfalls substituiert mit C₁-C₆-Alkoxy), Cyano, Formyl, -CH=NO-H, -CH=NO-C₁-C₆-Alkyl. -CH₌NO-Halo-C_{I}-C₆-Alkyl, -C(CH₃)=NO-H, -C(CH₃)=NO-C₁-C₆-Alkyl, -C(CH₃)=NO-Halo-C₁-C₆-Alkyl oder Benzyl (gegebenenfalls einfach oder mehrfach substituiert mit einem oder mehreren Substituenten unabhängig voneinander ausgewählt aus Halogen, C₁-C₆-Alkyl und C₁-C₆-Alkoxy).
- R¹: steht besonders bevorzugt für C₁-C₄-Alkyl (gegebenenfalls ein- oder zweifach substituiert mit Substituenten unabhängig voneinander ausgewählt aus C₁-C₄-Alkoxy, C₁-C₄-Alkylsulfanyl, C₁-C₄-Alkylcarbcnyl, C₁-C₄-Alkoxycarbonyl, Hydroxy, C₃-C₆- Cycloalkyl, Phenyl und Heterocyclyl), C₃-C₅-Cycloalkyl (gegebenenfalls substituiert mit einem oder mehreren Halogenatomen), Halo-C₁-C₄-alkyl (gegebenenfalls substituiert mit C₁-C₄-Alkoxy), Cyano, Formyl, -CH=NO-H, -CH=NO-C₁-C₄-Alkyl, -CH=NO-Halo-C₁-C₄-Alkyl, -C(CH₃)=NO-H, -C(CH₃)=NO-C₁₋C₄-Alkyl, -C(CH₃)=NO-Halo-C₁-C₄-Alkyl oder Benzyl (gegebenenfalls einfach oder mehrfach substituiert mit einem oder mehreren Substituenten unabhängig voneinander ausgewählt aus Halogen, C₁-C4-Alkyl und C₁-C4-Alkoxy).
- R¹: steht ganz besonders bevorzugt für Methyl, Ethyl, n-Propyl, i-Propyl, n-Butyl oder i- Butyl (jeweils gegebenenfalls ein- oder zweifach substituiert mit Substituenten unabhängig voneinander ausgewählt aus Methoxy, Ethoxy, Methylthio, Methoxycarbonyl, Trifluormethyl, Hydroxy, Cyclopropyl, Cyclohexyl, Phenyl und gegebenenfalls substituiertem 1-Pyrazolyl); für Cyclopropyl, Cyclobutyl oder Cyclopentyl (jeweils gegebenenfalls substituiert mit einem oder mehreren ausgewählt aus Fluor und Chlor); für Fluormethyl, Chlorfluormethyl, Difluormethyl, 1-Fluor-n- Propyl, 1-Fluor-n-Butyl, Difluorbrommethyl, Difluorchlormethyl, Dichlorfluormetyhl, 2-Fluor-i-Propyl, Perfluorethyl, 1,1,2,2-Tetrafluorethyl, 1,1-Difluorethyl, 2,2-Difluor-1- bromethyl, 2,2,2-Trifluor-1-methoxy-ethyl oder Trifluormethyl; für Cyano; für -CH=NO-H, -CH=NO-CH₃ oder -CH=NO-C₂H₅; oder für Benzyl (gegebenenfalls einfach oder mehrfach substituiert mit einem oder mehreren Substituenten unabhängig voneinander ausgewählt aus Fluor, Brom, Chlor, Methyl und Methöxy).
- R¹: steht insbesondere bevorzugt für Methyl, Ethyl, n-Propyl, i-Propyl, n-Butyl oder i-Butyl (jeweils gegebenenfalls ein- oder zweifach substituiert mit Substituenten unabhängig voneinander ausgewählt aus Methoxy, Ethoxy, Methylthio, Methoxycarbonyl, Trifluormethyl, Hydroxy, Cyclopropyl, Cyclohexyl und Phenyl); für Cyclopropyl, Cyclobutyl oder Cyclopentyl (jeweils gegebenenfalls substituiert mit einem oder mehreren ausgewählt aus Fluor und Chlor); für Fluormethyl, Chlorfluormethyl, Difluormethyl, 1-Fluor-n-Propyl, 1-Fluor-n-Butyl, Difluorbrommethyl, Difluorchlormethyl, Dichlorfluormetyhl, 2-Fluor-i-Propyl, Perfluorethyl, 1,1,2,2- Tetrafluorethyl, 1,1-Difluorethyl, 2,2-Difluor-1-bromethyl, 2,2,2-Trifluor-1-methoxy- ethyl oder Trifluormethyl; für Cyano; für -CH=NO-H, -CH=NO-CH₃ oder -CH=NO- C₂H₅; oder für Benzyl (gegebenenfalls einfach oder mehrfach substituiert mit einem oder mehreren Substituenten unabhängig voneinander ausgewählt aus Fluor, Brom, Chlor, Methyl und Methoxy).
- R²: steht bevorzugt für Amino, gegebenenfalls einfach substituiert mit C₁-C₆-Alkyl, C₁- C₆-Alkylcarbonyl, C₁-C₆-Alkoxycarbonyl, C₃-C₆-Alkenyloxycarbonyl, C₃-C₆- Alkinyloxycarbonyl, C₁-C₆-Alkoxycarbonyl-C₁-C₆-alkyl, C₃-C₅-Cycloalkyl, C₃-C₅- Cycloalkyl-C₁-C₃-alkyl, C₁-C₆-Alkenyl, C₁-C₆-Alkoxycarbonyl, C₁-C₆- Alkoxycarbonylcarbonyl, Benzyl oder Phenylcarbonyl (wobei der Phenylring in Benzyl und Phenylcarbonyl wiederum gegebenenfalls substituiert ist mit einem oder mehreren Halogenatomen) und
- R²: steht weiterhin bevorzugt für Amino, welches disubstituiert ist mit zwei Substituenten unabhängig voneinander ausgewählt aus Benzyl, C₁-C₆-Alkyl und C₁-C₆- Alkylcarbonyl, C₁-C₆-Alkoxycarbonyl, C₃-C₆-Alkenyloxycarbonyl und C₃-C₆- Alkinyloxycarbonyl, und
- R²: steht weiterhin bevorzugt für Piperidin, Pyrrolidin oder Morpholin, welche gegebenenfalls substituiert sind mit ein oder zwei C₁-C₆-Alkyl und
- R²: steht weiterhin bevorzugt für Fluor, Chlor, Brom oder Jod.
- R²: steht besonders bevorzugt für Amino, gegebenenfalls einfach substituiert mit Methyl, Ethyl, i-Propyl, i-Butyl, n-Propyl, n-Butyl, 2'-i-Pentyl, Methylcarbonyl, Cyclopropylmethyl, Cyclopentyl, Prop-1-en-3-yl, 2-Methylbut-2-en-4-yl, Methoxycarbonylcarbonyl, Ethoxycarbonylcarbonyl, Methoxycarbonyl, Ethoxycarbonyl, 2-Propenyloxycarbonyl, Propargyloxycarbonyl, Ethoxycarbonylmethyl, Benzyl oder Phenylcarbonyl (wobei der Phenylring in Benzyl und Phenylcarbonyl wiederum gegebenenfalls substituiert ist mit einem oder mehreren Fluor-, Brom- und/oder Chloratomen) und
- R²: steht weiterhin besonders bevorzugt für Amino, welches disubstituiert ist mit zwei Substituenten unabhängig voneinander ausgewählt aus Benzyl, Methyl, Ethyl, Methoxycarbonyl, Ethoxycarbonyl, 2-Propenyloxycarbonyl und Propargyloxycarbonyl, und
- R²: steht weiterhin besonders bevorzugt für Piperidin, Pyrrolidin oder Morpholin, welche gegebenenfalls einfach oder zweifach substituiert sind mit C₁₋C₆-Alkyl und
- R²: steht weiterhin besonders bevorzugt für Chlor, Brom oder Jod.
- R²: steht ganz besonders bevorzugt für Amino, gegebenenfalls einfach substituiert mit Methyl, Ethyl, i-Propyl, i-Butyl, n-Propyl, n-Butyl, 2'-i-Pentyl, Methylcarbonyl, Cyclopropylmethyl, Cyclopentyl, Prop-1-en-3-yl, 2-Methylbut-2-en-4-yl, Methoxycarbonylcarbonyl, Methoxycarbonyl, Ethoxycarbonyl, 2-Propenyloxycarbonyl, Propargyloxycarbonyl, Ethoxycarbonylcarbonyl, Ethoxycarbonylmethyl, Benzyl oder Phenylcarbonyl (wobei der Phenylring in Benzyl und Phenylcarbonyl wiederum gegebenenfalls substituiert ist mit einem oder mehreren Fluor-, Brom- und/oder Chloratomen) und
- R²: steht weiterhin ganz besonders bevorzugt für Amino, welches disubstituiert ist mit zwei identischen Substituenten ausgewählt aus Benzyl, Methyl, Ethyl, Methoxycarbonyl, Ethoxycarbonyl, 2-Propenyloxycarbonyl und Propargyloxycarbonyl, und
- R²: steht weiterhin ganz besonders bevorzugt für 4-Methylpiperidin, Pyrrolidin, Morpholin, 2-6-Dimethylmorpholin oder Piperidin und
- R²: steht weiterhin ganz besonders bevorzugt für Chlor, Brom oder Jod.
- R²: steht insbesondere bevorzugt für Amino, gegebenenfalls einfach substituiert mit Methyl, Ethyl, i-Propyl, i-Butyl, n-Propyl, n-Butyl, 2'-i-Pentyl, Methylcarbonyl, Cyclopropylmethyl, Cyclopentyl, Prop-1-en-3-yl, 2-Methylbut-2-en-4-yl, Methoxycarbonylcarbonyl, Methoxycarbonyl, Ethoxycarbonyl, Propargyloxycarbonyl, Ethoxycarbonylcarbonyl, Ethoxycarbonylmethyl, Benzyl oder Phenylcarbonyl (wobei der Phenylring in Benzyl und Phenylcarbonyl wiederum gegebenenfalls substituiert ist mit einem oder mehreren Brom- und/oder Chloratomen) und
- R²: steht weiterhin insbesondere bevorzugt für Amino, welches disubstituiert ist mit zwei identischen Substituenten ausgewählt aus Benzyl, Methyl, Ethyl, Methoxycarbonyl, Ethoxycarbonyl und Propargyloxycarbonyl, und
- R²: steht weiterhin insbesondere bevorzugt für 4-Methylpiperidin und
- R²: steht weiterhin insbesondere bevorzugt für Chlor, Brom oder Jod.
- R³: steht bevorzugt für Fluor, Chlor, Brom, C₁-C₆-Alkyl, Halo-C₁-C₆-alkyl, C₁-C₆- Alkoxy oder Di-C₁-C₆-alkylamino.
- R³: steht besonders bevorzugt für Fluor, Chlor, Methyl, Ethyl, i-Propyl, n-Propyl, Perhalo- C₁-C₃-alkyl, Methoxy, Ethoxy, n-Propoxy, i-Propoxy, Dimethylamino oder Diethylamino.
- R³: steht ganz besonders bevorzugt für Fluor, Chlor, Methyl, Trifluormethyl, Methoxy oder Dimethylamino.
- n: steht ganz besonders bevorzugt für 0.

Eine weitere bevorzugte Ausführungsform der vorliegenden Erfindung sind Verbindungen der Formel (I), mit X, R¹, R², R³ und n wie oben definiert, wobei wenn X für Phenyl *steht, substituiert durch C₁-C₅-Alkyl, Cl, F, Br, CF₃, C₂-C₃-Alkenyl, C₂-C₃-Alkinyl, C₁-C₄ Alkoxy, Alkoxyalkyl, Phenyl, Benzyl, O-Phenyl oder O-Benzyl, und R² für NH₂ steht und R¹ für CF₃, C₁-C₃-Alkyl oder C₅-C₃-Alkoxy-C₁-C₃-Alkyl steht und n für 0 steht, dann ist X mindestens zweifach substituiert und für den Fall dass X mit zwei identischen Substituenten substituiert ist, stehen diese zwei Substituenten weder in Position 2 und 4 noch in Position 3 und 4.

Eine weitere bevorzugte Ausführungsform der vorliegenden Erfindung sind Verbindungen der Formel (I), ausgewählt aus den Tabellen 1 bis 43, wobei
a) wenn die Verbindungen ausgewählt sind aus Tabelle 1, dann steht für den Fall, dass X einfach substituiert ist, der Substituent von X nicht für F, Cl oder Br und wenn X mit zwei identischen Substituenten ausgewählt aus F, Cl und Br substituiert ist, dann stehen diese zwei Substituenten weder in Position 2 und 4, noch in Position 3 und 4;
b) wenn die Verbindungen ausgewählt sind aus Tabelle 3, dann ist für den Fall, dass X einfach substituiert ist der Substituent nicht CF₃ und für den Fall, dass X zweifach mit CF₃ substituiert ist, dann stehen diese zwei Substituenten weder in Position 2 und 4, noch in Position 3 und 4;
c) wenn die Verbindungen ausgewählt sind aus Tabelle 8, dann stehen für den Fall, dass X mit zwei identischen Substituenten ausgewählt aus Methyl und Methoxy substituiert ist, diese zwei Substituenten weder in Position 2 und 4, noch in Position 3 und 4;
d) wenn die Verbindungen ausgewählt sind aus Tabelle 16 mit R¹ gleich C₁-C₃-Alkyl, dann steht für den Fall, dass X einfach substituiert ist, der Substituent von X nicht für F, Cl oder Br und für den Fall, dass X zweifach mit Cl substituiert ist, dann stehen diese zwei Substituenten weder in Position 2 und 4 noch in Position 3 und 4; und
e) wenn die Verbindungen ausgewählt sind aus Tabelle 17 mit R¹ gleich CH₂OMe, CH₂OEt oder CH(OMe)Me, dann steht für den Fall, dass X einfach substituiert ist, der Substituent von X nicht für F, Cl oder Br und wenn X mit zwei identischen Substituenten ausgewählt aus F, Cl und Br substituiert ist, dann stehen diese zwei Substituenten weder in Position 2 und 4, noch in Position 3 und 4.

Eine weitere bevorzugte Ausführungsform der vorliegenden Erfindung sind Verbindungen der Formel (I), mit X, R¹. R², R³ und n wie oben definiert, wobei X zweifach mit zwei nicht identischen Substituenten substituiert ist.

Eine weitere bevorzugte Ausführungsform der vorliegenden Erfindung sind Verbindungen der Formel (I), mit X, R¹, R², R³ und n wie oben definiert, wobei X zweifach substituiert ist und die Substituenten in Position 3 und 5 stehen.

Eine weitere bevorzugte Ausführungsform der vorliegenden Erfindung sind Verbindungen der Formel (I), mit X, R¹, R², R³ und n wie oben definiert, wobei X mindestens dreifach substituiert ist.

Eine weitere bevorzugte Ausführungsform der vorliegenden Erfindung sind Verbindungen der Formel (I), mit X, R¹, R¹, R³ und n wie oben definiert, wobei X mindestens dreifach substituiert ist und die Substituenten in Position 3, 4 und 5 oder Position 2, 3 und 5 stehen.

Eine weitere bevorzugte Ausführungsforin der vorliegenden Erfindung sind Verbindungen der Formel (I), mit X, R¹, R², R³ und n wie oben definiert, wobei R¹ ausgewählt ist aus Fluormethyl, Chlorfluormethyl, Difluormethyl, 1-Fluor-n-Propyl, 1-Fluor-n-Butyl, Difluorbrommethyl, Difluorchlormethyl, Dichlorfluormetyhl, 2-Fluor-i-Propyl, Perfluorethyl, 1,1,2,2-Tetrafluorethyl, 1,1-Difluorethyl, 2,2-Difluor-1-bromethyl oder 2,2,2-Trifiuor-I-methoxy-ethyl.

Durch Halogen substituierte Reste, z.B. Haloalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Bevorzugt, besonders bevorzugt, ganz besonders bevorzugt und insbesondere bevorzugt sind Verbindungen, welche jeweils die unter bevorzugt, besonders bevorzugt, ganz besonders bevorzugt und insbesondere bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die Verbindungen der Formel (I) lassen sich in Abhängigkeit von ihrem Substituenten R² in die Verbindungen der Formel (IA) und (IB) unterteilen: 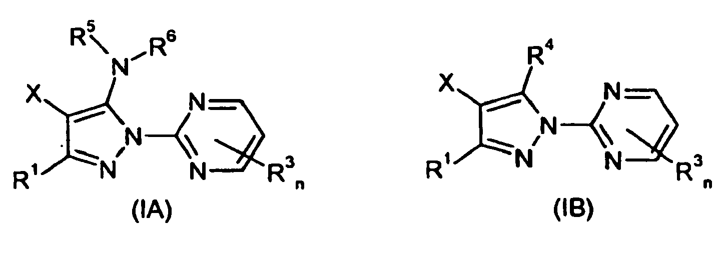 wobei
- X, R¹, R³ und n: die oben angegebenen Bedeutungen haben,
- R⁴: für Halogen steht,
- R⁵: für Wasserstoff, Alkyl, Alkylcarbonyl, Alkoxycarbonyl, Alkenyloxycarbonyl, Alkinyloxycarbonyl, Cycloalkyl, Cycloalkylalkyl, Alkenyl, Alkoxycarbonylcarbonyl, Benzyl oder Phenylcarbonyl (wobei der Phenylring in Benzyl und Phenylcarbonyl wiederum gegebenenfalls substituiert ist mit einem oder mehreren Fluor, Brom- und/oder Chloratomen) steht,
- R⁶: für Wasserstoff steht,
- R⁶: weiterhin für den Fall, dass R⁵ Benzyl, Alkyl, Alkylcarbonyl Alkoxycarbonyl, Alkenyloxycarbonyl, oder Alkinyloxycarbonyl, ist, ebenfalls Benzyl, Alkyl, Alkylcarbonyl, Alkoxycarbonyl, Alkenyloxycarbonyl oder Alkinyloxycarbonyl sein kann, oder
- R⁵ und R⁶: gemeinsam mit dem Stickstoffatom, an das sie gebunden sind, ein Piperidin-, Pyrrolidin- oder Morpholinrest bilden können.

Verbindungen der Formel (IA), bei denen R⁵ und R⁶ gleichzeitig für Wasserstoff stehen, werde im Folgenden als Verbindungen der Formel (IA-1) bezeichnet: 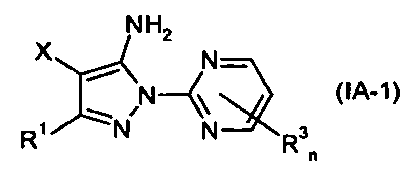 wobei X, R¹, R³ und n die oben angegebenen Bedeutungen haben.

### Verfahren (A)

Verbindungen der Formel (IA-1) werden z.B. nach folgendem Verfahren (A) synthetisiert: 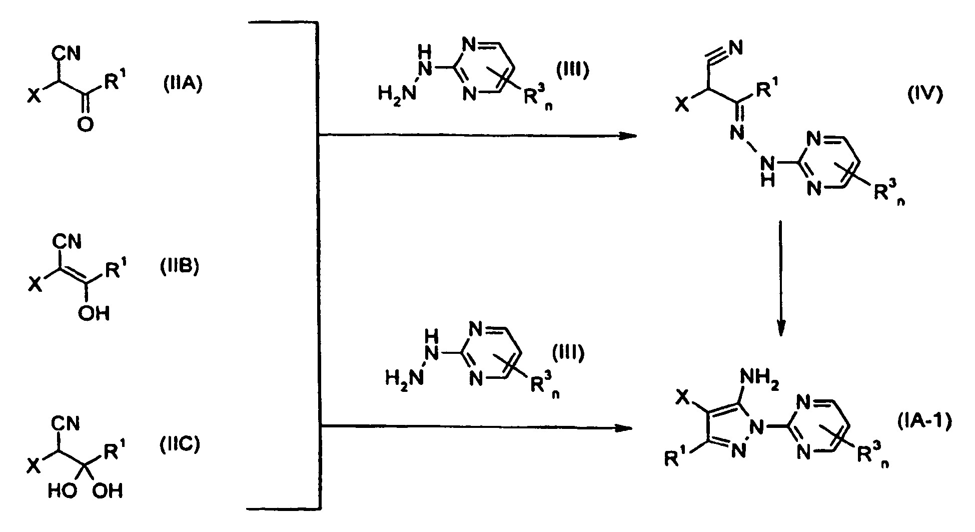 wobei X, R¹, R³ und n die oben angegebenen Bedeutungen haben.

Beim erfindungsgemässen Verfahren (A) zur Herstellung der Verbindungen der Formel (IA-1) werden Ketonitrile, deren Tautomere oder Hydrate der Formeln (IIA), (IIB) bzw. (IIC) mit Pyrimidinyl-Hydrazinen der Formel (III) kondensiert, wobei zunächst Hydrazone der Formel (IV) als Intermediat entstehen und bei längerer Reaktionszeit und höherer Temperatur der Ringschluss zum Aminopyrazol der Formel (IA) erfolgt. Dabei können Säuren als Katalysator zugesetzt werden, wobei anorganische Säuren wie Salzsäure und organische Säuren wie Sulfonsäuren oder Essigsäure geeignet sein können.

Die Synthese von strukturverwandten Aminopyrazolen wird beschrieben in
- R. Aggarwal et al, Bioorg. Med. Chem. 14 (2006), 6, 1785-1791
- S. P. Singh et al, Eur. J. Med. Chem. 40 (2005), 922-927
- DE-A 2643640
- US 3,041,342

Die Ketonitrile können in den tautomeren Formen (IIA) und (IIB) und als Hydrat (IIC) vorliegen. Die Ausgangsverbindungen können auch in Form ihrer Salze eingesetzt werden, z.B. können die Ketonitrile in Form ihrer Alkalisalze und die Pyrimdinyl-Hydrazine in Form ihrer Hydrochloride verwendet werden.

Die Ketonitrile der Formel (II) lassen sich nach bekannten Methoden herstellen:
- W. R. Nes, Alfred Burger, J. Amer. Chem. Soc. 72 (1950), 5409-5413

Die Pyrimidinyl-Hydrazine der Formel (III) sind zum Teil kommerziell verfügbar. Die Herstellung von Pyrimidinyl-Hydrazinen der Formel (III) erfolgt nach den Methoden beschrieben in:
- Methoden der Organischen Chemie (Houben-Weyl), Organische Stickstoff-Verbindungen, Band E 16a, Teil I, S. 678-775, Georg Thieme Verlag Stuttgart-New York, 1990.

### Verfahren (B)

Alternativ können Verbindungen der Formel (IA-1) nach dem erfindungsgemäßen Verfahren (B) synthetisiert werden:

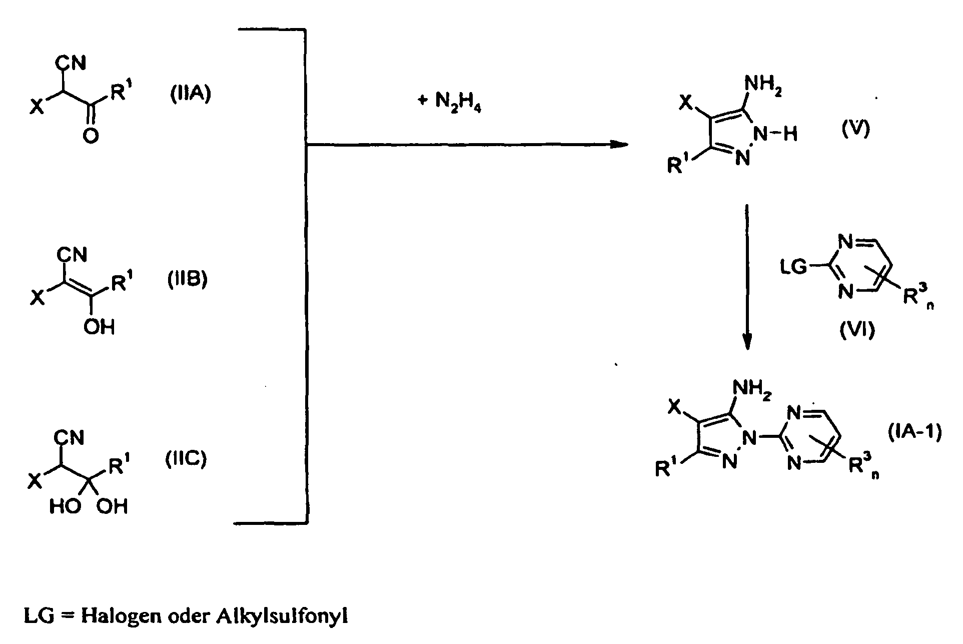

Beim erfindungsgemässen Verfahren (B) zur Herstellung der Verbindungen der Formel (IA-1) werden 1-H-Aminopyrazole der Formel (V) mit Pyrimidinyl-Halogeniden bzw. - Alkylsulfonen der Formel (VI) in Gegenwart einer Base in organischen Lösungsmitteln umgesetzt, wobei bevorzugt ein bestimmtes Isomer, das Aminopyrazol der Formel (IA-1), gebildet wird.

Die 1-H-Aminopyrazole der Formel (V) lassen sich nach bekannten Methoden herstellen:
- DE-A 2643640
- C. Chen et al, Bioorg. Med. Chem. Lett., 14 (2004), 14, 3669
- Gilligan, Paul J. et al, Bioorg. Med. Chem., 8, (2000), 1, 181 - 190

Die Pyrimidinyl-Halogenide oder -Alkylsulfone der Formel (VI) sind zum Teil kommerziell verfügbar oder lassen sich nach dem Fachmann bekannten Methoden synthetisieren.

### Verfahren (C)

Erfindungsgemäße Verbindungen der Formel (IA), in denen R⁵ oder R⁶ oder R⁵ und R⁶ nicht für Wasserstoff stehen, lassen sich nach dem erfindungsgemäßen Verfahren (C) synthetisieren:

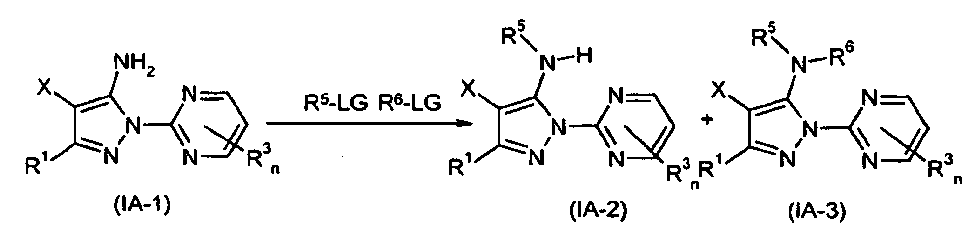

X, R¹, R³, R⁵, R⁶ und n die oben angegebenen Bedeutungen haben können (R⁵ und R⁶ aber nicht für Wasserstoff stehen) und LG für Halogen oder Alkylsulfonyl steht.

Beim erfindungsgemässen Verfahren (C) zur Herstellung der Verbindungen der Formel (IC) und (ID) werden Verbindungen der Formel (IA-1) mit einem oder zwei Alkylierungsmitteln oder Acylierungsmitteln R⁵-LG bzw. R⁶-LG umgesetzt, wobei durch Monosubstitution und Disubstitution Aminopyrazole der Formel (IA-2) und (IA-3) entstehen. Geeignete Alkylierungsmittel sind Alkylbromide, Alkyldibromide, Alkyliodide, Alkyldiiodide, Dialkylsulfate und Alkylsulfonate. Als Acylierungsmittel werden Carbonsäureanhydride und Carbonsäurechloride verwendet.

### Verfahren (D)

Erfindungsgemäße Verbindungen der Formel (IB) werden z.B. nach Verfahren (D) synthetisiert: 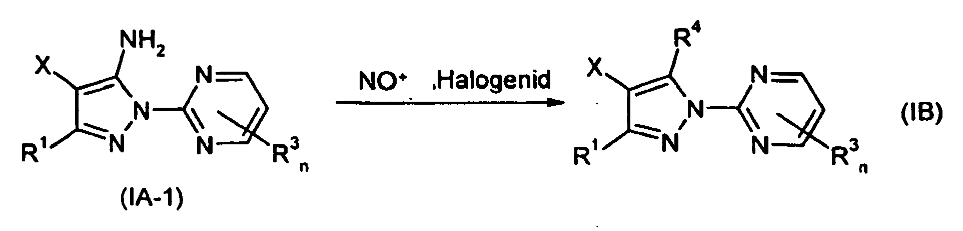 wobei X, R¹, R³, R⁴ und n die oben beschriebenen Bedeutungen haben.

Beim erfindungsgemässen Verfahren (D) zur Herstellung der Verbindungen der Formel (IB) werden Aminopyrazole der Formel (IA-1) mit Nitrosylspezies in Gegenwart von geeigneten Halogeniden umgesetzt, wobei nach der Bildung eines Diazo-Intermediates ein 5-Halogenpyrazol der Formel (IB) entsteht. Geeignete Quellen für Nitrosylspezies sind Alkalinitrite plus Säuren sowie Ester der salpetrigen Säure, z.B. Butylnitrit. Als Halogenide können Alkalihalogenide sowie organische Halogenide, z.B. Bromoform verwendet werden.

Die Synthese von strukturverwandten 5-Halogenpyrazolen wird beschrieben in
- WO 2003/074492, S. 34, Bayer CropScience
- Beck, James R. et al., J. Heterocycl. Chem., 24 (1987), 267-270

### Verfahren (E)

Erfindungsgemäße Verbindungen der Formel (IA-1), in denen R¹ für ein Oxim steht und die im Folgenden als (IA-1-2) bezeichnet werden, werden z.B. nach Verfahren (E) aus den Acetalen der Formel (IA-1-1) synthetisiert: 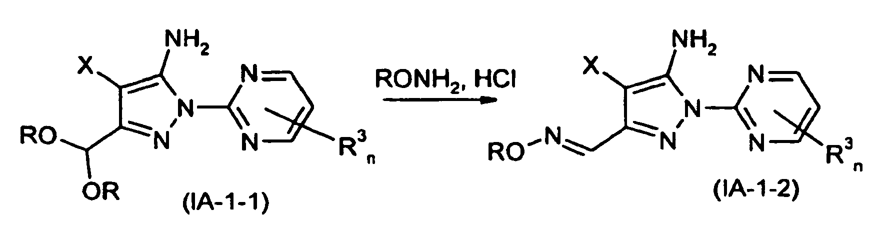 wobei X, R³ und n die oben beschriebenen Bedeutungen haben und R für Alkyl steht.

Beim erfindungsgemässen Verfahren (E) zur Herstellung von 3-Oxim-Pyrazolen der Formel (IA-1-2) werden 3-Acetalpyrazole der Formel (IA-1-1) mit O-Alkyl-Hydroxylamin in organischen Lösungsmitteln in Gegenwart von Säuren umgesetzt.

### Verfahren (F)

Erfindungsgemäße Verbindungen der Formel (IA-1), in denen R¹ für Cyano steht und die im Folgenden als Verbindungen der Formel (IA-1-3) bezeichnet werden, werden z.B. nach Verfahren (F) synthetisiert: 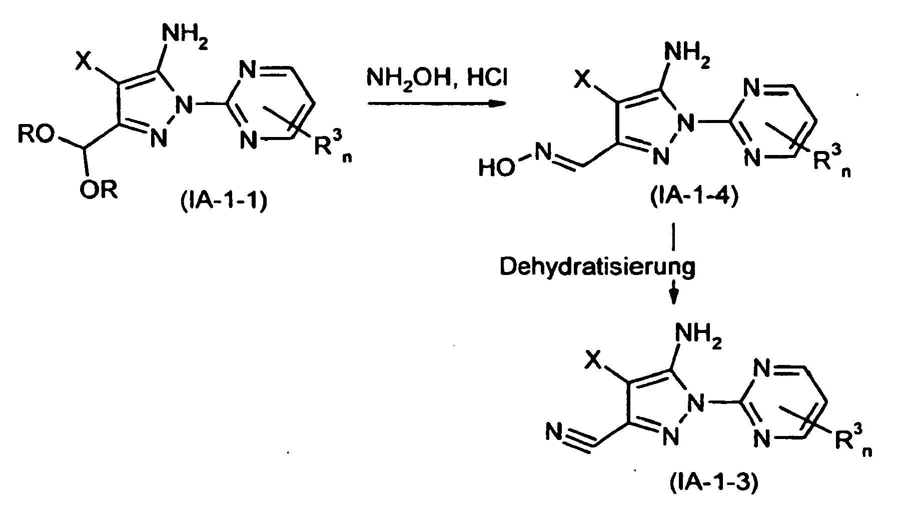 wobei X, R, R³ und n die oben beschriebenen Bedeutungen haben und wobei als wasserentziehende Mittel Säurechloride, z.B. Phosphoroxychlorid, oder Anhydride verwendet werden.

### Verfahren (G)

Verbindungen der Formel (IA-1) können alternativ auch nach folgendem Verfahren (G) synthetisiert werden: 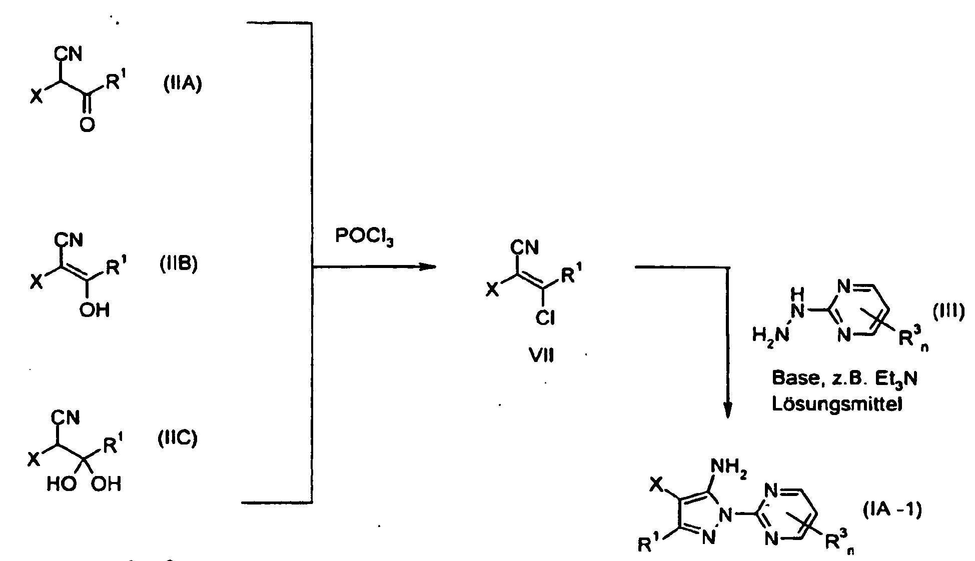 wobei X, R¹, R³ und n die oben angegebenen Bedeutungen haben.

Beim erfindungsgemässen Verfahren (G) zur Herstellung der Verbindungen der Formel (IA-1) werden Ketonitrile, deren Tautomere oder Hydrate der Formeln (IIA), (IIB) bzw. (IIC) mit Chlorierungsmitteln, z.B. Phosphorylchlorid, Thionylchlorid, Phosgen, Chlor oder Oxalylchlorid, gegebenenfalls in einem inerten organischen Lösungsmittel verdünnt, zu Chloracrylnitrilen (VII) umgesetzt, wobei die Reaktion im Temperaturbereich von -20 °C bis 120°C durchgeführt werden kann.

In einem anschließenden Schritt erfolgt die Kondensation mit Pyrimidinyl-Hydrazinen (III) in einem geeigneten organischen Lösungsmittel in Gegenwart von basischen Hilfsreagentien, z.B. Alkoholaten oder Stickstoffbasen, wobei die Reaktion im Temperaturbereich von -20 °C bis 120°C durchgeführt werden kann.

Die Ketonitrile können in den tautomeren Formen (IIA) und (IIB) und als Hydrat (IIC) vorliegen. Die Ausgangsverbindungen können auch in Form ihrer Salze eingesetzt werden, z.B. können die Ketonitrile in Form ihrer Alkalisalze und die Pyrimdinyl-Hydrazine in Form ihrer Hydrochloride verwendet werden.

Die Ketonitrile der Formel (II) lassen sich nach bekannten Methoden herstellen:
- W. R. Nes, Alfred Burger, J. Amer. Chem. Soc. 72 (1950), 5409-5413

Die Pyrimidinyl-Hydrazine der Formel (III) sind zum Teil kommerziell verfügbar. Die Herstellung von Pyrimidinyl-Hydrazinen der Formel (III) erfolgt nach den Methoden beschrieben in:
- Methoden der Organischen Chemie (Houben-Weyl), Organische Stickstoff-Verbindungen, Band E 16a, Teil 1, S. 678-775, Georg Thieme Verlag Stuttgart-New York, 1990.

Die Chlor-acrylnitrile der Formel (VII) lassen sich ebenfalls nach bekannten Methoden herstellen:
- JP08208620 (X = 4-CF₃C₆H₆ und R¹ = CF₃)

### Verfahren (H)

Verbindungen der Formel (IA-1) können alternativ auch nach folgendem Verfahren (H) synthetisiert werden: 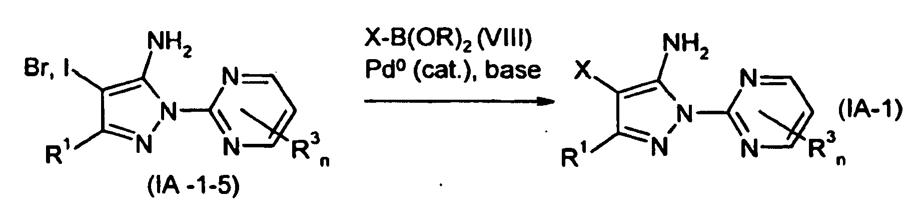 wobei X, R¹, R³ und n die oben angegebenen Bedeutungen haben.

Beim erfindungsgemässen Verfahren (H) zur Herstellung der Verbindungen der Formel (IA-1) werden Bromide oder Iodide der Formel (I-A-1-5) mit Boronsäuren oder Boronsäureestern der Formel (VIII) in Gegenwart von geeigneten Palladium-Katalysatoren und Basen (Suzuki-Reaktion) im Temperaturbereich von -20 °C bis 120°C in geeigneten Lösungsmitteln zur Reaktion gebracht.

Die Bromide oder Iodide der Formel (I-A-1-5) lassen sich nach bekannten Methoden herstellen, beschrieben z.B. in:
- W02005/11292
- Chemistry of Heterocyclic Compounds (New York, NY, United States), 41(I), 105-110; 2005
- Bioorganic & Medicinal Chemistry, 12(12), 3345-3355; 2004
- Journal of Medicinal Chemistry, 20(12), 1562-9; 1977

Die Boronsäuren oder Boronsäureester der Formel (VIII) sind zum Teil kommerziell verfügbar oder sie sind nach bekannten Methoden leicht herstellbar. Dies ist beispielsweise beschrieben in:
- WO 99/64428.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus. spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Stemechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (ptily-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nichtionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP-POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephalin und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:
**Fungizide:**
   Inhibitoren der Nucleinsäure Synthese
      Benalaxyl, Benalaxyl-M, Bupirimat, Chiralaxyl, Clozylacon, Dimethirimol, Ethirimol, Furalaxyl, Hymexazol, Mefenoxam, Metalaxyl, Metalaxyl-M, Ofurace, Oxadixyl, Oxolinsäure
   Inhibitoren der Mitose und Zellteilung
      Benomyl, Carbendazim, Diethofencarb, Ethaboxam, Fuberidazole, Pencycuron, Thiabendazol, Thiophanat-methyl, Zoxamid
   Inhibitoren der Atmungskette Komplex I
      Diflumetorim
   Inhibitoren der Atmungskette Komplex II
      Boscalid, Carboxin, Fenfuram, Flutolanil, Furametpyr, Furmecyclox, Mepronil, Oxycarboxin, Penthiopyrad, Thifluzamid
   Inhibitoren der Atmungskette Komplex III
      Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
   Entkoppler
      Dinocap, Fluazinam
   Inhibitoren der ATP Produktion
      Fentinacetat, Fentinchlorid, Fentinhydroxid, Silthiofam
   Inhibitoren der Aminosäure- und Proteinbiosynthese
      Andoprim, Blasticidin-S, Cyprodinil, Kasugamycin, Kasugamycinhydrochlorid Hydrat, Mepanipyrim, Pyrimethanil
   Inhibitoren der Signal-Transduktion
      Fenpiclonil, Fludioxonil, Quinoxyfen
   Inhibitoren der Fett- und Membran Synthese
      Chlozolinat, Iprodion, Procymidon, Vinclozolin
      Ampropylfos, Kalium-Ampropylfos, Edifenphos, Etridiazol, Iprobenfos (IBP), Isoprothiolan, Pyrazophos
      Tolclofos-methyl, Biphenyl
      Iodocarb, Propamocarb, Propamocarb hydrochlorid, Propamocarb-Fosetylat
   Inhibitoren der Ergosterol Biosynthese
      Fenhexamid,
      Azaconazol, Bitertanol, Bromuconazol, Cyproconazol, Diclobutrazol, Difenoconazol, Diniconazol, Diniconazol-M, Epoxiconazol, Etaconazol, Fenarimol, Fenbuconazol, Fluquinconazol, Flurprimidol, Flusilazol, Flutriafol, Furconazol, Furconazol-cis, Hexaconazol, Imazalil, Imazalilsulfat Imibenconazol, Ipconazol, Metconazol, Myclobutanil, Nuarimol, Oxpoconazol, Paclobutrazol, Penconazol, Pefurazoat Prochloraz, Propiconazol, Prothioconazol, Pyrifenox, Simeconazol, Tebuconazol, Tetraconazol, Triadimefon, Triadimenol, Triflumizol Triforin, Triticonazol, Uniconazol, Voriconazol, Viniconazol,
      Aldimorph, Dodemorph, Dodemorphacetat, Fenpropidin, Fenpropimorph, Spiroxamin, Tridemorph,
      Naftifin, Pyributicarb, Terbinafin
   Inhibitoren der Zellwand Synthese
      Benthiavalicarb, Bialaphos, Dimethomorph, Flumorph, Iprovalicarb, Mandipropamid, Polyoxins, Polyoxorim, Validamycin A
   Inhibitoren der Melanin Biosynthese
      Carpropamid, Diclocymet, Fenoxanil, Phthalid, Pyroquilon, Tricyclazol
   Resistenzinduktion
      Acibenzolar-S-methyl, Probenazol, Tiadinil
   Multisite
      Captafol, Captan, Chlorothalonil, Kupfersalze wie: Kupferhydroxid, Kupfernaphthenat Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metirarn, Metiram Zink, Propineb, Schwefel und Schwefelpräparate enthaltend Calciumpolysulphid, Thiram, Tolylfluanid, Zineb, Ziram
   Weitere Fungizide
      Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ferimzon, Flumetover, Flusulfamid, Fluopicolid, Fluoroimid, Fosetyl-Aluminium, Fosetyl-Caclcium, Fosetyl-Natrium, Hexachlorobenzol, 8-Hydroxychinolinsulfat, Irumamycin, Isotianil, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorphenol und Salze, 2-Phenylphenol und Salze, Piperalin, Propanosin-Natrium, Proquinazid, Pyribencarb, Pyrrolnitrin, Quintozen, Tecloftalam, Tecnazen, Triazoxid, Trichlamid, Valiphenal, Zarilamid,
      2-{2-{[6-(3-Chlor-2-methylphenoxy)-5-fluorpyrimidin-4-yl]oxy}phenyl)-2-(methoxyimino)-N-methylacetamid,
      2-[[[(1-[3(1Fluor-2-phenylethyl)oxy]phenyl]ethyliden]amino]oxy]methyl]-alpha-(methoxyimino)-N-methyl-alpha-benzacetamid,
      cis-1-(4-Chlorphenyl)-2-(1H-1,2,4-triazol-1-yl)-cycloheptanol,
      1-[(4-Methoxyphenoxy)methyl]-2,2-dimethylpropyl-1H-imidazol-1- carbonsäure,
      2,3,5,6-Tetrachlor-4-(methylsulfonyl)-pyridin,
      2-Butoxy-6-iod-3-propyl-benzopyranon-4-on,
      2-Chlor-N-(2,3-dihydro-1,1,3-trimethyl-1H-inden4-yl)-3-pyridincarboxamid,
      3,4,5-Trichlor-2,6-pyridindicarbonitril,
      3-[5-(4-Chlorphenyl)-2,3-dimethylisoxazolidin-3-yl]pyridin,
      5-Chfor-6-(2,4,6-trifluorophenyl)-N-[(1R)-1,2,2-trimethylpropyl][1,2,4]triazolo[1,5-a]pyrimidin-7-amin,
      5-Chlor-7-(4-methylpiperidin-1-yl)-6-(2,4,6-trifluorophenyl)[1,2,4]triazolo[1,5-a]pyrimidin,
      5-Chlor-N-[(1R)-1,2-dimethylpropyl]-6-(2,4,6-trifluorophenyl)[1,2,4]triazolo[1,5-a]pyrimidin-7-amin,
      Methyl 2-[[[cyclopropyl[(4-methoxyphenyl)imino]methyl]thio]methyl]-alpha-(methoxymethylen)- benzacetat,
      Methyl 1-(2.3-dihydro-2,2-dimethyl-1H-inden-1-yl)-1H-imidazole-5-carboxylat,
      N-(3',4'-dichlor-5-fluorbiphenyl-2-yl)-3-(difluormethyl)-1-methyl-1H-pyrazol-4-carboxamid,
      N-(3-Ethyl-3,5,5-trimethyl-cyclohexyl)-3-formylamino-2-hydroxy-benzamid,
      N-(4-Chlor-2-nitrophenyl)-N-ethyl-4-methyl-benzenesulfonamid,
      N-(4-chlorbenzyl)-3-[3-methoxy-4-(prop-2-yn-1-yloxy)phenyl]propanamid,
      N-[(4-chlorphenyl)(cyano)methyl]-3-[3-methoxy-4-(prop-2-yn-1-yloxy)phenyl]propanamid,
      N-(5-Brom-3-chlorpyridin-2-yl)methyl-2,4-dichlomicotinamid,
      N-[1-(5-Brom-3-chloropyridin-2-yl)ethyl]-2,4-dichloronicotinamid,
      (2S)-N-[2-[4-[[3-(4-chlorophenyl)-2-propinyl]oxy]-3-methoxyphenyl]ethyl]-3-methyl-2-[(methylsulfonyl)amino]-butanamid,
      N-{(Z)-[(cyclopropylmethoxy)imino][6-(difluormethoxy)-2,3-difluorphenyl]methyl}-2-benzacetamid,
      N-{2-[1,1'-bi(cyclopropyl)-2-yl]phenyl}-3-(difluormethyl)-1-methyl-1H-pyrazol-4-carboxamid,
      N-{2-[3-Chlor-5-(trifluormethyl)pyridin-2-yl]ethyl}-2-(trifluoromethyl)benzamid,
      N-ethyl-N-methyl-N'-{2-methyl-5-(trifluormethyl)-4-[3-(trimethylsilyl)propoxy]phenyl}imidoformamid,
      N-ethyl-N-methyl-N'-{2-methyl-5-(difluormethyl)-4-[3-(trimethylsilyl)propoxy]phenyl}imidoformamid,
      O-[1-[(4-Methoxyphenoxy)methyl]-2,2-dimethylpropyl]-1H-imidazol-1-carbothioic acid,
      2-Amino-4-methyl-N-phenyl-5-thiazolcarboxamid,
      2,4-Dihydro-5-methoxy-2-methyl-4-[[[[1-[3-(trifluoromethyl)-phenyl]-ethyliden]-amino]-oxy]-methyl]-phenyl]-3H-1,2,4-triazol-3-on (CAS Nr. 185336-79-2),
      N-(6-Methoxy-3-pyridinyl)-cyclopropan carboxamid,
**Bakterizide:**
   Branopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Acetylcholinesterase (AChE) Inhibitoren
      Carbamate,
      zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
      Organophosphate,
      zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyVethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
   Natrium-Kanal-Modulatoren / Spannungsabhängige Natrium-Kanal-Blocker
      Pyrethroide,
      zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (IR-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (IR-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
      DDT
      Oxadiazine,
      zum Beispiel Indoxacarb
      Semicarbazon,
      zum Beispiel Metaflumizon (BAS3201)
   Acetylcholin-Rezeptor-Agonisten/-Antagonisten
      Chloronicotinyle,
      zum Beispiel Acetamiprid, Clothianidin, Dinotefuran, Imidacloprid Nitenpyram, Nithiazine, Thiacloprid, Thiamethoxam
      Nicotine, Bensultap, Cartap
   Acetylcholin-Rezeptor-Modulatoren
      Spinosyne,
      zum Beispiel Spinosad, Spinetoram
   GABA-gesteuerte Chlorid-Kanal-Antagonisten
      Organochlorine,
      zum Beispiel Camphechlor, Chlordane, Endosulfan, Gamma-HCH, HCH, Heptachlor, Lindane, Methoxychlor
   Fiprole,
      zum Beispiel Acetoprole, Ethiprole, Fipronil, Pyrafluprole, Pyriprole, Vaniliprole
   Chlorid-Kanal-Aktivatoren
      Mectine,
      zum Beispiel Abamectin, Emamectin, Emamectin-benzoate, Ivermectin, Lepimectin, Milbemycin
   Juvenilhormon-Mimetika,
      zum Beispiel Diofenolan, Epofenonane, Fenoxycarb, Hydroprene, Kinoprene, Methoprene, Pyriproxifen, Triprene
   Ecdysonagonisten/disruptoren
      Diacylhydrazine,
      zum Beispiel Chromafenozide, Halofenozide, Methoxyfenozide, Tebufenozide
   Inhibitoren der Chitinbiosynthese
      Benzoylharnstoffe, zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
      Buprofezin
      Cyromazine
   Inhibitoren der oxidativen Phosphorylierung, ATP-Disruptoren
      Diafenthiuron
      Organozinnverbindungen,
      zum Beispiel Azocyclotin, Cyhexatin, Fenbutatin-oxide
   Entkoppler der oxidativen Phosphorylierung durch Unterbrechung des H-Protongradienten
      Pyrrole,
      zum Beispiel Chlorfenapyr
      Dinitrophenole,
      zum Beispiel Binapacyrl, Dinobuton, Dinocap, DNOC, Meptyldinocap
   Site-1-Elektronentransportinhibitoren
      METI's,
      zum Beispiel Fenazaquin, Fenpyroximate, Pyrimidifen, Pyridaben, Tebufenpyrad, Tolfenpyrad
      Hydramethylnon
      Dicofol
   Site-II-Elektronentransportinhibitoren
      Rotenone
   Site-III-Elektrenentransportinhibitoren
      Acequinocyl, Fluacrypyrim
   Mikrobielle Disruptoren der Insektendarmmembran
      Bacillus thuringiensis-Stämme
   Inhibitoren der Fettsynthese
      Tetronsäuren,
      zum Beispiel Spirodiclofen, Spiromesifen,
      Tetramsäuren,
      zum Beispiel Spirotetramat, cis-3-(2,5-dimethylphenyl)-4-hydroxy-8-methoxy-1-azaspiro[4.5]dec-3-en-2-on
      Carboxamide,
      zum Beispiel Flonicamid
      Oktopaminerge Agonisten,
      zum Beispiel Amitraz
   Inhibitoren der Magnesium-stimulierten ATPase,
      Propargite
      Nereistoxin-Analoge,
      zum Beispiel Thiocyclam hydrogen oxalate, Thiosultap-sodium
   Agonisten des Ryanodin-Rezeptors,
      Benzoesäuredicarboxamide,
      zum Beispiel Flubendiamid
      Anthranilamide,
      zum Beispiel Rynaxypyr (3-bromo-N-{4-chloro-2-methyl-6-[(methylamino)carbonyl]phenyl}-1-(3-chloropyridin-2-yl)-1H-pyrazole-5-carboxamide)
   Biologika, Hormone oder Pheromone
      Azadirachtin, Bacillus spec., Beauveria spec., Codlemone, Metarrhizium spec., Paecilomyces spec., Thuringiensin, Verticillium spec.
   Wirkstoffe mit unbekannten oder nicht spezifischen Wirkmechanismen
      Begasungsmittel,
      zum Beispiel Aluminium phosphide, Methyl bromide, Sulfuryl fluoride
      Fraßhemmer,
      zum Beispiel Cryolite, Flonicamid, Pymetrozine
      Milbenwachstumsinhibitoren,
      zum Beispiel Clofentezine, Etoxazole, Hexythiazox
      Amidoflumet, Benelothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), [M]® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von **I** bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfem, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die Erfindung wird durch die folgenden Beispiele erläutert, ohne dadurch in irgendeiner Weise zu begrenzen.

In den Tabellen werden folgende Abkürzungen benutzt:

| | |
|---|---|
| Me | CH₃ |
| Et | C₂H₅ |
| nPr | n-Propyl |
| iPr | i-Propyl |
| nBu | n-Butyl |
| secBu | s-Butyl |
| iBu | i-Butyl |
| tBu | t-Butyl |
| Ph | Phenyl |
| cPr | Cyclopropyl |
| cPent | Cyclopentyl |

### Synchesebeispiele

Gemäß den oben angegebenen allgemeinen Methoden lassen sich die in den folgenden Tabellen angegebenen Verbindungen herstellen.

**Tabelle 1**

| | | | | |
|---|---|---|---|---|
| 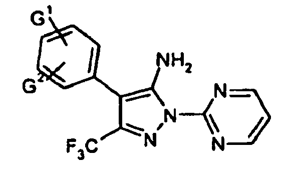 | | | | |

| Beispielnr. | | G¹ | G² | Physikalische Daten |
|---|---|---|---|---|
| 1- | 01 | 2-F | H | NMR siehe unten |
| 1- | 02 | 3-F | H | NMR siehe unten |
| 1- | 03 | 4-F | H | NMR siehe unten |
| 1- | 04 | 2-Cl | H | logP = 2,59 |
| 1- | 05 | 3-CI | H | logP = 2,96 |
| 1- | 06 | 4-Cl | H | NMR siehe unten |
| 1- | 07 | 2-Br | H | NMR siehe unten |
| 1- | 08 | 3-Br | H | NMR siehe unten |
| 1 - | 09 | 4-Br | H | NMR siehe unten |
| 1- | 10 | 2-I | H | NMR siehe unten |
| 1- | 11 | 3-I | H | NMR siehe unten |
| 1- | 12 | 4-I | H | NMR siehe unten |
| 1- | 13 | 2-F | 3-F | NMR siehe unten |
| 1 - | 14 | 2-F | 4-F | NMR siehe unten |
| 1 - | 15 | 2-F | 5-F | NMR siehe unten |
| 1- | 16 | 2-F | 6-F | |
| 1- | 17 | 2-F | 3-Cl | NMR siehe unten |
| 1- | 18 | 2-F | 4-Cl | NMR siehe unten |
| 1- | 19 | 2-F | 5-Cl | |
| 1- | 20 | 2-F | 6-Cl | NMR siehe unten |
| 1- | 21 | 2-F | 3-Br | |
| 1- | 22 | 2-F | 4-Br | NMR siehe unten |
| 1- | 23 | 2-F | 5-Br | NMR siehe unten |
| 1- | 24 | 2-F | 6-Br | |
| 1- | 25 | 2-F | 3-I | |
| 1- | 26 | 2-F | 4-I | |
| 1- | 27 | 2-F | 5-I | |
| 1- | 28 | 2-F | 6-I | |
| 1- | 29 | 2-Cl | 3-F | |
| 1- | 30 | 2-Cl | 4-F | NMR siehe unten |
| 1- | 31 | 2-Cl | 5-F | |
| 1- | 32 | 2-Cl | 3-Cl | NMR siehe unten |
| 1- | 33 | 2-Cl | 4-Cl | NMR siehe unten |
| 1- | 34 | 2-Cl | 5-Cl | NMR siehe unten |
| 1- | 35 | 2-Cl | 6-Cl | NMR siehe unten |
| 1- | 36 | 2-Cl | 3-Br | |
| 1- | 37 | 2-Cl | 4-Br | ¹H-NMR (d₆-DMSO): 8.92, d, 2H, 7.62, t, 1H |
| 1- | 38 | 2-Cl | 5-Br | NMR siehe unten |
| 1- | 39 | 2-Cl | 6-Br | |
| 1- | 40 | 2-Cl | 3-I | |
| 1- | 41 | 2-Cl | 4-1 | |
| 1- | 42 | 2-Cl | 5-I | |
| 1- | 43 | 2-Cl | 6-I | |
| 1- | 44 | 2-Br | 3-F | |
| 1- | 45 | 2-Br | 4-F | |
| 1- | 46 | 2-Br | 5-F | |
| 1- | 47 | 2-Br | 3-Cl | |
| 1- | 48 | 2-Br | 4-Cl | |
| 1- | 49 | 2-Br | 5-Cl | |
| 1- | 50 | 2-Br | 3-Br | |
| 1- | 51 | 2-Br | 4-Br | |
| 1- | 52 | 2-Br | 5-Br | |
| 1- | 53 | 2-Br | 6-Br | |
| 1- | 54 | 2-Br | 3-I | |
| 1- | 55 | 2-Br | 4-I | |
| 1- | 56 | 2-Br | 5-I | |
| 1- | 57 | 2-Br | 6-I | |
| 1- | 58 | 2-I | 3-F | |
| 1- | 59 | 2-I | 4-F | |
| 1- | 60 | 2-I | 5-F | |
| 1- | 61 | 2-I | 3-Cl | |
| 1- | 62 | 2-1 | 4-Cl | |
| 1- | 63 | 2-I | 5-Cl | |
| 1- | 64 | 2-I | 3-Br | |
| 1- | 65 | 2-I | 4-Br | |
| 1- | 66 | 2-I | 5-Br | |
| 1- | 67 | 2-I | 3-I | |
| 1- | 68 | 2-I | 4-1 | |
| 1- | 69 | 2-I | 5-1 | |
| 1- | 70 | 2-I | 6-I | |
| 1- | 71 | 3-F | 4-F | NMR siehe unten |
| 1- | 72 | 3-F | 5-F | |
| 1- | 73 | 3-F | 4-Cl | NMR siehe unten |
| 1- | 74 | 3-F | 5-Cl | NMR siehe unten |
| 1- | 75 | 3-F | 4-Br | NMR siehe unten |
| 1- | 76 | 3-F | 5-Br | |
| 1- | 77 | 3-F | 4-I | |
| 1- | 78 | 3-F | 5-I | |
| 1- | 79 | 3-CI | 4-F | Herstellungsbsp. |
| 1- | 80 | 3-Cl | 4·Cl | NMR siehe unten |
| 1- | 81 | 3-Cl | 5-CI | NMR siehe unten |
| 1- | 82 | 3-Cl | 4-Br | NMR siehe unten |
| 1- | 83 | 3-Cl | 5-Br | NMR siehe unten |
| 1- | 84 | 3-Cl | 4-I | ¹H-NMR (d₆-DMSO): 8.93, d, 2H, 7.07, dd, 1H |
| 1- | 85 | 3-Cl | 5-1 | |
| 1- | 86 | 3-Br | 4-F | |
| 1- | 87 | 3-Br | 4-Cl | NMR siehe unten |
| 1- | 88 | 3-Br | 4-Br | NMR siehe unten |
| 1- | 89 | 3-Br | 5-Br | NMR siehe unten |
| 1- | 90 | 3-Br | 4-I | |
| 1- | 91 | 3-Br | 5-I | |
| 1- | 92 | 3-I | 4-F | |
| 1- | 93 | 3-1 | 4-Cl | |
| 1- | 94 | 3-I | 4-Br | |
| 1- | 95 | 3-I | 4-I | |
| 1- | 96 | 3-1 | 4-1 | |

**Tabelle 2**

| | | | | | | |
|---|---|---|---|---|---|---|
| 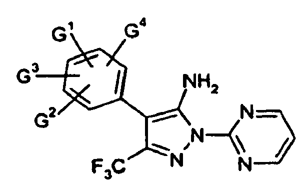 | | | | | | |

| Beispielnr. | | G¹ | G² | G³ | G⁴ | Physikalische Daten |
|---|---|---|---|---|---|---|
| 2- | 01 | 2-F | 3-F | 4-F | H | |
| 2- | 02 | 2-F | 3-F | 5-F | H | NMR siehe unten |
| 2- | 03 | 2-F | 3-F | 6-F | H | |
| 2- | 04 | 2-F | 4-F | 5-F | H | NMR siehe unten |
| 2- | 05 | 2-F | 4-F | 6-F | H | NMR siehe unten |
| 2- | 06 | 3-F | 4-F | 5-F | H | NMR siehe unten |
| 2- | 07 | 2-Cl | 3-Cl | 4-Cl | H | |
| 2- | 08 | 2-Cl | 3-Cl | 5-Cl | H | |
| 2- | 09 | 2-Cl | 3-Cl | 6-Cl | H | |
| 2- | 10 | 2-Cl | 4-Cl | 5-Cl | H | |
| 2- | 11 | 2-Cl | 4-Cl | 6-Cl | H | |
| 2- | 12 | 3-Cl | 4-Cl | 5-Cl | H | NMR siehe unten |
| 2- | 13 | 2-F | 4-Cl | 6-F | H | NMR siehe unten |
| 2- | 14 | 2-Cl | 4-Cl | 5-F | H | NMR siehe unten |
| 2- | 15 | 2-Cl | 4-F | 5-F | H | NMR siehe unten |
| 2- | 16 | 2-F | 4-F | 5-F | 3-Cl | NMR siehe unten |
| 2- | 17 | 2-F | 4-F | 3-Cl | H | NMR siehe unten |
| 2- | 18 | 2-Cl | 3-Cl | 6-F | H | |
| 2- | 19 | 3-Cl | 4-Cl | 5-CF₃ | H | ¹H-NMR (d₆-DMSO): 8.94, d, 2H, 7.53, t, 1H |
| 2- | 20 | 3-Cl | 4-CF₃ | 5-Cl | H | |
| 2- | 21 | 3-Cl | 4-F | 5-Cl | H | |

**Tabelle 3**

| | | | | |
|---|---|---|---|---|
| 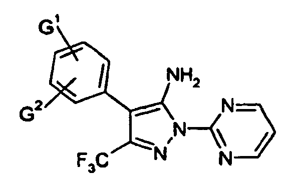 | | | | |

| Beispielnr. | | Gel | G² | Physikalische Daten |
|---|---|---|---|---|
| 3- | 01 | 2-CF₃ | H | |
| 3- | 02 | 3-CF₃ | H | NMR siehe unten |
| 3- | 03 | 4-CF₃ | H | NMR siehe unten |
| 3- | 04 | 4-C₂F₅ | H | |
| 3- | 05 | 4-nC₃F₇ | H | |
| 3- | 06 | 4-iC₃F₇ | H | |
| 3- | 07 | 4-CH₂CH₂CF₃ | H | |
| 3- | 08 | 4-CH(CH₃)CF₃ | H | |
| 3- | 09 | 2-CF₃ | 3-CF₃ | |
| 3- | 10 | 2-CF₃ | 4-CF₃ | |
| 3- | 11 | 2-CF₃ | 5-CF₃ | NMR siehe unten |
| 3- | 12 | 2-CF₃ | 6-CF₃ | |
| 3- | 13 | 3-CF₃ | 4-CF₃ | |
| 3- | 14 | 3-CF₃ | 5-CF₃ | NMR siehe unten |
| 3- | 15 | 4-CF₃ | 2-F | NMR siehe unten |
| 3- | 16 | 4-CF₃ | 2-Cl | |
| 3- | 17 | 4-CF₃ | 2-Br | |
| 3- | 18 | 4-CF₃ | 2-I | |
| 3- | 19 | 4-CF₃ | 3-F | |
| 3- | 20 | 4-CF₃ | 3-Cl | NMR siehe unten |
| 3- | 21 | 4-CF₃ | 3-Br | |
| 3- | 22 | 4-CF₃ | 3-I | |
| 3- | 23 | 3-CF₃ | 2-Cl | NMR siehe unten |
| 3- | 24 | 3-CF₃ | 4-Cl | NMR siehe unten |
| 3- | 25 | 3-CF₃ | 5-Cl | NMR siehe unten, s. a. detaillierte Synthesebeispiele |
| 3- | 26 | 5-CF₃ | 2-Cl | NMR siehe unten |
| 3- | 27 | 2-CF₃ | 3-Cl | |
| 3- | 28 | 2-CF₃ | 4-Cl | |
| 3- | 29 | 2-CF₃ | 5-Cl | |
| 3- | 30 | 2-CF₃ | 6-Cl | |
| 3- | 31 | 3-CF₃ | 4-F | NMR siehe unten |
| 3- | 32 | 3-CF₃ | 5-F | NMR siehe unten |
| 3- | 33 | 5-CF₃ | 2-Br | NMR siehe unten |
| 3- | 34 | 3-CF₃ | 2-F | NMR siehe unten |
| 3- | 35 | 3-CF₃ | 4-Br | |

**Tabelle 4**

| | | | | |
|---|---|---|---|---|
| 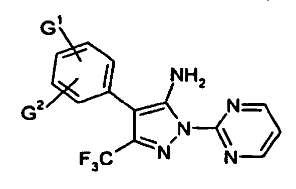 | | | | |

| Beispielnr. | | G¹ | G² | Physikalische Daten |
|---|---|---|---|---|
| 4- | 01 | 2-OCHF₂ | H | |
| 4- | 02 | 3-OCHF₂ | H | NMR siehe unten |
| 4- | 03 | 4-OCHF₂ | H | NMR siehe unten |
| 4- | 04 | 2-OCF₃ | H | |
| 4- | 05 | 3-OCF₃ | H | NMR siehe unten |
| 4- | 06 | 4-OCF₃ | H | NMR siehe unten |
| 4- | 07 | 4-OCF₂Cl | H | NMR siehe unten |
| 4- | 08 | 4-OCF₂Br | H | |
| 4- | 09 | 4-OCF₂CF₂H | H | |
| 4- | 10 | 4-OCF₂CFClH | H | NMR siehe unten |
| 4- | 11 | 4-OCF₂CF₃ | H | |
| 4- | 12 | 4-OCH₂CF₂H | H | |
| 4- | 13 | 4-OCH₂CF₃ | H | |
| 4- | 14 | 4-OCH₂CF₂CF₂H | H | |
| 4- | 15 | 4-OCH₂CF₂CF₃ | H | |
| 4- | 16 | 3-OCF₂CHFCF₃ | H | NMR siehe unten |
| 4- | 17 | 2-OCF₃ | 4-F | |
| 4- | 18 | 3-OCF₃ | 4-F | NMR siehe unten |
| 4- | 19 | 4-OCF₃ | 3-F | NMR siehe unten |
| 4- | 20 | 2-OCF₃ | 4-Cl | |
| 4- | 21 | 3-OCF₃ | 4-Cl | NMR siehe unten |
| 4- | 22 | 4-OCF₃ | 3-Cl | |
| 4- | 23 | 3-OCF₃ | 5-Cl | NMR siehe unten |
| 4- | 24 | 2-OCF₃ | 4-Br | |
| 4- | 25 | 3-OCF₃ | 4-Br | |
| 4- | 26 | 4-OCF₃ | 3-Br | |
| 4- | 27 | 2-OCF₃ | 4-I | |
| 4- | 28 | 3-OCF₃ | 4-I | |
| 4- | 29 | 4-OCF₃ | 3-I | |
| 4- | 30 | 4-OCHF₂ | 3-F | |
| 4- | 31 | 4-OCHF₂ | 3-Cl | |
| 4- | 32 | 4-OCHF₂ | 3-Br | |
| 4- | 33 | 4-OCHF₂ | 3-I | |
| 4- | 34 | 3-OCF₃ | 4-OCF₃ | NMR siehe unten |
| 4- | 35 | 4-OCH₂CF₃ | 3-F | |
| 4- | 36 | 4-OCH₂CF₃ | 3-Cl | 19F: -61.7 s, -74,4 tr, CF₃ |
| 4- | 37 | 4-OCH₂CF₃ | 3-Br | |
| 4- | 38 | 4-OCH₂CF₃ | 3-I | |
| 4- | 39 | 4-OCH₂CF₃ | 2-F | |
| 4- | 40 | 4-OCH₂CF₃ | 2-Cl | |
| 4- | 41 | 4-OCH₂CF₃ | 2-Br | |
| 4- | 42 | 4-OCH₂CF₃ | 2-I | |
| 4- | 43 | 5-OCF₃ | 2-F | NMR siehe unten |
| 4- | 44 | 5-OCF₃ | 2-Cl | |
| 4- | 45 | 5-OCF₃ | 2-Br | |
| 4- | 46 | 5-OCF₃ | 2-1 | |
| 4- | 47 | 5-OCF₃ | 3-F | |
| 4- | 48 | 5-OCF₃ | 3-Cl | |
| 4- | 49 | 5-OCF₃ | 3-Br | |
| 4- | 50 | 5-OCF₃ | 3-1 | |
| 4- | 51 | 4-OCF₃ | 2-F | |
| 4- | 52 | 4-OCF₃ | 2-Cl | |
| 4- | 53 | 4-OCF₃ | 2-Br | |
| 4- | 54 | 4-OCF₃ | 2-I | |
| 4- | 55 | 3-OCF₂Cl | H | NMR siehe unten |

**Tabelle 5**

| | | | |
|---|---|---|---|
| 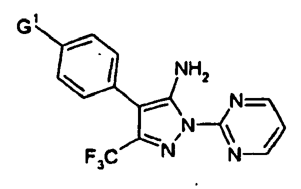 | | | |

| Beispielnr. | | G¹ | Physikalische Daten |
|---|---|---|---|
| 5- | 01 | H | NM R siehe unten |
| 5- | 02 | Me | NMR siehe unten |
| 5- | 03 | Et | |
| 5- | 04 | nPr | |
| 5- | 05 | iPr | 1H (d6DMSO): 2.93, 1H; 1.24, 6H; iPr |
| 5- | 06 | nBu | |
| 5- | 07 | secBu | |
| 5- | 08 | iBu | |
| 5- | 09 | tBu | 1H (d6DMSO): 1.32; tBu |
| 5- | 10 | n-C₅H₁₁ | |
| 5- | 11 | n-C₆H₁₃ | |
| 5- | 18 | CH₂OMe | |
| 5- | 19 | CH₂OEt | |
| 5- | 20 | CH₂OH | |
| 5- | 21 | OH | |
| 5- | 22 | OMe | NMR siehe unten |
| 5- | 23 | OEt | 1H: 4.07; OCH₂ |
| 5- | 24 | OnPr | |
| 5- | 25 | OiPr | 1H: 4.59, 1.37; OiPr |
| 5- | 26 | OnBu | |
| 5- | 27 | OsecBu | |
| 5- | 28 | OiBu | |
| 5- | 29 | OtBu | |
| 5- | 30 | OCH₂OMe | |
| 5- | 31 | OCH₂CH₂OMe | |
| 5- | 32 | OCH₂cPr | |
| 5- | 33 | OCH₂cPentyl | |
| 5- | 34 | OCH₂cHexyl | |
| 5- | 35 | OcPentyl | |
| 5- | 36 | OcHexyl | |
| 5- | 37 | OCH₂CH=CH₂ | |
| 5- | 38 | OCH₂CCH | |
| 5- | 39 | OCH₂Phenyl | |
| 5- | 40 | OPhenyl | logP 3.22 |
| 5- | 41 | O-(4-F-Phenyl) | |
| 5- | 42 | O-(4-Cl-Phenyt) | 1H: 7.04, 7.34, Phenoxy |
| 5- | 43 | O-(4-CF₃-Phenyl) | |
| 5- | 44 | O-(2-Cl-4-CF₃-Phenyl) | 19F: -61.4, -62.5, CF₃ |
| 5- | 45 | O-(4-Me-Phenyl) | |
| 5- | 46 | O-(2,4-Cl₂-Phenyl) | |
| 5- | 47 | O-(5-Cl-2-Pyridyl) | |
| 5- | 48 | O-(3,5-Cl₂-2-Pyridyl) | |
| 5- | 49 | O-(5-CF₃-2-Pyridyl) . | |
| 5- | 50 | O-(3-Cl-5-CF₃-2-Pyridyl) | 19F: -61.5, -62.1, CF₃ |
| 5- | 51 | Phenyl | |
| 5- | 52 | 4-F-Phenyl | |
| 5- | 53 | 4-Cl-Phenyl | 1H: 7.65, 7.56, 7.49, 7.43, 8H, PhH |
| 5- | 54 | 4-Br-Phenyl | |
| 5- | 55 | 2-Pyridyl | |
| 5- | 56 | 3-Pyridyl | |
| 5- | 57 | 4-Pyridyl | |
| 5- | 58 | SMe | NMR siehe unten |
| 5- | 59 | SOMe | NMR siehe unten |
| 5- | 60 | SO₂Me | NMR siehe unten |
| 5- | 61 | SO₂NMe₂ | |
| 5- | 62 | SCF₃ | NMR siehe unten |
| 5- | 63 | SOCF₃ | |
| 5- | 64 | SO₂CF₃ | |
| 5- | 65 | CHO | |
| 5- | 70 | COCH₃ | |
| 5- | 71 | CN | NMR siehe unten |
| 5- | 72 | COOH | |
| 5- | 73 | COOMe | |
| 5- | 74 | COOEt | |
| 5- | 75 | CONH₂ | |
| 5- | 76 | CONMe₂ | |
| 5- | 77 | SiMe₃ | |
| 5- | 88 | Benzyl | 1H(d6DMSO): 3.97ppm, CH₂ |
| 5 | 89 | NMe₂ | NMR siehe unten |

**Tabelle 6**

| | | | |
|---|---|---|---|
| 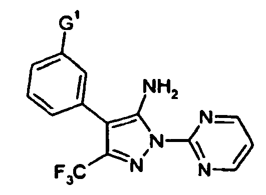 | | | |

| Beispielnr. | | G¹ | Physikalische Daten |
|---|---|---|---|
| 6- | 01 | Me | NMR siehe unten |
| 6- | 02 | Et | |
| 6- | 03 | nPr | |
| 6- | 04 | iPr | |
| 6- | 05 | nBu | |
| 6- | 06 | secBu | |
| 6- | 07 | iBu | |
| 6- | 08 | tBu | |
| 6- | 09 | n-C₅H₁₁ | |
| 6- | 10 | n-C₆H₁₃ | |
| 6- | 17 | CH₂OMe | |
| 6- | 18 | CH₂OEt | |
| 6- | 19 | CH₂OH | |
| 6- | 20 | OH | |
| 6- | 21 | OMe | |
| 6- | 22 | OEt | |
| 6- | 23 | OnPr | |
| 6- | 24 | OiPr | |
| 6- | 25 | OnBu | |
| 6- | 26 | OsecBu | |
| 6- | 27 | OiBu | |
| 6- | 28 | OtBu | |
| 6- | 29 | OCH₂OMe | |
| 6- | 30 | OCH₂CH₂OMe | |
| 6- | 31 | OCH₂cPr | |
| 6- | 32 | OCH₂cPentyl | |
| 6- | 33 | OCH₂cHexyl | |
| 6- | 34 | OcPentyl | |
| 6- | 35 | OcHexyl | |
| 6- | 36 | OCH₂CH=CH₂ | |
| 6- | 37 | OCH₂CCH | |
| 6- | 38 | OCH₂Phenyl | |
| 6- | 39 | OPhenyl | |
| 6- | 40 | O-(4-F-Phenyl) | |
| 6- | 41 | O-(4-Cl-Phenyl) | |
| 6- | 42 | O-(4-CF₃-Phenyl) | |
| 6- | 43 | O-(2-Cl-4-CF₃-Phenyl) | |
| 6- | 44 | O-(4-Me-Phenyl) | |
| 6- | 45 | O-(2,4-Cl₂-Phenyl) | |
| 6- | 46 | O-(5-Cl-2-Pyridyl) | |
| 6- | 47 | O-(3,5-Cl₂-2-Pyridyl) | |
| 6- | 48 | O-(5-CF₃-2-Pyridyl) | |
| 6- | 49 | O-(3-Cl-5-CF₃-2-Pyridyl) | |
| 6- | 50 | Phenyl | |
| 6- | 51 | 4-F-Phenyl | |
| 6- | 52 | 4-Cl-Phenyl | |
| 6- | 53 | 4-Br-Phenyl | |
| 6- | 54 | 2-Pyridyl | |
| 6- | 55 | 3-Pyridyl | |
| 6- | 56 | 4-Pyridyl | |
| 6- | 57 | SMe | |
| 6- | 58 | SOMe | |
| 6- | 59 | SO₂Me | |
| 6- | 60 | SO₂NMe₂ | |
| 6- | 61 | SCF₃ | NMR siehe unten |
| 6- | 62 | SOCF₃ | |
| 6- | 63 | SO₂CF₃ | |
| 6- | 64 | CHO | |
| 6- | 69 | COCH₃ | |
| 6- | 70 | CN | NMR siehe unten |
| 6- | 71 | COOH | |
| 6- | 72 | COOMe | NMR siehe unten |
| 6- | 73 | COOEt | |
| 6- | 74 | CONH₂ | |
| 6- | 75 | CONMe₂ | |
| 6- | 76 | SiMe₃ | |
| 6 | 77 | NMe₂ | NMR siehe unten |
| 6 | 78 | NO₂ | NMR siehe unten |

**Tabelle 7**

| | | | |
|---|---|---|---|
| 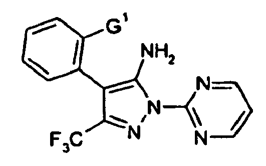 | | | |

| Beispielnr. | | G¹ | Physikalische Daten |
|---|---|---|---|
| 7- | 01 | Me | NMR siehe unten |
| 7- | 02 | Et | NMR siehe unten |
| 7- | 03 | nPr | |
| 7- | 04 | iPr | |
| 7- | 05 | nBu | |
| 7- | 06 | secBu | |
| 7- | 07 | iBu | |
| 7- | 08 | tBu | |
| 7- | 09 | n-C₅H₁₁ | |
| 7- | 10 | n-C₆H₁₃ | |
| 7- | 17 | CH₂OMe | |
| 7- | 18 | CH₂OEt | |
| 7- | 19 | CH₂OH | |
| 7- | 20 | OH | |
| 7- | 21 | OMe | |
| 7- | 22 | OEt | |
| 7- | 23 | OnPr | |
| 7- | 24 | OiPr | |
| 7- | 25 | OnBu | |
| 7- | 26 | OsecBu | |
| 7- | 27 | OiBu | |
| 7- | 28 | OtBu | |
| 7- | 29 | OCH₂OMe | |
| 7- | 30 | OCH₂CH₂OMe | |
| 7- | 31 | OCH₂cPr | |
| 7- | 32 | OCH₂cPentyl | |
| 7- | 33 | OCH₂cHexyl | |
| 7- | 34 | OcPentyl | |
| 7- | 35 | OcHexyl | |
| 7- | 36 | OCH₂CH=CH₂ | |
| 7- | 37 | OCH₂CCH | |
| 7- | 38 | OCH₂Phenyl | |
| 7- | 39 | OPhenyl | |
| 7- | 40 | O-(4-F-Phenyl) | |
| 7- | 41 | O-(4-Cl-Phenyl) | |
| 7- | 42 | O-(4-CF₃-Phenyl) | |
| 7- | 43 | O-(2-Cl-4-CF₃-Phenyl) | |
| 7- | 44 | O-(4-Me-Phenyl) | |
| 7- | 45 | O-(2,4-Cl₂-Phenyl) | |
| 7- | 46 | O-(5-Cl-2-Pyridyl) | |
| 7- | 47 | O-(3,5-Cl₂-2-Pyridyl) | |
| 7- | 48 | O-(5-CF₃-2-Pyridyl) | |
| 7- | 49 | O-(3-Cl-5-CF₃-2-Pyridyl) | |
| 7- | 50 | Phenyl | |
| 7- | 51 | 4-F-Phenyl | |
| 7- | 52 | 4-Cl-Phenyl | |
| 7- | 53 | 4-Br-Phenyl | |
| 7- | 54 | 2-Pyridyl | |
| 7- | 55 | 3-Pyridyl | |
| 7- | 56 | 4-Pyridyl | |
| 7- | 57 | SMe | |
| 7- | 58 | SOMe | |
| 7- | 59 | SO₂Me | |
| 7- | 60 | SO₂NMe₂ | |
| 7- | 61 | SCF₃ | |
| 7- | 62 | SOCF₃ | |
| 7- | 63 | SO₂CF₃ | |
| 7- | 64 | CHO | |
| 7- | 69 | COCH₃ | |
| 7- | 70 | CN | |
| 7- | 71 | COOH | |
| 7- | 72 | COOMe | |
| 7- | 73 | COOEt | |
| 7- | 74 | CONH₂ | |
| 7- | 75 | CONMe₂ | |
| 7- | 76 | SiMe₃ | |

**Tabelle 8**

| | | | | | |
|---|---|---|---|---|---|
| 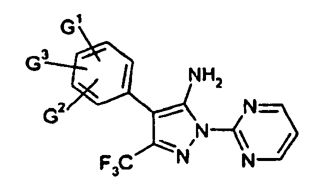 | | | | | |

| Beispielnr. | | G¹ | G² | G³ | Physikalische Daten |
|---|---|---|---|---|---|
| 8- | 01 | 2-Me | 3-Me | H | |
| 8- | 02 | 2-Me | 4-Me | H | |
| 8- | 03 | 2-Me | 5-Me | H | |
| 8- | 04 | 2-Me | 6-Me | H | |
| 8- | 05 | 3-Me | 4-Me | H | NMR siehe unten |
| 8- | 06 | 3-Me | 5-Me | H | NMR siehe unten |
| 8- | 07 | 2-OMe | 3-OMe | H | |
| 8- | 08 | 2-OMe | 4-OMe | H | |
| 8- | 09 | 2-OMe | 5-OMe | H | |
| 8- | 10 | 2-OMe | 6-OMe | H | |
| 8- | 11 | 3-OMe | 4-OMe | H | 1H: 3.89, 3.92ppm, OMe |
| 8- | 12 | 3-OMe | 5-OMe | H | |
| 8- | 13 | 2-Me | 3-Me | 4-Me | |
| 8- | 14 | 2-Me | 3-Me | 5-Me | |
| 8- | 15 | 2-Me | 3-Me | 6-Me | |
| 8- | 16 | 2-Me | 4-Me | 5-Me | |
| 8- | 17 | 2-Me | 4-Me | 6-Me | |
| 8- | 18 | 3-Me | 4-Me | 5-Me | |
| 8- | 19 | 2-OMe | 3-OMe | 4-OMe | |
| 8- | 20 | 2-OMe | 3-OMe | 5-OMe | |
| 8- | 21 | 2-OMe | 3-OMe | 6-OMe | |
| 8- | 22 | 2-OMe | 4-OMe | 5-OMe | |
| 8- | 23 | 2-OMe | 4-OMe | 6-OMe | |
| 8- | 24 | 3-OMe | 4-OMe | 5-OMe | 1H: 6.63ppm, PhH |
| 8- | 25 | 3-F | 4-OMe | H | NMR siehe unten |
| 8- | 26 | 3-Cl | 4-OMe | H | NMR siehe unten |
| 8- | 27 | 3-Br | 4-OMe | H | NMR siehe unten |
| 8- | 28 | 3-1 | 4-OMe | H | |
| 8- | 29 | 3-F | 4-OEt | H | |
| 8- | 30 | 3-Cl | 4-OEt | H | 1H: 7.00, 7.24, 7.42ppm, PhH |
| 8- | 31 | 3-Br | 4-OEt | H | 1H: 4.16ppm, OCH₂ |
| 8- | 32 | 3-I | 4-OEt | H | |
| 8- | 33 | 3-F | 4-OMe | 5-F | NMR siehe unten |
| 8- | 34 | 3-Cl | 4-OMe | 5-Cl | |
| 8- | 35 | 3-Br | 4-OMe | 5-Br | |
| 8- | 36 | 3-I | 4-OMe | 5-I | |
| 8- | 37 | 3-F | 4-OEt | 5-F | |
| 8- | 38 | 3-Cl | 4-OEt | 5-Cl | |
| 8- | 39 | 3-Br | 4-OEt | 5-Br | |
| 8- | 40 | 3-I | 4-OEt | 5-I | |
| 8- | 41 | 4-F | 3-OPh | H | NMR siehe unten |
| 8- | 42 | 3-F | 5-Me | H | NMR siehe unten |
| 8- | 43 | 3-F | 4-Me | H | NMR siehe unten |
| 8- | 44 | 2-OMe | 5-Br | H | NMR siehe unten |
| 8- | 45 | 3-OMe | 4-Me | H | NMR siehe unten |
| 8- | 46 | 3-OMe | 5-Me | H | |
| 8- | 47 | 2-OMe | 5-Me | H | |
| 8- | 48 | 2-OMe | 5-Cl | H | NMR siehe unten |
| 8- | 49 | 2-OMe | 5-F | H | |
| 8- | 50 | 2-F | 5-OMe | H | |
| 8- | 51 | 2-Cl | 5-OMe | H | |
| 8- | 52 | 2-Br | 5-OMe | H | |
| 8- | 53 | 2-1 | 5-OMe | H | |
| 8- | 54 | 2-Cl | 4-Me | 5-Cl | NMR siehe unten |
| 8- | 55 | 2-Br | 4-Me | 5-Br | |
| 8- | 56 | 2-Cl | 4-OMe | 5-Cl | |
| 8- | 57 | 2-Br | 4-OMe | 5-Cl | |
| 8- | 58 | 2-Cl | 4-Me | 6-Cl | |
| 8- | 59 | 2-Br | 4-Me | 6-Br | |
| 8- | 60 | 2-Cl | 4-OMe | 6-Cl | |
| 8- | 61 | 2-Br | 4-OMe | 6-Br | |
| 8- | 62 | 2-Cl | 3-OMe | 4-OMe | NMR siehe unten |
| 8- | 63 | 2-Br | 3-OMe | 4-OMe | |
| 8- | 64 | 2-F | 3-OMe | 4-OMe | |
| 8- | 65 | 2-F | 3-F | 4-OMe | NMR siehe unten |
| 8- | 66 | 4-OCHF₂ | 3-OMe | H | NMR siehe unten |
| 8- | 67 | 2-F | 4-OMe | 5-OMe | |
| 8- | 68 | 2-Cl | 4-OMe | 5-OMe | 1H: 6.85, 6.99ppm, PhH |
| 8- | 69 | 2-Br | 4-OMe | 5-OMe | 1H: 6.85, 7.14ppm, PhH |
| 8- | 70 | 2-I | 4-OMe | 5-OMe | |
| 8- | 71 | 2-OMe | 4-F | H | |
| 8- | 72 | 2-OMe | 4-Cl | H | |
| 8- | 73 | 2-OMe | 4-Br | H | 19F: -62.2ppm, CF₃ |
| 8- | 74 | 2-OMe | 4-I | H | |
| 8- | 75 | 2-OEt | 4-F | H | |
| 8- | 76 | 2-OEt | 4-Cl | H | |
| 8- | 77 | 2-OEt | 4-Br | H | |
| 8- | 78 | 2-OEt | 4-I | H | |
| 8- | 79 | 3-OMe | 4-F | H | |
| 8- | 80 | 3-OMe | 4-Cl | H | |
| 8- | 81 | 3-OMe | 4-Br | H | |
| 8- | 82 | 3-OMe | 4-I | H | NMR siehe unten |
| 8- | 83 | 3-OEt | 4-F | H | |
| 8- | 84 | 3-OEt | 4-Cl | H | |
| 8- | 85 | 3-OEt | 4-Br | H | |
| 8- | 86 | 3-OEt | 4-I | H | |
| 8- | 87 | 3-F | 4-OnPr | H | |
| 8- | 88 | 3-Cl | 4-OnPr | H | NMR siehe unten |
| 8- | 89 | 3-Br | 4-OnPr | H | 1H: 4.03, 1.86, 1.10ppm, nPrO |
| 8- | 90 | 3-1 | 4-OnPr | H | |
| 8- | 91 | 3-F | 4-OiPr | H | |
| 8- | 92 | 3-Cl | 4-OiPr | H | NMR siehe detaillierte Synthesebeschreibung |
| 8- | 93 | 3-Br | 4-OiPr | H | |
| 8- | 94 | 3-I | 4-OiPr | H | |
| 8- | 95 | 3-F | 4-OnBu | H | |
| 8- | 96 | 3-Cl | 4-OnBu | H | NMR siehe unten |
| 8- | 97 | 3-Br | 4-OnBu | H | |
| 8- | 98 | 3-1 | 4-OnBu | H | |
| 8- | 99 | 3-F | 4-OsecBu | H | |
| 8- | 100 | 3-Cl | 4-OsecBu | H | |
| 8- | 101 | 3-Br | 4-OsecBu | H | |
| 8- | 102 | 3-1 | 4-OsecBu | H | |
| 8- | 103 | 3-F | 4-OiBu | H | |
| 8- | 104 | 3-Cl | 4-OiBu | H | |
| 8- | 105 | 3-Br | 4-OiBu | H | |
| 8- | 106 | 3-1 | 4-OiBu | H | |
| 8- | 107 | 3,4-(O-CH₂-CH2-O) | | 5-Cl | NMR siehe unten |
| 8- | 108 | 2-OMe | 5-OCF₃ | H | NMR siehe unten |
| 8- | 109 | 3-OMe | 4-OnPr | H | ¹H-NMR (d₆-DMSO): 0.99, 1.74, 3.95 ppm, OnPr |
| 8- | 110 | 3-OMe | 4-OEt | H | ¹H-NMR (d₆-DMSO): 1.35, 4.05 ppm, OEt |
| 8- | 111 | 3-OMe | 5-CF₃ | H | NMR siehe unten |
| 8- | 112 | 3-OEt | 5-CF₃ | H | NMR siehe unten |
| 8- | 113 | 3-OEt | 5-F | H | NMR siehe unten |

**Tabelle 9**

| | | | |
|---|---|---|---|
| 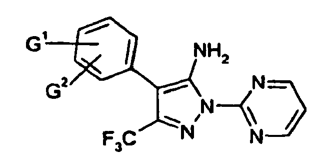 | | | |

| Beispielnr. | | G¹+G² | Physikalische Daten |
|---|---|---|---|
| 9- | 01 | 2.3-C₃H₆ | |
| 9- | 02 | 3,4-C₃H₆ | |
| 9- | 03 | 2,3-C₄H₈ | |
| 9- | 04 | 3,4-C₄H₈ | 1H(DMSO): 7.11, 7.25ppm, PhH |
| 9- | 05 | 2,3-OCH₂O | |
| 9- | 06 | 3,4-OCH₂O | NMR siehe unten |
| 9- | 07 | 2,3-OC₂H₄O | |
| 9- | 09 | 3,4-OC₂H₄O | NMR siehe unten |
| 9- | 09 | 2,3-OC₂H₄ | |
| 9- | 10 | 3,4-OC₂H₄ | |
| 9- | 11 | 2,3-C₂H₄O | |
| 9- | 12 | 3,4-C₂H₄O | |
| 9- | 13 | 2,3-OCF₂O | |
| 9- | 14 | 3,4-OCF₂O | |

**Tabelle 10**

| | | | | | | |
|---|---|---|---|---|---|---|
| 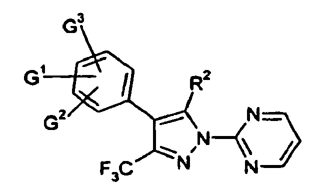 | | | | | | |

| Beispielnr. | | R² | G¹ | G² | G³ | Physikalische Daten |
|---|---|---|---|---|---|---|
| 10- | 01 | N(CH₂Ph)₂ | H | H | H | |
| 10- | 02 | N(CH₂Ph)₂ | 2-F | H | H | |
| 10- | 03 | N(CH₂Ph)₂ | 3-F | H | H | |
| 10- | 04 | N(CH₂Ph)₂ | 4-F | H | H | |
| 10- | 05 | N(CH₂Ph)₂ | 2-Cl | H | H | |
| 10- | 06 | N(CH₂Ph)₂ | 3-Cl | H | H | |
| 10- | 07 | N(CH₂Ph)₂ | 4-Cl | H | H | |
| 10- | 08 | N(CH₂Ph)₂ | 2-Br | H | H | |
| 10- | 09 | N(CH₂Ph)₂ | 3-Br | H | H | |
| 10- | 10 | N(CH₂Ph)₂ | 4-Br | H | H | |
| 10- | 11 | N(CH₂Ph)₂ | 2-I | H | H | |
| 10- | 12 | N(CH₂Ph)₂ | 3-I | H | H | |
| 10- | 13 | N(CH₂Ph)₂ | 4-I | H | H | NMR siehe unten |
| 10- | 14 | N(CH₂Ph)₂ | 2-Cl | 3-Cl | H | |
| 10- | 15 | N(CH₂Ph)₂ | 2-Cl | 4-Cl | H | |
| 10- | 16 | N(CH₂Ph)₂ | 2-Cl | 5-Cl | H | |
| 10- | 17 | N(CH₂Ph)₂ | 2-Cl | 6-Cl | H | |
| 10- | 18 | N(CH₂Ph)₂ | 2-Cl | 4-F | H | |
| 10- | 19 | N(CH₂Ph)₂ | 3-Cl | 4-Cl | H | |
| 10- | 20 | N(CH₂Ph)₂ | 3-Cl | 5-Cl | H | |
| 10- | 21 | N(CH₂Ph)₂ | 3-CF₃ | 4-Cl | H | NMR siehe unten |
| 10- | 22 | N(CH₂Ph)₂ | 3-F | 4-OMe | 5-F | NMR siehe unten |
| 10- | 23 | N(CH₂Ph)₂ | 3-CHF2 | H | H | NMR siehe unten |
| 10- | 24 | N(CH₂Ph)₂ | 3-F | 4-OMe | H | NMR siehe unten |
| 10- | 25 | N(CH₂Ph)₂ | 4-SMe | H | H | NMR siehe unten |
| 10- | 26 | N(CH₂Ph)₂ | 3-OCF₃ | 4-Cl | H | NMR siehe unten |
| 10- | 27 | N(CH₂Ph)₂ | 2-F | 3-Cl | H | NMR siehe unten |
| 10- | 28 | N(CH₂Ph)₂ | 4-OCHF₂ | H | H | NMR siehe unten |
| 10- | 29 | N(CH₂Ph)₂ | 3-Cl | 4-F | H | NMR siehe unten |

**Tabelle 11**

| | | | | |
|---|---|---|---|---|
| 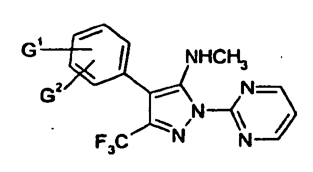 | | | | |

| Beispielnr. | | G¹ | G² | Physikalische Daten |
|---|---|---|---|---|
| 11- | 01 | H | H | |
| 11- | 02 | 2-F | H | |
| 11- | 03 | 3-F | H | |
| 11- | 04 | 4-F | H | |
| 11- | 05 | 2-Cl | H | |
| 11- | 06 | 3-Cl | H | |
| 11- | 07 | 4-Cl | H | NMR siehe unten |
| 11- | 08 | 2-Br | H | |
| 11- | 09 | 3-Br | H | |
| 11- | 10 | 4-Br | H | 19F: -61.85ppm |
| 11- | 11 | 2-I | H | |
| 11- | 12 | 3-I | H | |
| 11- | 13 | 4-I | H | |
| 11- | 14 | 2-F | 3-F | |
| 11- | 15 | 2-F | 4-F | |
| 11- | 16 | 2-F | 5-F | |
| 11- | 17 | 2-F | 6-F | |
| 11- | 18 | 2-F | 3-Cl | |
| 11- | 19 | 2-F | 4-Cl | |
| 11- | 20 | 2-F | 5-Cl | |
| 11- | 21 | 2-F | 6-Cl | |
| 11- | 22 | 2-F | 3-Br | |
| 11- | 23 | 2-F | 4-Br | |
| 11- | 24 | 2-F | 5-Br | |
| 11- | 25 | 2-F | 6-Br | |
| 11- | 26 | 2-F | 3-I | |
| 11- | 27 | 2-F | 4-1 | |
| 11- | 28 | 2-F | 5-1 | |
| 11- | 29 | 2-F | 6-1 | |
| 11- | 30 | 2-Cl | 3-F | |
| 11- | 31 | 2-Cl | 4-F | |
| 11- | 32 | 2-Cl | 5-F | |
| 11- | 33 | 2-Cl | 3-Cl | |
| 11- | 34 | 2-Cl | 4-Cl | |
| 11- | 35 | 2-Cl | 5-Cl | |
| 11- | 36 | 2-Cl | 6-Cl | |
| 11- | 37 | 2-Cl | 3-Br | |
| 11- | 38 | 2-Cl | 4-Br | |
| 11- | 39 | 2-Cl | 5-Br | |
| 11- | 40 | 2-Cl | 6-Br | |
| 11- | 41 | 2-Cl | 3-I | |
| 11- | 42 | 2-Cl | 4-1 | |
| 11- | 43 | 2-Cl | 5-1 | |
| 11- | 44 | 2-Cl | 6-1 | |
| 11- | 45 | 2-Br | 3-F | |
| 11- | 46 | 2-Br | 4-F | |
| 11- | 47 | 2-Br | 5-F | |
| 11- | 48 | 2-Br | 3-Cl | |
| 11- | 49 | 2-Br | 4-Cl | |
| 11- | 50 | 2-Br | 5-Cl | |
| 11- | 51 | 2-Br | 3-Br | |
| 11- | 52 | 2-Br | 4-Br | |
| 11- | 53 | 2-Br | 5-Br | |
| 11- | 54 | 2-Br | 6-Br | |
| 11- | 55 | 2-Br | 3-I | |
| 11- | 56 | 2-Br | 4-I | |
| 11- | 57 | 2-Br | 5-1 | |
| 11- | 58 | 2-Br | 6-1 | |
| 11- | 59 | 2-1 | 3-F | |
| 11- | 60 | 2-I | 4-F | |
| 11- | 61 | 2-I | 5-F | |
| 11- | 62 | 2-I | 3-Cl | |
| 11- | 63 | 2-I | 4-Cl | |
| 11- | 64 | 2-I | 5-Cl | |
| 11- | 65 | 2-I | 3-Br | |
| 11- | 66 | 2-I | 4-Br | |
| 11- | 67 | 2-I | 5-Br | |
| 11- | 68 | 2-I | 3-I | |
| 11- | 69 | 2-I | 4-I | |
| 11- | 70 | 2-I | 5-I | |
| 11- | 71 | 2-I | 6-I | |
| 11- | 72 | 3-F | 4-F | |
| 11- | 73 | 3-F | 5-F | |
| 11- | 74 | 3-F | 4-Cl | |
| 11- | 75 | 3-F | 5-Cl | |
| 11- | 76 | 3-F | 4-Br | |
| 11- | 77 | 3-F | 5-Br | |
| 11- | 78 | 3-F | 4-I | |
| 11- | 79 | 3-F | 5-I | |
| 11- | 80 | 3-Cl | 4-F | |
| 11- | 81 | 3-Cl | 4-Cl | |
| 11- | 82 | 3-Cl | 5-Cl | NMR siehe unten |
| 11- | 83 | 3-Cl | 4-Br | |
| 11- | 84 | 3-Cl | 5-Br | |
| 11- | 85 | 3-Cl | 4-I | |
| 11- | 86 | 3-Cl | 5-I | |
| 11- | 87 | 3-Br | 4-F | |
| 11- | 88 | 3-Br | 4-Cl | |
| 11- | 89 | 3-Br | 4-Br | |
| 11- | 90 | 3-Br | 5-Br | |
| 11- | 91 | 3-Br | 4-I | |
| 11- | 92 | 3-Br | 5-I | |
| 11- | 93 | 3-I | 4-F | |
| 11- | 94 | 3-I | 4-Cl | |
| 11- | 95 | 3-I | 4-Br | |
| 11- | 96 | 3-I | 4-I | |
| 11- | 97 | 3-I | 4-I | |

**Tabelle 12**

| | | | | | |
|---|---|---|---|---|---|
| 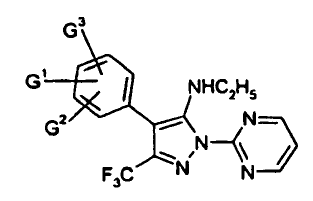 | | | | | |

| Beispielnr. | | G¹ | G² | G³ | Physikalische Daten |
|---|---|---|---|---|---|
| 12- | 01 | H | H | H | |
| 12- | 02 | 2-F | H | H | |
| 12- | 03 | 3-F | H | H | |
| 12- | 04 | 4-F | H | H | |
| 12- | 05 | 2-Cl | H | H | |
| 12- | 06 | 3-Cl | H | H | |
| 12- | 07 | 4-Cl | H | H | NMR siehe unten |
| 12- | 08 | 2-Br | H | H | |
| 12- | 09 | 3-Br | H | H | |
| 12- | 10 | 4-Br | H | H | |
| 12- | 11 | 2-I | H | H | |
| 12- | 12 | 3-I | H | H | NMR siehe unten |
| 12- | 13 | 4-I | H | H | |
| 12- | 14 | 2-F | 3-F | H | |
| 12- | 15 | 2-F | 4-F | H | |
| 12- | 16 | 2-F | 5-F | H | |
| 12- | 17 | 2-F | 6-F | H | |
| 12- | 18 | 2-F | 3-Cl | H | NMR siehe unten |
| 12- | 19 | 2-F | 4-Cl | H | |
| 12- | 20 | 2-F | 5-Cl | H | |
| 12- | 21 | 2-F | 6-Cl | H | |
| 12- | 22 | 2-F | 3-Br | H | |
| 12- | 23 | 2-F | 4-Br | H | |
| 12- | 24 | 2-F | 5-Br | H | |
| 12- | 25 | 2-F | 6-Br | H | |
| 12- | 26 | 2-F | 3-I | H | |
| 12- | 27 | 2-F | 4-I | H | |
| 12- | 28 | 2-F | 5-I | H | |
| 12- | 29 | 2-F | 6-I | H | |
| 12- | 30 | 2-Cl | 3-F | H | |
| 12- | 31 | 2-Cl | 4-F | H | |
| 12- | 32 | 2-Cl | 5-F | H | |
| 12- | 33 | 2-Cl | 3-Cl | H | |
| 12- | 34 | 2-Cl | 4-Cl | H | |
| 12- | 35 | 2-Cl | 5-Cl | H | |
| 12- | 36 | 2-Cl | 6-Cl | H | |
| 12- | 37 | 2-Cl | 3-Br | H | |
| 12- | 38 | 2-Cl | 4-Br | H | |
| 12- | 39 | 2-Cl | 5-Br | H | |
| 12- | 40 | 2-Cl | 6-Br | H | |
| 12- | 41 | 2-Cl | 3-I | H | |
| 12- | 42 | 2-Cl | 4-I | H | |
| 12- | 43 | 2-Cl | 5-I | H | |
| 12- | 44 | 2-Cl | 6-I | H | |
| 12- | 45 | 2-Br | 3-F | H | |
| 12- | 46 | 2-Br | 4-F | H | |
| 12- | 47 | 2-Br | 5-F | H | |
| 12- | 48 | 2-Br | 3-Cl | H | |
| 12- | 49 | 2-Br | 4-Cl | H | |
| 12- | 50 | 2-Br | 5-Cl | H | |
| 12- | 51 | 2-Br | 3-Br | H | |
| 12- | 52 | 2-Br | 4-Br | H | |
| 12- | 53 | 2-Br | 5-Br | H | |
| 12- | 54 | 2-Br | 6-Br | H | |
| 12- | 55 | 2-Br | 3-I | H | |
| 12- | 56 | 2-Br | 4-I | H | |
| 12- | 57 | 2-Br | 5-I | H | |
| 12- | 58 | 2-Br | 6-I | H | |
| 12- | 59 | 2-I | 3-F | H | |
| 12- | 60 | 2-I | 4-F | H | |
| 12- | 61 | 2-I | 5-F | H | |
| 12- | 62 | 2-I | 3-Cl | H | |
| 12- | 63 | 2-I | 4-Cl | H | |
| 12- | 64 | 2-I | 5-Cl | H | |
| 12- | 65 | 2-I | 3-Br | H | |
| 12- | 66 | 2-I | 4-Br | H | |
| 12- | 67 | 2-I | 5-Br | H | |
| 12- | 68 | 2-I | 3-I | H | |
| 12- | 69 | 2-I | 4-I | H | |
| 12- | 70 | 2-I | 5-I | H | |
| 12- | 71 | 2-I | 6-I | H | |
| 12- | 72 | 3-F | 4-F | H | |
| 12- | 73 | 3-F | 5-F | H | |
| 12- | 74 | 3-F | 4-Cl | H | |
| 12- | 75 | 3-F | 5-Cl | H | |
| 12- | 76 | 3-F | 4-Br | H | |
| 12- | 77 | 3-F | 5-Br | H | |
| 12- | 78 | 3-F | 4-I | H | |
| 12- | 79 | 3-F | 5-I | H | |
| 12- | 80 | 3-Cl | 4-F | H | NMR siehe unten |
| 12- | 81 | 3-Cl | 4-Cl | H | |
| 12- | 82 | 3-Cl | 5-Cl | H | NMR siehe unten |
| 12- | 83 | 3-Cl | 4-Br | H | |
| 12- | 84 | 3-Cl | 5-Br | H | |
| 12- | 85 | 3-Cl | 4-I | H | |
| 12- | 86 | 3-Cl | 5-I | H | |
| 12- | 87 | 3-Br | 4-F | H | |
| 12- | 88 | 3-Br | 4-Cl | H | |
| 12- | 89 | 3-Br | 4-Br | H | |
| 12- | 90 | 3-Br | 5-Br | H | |
| 12- | 91 | 3-Br | 4-I | H | |
| 12- | 92 | 3-Br | 5-I | H | |
| 12- | 93 | 3-I | 4-F | H | |
| 12- | 94 | 3-I | 4-Cl | H | |
| 12- | 95 | 3-I | 4-Br | H | |
| 12- | 96 | 3-I | 4-I | H | |
| 12- | 97 | 3-I | 4-I | H | |
| 12- | 98 | 3-F | 4-OMe | 5-F | NMR siehe unten |
| 12- | 99 | 3-CF₃ | 4-Cl | H | NMR siehe unten |
| 12- | 100 | 4-SMe | H | H | NMR siehe unten |
| 12- | 101 | 3-F | 4-OMe | H | NMR siehe unten |
| 12- | 102 | 3-OCHF₂ | H | H | NMR siehe unten |
| 12- | 103 | 4-OCHF₂ | H | H | NMR siehe unten |
| 12- | 104 | 3-OMe | 4-Me | H | NMR siehe unten |

**Tabelle 13**

| | | | | | | |
|---|---|---|---|---|---|---|
| 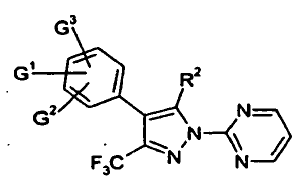 | | | | | | |

| Beispielnr. | | G¹ | G² | G³ | R² | Physikalische Daten |
|---|---|---|---|---|---|---|
| 13- | 01 | H | H | H | NMe₂ | |
| 13- | 02 | 2-F | H | H | NMe₂ | |
| 13- | 03 | 3-F | H | H | NMe₂ | |
| 13- | 04 | 4-F | H | H | NMe₂ | |
| 13- | 05 | 2-Cl | H | H | NMe₂ | |
| 13- | 06 | 3-Cl | H | H | NMe₂ | |
| 13- | 07 | 4-Cl | H | H | NMe₂ | |
| 13- | 08 | 2-Br | H | H | NMe₂ | |
| 13- | 09 | 3-Br | H | H | NMe₂ | |
| 13- | 10 | 4-Br | H | H | NMe₂ | |
| 13- | 11 | 2-I | H | H | NMe₂ | |
| 13- | 12 | 3-I | H | H | NMe₂ | |
| 13- | 13 | 4-I | H | H | NMe₂ | |
| 13- | 14 | 2-Cl | 3-Cl | H | NMe₂ | |
| 13- | 15 | 2-Cl | 4-Cl | H | NMe₂ | |
| 13- | 16 | 2-Cl | 5-Cl | H | NMe₂ | |
| 13- | 17 | 2-Cl | 6-Cl | H | NMe₂ | |
| 13- | 18 | 3-Cl | 4-Cl | H | NMe₂ | |
| 13- | 19 | 3-Cl | 5-Cl | H | NMe₂ | NMR siehe unten |
| 13- | 20 | H | H | H | NEt₂ | |
| 13- | 21 | 2-F | H | H | NEt₂ | |
| 13- | 22 | 3-F | H | H | NEt₂ | |
| 13- | 23 | 4-F | H | H | NEt₂ | |
| 13- | 24 | 2-Cl | H | H | NEt₂ | |
| 13- | 25 | 3-Cl | H | H | NEt₂ | |
| 13- | 26 | 4-Cl | H | H | NEt₂ | |
| 13- | 27 | 2-Br | H | H | NEt₂ | |
| 13- | 28 | 3-Br | H | H | NEt₂ | |
| 13- | 29 | 4-Br | H | H | NEt₂ | |
| 13- | 30 | 2-I | H | H | NEt₂ | |
| 13- | 31 | 3-I | H | H | NEt₂ | NMR siehe unten |
| 13- | 32 | 4-I | H | H | NEt₂ | NMR siehe unten |
| 13- | 33 | 2-Cl | 3-Cl | H | NEt₂ | |
| 13- | 34 | 2-Cl | 4-Cl | H | NEt₂ | |
| 13- | 35 | 2-Cl | 5-Cl | H | NEt₂ | |
| 13- | 36 | 2-Cl | 6-Cl | H | NEt₂ | |
| 13- | 37 | 3-Cl | 4-Cl | H | NEt₂ | |
| 13- | 38 | 3-Cl | 5-Cl | H | NEt₂ | |
| 13- | 39 | H | H | H | NMeEt | |
| 13- | 40 | 2-F | H | H | NMeEt | |
| 13- | 41 | 3-F | H | H | NMeEt | |
| 13- | 42 | 4-F | H | H | NMeEt | |
| 13- | 43 | 2-Cl | H | H | NMeEt | |
| 13- | 44 | 3-Cl | H | H | NMeEt | |
| 13- | 45 | 4-Cl | H | H | NMeEt | |
| 13- | 46 | 2-Br | H | H | NMeEt | |
| 13- | 47 | 3-Br | H | H | NMeEt | |
| 13- | 48 | 4-Br | H | H | NMeEt | |
| 13- | 49 | 2-I | H | H | NMeEt | |
| 13- | 50 | 3-I | H | H | NMeEt | |
| 13- | 51 | 4-I | H | H | NMeEt | |
| 13- | 52 | 2-Cl | 3-Cl | H | NMeEt | |
| 13- | 53 | 2-Cl | 4-Cl | H | NMeEt | |
| 13- | 54 | 2-Cl | 5-Cl | H | NMeEt | |
| 13- | 55 | 2-Cl | 6-Cl | H | NMeEt | |
| 13- | 56 | 3-Cl | 4-Cl | H | NMeEt | |
| 13- | 57 | 3-Cl | 5-Cl | H | NMeEt | |
| 13- | 58 | H | H | H | N(C₄H₈) | |
| 13- | 59 | 2-F | H | H | N(C₄H₈) | |
| 13- | 60 | 3-F | H | H | N(C₄H₈) | |
| 13- | 61 | 4-F | H | H | N(C₄H₈) | |
| 13- | 62 | 2-Cl | H | H | N(C₄H₈) | |
| 13- | 63 | 3-Cl | H | H | N(C₄H₈) | |
| 13- | 64 | 4-Cl | H | H | N(C₄H₈) | |
| 13- | 65 | 2-Br | H | H | N(C₄H₈) | |
| 13- | 66 | 3-Br | H | H | N(C₄H₈) | |
| 13- | 67 | 4-Br | H | H | N(C₄H₈) | |
| 13- | 68 | 2-I | H | H | N(C₄H₈) | |
| 13- | 69 | 3-I | H | H | N(C₄H₈) | |
| 13- | 70 | 4-I | H | H | N(C₄H₈) | |
| 13- | 71 | 2-Cl | 3-Cl | H | N(C₄H₈) | |
| 13- | 72 | 2-Cl | 4-Cl | H | N(C₄H₈) | |
| 13- | 73 | 2-Cl | 5-Cl | H | N(C₄H₈) | |
| 13- | 74 | 2-Cl | 6-Cl | H | N(C₄H₈) | |
| 13- | 75 | 3-Cl | 4-Cl | H | N(C₄H₈) | |
| 13- | 76 | 3-Cl | 5-Cl | H | N(C₄H₈) | |
| 13- | 77 | H | H | H | N(C₅H10) | |
| 13- | 78 | 2-F | H | H | N(C₅H10) | |
| 13- | 79 | 3-F | H | H | N(C₅H10) | |
| 13- | 80 | 4-F | H | H | N(C₅H10) | |
| 13- | 81 | 2-Cl | H | H | N(C₅H10) | |
| 13- | 82 | 3-Cl | H | H | N(C₅H10) | |
| 13- | 83 | 4-Cl | H | H | N(C₅H10) | |
| 13- | 84 | 2-Br | H | H | N(C₅H10) | |
| 13- | 85 | 3-Br | H | H | N(C₅H10) | |
| 13- | 86 | 4-Br | H | H | N(C₅H110) | |
| 13- | 87 | 2-I | H | H | N(C₅H10) | |
| 13- | 88 | 3-I | H | H | N(C₅H10) | |
| 13- | 89 | 4-I | H | H | N(C₅H10) | |
| 13- | 90 | 2-Cl | 3-Cl | H | N(C₅H10) | |
| 13- | 91 | 2-Cl | 4-Cl | H | N(C₅H10) | |
| 13- | 92 | 2-Cl | 5-Cl | H | N(C₅H10) | |
| 13- | 93 | 2-Cl | 6-Cl | H | N(C₅H10) | |
| 13- | 94 | 3-Cl | 4-Cl | H | N(C₅H10) | |
| 13- | 95 | 3-Cl | 5-Cl | H | N(C₅H10) | |
| 13- | 96 | H | H | H | N(C₂H₄OC₂H₄) | |
| 13- | 97 | 2-F | H | H | N(C₂H₄OC₂H₄) | |
| 13- | 98 | 3-F | H | H | N(C₂H₄OC₂H₄) | |
| 13- | 99 | 4-F | H | H | N(C₂H₄OC₂H₄) | |
| 13- | 100 | 2-Cl | H | H | N(C₂H₄OC₂H₄) | |
| 13- | 101 | 3-Cl | H | H | N(C₂H₄OC₂H₄) | |
| 13- | 102 | 4-Cl | H | H | N(C₂H₄OC₂H₄) | |
| 13- | 103 | 2-Br | H | H | N(C₂H₄OC₂H₄) | |
| 13- | 104 | 3-Br | H | H | N(C₂H₄OC₂H₄) | |
| 13- | 105 | 4-Br | H | H | N(C₂H₄OC₂H₄) | |
| 13- | 106 | 2-I | H | H | N(C₂H₄OC₂H₄) | |
| 13- | 107 | 3-I | H | H | N(C₂H₄OC₂H₄) | |
| 13- | 108 | 4-I | H | H | N(C₂H₄OC₂H₄) | |
| 13- | 109 | 2-Cl | 3-Cl | H | N(C₂H₄OC₂H₄) | |
| 13- | 110 | 2-Cl | 4-Cl | H | N(C₂H₄OC₂H₄) | |
| 13- | 111 | 2-Cl | 5-Cl | H | N(C₂H₄OC₂H₄) | |
| 13- | 112 | 2-Cl | 6-Cl | H | N(C₂H₄OC₂H₄) | |
| 13- | 113 | 3-Cl | 4-Cl | H | N(C₂H₄OC₂H₄) | |
| 13- | 114 | 3-Cl | 5-Cl | H | N(C₂H₄OC₂H₄) | |
| 13- | 115 | 3-Cl | 4-F | H | NEt₂ | NMR siehe unten |
| 13- | 116 | 3-OCF₃ | 4-Cl | H | NEt₂ | NMR siehe unten |
| 13- | 117 | 3-F | 4-OMe | 5-F | NEt₂ | NMR siehe unten |
| 13- | 118 | 3-Cl | 5-Cl | H | N(C₂H₄CH(CH₃)C₂H₄) | NMR siehe unten |

**Tabelle 14**

| | | | | | |
|---|---|---|---|---|---|
| 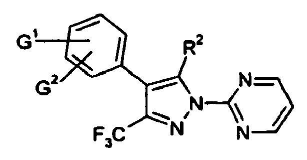 | | | | | |

| Beispielnr. | | G¹ | G² | R² | Physikalische Daten |
|---|---|---|---|---|---|
| 14- | 01 | H | H | Cl | |
| 14- | 02 | 2-F | H | Cl | |
| 14- | 03 | 3-F | H | Cl | |
| 14- | 04 | 4-F | H | Cl | |
| 14- | 05 | 2-Cl | H | Cl | |
| 14- | 06 | 3-Cl | H | Cl | IgP = 3,88 |
| 14- | 07 | 4-Cl | H | Cl | siehe Herstellungsbsp. |
| 14- | 08 | 2-Br | H | Cl | |
| 14- | 09 | 3-Br | H | Cl | |
| 14- | 10 | 4-Br | H | Cl | |
| 14- | 11 | 2-I | H | Cl | |
| 14- | 12 | 3-I | H | Cl | |
| 14- | 13 | 4-I | H | Cl | |
| 14- | 14 | 2-Cl | 3-Cl | Cl | |
| 14- | 15 | 2-Cl | 4-Cl | Cl | |
| 14- | 16 | 2-Cl | 5-Cl | Cl | |
| 14- | 17 | 2-Cl | 6-Cl | Cl | |
| 14- | 18 | 3-Cl | 4-Cl | Cl | NMR siehe unten |
| 14- | 19 | 3-Cl | 5-Cl | Cl | |
| 14- | 20 | H | H | Br | |
| 14- | 21 | 2-F | H | Br | |
| 14- | 22 | 3-F | H | Br | |
| 14- | 23 | 4-F | H | Br | |
| 14- | 24 | 2-Cl | H | Br | |
| 14- | 25 | 3-Cl | H | Br | 1gP = 3,89 |
| 14- | 26 | 4-Cl | H | Br | |
| 14- | 27 | 2-Br | H | Br | |
| 14- | 28 | 3-Br | H | Br | |
| 14- | 29 | 4-Br | H | Br | logP 3.75 |
| 14- | 30 | 2-I | H | Br | |
| 14- | 31 | 3-I | H | Br | |
| 14- | 32 | 4-I | H | Br | |
| 14- | 33 | 2-Cl | 3-Cl | Br | |
| 14- | 34 | 2-Cl | 4-Cl | Br | |
| 14- | 35 | 2-Cl | 5-Cl | Br | |
| 14- | 36 | 2-Cl | 6-Cl | Br | |
| 14- | 37 | 3-Cl | 4-Cl | Br | NMR siehe unten |
| 14- | 38 | 3-Cl | 5-Cl | Br | lgP = 4,62 |
| 14- | 39 | H | H | I | |
| 14- | 40 | 2-F | H | I | |
| 14- | 41 | 3-F | H | I | |
| 14- | 42 | 4-F | H | I | |
| 14- | 43 | 2-Cl | H | I | |
| 14- | 44 | 3-Cl | H | I | lgP = 3,78 |
| 14- | 45 | 4-Cl | H | I | |
| 14- | 46 | 2-Br | H | I | |
| 14- | 47 | 3-Br | H | I | |
| 14- | 48 | 4-Br | H | I | |
| 14- | 49 | 2-I | H | I | |
| 14- | 50 | 3-I | H | I | |
| 14- | 51 | 4-I | H | I | |
| 14- | 52 | 2-Cl | 3-Cl | I | |
| 14- | 53 | 2-Cl | 4-Cl | I | |
| 14- | 54 | 2-Cl | 5-Cl | I | |
| 14- | 55 | 2-Cl | 6-Cl | I | |
| 14- | 56 | 3-Cl | 4-Cl | I | lgP = 4,39 |
| 14- | 57 | 3-Cl | 5-Cl | I | lgP = 4,51 |
| 14- | 58 | 3-Cl | 5-CF₃ | Br | lgP = 4,64 |
| 14- | 59 | 3-Cl | 5-CF₃ | I | lgP = 4,39 |

**Tabelle 15**

| | | | | | |
|---|---|---|---|---|---|
| 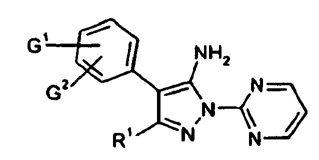 | | | | | |

| Beispielnr. | | G¹ | G² | R¹ | Physikalische Daten |
|---|---|---|---|---|---|
| 15- | 01 | H | H | CH₂F | |
| 15- | 02 | 2-F | H | CH₂F | |
| 15- | 03 | 3-F | H | CH₂F | |
| 15- | 04 | 4-F | H | CH₂F | |
| 15- | 05 | 2-Cl | H | CH₂F | |
| 15- | 06 | 3-Cl | H | CH₂F | |
| 15- | 07 | 4-Cl | H | CH₂F | NMR siehe unten |
| 15- | 08 | 2-Br | H | CH₂F | |
| 15- | 09 | 3-Br | H | CH₂F | |
| 15- | 10 | 4-Br | H | CH₂F | |
| 15- | 11 | 2-I | H | CH₂F | |
| 15- | 12 | 3-I | H | CH₂F | |
| 15- | 13 | 4-I | H | CH₂F | |
| 15- | 14 | 2-Cl | 3-Cl | CH₂F | |
| 15- | 15 | 2-Cl | 4-Cl | CH₂F | |
| 15- | 16 | 2-Cl | 5-Cl | CH₂F | |
| 15- | 17 | 2-Cl | 6-Cl | CH₂F | |
| 15- | 18 | 3-Cl | 4-Cl | CH₂F | |
| 15- | 19 | 3-Cl | 5-Cl | CH₂F | NMR siehe unten |
| 15- | 20 | H | H | CHF₂ | |
| 15- | 21 | 2-F | H | CHF₂ | |
| 15- | 22 | 3-F | H | CHF₂ | |
| 15- | 23 | 4-F | H | CHF₂ | |
| 15- | 24 | 2-Cl | H | CHF₂ | |
| 15- | 25 | 3-Cl | H | CHF₂ | |
| 15- | 26 | 4-Cl | H | CH₂ | |
| 15- | 27 | 2-Br | H | CHF₂ | |
| 15- | 28 | 3-Br | H | CHF₂ | |
| 15- | 29 | 4-Br | H | CHF₂ | |
| 15- | 30 | 2-I | H | CHF₂ | |
| 15- | 31 | 3-I | H | CHF₂ | |
| 15- | 32 | 4-I | H | CHF₂ | |
| 15- | 33 | 2-Cl | 3-Cl | CHF₂ | |
| 15- | 34 | 2-Cl | 4-Cl | CHF₂ | |
| 15- | 35 | 2-Cl | 5-Cl | CHF₂ | |
| 15- | 36 | 2-Cl | 6-Cl | CHF₂ | |
| 15- | 37 | 3-Cl | 4-Cl | CHF₂ | |
| 15- | 38 | 3-Cl | 5-Cl | CHF₂ | NMR siehe unten |
| 15- | 39 | H | H | CClF₂ | |
| 15- | 40 | 2-F | H | CClF₂ | |
| 15- | 41 | 3-F | H | CClF₂ | |
| 15- | 42 | 4-F | H | CClF₂ | |
| 15- | 43 | 2-Cl | H | CClF₂ | |
| 15- | 44 | 3-Cl | H | CClF₂ | |
| 15- | 45 | 4-Cl | H | CClF₂ | NMR siehe unten |
| 15- | 46 | 2-Br | H | CClF₂ | |
| 15- | 47 | 3-Br | H | CClF₂ | |
| 15- | 48 | 4-Br | H | CClF₂ | NMR siehe unten |
| 15- | 49 | 2-I | H | CClF₂ | |
| 15- | 50 | 3-I | H | CClF₂ | |
| 15- | 51 | 4-I | H | CClF₂ | NMR siehe unten |
| 15- | 52 | 2-Cl | 3-Cl | CClF₂ | |
| 15- | 53 | 2-Cl | 4-Cl | CClF₂ | |
| 15- | 54 | 2-Cl | 5-Cl | CClF₂ | |
| 15- | 55 | 2-Cl | 6-Cl | CClF₂ | |
| 15- | 56 | 3-Cl | 4-Cl | CClF₂ | |
| 15- | 57 | 3-Cl | 5-Cl | CClF₂ | NMR siehe unten |
| 15- | 58 | H | H | CCl₂F | |
| 15- | 59 | 2-F | H | CCl₂F | |
| 15- | 60 | 3-F | H | CCl₂F | |
| 15- | 61 | 4-F | H | CCl₂F | |
| 15- | 62 | 2-Cl | H | CCl₂F | |
| 15- | 63 | 3-Cl | H | CCl₂F | |
| 15- | 64 | 4-Cl | H | CCl₂F | |
| 15- | 65 | 2-Br | H | CCl₂F | |
| 15- | 66 | 3-Br | H | CCl₂F | |
| 15- | 67 | 4-Br | H | CCl₂F | |
| 15- | 68 | 2-I | H | CCl₂F | |
| 15- | 69 | 3-I | H | CCl₂F | |
| 15- | 70 | 4-I | H | CCl₂F | |
| 15- | 71 | 2-Cl | 3-Cl | CCl₂F | |
| 15- | 72 | 2-Cl | 4-Cl | CCl₂F | |
| 15- | 73 | 2-Cl | 5-Cl | CCl₂F | |
| 15- | 74 | 2-Cl | 6-Cl | CCl₂F | |
| 15- | 75 | 3-Cl | 4-Cl | CCl₂F | |
| 15- | 76 | 3-Cl | 5-Cl | CCl₂F | |
| 15- | 77 | H | H | CHClF | |
| 15- | 78 | 2-F | H | CHClF | |
| 15- | 79 | 3-F | H | CHClF | |
| 15- | 80 | 4-F | H | CHClF | |
| 15- | 81 | 2-Cl | H | CHClF | |
| 15- | 82 | 3-Cl | H | CHClF | |
| 15- | 83 | 4-Cl | H | CHClF | |
| 15- | 84 | 2-Br | H | CHClF | |
| 15- | 85 | 3-Br | H | CHClF | |
| 15- | 86 | 4-Br | H | CHClF | |
| 15- | 87 | 2-I | H | CHClF | |
| 15- | 88 | 3-I | H | CHClF | |
| 15- | 89 | 4-I | H | CHClF | |
| 15- | 90 | 2-Cl | 3-Cl | CHClF | |
| 15- | 91 | 2-Cl | 4-Cl | CHClF | |
| 15- | 92 | 2-Cl | 5-Cl | CHClF | |
| 15- | 93 | 2-Cl | 6-Cl | CHClF | |
| 15- | 94 | 3-Cl | 4-Cl | CHClF | |
| 15- | 95 | 3-Cl | 5-Cl | CHClF | NMR siehe unten |
| 15- | 96 | H | H | CBrF₂ | |
| 15- | 97 | 2-F | H | CBrF₂ | |
| 15- | 98 | 3-F | H | CBrF₂ | |
| 15- | 99 | 4-F | H | CBrF₂ | |
| 15- | 100 | 2-Cl | H | CBrF₂ | |
| 15- | 101 | 3-Cl | H | CBrF₂ | |
| 15- | 102 | 4-Cl | H | CBrF₂ | |
| 15- | 103 | 2-Br | H | CBrF₂ | |
| 15- | 104 | 3-Br | H | CBrF₂ | |
| 15- | 105 | 4-Br | H | CBrF₂ | NMR siehe unten |
| 15- | 106 | 2-I | H | CBrF₂ | |
| 15- | 107 | 3-I | H | CBrF₂ | |
| 15- | 108 | 4-I | H | CBrF₂ | NMR siehe unten |
| 15- | 109 | 2-Cl | 3-Cl | CBrF₂ | |
| 15- | 110 | 2-Cl | 4-Cl | CBrF₂ | |
| 15- | 111 | 2-Cl | 5-Cl | CBrF₂ | |
| 15- | 112 | 2-Cl | 6-Cl | CBrF₂ | |
| 15- | 113 | 3-Cl | 4-Cl | CBrF₂ | |
| 15- | 114 | 3-Cl | 5-Cl | CBrF₂ | NMR siehe unten |
| 15- | 115 | H | H | CBr₂F | |
| 15- | 116 | 2-F | H | CBr₂F | |
| 15- | 117 | 3-F | H | CBr₂F | |
| 15- | 118 | 4-F | H | CBr₂F | |
| 15- | 119 | 2-Cl | H | CBr₂F | |
| 15- | 120 | 3-Cl | H | CBr₂F | |
| 15- | 121 | 4-Cl | H | CBr₂F | |
| 15- | 122 | 2-Br | H | CBr₂F | |
| 15- | 123 | 3-Br | H | CBr₂F | |
| 15- | 124 | 4-Br | H | CBr₂F | |
| 15- | 125 | 2-I | H | CBr₂F | |
| 15- | 126 | 3-I | H | CBr₂F | |
| 15- | 127 | 4-I | H | CBr₂F | |
| 15- | 128 | 2-Cl | 3-Cl | CBr₂F | |
| 15- | 129 | 2-Cl | 4-Cl | CBr₂F | |
| 15- | 130 | 2-Cl | 5-Cl | CBr₂F | |
| 15- | 131 | 2-Cl | 6-Cl | CBr₂F | |
| 15- | 132 | 3-Cl | 4-Cl | CBr₂F | |
| 15- | 133 | 3-Cl | 5-Cl | CBr₂F | |
| 15- | 134 | H | H | CHBrF | |
| 15- | 135 | 2-F | H | CHBrF | |
| 15- | 136 | 3-F | H | CHBrF | |
| 15- | 137 | 4-F | H | CHBrF | |
| 15- | 138 | 2-Cl | H | CHBrF | |
| 15- | 139 | 3-Cl | H | CHBrF | |
| 15- | 140 | 4-Cl | H | CHBrF | |
| 15- | 141 | 2-Br | H | CHBrF | |
| 15- | 142 | 3-Br | H | CHBrF | |
| 15- | 143 | 4-Br | H | CHBrF | |
| 15- | 144 | 2-I | H | CHBrF | |
| 15- | 145 | 3-I | H | CHBrF | |
| 15- | 146 | 4-I | H | CHBrF | |
| 15- | 147 | 2-CI | 3-CI | CHBrF | |
| 15- | 148 | 2-CI | 4-CI | CHBrF | |
| 15- | 149 | 2-CI | 5-CI | CHBrF | |
| 15- | 150 | 2-CI | 6-CI | CHBrF | |
| 15- | 151 | 3-CI | 4-CI | CHBrF | |
| 15- | 152 | 3-CI | 5-CI | CHBrF | |
| 15- | 153 | 3-OCF₃ | 4-CI | CCIF₂ | NMR siehe unten |
| 15- | 154 | 3-OCF₃ | 4-CI | CBrF₂ | NMR siehe unten |
| 15- | 155 | 3-CF₃ | 4-CI | CClF₂ | NMR siehe unten |
| 15- | 156 | 3-CF₃ | 4-CI | CBrF₂ | NMR siehe unten |
| 15- | 157 | 3-Br | 5-Br | CClF₂ | NMR siehe unten |
| 15- | 158 | 3-Br | 5-Br | CBrF₂ | NMR siehe unten |
| 15- | 159 | 3-Cl | 5-OCF₃ | CClF₂ | NMR siehe unten |
| 15- | 160 | 3-CI | 4-Br | CClF₂ | NMR siehe unten |
| 15- | 161 | 3-CI | 5-CF₃ | CCIF₂ | NMR siehe unten |
| 15- | 162 | 3-CI | 5-CF₃ | CBrF₂ | NMR siehe unten |
| 15- | 163 | 3-CF₃ | 5-CF₃ | CClF₂ | NMR siehe unten |
| 15- | 164 | 3-CF₃ | 5-CF₃ | CBrF₂ | NMR siehe unten |
| 15- | 165 | 3-F | 5-CF₃ | CClF₂ | NMR siehe unten |
| 15- | 166 | 3-F | 5-CF₃. | CBrF₂ | NMR siehe unten |
| 15- | 167 | 3-CI | 5-CF₃ | CH₂F | NMR siehe unten |
| 15- | 168 | 3-CI | 4-Br | CF₂Br | NMR siehe unten |
| 15- | 169 | 3-CI | 5-OCF₃ | CF₂Br | NMR siehe unten |
| 15- | 170 | 3-CI | 5-CF₃ | CCl₂F | |
| 15- | 171 | 3-CI | 4-CF₃ | CCl₂F | |
| 15- | 172 | 3-CF₃ | 5-CF₃ | CCl₂F | |
| 15- | 173 | 2-F | 4-Br | CClF₂ | |
| 15- | 174 | 2-F | 4-Br | CBrF₂ | |
| 15- | 175 | 2-F | 4-Br | CCl₂F | |
| 15- | 176 | 2-CI | 4-Br | CClF₂ | |
| 15- | 177 | 2-CI | 4-Br | CBrF₂ | |
| 15- | 178 | 2-CI | 4-Br | CCl₂F | |
| 15- | 179 | 3-CI | 4-Br | CClF₂ | |
| 15- | 180 | 3-CI | 4-Br | CBrF₂ | |
| 15- | 181 | 3-CI | 4-Br | CCl₂F | |
| 15- | 182 | 3-CI | 5-Br | CClF₂ | |
| 15- | 183 | 3-CI | 5-Br | CBrF₂ | |
| 15- | 184 | 3-CI | 5-Br | CCl₂F | |
| 15- | 185 | 3-CF₃ | 4-Br | CClF₂ | |
| 15- | 186 | 3-CF₃ | 4-Br | CBrF₂ | |
| 15- | 187 | 3-CF₃ | 4-Br | CCl₂F | |
| 15- | 188 | 3-CI | 4-I | CClF₂ | |
| 15- | 189 | 3-CI | 4-I | CBrF₂ | |
| 15- | 190 | 3-CI | 4-I | CCl₂F | |

**Tabelle 16**

| | | | | | |
|---|---|---|---|---|---|
| 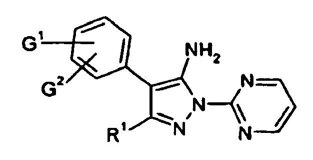 | | | | | |

| Beispielnr. | | G¹ | G² | R¹ | Physikalische Daten |
|---|---|---|---|---|---|
| 16- | 01 | H | H | Me | |
| 16- | 02 | 2-F | H | Me | |
| 16- | 03 | 3-F | H | Me | |
| 16- | 04 | 4-F | H | Me | NMR siehe unten |
| 16- | 05 | 2-CI | H | Me | NMR siehe unten |
| 16- | 06 | 3-CI | H | Me | NMR siehe unten |
| 16- | 07 | 4-CI | H | Me | NMR siehe unten |
| 16- | 08 | 2-Br | H | Me | |
| 16- | 09 | 3-Br | H | Me | |
| 16- | 10 | 4-Br | H | Me | |
| 16- | 11 | 2-I | H | Me | |
| 16- | 12 | 3-I | H | Me | |
| 16- | 13 | 4.I | H | Me | |
| 16- | 14 | 2-CI | 3-CI | Me | |
| 16- | 15 | 2-CI | 4-CI | Me | NMR siehe unten |
| 16- | 16 | 2-CI | 5-CI | Me | |
| 16- | 17 | 2-CI | 6-CI | Me | |
| 16- | 18 | 2-CI | 4-F | Me | logP = 1,71 |
| 16- | 19 | 3-CI | 4-CI | Me | |
| 16- | 20 | 3-CI | 5-CI | Me | |
| 16- | 21 | H | H | Et | |
| 16- | 22 | 2-F | H | Et | |
| 16- | 23 | 3-F | H | Et | |
| 16- | 24 | 4-F | H | Et | |
| 16- | 25 | 2-CI | H | Et | |
| 16- | 26 | 3-CI | H | Et | |
| 16- | 27 | 4-CI | H | Et | |
| 16- | 28 | 2-Br | H | Et | |
| 16- | 29 | 3-Br | H | Et | |
| 16- | 30 | 4-Br | H | Et | 1H(DMSO): 1.08, 2.56ppm, Et |
| 16- | 31 | 2-I | H | Et | |
| 16- | 32 | 3-I | H | Et | |
| 16- | 33 | 4-I | H | Et | |
| 16- | 34 | 2-CI | 3-CI | Et | |
| 16- | 35 | 2-CI | 4-CI | Et | |
| 16- | 36 | 2-CI | 5-CI | Et | |
| 16- | 37 | 2-CI | 6-CI | Et | |
| 16- | 38 | 3-CI | 4-CI | Et | 1H: 1.17, 2.70ppm, Et |
| 16- | 39 | 3-CI | 5-CI | Et | NMR siehe unten |
| 16- | 40 | H | H | nPr | |
| 16- | 41 | 2-F | H | nPr | |
| 16- | 42 | 3-F | H | nPr | |
| 16- | 43 | 4-F | H | nPr | |
| 16- | 44 | 2-CI | H | nPr | |
| 16- | 45 | 3-CI | H | nPr | |
| 16- | 46 | 4-CI | H | nPr | |
| 16- | 47 | 2-Br | H | nPr | |
| 16- | 48 | 3-Br | H | nPr | |
| 16- | 49 | 4-Br | H | nPr | 1H: 2.65, 1.60, 0.87ppm, nPr |
| 16- | 50 | 2-I | H | nPr | |
| 16- | 51 | 3-I | H | nPr | |
| 16- | 52 | 4-I | H | nPr | |
| 16- | 53 | 2-CI | 3-CI | nPr | |
| 16- | 54 | 2-CI | 4-CI | nPr | |
| 16- | 55 | 2-CI | 5-CI | nPr | |
| 16- | 56 | 2-CI | 6-CI | nPr | |
| 16- | 57 | 3-CI | 4-CI | nPr | |
| 16- | 58 | 3-CI | 5-CI | nPr | |
| 16- | 59 | H | H | iPr | |
| 16- | 60 | 2-F | H | iPr | |
| 16- | 61 | 3-F | H | iPr | |
| 16- | 62 | 4-F | H | iPr | |
| 16- | 63 | 2-CI | H | iPr | |
| 16- | 64 | 3-CI | H | iPr | |
| 16- | 65 | 4-CI | H | iPr | |
| 16- | 66 | 2-Br | H | iPr | |
| 16- | 67 | 3-Br | H | iPr | |
| 16- | 68 | 4-Br | H | iPr | 1H(d6DMSO): 2.96, 1.14ppm, iPr |
| 16- | 69 | 2-I | H | iPr | |
| 16- | 70 | 3-I | H | iPr | |
| 16- | 71 | 4-I | H | iPr | |
| 16- | 72 | 2-CI | 3-CI | iPr | |
| 16- | 73 | 2-CI | 4-CI | iPr | |
| 16- | 74 | 2-CI | 5-CI | iPr | |
| 16- | 75 | 2-CI | 6-CI | iPr | |
| 16- | 76 | 3-CI | 4-CI | iPr | |
| 16- | 77 | 3-CI | 5-CI | iPr | |
| 16- | 78 | H | H | nBu | |
| 16- | 79 | 2-F | H | nBu | |
| 16- | 80 | 3-F | H | nBu | |
| 16- | 81 | 4-F | H | nBu | |
| 16- | 82 | 2-CI | H | nBu | |
| 16- | 83 | 3-CI | H | nBu | |
| 16- | 84 | 4-CI | H | nBu | |
| 16- | 85 | 2-Br | H | nBu | |
| 16- | 86 | 3-Br | H | nBu | |
| 16- | 87 | 4-Br | H | nBu | 1H(d6DMSO): 0.82, 1.27, 1.48, 2.53, nBu |
| 16- | 88 | 2-I | H | nBu | |
| 16- | 89 | 3-I | H | nBu | |
| 16- | 90 | 4-I | H | nBu | |
| 16- | 91 | 2-CI | 3-CI | nBu | |
| 16- | 92 | 2-CI | 4-CI | nBu | |
| 16- | 93 | 2-CI | 5-CI | nBu | |
| 16- | 94 | 2-CI | 6-CI | nBu | |
| 16- | 95 | 3-CI | 4-CI | nBu | |
| 16- | 96 | 3-CI | 5-CI | nBu | |
| 16- | 97 | H | H | secBu | |
| 16- | 98 | 2-F | H | secBu | |
| 16- | 99 | 3-F | H | secBu | |
| 16- | 100 | 4-F | H | secBu | |
| 16- | 101 | 2-CI | H | secBu | |
| 16- | 102 | 3-CI | H | secBu | |
| 16- | 103 | 4-CI | H | secBu | |
| 16- | 104 | 2-Br | H | secBu | |
| 16- | 105 | 3-Br | H | secBu | |
| 16- | 106 | 4-Br | H | secBu | |
| 16- | 107 | 2-I | H | secBu | |
| 16- | 108 | 3-I | H | secBu | |
| 16- | 109 | 4-I | H | secBu | |
| 16- | 110 | 2-CI | 3-CI | secBu | |
| 16- | 111 | 2-CI | 4-CI | secBu | |
| 16- | 112 | 2-CI | 5-CI | secBu | |
| 16- | 113 | 2-CI | 6-CI | secBu | |
| 16- | 114 | 3-CI | 4-CI | secBu | |
| 16- | 115 | 3-CI | 5-CI | secBu | |
| 16- | 116 | H | H | iBu | |
| 16- | 117 | 2-F | H | iBu | |
| 16- | 118 | 3-F | H | iBu | |
| 16- | 119 | 4-F | H | iBu | |
| 16- | 120 | 2-CI | H | iBu | |
| 16- | 121 | 3-CI | H | iBu | |
| 16- | 122 | 4-CI | H | iBu | |
| 16- | 123 | 2-Br | H | iBu | |
| 16- | 124 | 3-Br | H | iBu | |
| 16- | 125 | 4-Br | H | iBu | |
| 16- | 126 | 2-I | H | iBu | |
| 16- | 127 | 3-I | H | iBu | |
| 16- | 128 | 4-I | H | iBu | |
| 16- | 129 | 2-CI | 3-CI | iBu | |
| 16- | 130 | 2-CI | 4-CI | iBu | |
| 16- | 131 | 2-CI | 5-CI | iBu | |
| 16- | 132 | 2-CI | 6-CI | iBu | |
| 16- | 133 | 3-CI | 4-CI | iBu | |
| 16- | 134 | 3-CI | 5-CI | iBu | NMR siehe unten |
| 16- | 135 | H | H | tBu | |
| 16- | 136 | 2-F | H | tBu | |
| 16- | 137 | 3-F | H | tBu | |
| 16- | 138 | 4-F | H | tBu | |
| 16- | 139 | 2-CI | H | tBu | |
| 16- | 140 | 3-CI | H | tBu | |
| 16- | 141 | 4-CI | H | tBu | |
| 16- | 142 | 2-Br | H | tBu | |
| 16- | 143 | 3-Br | H | tBu | |
| 16- | 144 | 4-Br | H | tBu | |
| 16- | 145 | 2-I | H | tBu | |
| 16- | 146 | 3-I | H | tBu | |
| 16- | 147 | 4-I | H | tBu | |
| 16- | 148 | 2-CI | 3-CI | tBu | |
| 16- | 149 | 2-CI | 4-CI | tBu | |
| 16- | 150 | 2-CI | 5-CI | tBu | |
| 16- | 151 | 2-CI | 6-CI | tBu | |
| 16- | 152 | 3-CI | 4-CI | tBu | |
| 16- | 153 | 3-CI | 5-CI | tBu | |
| 16- | 153 | H | H | tBuCH₂ | |
| 16- | 154 | 2-F | H | tBuCH₂ | |
| 16- | 155 | 3-F | H | tBuCH₂ | |
| 16- | 156 | 4-F | H | tBuCH₂ | |
| 16- | 157 | 2-CI | H | tBuCH₂ | |
| 16- | 158 | 3-CI | H | tBuCH₂ | |
| 16- | 159 | 4-CI | H | tBuCH₂ | |
| 16- | 160 | 2-Br | H | tBuCH₂ | |
| 16- | 161 | 3-Br | H | tBuCH₂ | ¹H-NMR (CDCl₃): 0.79 (s, 9H), 2.64 ppm (s, 2H), tBuCH₂ |
| 16- | 162 | 4-Br | H | tBuCH₂ | |
| 16- | 163 | 2-I | H | tBuCH₂ | |
| 16- | 164 | 3-I | H | tBuCH₂ | |
| 16- | 165 | 4-I | H | tBuCH₂ | |
| 16- | 166 | 2-CI | 3-CI | tBuCH₂ | |
| 16- | 167 | 2-CI | 4-CI | tBuCH₂ | |
| 16- | 168 | 2-CI | 5-CI | tBuCH₂ | |
| 16- | 169 | 2-CI | 6-CI | tBuCH₂ | |
| 16- | 170 | 3-CI | 4-CI | tBuCH₂ | |
| 16- | 171 | 3-CI | 5-CI | tBuCH₂ | |
| 16- | 172 | 3-CI | 4-CF₃ | Me | ¹H-NMR (d₆-DMSO): 8.83, d, 2H, 7.36, t, 1H, 6.82, br s, 2H |
| 16- | 173 | 3-CI | 5-CF₃ | Me | |
| 16- | 174 | 5-CF₃ | 5-CF₃ | Me | |
| 16- | 175 | 3-CI | 4-CF₃ | Et | |
| 16- | 176 | 3-CI | 5-CF₃ | Et | |
| 16- | 177 | 5-CF₃ | 5-CF₃ | Et | |
| 16- | 178 | 3-CI | 4-CF₃ | iPᵣ | |
| 16- | 179 | 3-CI | 5-CF₃ | iPr | |
| 16- | 180 | 5-CF₃ | 5-CF₃ | iPr | |
| 16- | 181 | 3-CI | 4-CF₃ | nPr | |
| 16- | 182 | 3-CI | 5-CF₃ | nPr | |
| 16- | 183 | 5-CF₃ | 5-CF₃ | nPr | |
| 16- | 184 | 3-CI | 4-CF₃ | tBu | |
| 16- | 185 | 3-CI | 5-CF₃ | tBu | |
| 16- | 186 | 5-CF₃ | 5-CF₃ | tBu | |

**Tabelle 17**

| | | | | | |
|---|---|---|---|---|---|
| 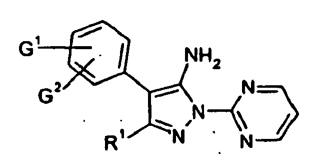 | | | | | |

| Beispielnr. | | G¹ | G² | R¹ | Physikalische Daten |
|---|---|---|---|---|---|
| 17- | 01 | H | H | CH₂OH | |
| 17- | 02 | 2-F | H | CH₂OH | |
| 17- | 03 | 3-F | H | CH₂OH | |
| 17- | 04 | 4-F | H | CH₂OH | |
| 17- | 05 | 2-CI | H | CH₂OH | |
| 17- | 06 | 3-CI | H | CH₂OH | |
| 17- | 07 | 4-CI | H | CH₂OH | |
| 17- | 08 | 2-Br | H | CH₂OH | |
| 17- | 09 | 3-Br | H | CH₂OH | |
| 17- | 10 | 4-Br | H | CH₂OH | 1H: 4.67ppm, CH₂O |
| 17- | 11 | 2-I | H | CH₂OH | |
| 17- | 12 | 3-I | H | CH₂OH | |
| 17- | 13 | 4-I | H | CH₂OH | |
| 17- | 14 | 2-CI | 3-CI | CH₂OH | |
| 17- | 15 | 2-CI | 4-CI | CH₂OH | |
| 17- | 16 | 2-CI | 5-CI | CH₂OH | |
| 17- | 17 | 2-CI | 6-CI | CH₂OH | |
| 17- | 18 | 3-CI | 4-CI | CH₂OH | |
| 17- | 19 | 3-CI | 5-CI | CH₂OH | |
| 17- | 20 | H | H | CH₂OMe | |
| 17- | 21 | 2-F | H | CH₂OMe | |
| 17- | 22 | 3-F | H | CH₂OMe | |
| 17- | 23 | 4-F | H | CH₂OMe | |
| 17- | 24 | 2-CI | H | CH₂OMe | |
| 17- | 25 | 3-CI | H | CH₂OMe | |
| 17- | 26 | 4-CI | H | CH₂OMe | |
| 17- | 27 | 2-Br | H | CH₂OMe | |
| 17- | 28 | 3-Br | H | CH₂OMe | |
| 17- | 29 | 4-Br | H | CH₂OMe | |
| 17- | 30 | 2-I | H | CH₂OMe | |
| 17- | 31 | 3-I | H | CH₂OMe | |
| 17- | 32 | 4-I | H | CH₂OMe | |
| 17- | 33 | 2-CI | 3-CI | CH₂OMe | |
| 17- | 34 | 2-CI | 4-CI | CH₂OMe | |
| 17- | 35 | 2-CI | 5-CI | CH₂OMe | |
| 17- | 36 | 2-CI | 6-CI | CH₂OMe | |
| 17- | 37 | 3-CI | 4-CI | CH₂OMe | 1H: 4.30ppm, CH₂O |
| 17- | 38 | 3-CI | 5-CI | CH₂OMe | |
| 17- | 39 | H | H | CH₂OEt | |
| 17- | 40 | 2-F | H | CH₂OEt | |
| 17- | 41 | 3-F | H | CH₂OEt | |
| 17- | 42 | 4-F | H | CH₂OEt | |
| 17- | 43 | 2-CI | H | CH₂OEt | |
| 17- | 44 | 3-CI | H | CH₂OEt | |
| 17- | 45 | 4-CI | H | CH₂OEt | |
| 17- | 46 | 2-Br | H | CH₂OEt | |
| 17- | 47 | 3-Br | H | CH₂OEt | |
| 17- | 48 | 4-Br | H | CH₂OEt | 1H: 4.49ppm, OCH₂ |
| 17- | 49 | 2-I | H | CH₂OEt | |
| 17- | 50 | 3-I | H | CH₂OEt | |
| 17- | 51 | 4-I | H | CH₂OEt | |
| 17- | 52 | 2-CI | 3-CI | CH₂OEt | |
| 17- | 53 | 2-CI | 4-CI | CH₂OEt | |
| 17- | 54 | 2-CI | 5-CI | CH₂OEt | |
| 17- | 55 | 2-CI | 6-CI | CH₂OEt | |
| 17- | 56 | 3-CI | 4-CI | CH₂OEt | |
| 17- | 57 | 3-CI | 5-CI | CH₂OEt | |
| 17- | 58 | H | H | CH₂SMe | |
| 17- | 59 | 2-F | H | CH₂SMe | |
| 17- | 60 | 3-F | H | CH₂SMe | |
| 17- | 61 | 4-F | H | CH₂SMe | |
| 17- | 62 | 2-CI | H | CH₂SMe | |
| 17- | 63 | 3-CI | H | CH₂SMe | |
| 17- | 64 | 4-CI | H | CH₂SMe | |
| 17- | 65 | 2-Br | H | CH₂SMe | |
| 17- | 66 | 3-Br | H | CH₂SMe | |
| 17- | 67 | 4-Br | H | CH₂SMe | 1H: 3.68ppm, CH₂S |
| 17- | 68 | 2-I | H | CH₂SMe | |
| 17- | 69 | 3-I | H | CH₂SMe | |
| 17- | 70 | 4-I | H | CH₂SMe | |
| 17- | 71 | 2-CI | 3-CI | CH₂SMe | |
| 17- | 72 | 2-CI | 4-CI | CH₂SMe | |
| 17- | 73 | 2-CI | 5-CI | CH₂SMe | |
| 17- | 74 | 2-CI | 6-CI | CH₂SMe | |
| 17- | 75 | 3-CI | 4-CI | CH₂SMe | |
| 17- | 76 | 3-CI | 5-CI | CH₂SMe | |
| 17- | 77 | H | H | CH₂SOMe | |
| 17- | 78 | 2-F | H | CH₂SOMe | |
| 17- | 79 | 3-F | H | CH₂SOMe | |
| 17- | 80 | 4-F | H | CH₂SOMe | |
| 17- | 81 | 2-CI | H | CH₂SOMe | |
| 17- | 82 | 3-CI | H | CH₂SOMe | |
| 17- | 83 | 4-CI | H | CH₂SOMe | |
| 17- | 84 | 2-Br | H | CH₂SOMe | |
| 17- | 85 | 3-Br | H | CH₂SOMe | |
| 17- | 86 | 4-Br | H | CH₂SOMe | |
| 17- | 87 | 2-I | H | CH₂SOMe | |
| 17- | 88 | 3-I | H | CH₂SOMe | |
| 17- | 89 | 4-I | H | CH₂SOMe | |
| 17- | 90 | 2-CI | 3-CI | CH₂SOMe | |
| 17- | 91 | 2-CI | 4-CI | CH₂SOMe | |
| 17- | 92 | 2-CI | 5-CI | CH₂SOMe | |
| 17- | 93 | 2-CI | 6-CI | CH₂SOMe | |
| 17- | 94 | 3-CI | 4-CI | CH₂SOMe | |
| 17- | 95 | 3-CI | 5-CI | CH₂SOMe | |
| 17- | 96 | H | H | CH₂SO₂Me | |
| 17- | 97 | 2-F | H | CH₂SO₂Me | |
| 17- | 98 | 3-F | H | CH₂SO₂Me | |
| 17- | 99 | 4-F | H | CH₂SO₂Me | |
| 17- | 100 | 2-CI | H | CH₂SO₂Me | |
| 17- | 101 | 3-CI | H | CH₂SO₂Me | |
| 17- | 102 | 4-CI | H | CH₂SO₂Me | |
| 17- | 103 | 2-Br | H | CH₂SO₂Me | |
| 17- | 104 | 3-Br | H | CH₂SO₂Me | |
| 17- | 105 | 4-Br | H | CH₂SO₂Me | |
| 17- | 106 | 2-I | H | CH₂SO₂Me | |
| 17- | 107 | 3-I | H | CH₂SO₂Me | |
| 17- | 108 | 4-I | H | CH₂SO₂Me | |
| 17- | 109 | 2-CI | 3-CI | CH₂SO₂Me | |
| 17- | 110 | 2-CI | 4-CI | CH₂SO₂Me | |
| 17- | 111 | 2-CI | 5-CI | CH₂SO₂Me | |
| 17- | 112 | 2-CI | 6-CI | CH₂SO₂Me | |
| 17- | 113 | 3-CI | 4-CI | CH₂SO₂Me | |
| 17- | 114 | 3-CI | 5-CI | CH₂SO₂Me | |
| 17- | 115 | H | H | CH(OMe)Me | |
| 17- | 116 | 2-F | H | CH(OMe)Me | |
| 17- | 117 | 3-F | H | CH(OMe)Me | |
| 17- | 118 | 4-F | H | CH(OMe)Me | |
| 17- | 119 | 2-CI | H | CH(OMe)Me | |
| 17- | 120 | 3-CI | H | CH(OMe)Me | |
| 17- | 121 | 4-CI | H | CH(OMe)Me | |
| 17- | 122 | 2-Br | H | CH(OMe)Me | |
| 17- | 123 | 3-Br | H | CH(OMe)Me | |
| 17- | 124 | 4-Br | H | CH(OMe)Me | 1H(DMSO): 4.36ppm, CHOMe |
| 17- | 125 | 2-I | H | CH(OMe)Me | |
| 17- | 126 | 3-I | H | CH(OMe)Me | |
| 17- | 127 | 4-I | H | CH(OMe)Me | |
| 17- | 128 | 2-Cl | 3-Cl | CH(OMe)Me | |
| 17- | 129 | 2-Cl | 4-Cl | CH(OMe)Me | |
| 17- | 130 | 2-Cl | 5-Cl | CH(OMe)Me | |
| 17- | 131 | 2-Cl | 6-Cl | CH(OMe)Me | |
| 17- | 132 | 3-Cl | 4-Cl | CH(OMe)Me | |
| 17- | 133 | 3-Cl | 5-Cl | CH(OMe)Me | |
| 17- | 153 | H | H | CH₂CH₂COOMe | |
| 17- | 154 | 2-F | H | CH₂CH₂COOMe | |
| 17- | 155 | 3-F | H | CH₂CH₂COOMe | |
| 17- | 156 | 4-F | H | CH₂CH₂COOMe | |
| 17- | 157 | 2-Cl | H | CH₂CH₂COOMe | |
| 17- | 158 | 3-Cl | H | CH₂CH₂COOMe | |
| 17- | 159 | 4-Cl | H | CN₂CH₂COOMe | |
| 17- | 160 | 2-Br | H | CH₂CH₂COOMe | |
| 17- | 161 | 3-Br | H | CH₂CH₂COOMe | |
| 17- | 162 | 4-Br | H | CH₂CH₂COOMe | 1H: 2.69, 3.00ppm, CH₂CH₂ |
| 17- | 163 | 2-I | H | CH₂CH₂COOMe | |
| 17- | 164 | 3-I | H | CH₂CH₂COOMe | |
| 17- | 165 | 4-I | H | CH₂CH₂COOMe | |
| 17- | 166 | 2-Cl | 3-Cl | CH₂CH₂COOMe | |
| 17- | 167 | 2-Cl | 4-Cl | CH₂CH₂COOMe | |
| 17- | 168 | 2-Cl | 5-Cl | CH₂CH₂COOMe | |
| 17- | 169 | 2-Cl | 6-Cl | CH₂CH₂COOMe | |
| 17- | 170 | 3-Cl | 4-Cl | CH₂CH₂COOMe | |
| 17- | 171 | 3-Cl | 5-Cl | CH₂CH₂COOMe | |
| 17- | 172 | 3-Cl | 4-CF₃ | CH₂OMe | ¹H-NMR (d₆-DMSO): 8.87, d, 2H, 7.41, t, 1H, 4.31, s, (MeO) |

**Tabelle 18**

| | | | | | |
|---|---|---|---|---|---|
| 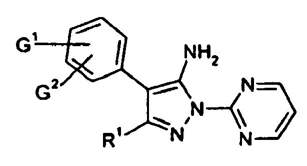 | | | | | |

| Beispielnr. | | G¹ | G² | R¹ | Physikalische Daten |
|---|---|---|---|---|---|
| 18- | 01 | H | H | CHFCH₃ | |
| 18- | 02 | 2-F | H | CHFCH₃ | |
| 18- | 03 | 3-F | H | CHFCH₃ | |
| 18- | 04 | 4-F | H | CHFCH₃ | |
| 18- | 05 | 2-Cl | H | CHFCH₃ | |
| 18- | 06 | 3-Cl | H | CHFCH₃ | |
| 18- | 07 | 4-Cl | H | CHFCF₃ | NMR siehe unten |
| 18- | 08 | 2-Br | H | CHFCH₃ | |
| 18- | 09 | 3-Br | H | CHFCH₃ | |
| 18- | 10 | 4-Br | H | CHFCH₃ | 19F: -164.0ppm, CHF |
| 18- | 11 | 2-I | H | CHFCH₃ | |
| 18- | 12 | 3-I | H | CHFCH₃ | |
| 18- | 13 | 4-I | H | CHFCH₃ | |
| 18- | 14 | 2-Cl | 3-Cl | CHFCH₃ | |
| 18- | 15 | 2-Cl | 4-Cl | CHFCH₃ | |
| 18- | 16 | 2-Cl | 5-Cl | CHFCF₃ | |
| 18- | 17 | 2-Cl | 6-Cl | CHFCH₃ | |
| 18- | 18 | 3-Cl | 4-Cl | CHFCH₃ | |
| 18- | 19 | 3-Cl | 5-Cl | CHFCH₃ | NMR siehe unten |
| 18- | 20 | H | H | CF₂CH₃ | |
| 18- | 21 | 2-F | H | CF₂CH₃ | |
| 18- | 22 | 3-F | H | CF₂CH₃ | |
| 18- | 23 | 4-F | H | CF₂CH₃ | |
| 18- | 24 | 2-Cl | H | CF₂CH₃ | |
| 18- | 25 | 3-Cl | H | CF₂CH₃ | |
| 18- | 26 | 4-Cl | H | CF₂CH₃ | NMR siehe unten |
| 18- | 27 | 2-Br | H | CF₂CH₃ | |
| 18- | 28 | 3-Br | H | CF₂CH₃ | |
| 18- | 29 | 4-Br | H | CF₂CH₃ | |
| 18- | 30 | 2-I | H | CF₂CH₃ | |
| 18- | 31 | 3-I | H | CF₂CH₃ | |
| 18- | 32 | 4-I | H | CF₂CH₃ | NMR siehe unten |
| 18- | 33 | 2-Cl | 3-Cl | CF₂CH₃ | |
| 18- | 34 | 2-Cl | 4-Cl | CF₂CH₃ | NMR siehe unten |
| 18- | 35 | 2-Cl | 5-Cl | CF₂CH₃ | |
| 18- | 36 | 2-Cl | 6-Cl | CF₂CH₃ | |
| 18- | 37 | 3-Cl | 4-Cl | CF₂CH₃ | |
| 18- | 38 | 3-Cl | 5-Cl | CF₂CH₃ | NMR siehe unten |
| 18- | 39 | H | H | CF₂CF₃ | |
| 18- | 40 | 2-F | H | CF₂CF₃ | |
| 18- | 41 | 3-F | H | CF₂CF₃ | |
| 18- | 42 | 4-F | H | CF₂CF₃ | |
| 18- | 43 | 2-Cl | H | CF₂CF₃ | |
| 18- | 44 | 3-Cl | H | CF₂CF₃ | |
| 18- | 45 | 4-Cl | H | CF₂CF₃ | NMR siehe unten |
| 18- | 46 | 2-Br | H | CF₂CF₃ | |
| 18- | 47 | 3-Br | H | CF₂CF₃ | |
| 18- | 48 | 4-Br | H | CF₂CF₃ | NMR siehe unten |
| 18- | 49 | 2-I | H | CF₂CF₃ | |
| 18- | 50 | 3-I | H | CF₂CF₃ | |
| 18- | 51 | 4-I | H | CF₂CF₃ | NMR siehe unten |
| 18- | 52 | 2-Cl | 3-Cl | CF₂CF₃ | |
| 18- | 53 | 2-Cl | 4-Cl | CF₂CF₃ | |
| 18- | 54 | 2-Cl | 5-Cl | CF₂CF₃ | |
| 18- | 55 | 2-Cl | 6-Cl | CF₂CF₃ | |
| 18- | 56 | 3-Cl | 4-Cl | CF₂CF₃ | |
| 18- | 57 | 3-Cl | 5-Cl | CF₂CF₃ | NMR siehe unten |
| 18- | 58 | H | H | CHFCF₃ | |
| 18- | 59 | 2-F | H | CHFCF₃ | |
| 18- | 60 | 3-F | H | CHFCF₃ | |
| 18- | 61 | 4-F | H | CHFCF₃ | |
| 18- | 62 | 2-Cl | H | CHFCF₃ | |
| 18- | 63 | 3-Cl | H | CHFCF₃ | |
| 18- | 64 | 4-Cl | H | CHFCF₃ | |
| 18- | 65 | 2-Br | H | CHFCF₃ | |
| 18- | 66 | 3-Br | H | CHFCF₃ | |
| 18- | 67 | 4-Br | H | CHFCF₃ | |
| 18- | 68 | 2-I | H | CHFCF₃ | |
| 18- | 69 | 3-I | H | CHFCF₃ | |
| 18- | 70 | 4-I | H | CHFCF₃ | |
| 18- | 71 | 2-Cl | 3-Cl | CHFCF₃ | |
| 18- | 72 | 2-Cl | 4-Cl | CHFCF₃ | |
| 18- | 73 | 2-Cl | 5-Cl | CHFCF₃ | |
| 18- | 74 | 2-Cl | 6-Cl | CHFCF₃ | |
| 18- | 75 | 3-Cl | 4-Cl | CHFCF₃ | |
| 18- | 76 | 3-Cl | 5-Cl | CHFCF₃ | |
| 18- | 77 | H | H | CF₂CF₂H | |
| 18- | 78 | 2-F | H | CF₂CF₂H | |
| 18- | 79 | 3-F | H | CF₂CF₂H | |
| 18- | 80 | 4-F | H | CF₂CF₂H | |
| 18- | 81 | 2-Cl | H | CF₂CF₂H | |
| 18- | 82 | 3-Cl | H | CF₂CF₂H | |
| 18- | 83 | 4-Cl | H | CF₂CF₂H | |
| 18- | 84 | 2-Br | H | CF₂CF₂H | |
| 18- | 85 | 3-Br | H | CF₂CF₂H | |
| 18- | 86 | 4-Br | H | CF₂CF₂H | |
| 18- | 87 | 2-I | H | CF₂CF₂H | |
| 18- | 88 | 3-I | H | CF₂CF₂H | |
| 18- | 89 | 4-I | H | CF₂CF₂H | |
| 18- | 90 | 2-Cl | 3-Cl | CF₂CF₂H | |
| 18- | 91 | 2-Cl | 4-Cl | CF₂CF₂H | |
| 18- | 92 | 2-Cl | S-Cl | CF₂CF₂H | |
| 18- | 93 | 2-Cl | 6-Cl | CF₂CF₂H | |
| 18- | 94 | 3-Cl | 4-Cl | CF₂CF₂H | |
| 18- | 95 | 3-Cl | 5-Cl | CF₂CF₂H | |
| 18- | 96 | H | H | CF₂CF₂CF₃ | |
| 18- | 97 | 2-F | H | CF₂CF₂CF₃ | |
| 18- | 98 | 3-F | H | CF₂CF₂CF₃ | |
| 18- | 99 | 4-F | H | CF₂CF₂CF₃ | |
| 18- | 100 | 2-Cl | H | CF₂CF₂CF₃ | |
| 18- | 101 | 3-Cl | H | CF₂CF₂CF₃ | |
| 18- | 102 | 4-Cl | H | CF₂CF₂CF₃ | |
| 18- | 103 | 2-Br | H | CF₂CF₂CF₃ | |
| 18- | 104 | 3-Br | H | CF₂CF₂CF₃ | |
| 18- | 105 | 4-Br | H | CF₂CF₂CF₃ | |
| 18- | 106 | 2-I | H | CF₂CF₂CF₃ | |
| 18- | 107 | 3-I | H | CF₂CF₂CF₃ | |
| 18- | 108 | 4-I | H | CF₂CF₂CF₃ | |
| 18- | 109 | 2-Cl | 3-Cl | CF₂CF₂CF₃ | |
| 18- | 110 | 2-Cl | 4-Cl | CF₂CF₂CF₃ | |
| 18- | 111 | 2-Cl | 5-Cl | CF₂CF₂CF₃ | |
| 18- | 112 | 2-Cl | 6-Cl | CF₂CF₂CF₃ | |
| 18- | 113 | 3-Cl | 4-Cl | CF₂CF₂CF₃ | |
| 18- | 114 | 3-Cl | 5-Cl | CF₂CF₂CF₃ | |
| 18- | 115 | H | H | CF(CH₃)₂ | |
| 18- | 116 | 2-F | H | CF(CH₃)₂ | |
| 18- | 117 | 3-F | H | CF(CH₃)₂ | |
| 18- | 118 | 4-F | H | CF(CH₃)₂ | |
| 18- | 119 | 2-Cl | H | CF(CH₃)₂ | |
| 18- | 120 | 3-Cl | H | CF(CH₃)₂ | |
| 18- | 121 | 4-Cl | H | CF(CH₃)₂ | |
| 18- | 122 | 2-Br | H | CF(CH₃)₂ | |
| 18- | 123 | 3-Br | H | CF(CH₃)₂ | |
| 18- | 124 | 4-Br | H | CF(CH₃)₂ | 1H: 1.61 ppm, CH₃ |
| 18- | 125 | 2-I | H | CF(CH₃)₂ | |
| 18- | 126 | 3-I | H | CF(CH₃)₂ | |
| 18- | 127 | 4-I | H | CF(CH₃)₂ | |
| 18- | 128 | 2-Cl | 3-Cl | CF(CH₃)₂ | |
| 18- | 129 | 2-Cl | 4-Cl | CF(CH₃)₂ | |
| 18- | 130 | 2-Cl | 5-Cl | CF(CH₃)₂ | |
| 18- | 131 | 2-Cl | 6-Cl | CF(CH₃)₂ | |
| 18- | 132 | 3-Cl | 4-Cl | CF(CH₃)₂ | |
| 18- | 133 | 3-Cl | 5-Cl | CF(CH₃)₂ | |
| 18- | 134 | H | H | CH₂CH₂CF₃ | |
| 18- | 135 | 2-F | H | CH₂CH₂CF₃ | |
| 18- | 136 | 3-F | H | CH₂CH₂CF₃ | |
| 18- | 137 | 4-F | H | CH₂CH₂CF₃ | |
| 18- | 138 | 2-Cl | H | CH₂CH₂CF₃ | |
| 18- | 139 | 3-Cl | H | CH₂CH₂CF₃ | |
| 18- | 140 | 4-Cl | H | CH₂CH₂CF₃ | |
| 18- | 141 | 2-Br | H | CH₂CH₂CF₃ | |
| 18- | 142 | 3-Br | H | CH₂CH₂CF₃ | |
| 18- | 143 | 4-Br | H | CH₂CH₂CF₃ | 1H(d6DMSO): 2.79, 2.59; CH₂CH₂ |
| 18- | 144 | 2-I | H | CH₂CH₂CF₃ | |
| 18- | 145 | 3-I | H | CH₂CH₂CF₃ | |
| 18- | 146 | 4-I | H | CH₂CH₂CF₃ | |
| 18- | 147 | 2-Cl | 3-Cl | CH₂CH₂CF₃ | |
| 18- | 148 | 2-Cl | 4-Cl | CH₂CH₂CF₃ | |
| 18- | 149 | 2-Cl | 5-Cl | CH₂CH₂CF₃ | |
| 18- | 150 | 2-Cl | 6-Cl | CH₂CH₂CF₃ | |
| 18- | 151 | 3-Cl | 4-Cl | CH₂CH₂CF₃ | |
| 18- | 152 | 3-Cl | 5-Cl | CH₂CH₂CF₃ | |
| 18- | 153 | H | H | CH(CH₃)CF₃ | |
| 18- | 154 | 2-F | H | CH(CH₃)CF₃ | |
| 18- | 155 | 3-F | H | CH(CH₃)CF₃ | |
| 18- | 156 | 4-F | H | CH(CH₃)CF₃ | |
| 18- | 157 | 2-Cl | H | CH(CH₃)CF₃ | |
| 18- | 158 | 3-Cl | H | CH(CH₃)CF₃ | |
| 18- | 159 | 4-Cl | H | CH(CH₃)CF₃ | |
| 18- | 160 | 2-Br | H | CH(CH₃)CF₃ | |
| 18- | 161 | 3-Br | H | CH(CH₃)CF₃ | |
| 18- | 162 | 4-Br | H | CH(CH₃)CF₃ | |
| 18- | 163 | 2-I | H | CH(CH₃)CF₃ | |
| 18- | 164 | 3-I | H | CH(CH₃)CF₃ | |
| 18- | 165 | 4-I | H | CH(CH₃)CF₃ | |
| 18- | 166 | 2-Cl | 3-Cl | CH(CH₃)CF₃ | |
| 18- | 167 | 2-Cl | 4-Cl | CH(CH₃)CF₃ | |
| 18- | 168 | 2-Cl | 5-Cl | CH(CH₃)CF₃ | |
| 18- | 169 | 2-Cl | 6-Cl | CH(CH₃)CF₃ | |
| 18- | 170 | 3-Cl | 4-Cl | CH(CH₃)CF₃ | |
| 18- | 171 | 3-Cl | 5-Cl | CH(CH₃)CF₃ | |
| 18- | 172 | H | H | CHFC₂H₅ | |
| 18- | 173 | 2-F | H | CHFC₂H₅ | |
| 18- | 174 | 3-F | H | CHFC₂H₅ | |
| 18- | 175 | 4-F | H | CHFC₂H₅ | |
| 18- | 176 | 2-Cl | H | CHFC₂H₅ | |
| 18- | 177 | 3-Cl | H | CHFC₂H₅ | |
| 18- | 178 | 4-Cl | H | CHFC₂H₅ | |
| 18- | 179 | 2-Br | H | CHFC₂H₅ | |
| 18- | 180 | 3-Br | H | CHFC₂H₅ | |
| 18- | 181 | 4-Br | H | CHFC₂H₅ | 19F: -171,6ppm, CHF |
| 18- | 182 | 2-I | H | CHFC₂H₅ | |
| 18- | 183 | 3-I | H | CHFC₂H₅ | |
| 18- | 184 | 4-I | H | CHFC₂H₅ | |
| 18- | 185 | 2-Cl | 3-Cl | CHFC₂H₅ | |
| 18- | 186 | 2-Cl | 4-Cl | CHFC₂H₅ | |
| 18- | 187 | 2-Cl | 5-Cl | CHFC₂H₅ | |
| 18- | 188 | 2-Cl | 6-Cl | CHFC₂H₅ | |
| 18- | 189 | 3-Cl | 4-Cl | CHFC₂H₅ | |
| 18- | 190 | 3-Cl | 5-Cl | CHFC₂H₅ | NMR siehe unten |
| 18- | 191 | H | H | CHFnPr | |
| 18- | 192 | 2-F | H | CHFnPr | |
| 18- | 193 | 3-F | H | CHFnPr | |
| 18- | 194 | 4-F | H | CHFnPr | |
| 18- | 195 | 2-Cl | H | CHFnPr | |
| 18- | 196 | 3-Cl | H | CHFnPr | |
| 18- | 197 | 4-Cl | H | CHFnPr | |
| 18- | 198 | 2-Br | H | CHFnPr | |
| 18- | 199 | 3-Br | H | CHFnPr | |
| 18- | 200 | 4-Br | H | CHFnPr | 1H: 5.54ppm, CHF |
| 18- | 201 | 2-I | H | CHFnPr | |
| 18- | 202 | 3-I | H | CHFnPr | |
| 18- | 203 | 4-I | H | CHFnPr | |
| 18- | 204 | 2-Cl | 3-Cl | CHFnPr | |
| 18- | 205 | 2-Cl | 4-Cl | CHFnPr | |
| 18- | 206 | 2-Cl | 5-Cl | CHFnPr | |
| 18- | 207 | 2-Cl | 6-Cl | CHFnPr | |
| 18- | 208 | 3-Cl | 4-Cl | CHFnPr | |
| 18- | 209 | 3-Cl | 5-Cl | CHFnPr | NMR siehe unten |
| 18- | 210 | H | H | CH₂CH(CF₃)CH₃ | |
| 18- | 211 | 2-F | H | CH₂CH(CF₃)CH₃ | |
| 18- | 212 | 3-F | H | CH₂CH(CF₃)CH₃ | |
| 18- | 213 | 4-F | H | CH₂CH(CF₃)CH₃ | |
| 18- | 214 | 2-Cl | H | CH₂CH(CF₃)CH₃ | |
| 18- | 215 | 3-Cl | H | CH₂CH(CF₃)CH₃ | |
| 18- | 216 | 4-Cl | H | CH₂CH(CF₃)CH₃ | |
| 18- | 217 | 2-Br | H | CH₂CH(CF₃)CH₃ | |
| 18- | 218 | 3-Br | H | CH₂CH(CF₃)CH₃ | |
| 18- | 219 | 4-Br | H | CH₂CH(CF₃)CH₃ | 1H: 1.01 ppm,d, CH₃ |
| 18- | 220 | 2-I | H | CH₂CH(CF₃)CH₃ | |
| 18- | 221 | 3-I | H | CH₂CH(CF₃)CH₃ | |
| 18- | 222 | 4-I | H | CH₂CH(CF₃)CH₃ | |
| 18- | 223 | 2-Cl | 3-Cl | CH₂CH(CF₃)CH₃ | |
| 18- | 224 | 2-Cl | 4-Cl | CH₂CH(CF₃)CH₃ | |
| 18- | 225 | 2-Cl | 5-Cl | CH₂CH(CF₃)CH₃ | |
| 18- | 226 | 2-Cl | 6-Cl | CH₂CH(CF₃)CH₃ | |
| 18- | 227 | 3-Cl | 4-Cl | CH₂CH(CF₃)CH₃ | |
| 18- | 228 | 3-Cl | 5-Cl | CH₂CH(CF₃)CH₃ | |
| 18- | 229 | 3-Cl | 5-Cl | CF₂CF₂OMe | NMR siehe unten |
| 18- | 230 | 3-Br | 5-Br | CF₂CH₃ | NMR siehe unten |
| 18- | 231 | 3-CF₃ | 5-CF₃ | CHFCH₃ | NMR siehe unten |
| 18- | 232 | 3-Cl | 5-CF₃ | CHFCH₃ | NMR siehe unten |
| 18- | 233 | 3-CF₃ | 5-CF₃ | CF₂CH₃ | NMR siehe unten |
| 18- | 234 | 3-CF₃ | 5-CF₃ | CF₂CF₃ | NMR siehe unten |
| 18- | 235 | 3-Cl | 5-CF₃ | CF₂CH₃ | NMR siehe unten |
| 18- | 236 | 3-Cl | 5-CF₃ | CF₂CF₃ | NMR siehe unten |
| 18- | 237 | 3-F | 5-CF₃ | CF₂CF₃ | NMR siehe unten |
| 18- | 238 | 3-CF₃ | 4-Cl | CF₂CH₂Br | ¹H-NMR (CD₃CN): 8.82 (d, 2H), 7.36 (t, 1H), 4.08 ppm (t, 2H) |
| 18- | 239 | 3-Cl | 5-CF₃ | (S)-CHFCH₃ | ¹H-NMR (d₆-DMSO): 8.89 (d, 2H), 7.44 ppm (t, 1H) |
| 18- | 240 | 3-F | 5-CF₃ | CF₂CH₃ | NMR siehe unten |
| 18- | 241 | 3-Cl | 5-OCF₃ | CF₂CF₃ | NMR siehe unten |
| 18- | 242 | 3-Br | 5-Br | CF₂CF₃ | NMR siehe unten |
| 18- | 243 | 3-Br | 5-Br | CHFCH₃ | NMR siehe unten |
| 18- | 244 | 3-CF₃ | 4-Cl | CF₂CF₃ | NMR siehe unten |
| 18- | 245 | 3-CF₃ | 4-Cl | CHFCH₃ | NMR siehe unten |
| 18- | 246 | 3-Cl | 4-Br | CF₂CF₃ | NMR siehe unten |
| 18- | 247 | 3-CF₃ | 4-Cl | CF₂CH₃ | |
| 18- | 248 | 2-F | 4-Br | CHFCH₃ | |
| 18- | 249 | 2-F | 4-Br | CF₂CF₃ | |
| 18- | 250 | 2-F | 4-Br | CF₂CH₃ | |
| 18- | 251 | 2-Cl | 4-Br | CHFCH₃ | |
| 18- | 252 | 2-Cl | 4-Br | CF₂CF₃ | |
| 18- | 253 | 2-Cl | 4-Br | CF₂CH₃ | |
| 18- | 254 | 3-Cl | 5-Br | CHFCH₃ | |
| 18- | 255 | 3-Cl | 5-Br | CF₂CF₃ | |
| 18- | 256 | 3-Cl | 5-Br | CF₂CH₃ | |
| 18- | 257 | 3-Cl | 4-Br | CHFCH₃ | |
| 18- | 258 | 3-Cl | 4-Br | CF₂CF₃ | |
| 18- | 259 | 2-Cl | 4-Br | CF₂CH₃ | |
| 18- | 260 | 3-CF₃ | 4-Br | CHFCH₃ | |
| 18- | 261 | 3-CF₃ | 4-Br | CF₂CF₃ | |
| 18- | 262 | 3-CF₃ | 4-Br | CF₂CH₃ | |
| 18- | 263 | 3-Cl | 4-I | CHFCH₃ | |
| 18- | 264 | 3-Cl | 4-I | CF₂CF₃ | |
| 18- | 265 | 3-Cl | 4-1 | CF₂CH₃ | |

**Tabelle 19**

| | | | | | |
|---|---|---|---|---|---|
| 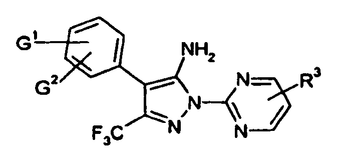 | | | | | |

| Beispielnr. | | R³ | G¹ | G² | Physikalische Daten |
|---|---|---|---|---|---|
| 19- | 01 | 4-Me | H | H | |
| 19- | 02 | 4-Me | 2-F | H | |
| 19- | 03 | 4-Me | 3-F | H | |
| 19- | 04 | 4-Me | 4-F | H | |
| 19- | 05 | 4-Me | 2-Cl | H | |
| 19- | 06 | 4-Me | 3-Cl | H | |
| 19- | 07 | 4-Me | 4-Cl | H | ¹H-NMR (d₆ DMSO): 8.75 ppm, (d, 1H) |
| 19- | 08 | 4-Me | 2-Br | H | |
| 19- | 09 | 4-Me | 3-Br | H | lgP = 3,84 |
| 19- | 10 | 4-Me | 4-Br | H | IgP = 3,37 |
| 19- | 11 | 4-Me | 2-I | H | |
| 19- | 12 | 4-Me | 3-I | H | |
| 19- | 13 | 4-Me | 4-I | H | |
| 19- | 14 | 4-Me | 2-Cl | 3-Cl | |
| 19- | 15 | 4-Me | 2-Cl | 4-Cl | |
| 19- | 16 | 4-Me | 2-Cl | 5-Cl | |
| 19- | 17 | 4-Me | 2-Cl | 6-Cl | |
| 19- | 18 | 4-Me | 3-Cl | 4-Cl | IgP = 3,73 |
| 19- | 19 | 4-Me | 3-Cl | 5-Cl | |
| 19- | 20 | 5-Me | H | H | |
| 19- | 21 | 5-Me | 2-F | H | |
| 19- | 22 | 5-Me | 3-F | H | |
| 19- | 23 | 5-Me | 4-F | H | |
| 19- | 24 | 5-Me | 2-Cl | H | |
| 19- | 25 | 5-Me | 3-Cl | H | |
| 19- | 26 | 5-Me | 4-Cl | H | NMR siehe unten |
| 19- | 27 | 5-Me | 2-Br | H | |
| 19- | 28 | 5-Me | 3-Br | H | |
| 19- | 29 | 5-Me | 4-Br | H | |
| 19- | 30 | 5-Me | 2-I | H | |
| 19- | 31 | 5-Me | 3-I | H | |
| 19- | 32 | 5-Me | 4-I | H | |
| 19- | 33 | 5-Me | 2-Cl | 3-Cl | |
| 19- | 34 | 5-Me | 2-Cl | 4-Cl | |
| 19- | 35 | 5-Me | 2-Cl | 5-Cl | |
| 19- | 36 | 5-Me | 2-Cl | 6-Cl | |
| 19- | 37 | 5-Me | 3-Cl | 4-Cl | NMR siehe unten |
| 19- | 38 | 5-Me | 3-Cl | 5-Cl | |
| 19- | 39 | 5-F | H | H | |
| 19- | 40 | 5-F | 2-F | H | |
| 19- | 41 | 5-F | 3-F | H | |
| 19- | 42 | 5-F | 4-F | H | |
| 19- | 43 | 5-F | 2-Cl | H | |
| 19- | 44 | 5-F | 3-Cl | H | |
| 19- | 45 | 5-F | 4-Cl | H | NMR siehe unten |
| 19- | 46 | 5-F | 2-Br | H | |
| 19- | 47 | 5-F | 3-Br | H | |
| 19- | 48 | 5-F | 4-Br | H | IgP = 3,19 |
| 19- | 49 | 5-F | 2-I | H | |
| 19- | 50 | 5-F | 3-I | H | |
| 19- | 51 | 5-F | 4-I | H | |
| 19- | 52 | 5-F | 2-Cl | 3-Cl | |
| 19- | 53 | 5-F | 2-Cl | 4-Cl | |
| 19- | 54 | 5-F | 2-Cl | 5-Cl | |
| 19- | 55 | 5-F | 2-Cl | 6-Cl | |
| 19- | 56 | 5-F | 3-Cl | 4-Cl | NMR siehe unten |
| 19- | 57 | 5-F | 3-Cl | 5-Cl | IgP = 3,63 |
| 19- | 58 | 5-Cl | H | H | |
| 19- | 59 | 5-Cl | 2-F | H | |
| 19- | 60 | 5-Cl | 3-F | H | |
| 19- | 61 | 5-Cl | 4-F | H | |
| 19- | 62 | 5-Cl | 2-Cl | H | |
| 19- | 63 | 5-Cl | 3-Cl | H | |
| 19- | 64 | 5-Cl | 4-Cl | H | IgP = 3,53 |
| 19- | 65 | 5-Cl | 2-Br | H | |
| 19- | 66 | 5-Cl | 3-Br | H | |
| 19- | 67 | 5-Cl | 4-Br | H | |
| 19- | 68 | 5-Cl | 2-I | H | |
| 19- | 69 | 5-Cl | 3-I | H | |
| 19- | 70 | 5-Cl | 4-I | H | |
| 19- | 71 | 5-Cl | 2-Cl | 3-Cl | |
| 19- | 72 | 5-Cl | 2-Cl | 4-Cl | |
| 19- | 73 | 5-Cl | 2-Cl | 5-Cl | |
| 19- | 74 | 5-Cl | 2-Cl | 6-Cl | |
| 19- | 75 | 5-Cl | 3-Cl | 4-Cl | NMR siehe unten |
| 19- | 76 | 5-Cl | 3-Cl | 5-Cl | IgP = 4,10 |
| 19- | 77 | 5-Br | H | H | |
| 19- | 78 | 5-Br | 2-F | H | |
| 19- | 79 | 5-Br | 3-F | H | |
| 19- | 80 | 5-Br | 4-F | H | |
| 19- | 81 | 5-Br | 2-Cl | H | |
| 19- | 82 | 5-Br | 3-Cl | H | |
| 19- | 83 | 5-Br | 4-Cl | H | |
| 19- | 84 | 5-Br | 2-Br | H | |
| 19- | 85 | 5-Br | 3-Br | H | |
| 19- | 86 | 5-Br | 4-Br | H | |
| 19- | 87 | 5-Br | 2-I | H | |
| 19- | 88 | 5-Br | 3-I | H | |
| 19- | 89 | 5-Br | 4-I | H | |
| 19- | 90 | 5-Br | 2-Cl | 3-Cl | |
| 19- | 91 | 5-Br | 2-Cl | 4-Cl | |
| 19- | 92 | 5-Br | 2-Cl | 5-Cl | |
| 19- | 93 | 5-Br | 2-Cl | 6-Cl | |
| 19- | 94 | 5-Br | 3-Cl | 4-Cl | |
| 19- | 95 | 5-Br | 3-Cl | 5-Cl | IgP = 4,15 |
| 19- | 96 | 5-I | H | H | |
| 19- | 97 | 5-I | 2-F | H | |
| 19- | 98 | 5-I | 3-F | H | |
| 19- | 99 | 5-I | 4-F | H | |
| 19- | 100 | 5-I | 2-Cl | H | |
| 19- | 101 | 5-I | 3-Cl | H | |
| 19- | 102 | 5-I | 4-Cl | H | |
| 19- | 103 | 5-I | 2-Br | H | |
| 19- | 104 | 5-I | 3-Br | H | |
| 19- | 105 | 5-I | 4-Br | H | |
| 19- | 106 | 5-I | 2-I | H | |
| 19- | 107 | 5-I | 3-I | H | |
| 19- | 108 | 5-I | 4-I | H | |
| 19- | 109 | 5-I | 2-Cl | 3-Cl | |
| 19- | 110 | 5-I | 2-Cl | 4-Cl | |
| 19- | 111 | 5-I | 2-Cl | 5-Cl | |
| 19- | 112 | 5-I | 2-Cl | 6-Cl | |
| 19- | 113 | 5-I | 3-Cl | 4-Cl | |
| 19- | 114 | 5-I | 3-Cl | 5-Cl | |
| 19- | 115 | 5-CF₃ | H | H | |
| 19- | 116 | 5-CF₃ | 2-F | H | |
| 19- | 117 | 5-CF₃ | 3-F | H | |
| 19- | 118 | 5-CHF₃ | 4-F | H | |
| 19- | 119 | 5-CF₃ | 2-Cl | H | |
| 19- | 120 | 5-CF₃ | 3-Cl | H | |
| 19- | 121 | 5-CF₃ | 4-Cl | H | NMR siehe unten |
| 19- | 122 | 5-CF₃ | 2-Br | H | |
| 19- | 123 | 5-CF₃ | 3-Br | H | |
| 19- | 124 | 5-CF₃ | 4-Br | H | |
| 19- | 125 | 5-CF₃ | 2-I | H | |
| 19- | 126 | 5-CF₃ | 3-I | H | |
| 19- | 127 | 5-CF₃ | 4-I | H | |
| 19- | 128 | 5-CF₃ | 2-Cl | 3-Cl | |
| 19- | 129 | 5-CF₃ | 2-Cl | 4-Cl | |
| 19- | 130 | 5-CF₃ | 2-Cl | 5-Cl | |
| 19- | 131 | 5-CF₃ | 2-Cl | 6-Cl | |
| 19- | 132 | 5-CF₃ | 3-Cl | 4-Cl | |
| 19- | 133 | 5-CF₃ | 3-Cl | 5-Cl | |
| 19- | 134 | 4-CF₃ | H | H | |
| 19- | 135 | 4-CF₃ | 2-F | H | |
| 19- | 136 | 4-CF₃ | 3-F | H | |
| 19- | 137 | 4-CF₃ | 4-F | H | |
| 19- | 138 | 4-CF₃ | 2-Cl | H | |
| 19- | 139 | 4-CF₃ | 3-Cl | H | |
| 19- | 140 | 4-CF₃ | 4-Cl | H | |
| 19- | 141 | 4-CF₃ | 2-Br | H | |
| 19- | 142 | 4-CF₃ | 3-Br | H | |
| 19- | 143 | 4-CF₃ | 4-Br | H | 19F: -61.9, -70.4ppm |
| 19- | 144 | 4-CF₃ | 2-I | H | |
| 19- | 145 | 4-CF₃ | 3-I | H | |
| 19- | 146 | 4-CF₃ | 4-I | H | |
| 19- | 147 | 4-CF₃ | 2-Cl | 3-Cl | |
| 19- | 148 | 4-CF₃ | 2-Cl | 4-Cl | |
| 19- | 149 | 4-CF₃ | 2-Cl | 5-Cl | |
| 19- | 150 | 4-CF₃ | 2-Cl | 6-Cl | |
| 19- | 151 | 4-CF₃ | 3-Cl | 4-Cl | |
| 19- | 152 | 4-CF₃ | 3-Cl | 5-Cl | IgP = 4,25 |
| 19- | 153 | 4-OMe | H | H | |
| 19- | 154 | 4-OMe | 2-F | H | |
| 19- | 155 | 4-OMe | 3-F | H | |
| 19- | 156 | 4-OMe | 4-F | H | |
| 19- | 157 | 4-OMe | 2-Cl | H | |
| 19- | 158 | 4-OMe | 3-Cl | H | |
| 19- | 159 | 4-OMe | 4-Cl | H | |
| 19- | 160 | 4-OMe | 2-Br | H | |
| 19- | 161 | 4-OMe | 3-Br | H | |
| 19- | 162 | 4-OMe | 4-Br | H | 1H: 4.10ppm, OCH₃ |
| 19- | 163 | 4-OMe | 2-I | H | |
| 19- | 164 | 4-OMe | 3-I | H | |
| 19- | 165 | 4-OMe | 4-I | H | |
| 19- | 166 | 4-OMe | 2-Cl | 3-Cl | |
| 19- | 167 | 4-OMe | 2-Cl | 4-Cl | |
| 19- | 168 | 4-OMe | 2-Cl | 5-Cl | |
| 19- | 169 | 4-OMe | 2-Cl | 6-Cl | |
| 19- | 170 | 4-OMe | 3-Cl | 4-Cl | |
| 19- | 171 | 4-OMe | 3-Cl | 5-Cl | IgP = 2,93 |
| 19- | 172 | 4-NMe₂ | H | H | |
| 19- | 173 | 4-NMe₂ | 2-F | H | |
| 19- | 174 | 4-NMe₂ | 3-F | H | |
| 19- | 175 | 4-NMe₂ | 4-F | H | |
| 19- | 176 | 4-NMe₂ | 2-Cl | H | |
| 19- | 177 | 4-NMe₂ | 3-Cl | H | |
| 19- | 178 | 4-NMe₂ | 4-Cl | H | |
| 19- | 179 | 4-NMe₂ | 2-Br | H | |
| 19- | 180 | 4-NMe₂ | 3-Br | H | |
| 19- | 181 | 4-NMe₂ | 4-Br | H | 1H: 6.41, 8.23ppm; Pyrimidin-H |
| 19- | 182 | 4-NMe₂ | 2-I | H | |
| 19- | 183 | 4-NMe₂ | 3-I | H | |
| 19- | 184 | 4-NMe₂ | 4-I | H | |
| 19- | 185 | 4-NMe₂ | 2-Cl | 3-Cl | |
| 19- | 186 | 4-NMe₂ | 2-Cl | 4-Cl | |
| 19- | 187 | 4-NMe₂ | 2-Cl | 5-Cl | |
| 19- | 188 | 4-NMe₂ | 2-Cl | 6-Cl | |
| 19- | 189 | 4-NMe₂ | 3-Cl | 4-Cl | |
| 19- | 190 | 4-NMe₂ | 3-Cl | 5-Cl | |
| 19- | 191 | 5-OCH₃ | 4-Cl | H | ¹H-NMR (d₆-DMSO): 8.66 ppm, (s, 2H) |
| 19- | 192 | 5-OCH₃ | 3-Cl | 5-Cl | ¹H-NMR (d₆-DMSO): 8.66 ppm, (s, 2H) |
| 19- | 193 | 5-OCH₃ | 3-Cl | 4-Cl | ¹H-NMR (d₆-DMSO): 8.66 ppm, (s, 2H) |
| 19- | 194 | 5-CN | 3-Cl | 5-Cl | ¹H-NMR(d₆-DMSO): 9.36 ppm, (s, 2H) |
| 19- | 195 | 4-CF₃ | 3-Br | 4-Cl | NMR siehe unten |
| 19- | 196 | 4-CF₃ | 3-Cl | 4-Br | NMR siehe unten |
| 19- | 197 | 4-CF₃ | 3-CF₃ | 5-CF₃ | NMR siehe unten |
| 19- | 198 | 4-CH₃ | 3-Cl | 5-F | IgP = 3,42 |
| 19- | 199 | 4-CH₃ | 3-F | 5-F | IgP = 3,06 |
| 19- | 200 | 4-CH₃ | 2-F | 4-Br | IgP = 3,28 |
| 19- | 201 | 4-CH₃ | 3-F | 4-F | IgP = 3,02 |
| 19- | 202 | 4-CH₃ | 3-CH₃ | 5-CH₃ | IgP = 3,42 |
| 19- | 203 | 5-CH₃ | 3-Cl | 5-F | IgP = 3,42 |
| 19- | 204 | 5-CH₃ | 3-F | 5-F | IgP = 3,06 |
| 19- | 205 | 5-CH₃ | 2-F | 4-Br | IgP = 3,28 |
| 19- | 206 | 5-CH₃ | 3-F | 4-F | IgP = 3,02 |
| 19- | 207 | 5-CH₃ | 3-Br | 5.Br | IgP = 4,05 |
| 19- | 208 | 5-CH₃ | 3-CH₃ | 5-CH₃ | IgP = 3,47 |
| 19- | 209 | 4-CN | 3-Cl | 5-Cl | IgP = 3,84 |

**Tabelle 24**

| | | | | |
|---|---|---|---|---|
| 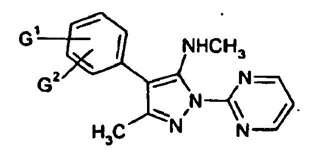 | | | | |

| Beispielnr. | | G¹ | G² | Physikalische Daten |
|---|---|---|---|---|
| 24- | 01 | H | H | |
| 24- | 02 | 2-F | H | |
| 24- | 03 | 3-F | H | |
| 24- | 04 | 4-F | H | |
| 24- | 05 | 2-Cl | H | |
| 24- | 06 | 3-Cl | H | NMR siehe unten |
| 24- | 07 | 4-Cl | H | |
| 24- | 08 | 2-Br | H | |
| 24- | 09 | 3-Br | H | |
| 24- | 10 | 4-Br | H | |
| 24- | 11 | 2-I | H | |
| 24- | 12 | 3-I | H | |
| 24- | 13 | 4-I | H | |
| 24- | 14 | 2-F | 3-F | |
| 24- | 15 | 2-F | 4-F | |
| 24- | 16 | 2-F | 5-F | |
| 24- | 17 | 2-F | 6-F | |
| 24- | 18 | 2-F | 3-Cl | |
| 24- | 19 | 2-F | 4-Cl | |
| 24- | 20 | 2-F | 5-Cl | |
| 24- | 21 | 2-F | 6-Cl | |
| 24- | 22 | 2-F | 3-Br | |
| 24- | 23 | 2-F | 4-Br | |
| 24- | 24 | 2-F | 5-Br | |
| 24- | 25 | 2-F | 6-Br | |
| 24- | 26 | 2-F | 3-I | |
| 24- | 27 | 2-F | 4-I | |
| 24- | 28 | 2-F | 5-I | |
| 24- | 29 | 2-F | 6-I | |
| 24- | 30 | 2-Cl | 3-F | |
| 24- | 31 | 2-Cl | 4-F | |
| 24- | 32 | 2-Cl | 5-F | |
| 24- | 33 | 2-Cl | 3-Cl | |
| 24- | 34 | 2-Cl | 4-Cl | |
| 24- | 35 | 2-Cl | 5-Cl | |
| 24- | 36 | 2-Cl | 6-Cl | |
| 24- | 37 | 2-Cl | 3-Br | |
| 24- | 38 | 2-Cl | 4-Br | |
| 24- | 39 | 2-Cl | 5-Br | |
| 24- | 40 | 2-Cl | 6-Br | |
| 24- | 41 | 2-Cl | 3-I | |
| 24- | 42 | 2-Cl | 4-I | |
| 24- | 43 | 2-Cl | 5-I | |
| 24- | 44 | 2-Cl | 6-I | |
| 24- | 45 | 2-Br | 3-F | |
| 24- | 46 | 2-Br | 4-F | |
| 24- | 47 | 2-Br | 5-F | |
| 24- | 48 | 2-Br | 3-Cl | |
| 24- | 49 | 2-Br | 4-Cl | |
| 24- | 50 | 2-Br | 5-Cl | |
| 24- | 51 | 2-Br | 3-Br | |
| 24- | 52 | 2-Br | 4-Br | |
| 24- | 53 | 2-Br | 5-Br | |
| 24- | 54 | 2-Br | 6-Br | |
| 24- | 55 | 2-Br | 3-I | |
| 24- | 56 | 2-Br | 4-1 | |
| 24- | 57 | 2-Br | 5-I | |
| 24- | 58 | 2-Br | 6-I | |
| 24- | 59 | 2-I | 3-F | |
| 24- | 60 | 2-I | 4-F | |
| 24- | 61 | 2-I | 5-F | |
| 24- | 62 | 2-I | 3-Cl | |
| 24- | 63 | 2-I | 4-Cl | |
| 24- | 64 | 2-I | 5-Cl | |
| 24- | 65 | 2-I | 3-Br | |
| 24- | 66 | 2-I | 4-Br | |
| 24- | 67 | 2-I | 5-Br | |
| 24- | 68 | 2-I | 3-I | |
| 24- | 69 | 2-I | 4-I | |
| 24- | 70 | 2-I | 5-I | |
| 24- | 71 | 2-I | 6-I | |
| 24- | 72 | 3-F | 4-F | |
| 24- | 73 | 3-F | 5-F | |
| 24- | 74 | 3-F | 4-Cl | |
| 24- | 75 | 3-F | 5-Cl | |
| 24- | 76 | 3-F | 4-Br | |
| 24- | 77 | 3-F | 5-Br | |
| 24- | 78 | 3-F | 4-I | |
| 24- | 79 | 3-F | 5-I | |
| 24- | 80 | 3-Cl | 4-F | |
| 24- | 81 | 3-Cl | 4-Cl | |
| 24- | 82 | 3-Cl | 5-Cl | |
| 24- | 83 | 3-Cl | 4-Br | |
| 24- | 84 | 3-Cl | 5-Br | |
| 24- | 85 | 3-Cl | 4-I | |
| 24- | 86 | 3-Cl | 5-1 | |
| 24- | 87 | 3-Br | 4-F | |
| 24- | 88 | 3-Br | 4-Cl | |
| 24- | 89 | 3-Br | 4-Br | |
| 24- | 90 | 3-Br | 5-Br | |
| 24- | 91 | 3-Br | 4-1 | |
| 24- | 92 | 3-Br | 5-1 | |
| 24- | 93 | 3-I | 4-F | |
| 24- | 94 | 3-I | 4-Cl | |
| 24- | 95 | 3-I | 4-Br | |
| 24- | 96 | 3-I | 4-1 | |
| 24- | 97 | 3-I | 4-1 | |

**Tabelle 25**

| | | | | |
|---|---|---|---|---|
| 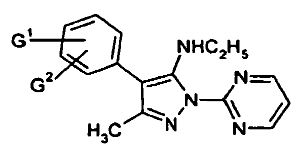 | | | | |

| Beispielnr. | | G¹ | G² | Physikalische Daten |
|---|---|---|---|---|
| 25- | 01 | H | H | |
| 25- | 02 | 2-F | H | |
| 25- | 03 | 3-F | H | |
| 25- | 04 | 4-F | H | logP = 2,59 |
| 25- | 05 | 2-Cl | H | logP = 2,69 |
| 25- | 06 | 3-Cl | H | logP = 3,02 |
| 25- | 07 | 4-Cl | H | NMR siehe unten |
| 25- | 08 | 2-Br | H | |
| 25- | 09 | 3-Br | H | |
| 25- | 10 | 4-Br | H | |
| 25- | 11 | 2-I | H | |
| 25- | 12 | 3-I | H | |
| 25- | 13 | 4-I | H | |
| 25- | 14 | 2-F | 3-F | |
| 25- | 15 | 2-F | 4-F | |
| 25- | 16 | 2-F | 5-F | |
| 25- | 17 | 2-F | 6-F | |
| 25- | 18 | 2-F | 3-Cl | |
| 25- | 19 | 2-F | 4-Cl | |
| 25- | 20 | 2-F | 5-Cl | |
| 25- | 21 | 2-F | 6-Cl | |
| 25- | 22 | 2-F | 3-Br | |
| 25- | 23 | 2-F | 4-Br | |
| 25- | 24 | 2-F | 5-Br | |
| 25- | 25 | 2-F | 6-Br | |
| 25- | 26 | 2-F | 3-1 | |
| 25- | 27 | 2-F | 4-I | |
| 25- | 28 | 2-F | 5-I | |
| 25- | 29 | 2-F | 6-I | |
| 25- | 30 | 2-Cl | 3-F | |
| 25- | 31 | 2-Cl | 4-F | logP =2,79 |
| 25- | 32 | 2-Cl | 5-F | |
| 25- | 33 | 2-Cl | 3-Cl | |
| 25- | 34 | 2-Cl | 4-Cl | NMR siehe unten |
| 25- | 35 | 2-Cl | 5-Cl | |
| 25- | 36 | 2-Cl | 6-Cl | |
| 25- | 37 | 2-Cl | 3-Br | |
| 25- | 38 | 2-Cl | 4-Br | |
| 25- | 39 | 2-Cl | 5-Br | |
| 25- | 40 | 2-Cl | 6-Br | |
| 25- | 41 | 2-Cl | 3-1 | |
| 25- | 42 | 2-Cl | 4-I | |
| 25- | 43 | 2-Cl | 5-1 | |
| 25- | 44 | 2-Cl | 6-1 | |
| 25- | 45 | 2-Br | 3-F | |
| 25- | 46 | 2-Br | 4-F | |
| 25- | 47 | 2-Br | 5-F | |
| 25- | 48 | 2-Br | 3-Cl | |
| 25- | 49 | 2-Br | 4-Cl | |
| 25- | 50 | 2-Br | 5-Cl | |
| 25- | 51 | 2-Br | 3-Br | |
| 25- | 52 | 2-Br | 4-Br | |
| 25- | 53 | 2-Br | 5-Br | |
| 25- | 54 | 2-Br | 6-Br | |
| 25- | 55 | 2-Br | 3-1 | |
| 25- | 56 | 2-Br | 4-1 | |
| 25- | 57 | 2-Br | 5-I | |
| 25- | 58 | 2-Br | 6-I | |
| 25- | 59 | 2-I | 3-F | |
| 25- | 60 | 2-I | 4-F | |
| 25- | 61 | 2-I | 5-F | |
| 25- | 62 | 2-I | 3-Cl | |
| 25- | 63 | 2-I | 4-Cl | |
| 25- | 64 | 2-I | 5-Cl | |
| 25- | 65 | 2-I | 3-Br | |
| 25- | 66 | 2-I | 4-Br | |
| 25- | 67 | 2-I | 5-Br | |
| 25- | 68 | 2-I | 3-I | |
| 25- | 69 | 2-I | 4-I | |
| 25- | 70 | 2-I | 5-I | |
| 25- | 71 | 2-I | 6-I | |
| 25- | 72 | 3-F | 4-F | |
| 25- | 73 | 3-F | 5-F | |
| 25- | 74 | 3-F | 4-Cl | |
| 25- | 75 | 3-F | 5-Cl | |
| 25- | 76 | 3-F | 4-Br | |
| 25- | 77 | 3-F | 5-Br | |
| 25- | 78 | 3-F | 4-I | |
| 25- | 79 | 3-F | 5-I | |
| 25- | 80 | 3-Cl | 4-F | |
| 25- | 81 | 3-Cl | 4-Cl | |
| 25- | 82 | 3-Cl | 5-Cl | |
| 25- | 83 | 3-Cl | 4-Br | |
| 25- | 84 | 3-Cl | 5-Br | |
| 25- | 85 | 3-Cl | 4-I | |
| 25- | 86 | 3-Cl | 5-I | |
| 25- | 87 | 3-Br | 4-F | |
| 25- | 88 | 3-Br | 4-Cl | |
| 25- | 89 | 3-Br | 4-Br | |
| 25- | 90 | 3-Br | 5-Br | |
| 25- | 91 | 3-Br | 4-I | |
| 25- | 92 | 3-Br | 5-1 | |
| 25- | 93 | 3-I | 4-F | |
| 25- | 94 | 3-I | 4-Cl | |
| 25- | 95 | 3-I | 4-Br | |
| 25- | 96 | 3-1 | 4-I | |
| 25- | 97 | 3-I | 4-I | |

**Tabelle 26**

| | | | | | |
|---|---|---|---|---|---|
| 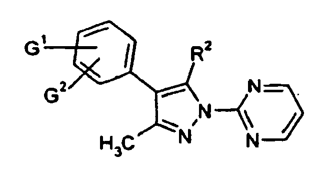 | | | | | |

| Beispielnr. | | G¹ | G² | R² | Physikalische Daten |
|---|---|---|---|---|---|
| 26- | 01 | H | H | NHnPr | |
| 26- | 02 | 2-F | H | NHnPr | |
| 26- | 03 | 3-F | H | NHnPr | |
| 26- | 04 | 4-F | H | NHnPr | |
| 26- | 05 | 2-Cl | H | NHnPr | |
| 26- | 06 | 3-Cl | H | NHnPr | logP = 3,37 |
| 26- | 07 | 4-Cl | H | NHnPr | logP = 3,42 |
| 26- | 08 | 2-Br | H | NHnPr | |
| 26- | 09 | 3-Br | H | NHnPr | |
| 26- | 10 | 4-Br | H | NHnPr | |
| 26- | 11 | 2-I | H | NHnPr | |
| 26- | 12 | 3-I | H | NHnPr | |
| 26- | 13 | 4-I | H | N HnPr | |
| 26- | 14 | 2-Cl | 3-Cl | NHnPr | |
| 26- | 15 | 2-Cl | 4-Cl | NHnPr | |
| 26- | 16 | 2-Cl | 5-Cl | NHnPr | |
| 26- | 17 | 2-Cl | 6-Cl | NHnPr | |
| 26- | 18 | 3-Cl | 4-Cl | NHnPr | |
| 26- | 19 | 3-Cl | 5-Cl | NHNPR | |
| 26- | 20 | H | H | NHiPr | |
| 26- | 21 | 2-F | H | NHiPr | |
| 26- | 22 | 3-F | H | NHiPr | |
| 26- | 23 | 4-F | H | NHiPr | NMR siehe unten |
| 26- | 24 | 2-Cl | H | NHiPr | NMR siehe unten |
| 26- | 25 | 3-Cl | H | NHiPr | |
| 26- | 26 | 4-Cl | H | NHiPr | NMR siehe unten |
| 26- | 27 | 2-Br | H | NHiPr | |
| 26- | 28 | 3-Br | H | NHiPr | |
| 26- | 29 | 4-Br | H | NHiPr | |
| 26- | 30 | 2-I | H | NHiPr | |
| 26- | 31 | 3-I | H | NHiPr | |
| 26- | 32 | 4-I | H | NHiPr | |
| 26- | 33 | 2-Cl | 3-Cl | NHiPr | |
| 26- | 34 | 2-Cl | 4-Cl | NHiPr | |
| 26- | 35 | 2-Cl | 5-Cl | NHiPr | |
| 26- | 36 | 2-Cl | 6-Cl | NHiPr | |
| 26- | 37 | 2-Cl | 4-F | NHiPr | logP = 2,91 |
| 26- | 38 | 3-Cl | 4-Cl | NHiPr | |
| 26- | 39 | 3-Cl | 5-Cl | NHiPr | |
| 26- | 40 | H | H | NHCH₂Phenyl | |
| 26- | 41 | 2-F | H | NHCH₂Phenyl | |
| 26- | 42 | 3-F | H | NHCH₂Phenyl | |
| 26- | 43 | 4-F | H | NHCH₂Phenyl | |
| 26- | 44 | 2-Cl | H | NHCH₂Phenyl | logP=3,37 |
| 26- | 45 | 3-Cl | H | NHCH₂Phenyl | |
| 26- | 46 | 4-Cl | H | NHCH₂Phenyl | NMR siehe unten |
| 26- | 47 | 2-Br | H | NHCH₂Phenyl | |
| 26- | 48 | 3-Br | H | NHCH₂Phenyl | |
| 26- | 49 | 4-Br | H | NHCH₂Phenyl | |
| 26- | 50 | 2-I | H | NHCH₂Phenyl | |
| 26- | 51 | 3-I | H | NHCH₂Phenyl | |
| 26- | 52 | 4-I | H | NHCH₂Phenyl | |
| 26- | 53 | 2-Cl | 3-Cl | NHCH₂Phenyl | |
| 26- | 54 | 2-Cl | 4-Cl | NHCH₂Phenyl | logP = 4,23 |
| 26- | 55 | 2-Cl | 5-Cl | NHCH₂Phenyl | |
| 26- | 56 | 2-Cl | 6-Cl | NHCH₂Phenyl | |
| 26- | 57 | 3-Cl | 4-Cl | NHCH₂Phenyl | |
| 26- | 58 | 3-Cl | 5-Cl | NNCH₂Phenyl | |
| 26- | 59 | H | H | NHCH₂cPr | |
| 26- | 60 | 2-F | H | NHCH₂cPr | |
| 26- | 61 | 3-F | H | NHCH₂cPr | |
| 26- | 62 | 4-F | H | NHCH₂cPr | logP = 3,15 |
| 26- | 63 | 2-Cl | H | NHCH₂cPr | |
| 26- | 64 | 3-Cl | H | NHCH₂cPr | |
| 26- | 65 | 4-Cl | H | NHCH₂cPr | NMR siehe unten |
| 26- | 66 | 2-Br | H | NHCH₂cPr | |
| 26- | 67 | 3-Br | H | NHCH₂cPr | |
| 26- | 68 | 4-Br | H | NHCH₂cPr | |
| 26- | 69 | 2-I | H | NHCH₂cPr | |
| 26- | 70 | 3-I | H | NHCH₂cPr | |
| 26- | 71 | 4-I | H | NHCH₂cPr | |
| 26- | 72 | 2-Cl | 3-Cl | NHCH₂cPr | |
| 26- | 73 | 2-Cl | 4-Cl | NHCH₂cPr | |
| 26- | 74 | 2-Cl | 5-Cl | NHCH₂cPr | |
| 26- | 75 | 2-Cl | 6-Cl | NHCH₂cPr | |
| 26- | 76 | 3-Cl | 4-Cl | NHCH₂cPr | |
| 26- | 77 | 3-Cl | 5-Cl | NHCH₂cPr | |
| 26- | 78 | H | H | NHCH₂C=CMe₂ | |
| 26- | 79 | 2-F | H | NHCH₂C=CMe₂ | |
| 26- | 80 | 3-F | H | NHCH₂C=CMe₂ | |
| 26- | 81 | 4-F | H | NHCH₂C=CMe₂ | |
| 26- | 82 | 2-Cl | H | NHCH₂C=CMe₂ | |
| 26- | 83 | 3-Cl | H | NHCH₂C=CMe₂ | |
| 26- | 84 | 4-Cl | H | NHCH₂C=CMe₂ | logP = 3,84 |
| 26- | 85 | 2-Br | H | NHCH₂C=CMe₂ | |
| 26- | 86 | 3-Br | H | NHCH₂C=CMe₂ | |
| 26- | 87 | 4-Br | H | NHCH₂C=CMe₂ | |
| 26- | 88 | 2-I | H | NHCH₂C=CMe₂ | |
| 26- | 89 | 3-I | H | NHCH₂C=CMe₂ | |
| 26- | 90 | 4-I | H | NHCH₂C=CMe₂ | |
| 26- | 91 | 2-Cl | 3-Cl | NHCH₂C=CMe₂ | |
| 26- | 92 | 2-Cl | 4-Cl | NHCH₂C=CMe₂ | |
| 26- | 93 | 2-Cl | 5-Cl | NHCH₂C=CMe₂ | |
| 26- | 94 | 2-Cl | 6-Cl | NHCH₂C=CMe₂ | |
| 26- | 95 | 3-Cl | 4-Cl | NHCH₂C=CMe₂ | |
| 26- | 96 | 3-Cl | 5-Cl | NHCH₂C=CMe₂ | |
| 26- | 97 | H | H | NH(2'-i-Pentyl) | |
| 26- | 98 | 2-F | H | NH(2'-i-Pentyl) | |
| 26- | 99 | 3-F | H | NH(2'-i-Pentyl) | |
| 26- | 100 | 4-F | H | NH(2'-i-Pentyl) | NMR siehe unten |
| 26- | 101 | 2-Cl | H | NH(2'-i-Pentyl) | NMR siehe unten |
| 26- | 102 | 3-Cl | H | NH(2'-i-Pentyl) | NMR siehe unten |
| 26- | 103 | 4-Cl | H | NH(2'-i-Pentyl) | NMR siehe unten |
| 26- | 104 | 2-Br | H | NH(2'-i-Pentyl) | |
| 26- | 105 | 3-Br | H | NH(2'-i-Pentyl) | |
| 26- | 106 | 4-Br | H | NH(2'-i-Pentyl) | |
| 26- | 107 | 2-I | H | NH(2'-i-Pentyl) | |
| 26- | 108 | 3-I | H | NH(2'-i-Pentyl) | |
| 26- | 109 | 4-I | H | NH(2'-i-Pentyl) | |
| 26- | 110 | 2-Cl | 3-Cl | NH(2'-i-Pentyl) | |
| 26- | 111 | 2-Cl | 4-Cl | NH(2'-i-Pentyl) | NMR siehe unten |
| 26- | 112 | 2-Cl | 5-Cl | NH(2'-i-Pentyl) | |
| 26- | 113 | 2-Cl | 6-Cl | NH(2'-i-Pentyl) | |
| 26- | 114 | 3-Cl | 4-Cl | NH(2'-i-Pentyl) | |
| 26- | 115 | 3-Cl | 5-Cl | NH(2'-i-Pentyl) | |
| 26- | 116 | 4-Cl | H | NHCH₂CO₂Et | NMR siehe unten |
| 26- | 117 | 2-Cl | 4-F | NH(2'-i-Pentyl) | NMR siehe unten |
| 26- | 118 | 2-Cl | 4-F | NH(2'-i-Pentyl) | NMR siehe unten |
| 26- | 119 | H | H | NHnBu | |
| 26- | 120 | 2-F | H | NHnBu | |
| 26- | 121 | 3-F | H | NHnBu | |
| 26- | 122 | 4-F | H | NHnBu | |
| 26- | 123 | 2-Cl | H | NHnBu | NMR siehe unten |
| 26- | 124 | 3-Cl | H | NHnBu | NMR siehe unten |
| 26- | 125 | 4-Cl | H | NHnBu | NMR siehe unten |
| 26- | 126 | 2-Br | H | NHnBu | |
| 26- | 127 | 3-Br | H | NHnBu | |
| 26- | 128 | 4-Br | H | NHnBu | |
| 26- | 129 | 2-I | H | NHnBu | |
| 26- | 130 | 3-I | H | NHnBu | |
| 26- | 131 | 4-I | H | NHnBu | |
| 26- | 132 | 2-Cl | 3-Cl | NHnBu | |
| 26- | 133 | 2-Cl | 4-Cl | NHnBu | |
| 26- | 134 | 2-Cl | 5-Cl | NHnBu | |
| 26- | 135 | 2-Cl | 6-Cl | NHnBu | |
| 26- | 136 | 3-Cl | 4-Cl | NHnBu | |
| 26- | 137 | 3-Cl | 5-Cl | NHnBu | |
| 26- | 138 | 2-Cl | 4-F | NHnBu | NMR siehe unten |
| 26- | 139 | 3-Cl | H | NHiBu | |
| 26- | 140 | 4-Cl | H | NHiBu | |
| 26- | 141 | 3-Cl | H | NHCH₂CO₂Et | NMR siehe unten |
| 26 | 142 | 2-Cl | 4-F | NHnPr | |

**Tabelle 27**

| | | | | | | |
|---|---|---|---|---|---|---|
| 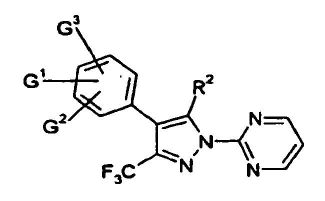 | | | | | | |

| Beispielnr. | | G¹ | G² | G³ | R² | Physikalische Daten |
|---|---|---|---|---|---|---|
| 27- | 01 | H | H | H | NHnPr | |
| 27- | 02 | H | 2-F | H | NHnPr | |
| 27- | 03 | H | 3-F | H | NHnPr | |
| 27- | 04 | H | 4-F | H | NHnPr | |
| 27- | 05 | H | 2-Cl | H | NHnPr | |
| 27- | 06 | H | 3-Cl | H | NHnPr | |
| 27- | 07 | H | 4-Cl | H | NHnPr | |
| 27- | 08 | H | 2-Br | H | NHnPr | |
| 27- | 09 | H | 3-Br | H | NHnPr | |
| 27- | 10 | H | 4-Br | H | NHnPr | |
| 27- | 11 | H | 2-I | H | NHnPr | |
| 27- | 12 | H | 3-I | H | NHnPr | |
| 27- | 13 | H | 4-I | H | NHnPr | |
| 27- | 14 | 3-Cl | 2-Cl | H | NHnPr | |
| 27- | 15 | 4-Cl | 2-Cl | H | NHnPr | |
| 27- | 16 | 5-Cl | 2-Cl | H | NHnPr | |
| 27- | 17 | 6-Cl | 2-Cl | H | NHnPr | |
| 27- | 18 | 4-Cl | 3-Cl | H | NHnPr | |
| 27- | 19 | 5-Cl | 3-Cl | H | NHnPr | NMR siehe unten |
| 27- | 20 | H | H | H | NHiPr | |
| 27- | 21 | H | 2-F | H | NHiPr | |
| 27- | 22 | H | 3-F | H | NHiPr | |
| 27- | 23 | H | 4-F | H | NHiPr | |
| 27- | 24 | H | 2-Cl | H | NHiPr | |
| 27- | 25 | H | 3-Cl | H | NHiPr | |
| 27- | 26 | H | 4-Cl | H | NHiPr | NMR siehe unten |
| 27- | 27 | H | 2-Br | H | NHiPr | |
| 27- | 28 | H | 3-Br | H | NHiPr | |
| 27- | 29 | H | 4-Br | H | NHiPr | |
| 27- | 30 | H | 2-I | H | NHiPr | |
| 27- | 31 | H | 3-I | H | NHiPr | |
| 27- | 32 | H | 4-I | H | NHiPr | |
| 27- | 33 | 3-Cl | 2-Cl | H | NHiPr | |
| 27- | 34 | 4-Cl | 2-Cl | H | NHiPr | |
| 27- | 35 | 5-Cl | 2-Cl | H | NHiPr | |
| 27- | 36 | 6-Cl | 2-Cl | H | NHiPr | |
| 27- | 37 | 4-Cl | 3-Cl | H | NHiPr | |
| 27- | 38 | 5-Cl | 3-Cl | H | NHiPr | NMR siehe unten |
| 27- | 39 | H | H | H | NHCH₂Phenyl | |
| 27- | 40 | H | 2-F | H | NHCH₂Phenyl | |
| 27- | 41 | H | 3-F | H | NHCH₂Phenyl | |
| 27- | 42 | H | 4-F | H | NHCH₂Phenyl | |
| 27- | 43 | H | 2-Cl | H | NHCH₂Phenyl | |
| 27- | 44 | H | 3-Cl | H | NHCH₂Phenyl | |
| 27- | 45 | H | 4-Cl | H | NHCH₂Phenyl | NMR siehe unten |
| 27- | 46 | H | 2-Br | H | NHCH₂Phenyl | |
| 27- | 47 | H | 3-Br | H | NHCH₂Phenyl | |
| 27- | 48 | H | 4-Br | H | NHCH₂Phenyl | |
| 27- | 49 | H | 2-I | H | NHCH₂Phenyl | |
| 27- | 50 | H | 3-I | H | NHCH₂Phenyl | NMR siehe unten |
| 27- | 51 | H | 4-I | H | NHCH₂Phenyl | NMR siehe unten |
| 27- | 52 | 3-Cl | 2-Cl | H | NHCH₂Phenyl | |
| 27- | 53 | 4-Cl | 2-Cl | H | NHCH₂Phenyl | |
| 27- | 54 | 5-Cl | 2-Cl | H | NHCH₂Phenyl | |
| 27- | 55 | 6-Cl | 2-Cl | H | NHCH₂Phenyl | |
| 27- | 56 | 4-Cl | 3-Cl | H | NHCH₂Phenyl | |
| 27- | 57 | 5-Cl | 3-Cl | H | NHCH₂Phenyl | |
| 27- | 58 | H | H | H | NHCH₂cPr | |
| 27- | 59 | H | 2-F | H | NHCH₂cPr | |
| 27- | 60 | H | 3-F | H | NHCH₂cPr | |
| 27- | 61 | H | 4-F | H | NHCH₂cPr | |
| 27- | 62 | H | 2-Cl | H | NHCH₂cPr | |
| 27- | 63 | H | 3-Cl | H | NHCH₂cPr | |
| 27- | 64 | H | 4-Cl | H | NHCH₂cPr | |
| 27- | 65 | H | 2-Br | H | NHCH₂cPr | |
| 27- | 66 | H | 3-Br | H | NHCH₂cPr | |
| 27- | 67 | H | 4-Br | H | NHCH₂cPr | |
| 27- | 68 | H | 2-I | H | NHCH₂cPr | |
| 27- | 69 | H | 3-I | H | NHCH₂cPr | |
| 27- | 70 | H | 4-I | H | NHCH₂cPr | |
| 27- | 71 | 3-Cl | 2-Cl | H | NHCH₂cPr | |
| 27- | 72 | 4-Cl | 2-Cl | H | NHCH₂cPr | |
| 27- | 73 | 5-Cl | 2-Cl | H | NHCH₂cPr | |
| 27- | 74 | 6-Cl | 2-Cl | H | NHCH₂cPr | |
| 27- | 75 | 4-Cl | 3-Cl | H | NHCH₂cPr | |
| 27- | 76 | 5-Cl | 3-Cl | H | NHCH₂cPr | NMR siehe unten |
| 27- | 77 | H | H | H | NHCH₂C=CMe₂ | |
| 27- | 78 | H | 2-F | H | NHCH₂C=CMe₂ | |
| 27- | 79 | H | 3-F | H | NHCH₂C=CMe₂ | |
| 27- | 80 | H | 4-F | H | NHCH₂C=CMe₂ | |
| 27- | 81 | H | 2-Cl | H | NHCH₂C=CMe₂ | |
| 27- | 82 | H | 3-Cl | H | NHCH₂C=CMe₂ | |
| 27- | 83 | H | 4-Cl | H | NHCH₂C=CMe₂ | |
| 27- | 84 | H | 2-Br | H | NHCH₂C=CMe₂ | |
| 27- | 85 | H | 3-Br | H | NHCH₂C=CMe₂ | |
| 27- | 86 | H | 4-Br | H | NHCH₂C=CMe₂ | |
| 27- | 87 | H | 2-I | H | NHCH₂C=CMe₂ | |
| 27- | 88 | H | 3-I | H | NHCH₂C=CMe₂ | |
| 27- | 89 | H | 4-I | H | NHCH₂C=CMe₂ | |
| 27- | 90 | 3-Cl | 2-Cl | H | NHCH₂C=CMe₂ | |
| 27- | 91 | 4-Cl | 2-Cl | H | NHCH₂C=CMe₂ | |
| 27- | 92 | 5-Cl | 2-Cl | H | NHCH₂C=CMe₂ | |
| 27- | 93 | 6-Cl | 2-Cl | H | NHCH₂C=CMe₂ | |
| 27- | 94 | 4-Cl | 3-Cl | H | NHCH₂C=CMe₂ | |
| 27- | 95 | 5-Cl | 3-Cl | H | NHCH₂C=CMe₂ | |
| 27- | 96 | H | H | H | NHCH₂CH=CH₂ | |
| 27- | 97 | H | 2-F | H | NHCH₂CH=CH₂ | |
| 27- | 98 | H | 3-F | H | NHCH₂CH=CH₂ | |
| 27- | 99 | H | 4-F | H | NHCH₂CH=CH₂ | |
| 27- | 100 | H | 2-Cl | H | NHCH₂CH=CH₂ | |
| 27- | 101 | H | 3-Cl | H | NHCH₂CH=CH₂ | |
| 27- | 102 | H | 4-Cl | H | NHCH₂CH=CH₂ | |
| 27- | 103 | H | 2-Br | H | NHCH₂CH=CH₂ | |
| 27- | 104 | H | 3-Br | H | NHCH₂CH=CH₂ | |
| 27- | 105 | H | 4-Br | H | NHCH₂CH=CH₂ | |
| 27- | 106 | H | 2-I | H | NHCH₂CH=CH₂ | |
| 27- | 107 | H | 3-I | H | NHCH₂CH=CH₂ | |
| 27- | 108 | H | 4-I | H | NHCH₂CH=CH₂ | |
| 27- | 109 | 3-Cl | 2-Cl | H | NHCH₂CH=CH₂ | |
| 27- | 110 | 4-Cl | 2-Cl | H | NHCH₂CH=CH₂ | |
| 27- | 111 | 5-Cl | 2-Cl | H | NHCH₂CH=CH₂ | |
| 27- | 112 | 6-Cl | 2-Cl | H | NHCH₂CH=CH₂ | |
| 27- | 113 | 4-Cl | 3-Cl | H | NHCH₂CH=CH₂ | |
| 27- | 114 | 5-Cl | 3-Cl | H | NHCH₂CH=CH₂ | NMR siehe unten |
| 27- | 115 | H | H | H | NHiBu | |
| 27- | 116 | H | 2-F | H | NHiBu | |
| 27- | 117 | H | 3-F | H | NHiBu | |
| 27- | 118 | H | 4-F | H | NHiBu | |
| 27- | 119 | H | 2-Cl | H | NHiBu | |
| 27- | 120 | H | 3-Cl | H | NHiBu | |
| 27- | 121 | H | 4-Cl | H | NHiBu | |
| 27- | 122 | H | 2-Br | H | NHiBu | |
| 27- | 123 | H | 3-Br | H | NHiBu | |
| 27- | 124 | H | 4-Br | H | NHiBu | |
| 27- | 125 | H | 2-I | H | NHiBu | |
| 27- | 126 | H | 3-I | H | NHiBu | |
| 27- | 127 | H | 4-I | H | NHiBu | |
| 27- | 128 | 3-Cl | 2-Cl | H | NHiBu | |
| 27- | 129 | 4-Cl | 2-Cl | H | NHiBu | |
| 27- | 130 | 5-Cl | 2-Cl | H | NHiBu | |
| 27- | 131 | 6-Cl | 2-Cl | H | NHiBu | |
| 27- | 132 | 4-Cl | 3-Cl | H | NHiBu | |
| 27- | 133 | 5-Cl | 3-Cl | H | NHiBu | NMR siehe unten |
| 27- | 134 | H | H | H | NHCH₂-4-Cl-Ph | |
| 27- | 135 | H | 2-F | H | NHCH₂-4-Cl-Ph | |
| 27- | 136 | H | 3-F | H | NHCH₂-4-Cl-Ph | |
| 27- | 137 | H | 4-F | H | NHCH₂-4-Cl-Ph | |
| 27- | 138 | H | 2-Cl | H | NHCH₂-4-Cl-Ph | |
| 27- | 139 | H | 3-Cl | H | NHCH₂-4-Cl-Ph | |
| 27- | 140 | H | 4-Cl | H | NHCH₂-4-Cl-Ph | NMR siehe unten |
| 27- | 141 | H | 2-Br | H | NHCH₂-4-Cl-Ph | |
| 27- | 142 | H | 3-Br | H | NHCH₂-4-Cl-Ph | |
| 27- | 143 | H | 4-Br | H | NHCH₂-4-Cl-Ph | |
| 27- | 144 | H | 2-I | H | NHCH₂-4-Cl-Ph | |
| 27- | 145 | H | 3-I | H | NHCH₂-4-Cl-Ph | |
| 27- | 146 | H | 4-I | H | NHCH₂-4-Cl-Ph | |
| 27- | 147 | 3-Cl | 2-Cl | H | NHCH₂-4-Cl-Ph | |
| 27- | 148 | 4-Cl | 2-Cl | H | NHCH₂-4-Cl-Ph | |
| 27- | 149 | 5-Cl | 2-Cl | H | NHCH₂-4-Cl-Ph | |
| 27- | 150 | 6-Cl | 2-Cl | H | NHCH₂-4-Cl-Ph | |
| 27- | 151 | 4-Cl | 3-Cl | H | NHCH₂-4-Cl-Ph | |
| 27- | 152 | 5-Cl | 3-Cl | H | NHCH₂-4-Cl-Ph | |
| 27- | 153 | 5-Cl | 3-Cl | H | NHCH₂-3-Br-5-Br-Ph | NMR siehe unten |
| 27- | 154 | 5-Cl | 3-Cl | H | NH-cPent | NMR siehe unten |
| 27- | 155 | 4-F | 3-Cl | H | NHCH₂Phenyl | NMR siehe unten |
| 27- | 156 | H | 4-OCHF₂ | H | NHCH₂Phenyl | NMR siehe unten |
| 27- | 157 | 3-Cl | 2-F | H | NHCH₂Phenyl | NMR siehe unten |
| 27- | 158 | 4-Cl | 3-CF₃ | H | NHCH₂Phenyl | NMR siehe unten |
| 27- | 159 | 4-Me | 3-OMe | H | NHCH₂Phenyl | NMR siehe unten |
| 27- | 160 | 4-Cl | 3-OCF₃ | H | NHCH₂Phenyl | NMR siehe unten |
| 27- | 161 | H | 4-SMe | H | NHCH₂Phenyl | NMR siehe unten |
| 27- | 162 | H | 3-OCHF₂ | H | NHCH₂Phenyl | NMR siehe unten |
| 27- | 163 | 4-OMe | 3-F | 5-F | NHCH₂Phenyl | NMR siehe unten |
| 27- | 164 | 3-Cl | 5-CF₃ | H | NHCH₂Phenyl | |
| 27- | 165 | 3-CF₃ | 4-Cl | H | NHCH₂Phenyl | |
| 27 | 166 | 3-CF₃ | 5-CF₃ | H | NHCH₂Phenyl | |
| 27- | 167 | 3-Cl | 5-CF₃ | H | NHCH₂-4-Cl-Ph | |
| 27- | 168 | 3-CF₃ | 4-Cl | H | NHCH₂-4-Cl-Ph | |
| 27- | 169 | 3-CF₃ | 5-CF₃ | H | NHCH₂-4-Cl-Ph | |
| 27- | 170 | 3-Cl | 5-CF₃ | H | NHCH₂CH=CH₂ | |
| 27- | 171 | 3-CF₃ | 4-Cl | H | NHCH₂CH=CH₂ | |
| 27- | 172 | 3-CF₃ | 5-CF₃ | H | NHCH₂CH=CH₂ | |

**Tabelle 28**

| | | | | | |
|---|---|---|---|---|---|
| 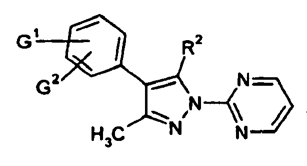 | | | | | |

| Beispielnr. | | G¹ | G² | R² | Physikalische Daten |
|---|---|---|---|---|---|
| 28- | 01 | H | H | NHCOCH₃ | |
| 28- | 02 | 2-F | H | NHCOCH₃ | |
| 28- | 03 | 3-F | H | NHCOCH₃ | |
| 28- | 04 | 4-F | H | NHCOCH₃ | |
| 28- | 05 | 2-Cl | H | NHCOCH₃ | |
| 28- | 06 | 3-Cl | H | NHCOCH₃ | |
| 28- | 07 | 4-Cl | H | NHCOCH₃ | NMR siehe unten |
| 28- | 08 | 2-Br | H | NHCOCH₃ | |
| 28- | 09 | 3-Br | H | NHCOCH₃ | |
| 28- | 10 | 4-Br | H | NHCOCH₃ | |
| 28- | 11 | 2-I | H | NHCOCH₃ | |
| 28- | 12 | 3-I | H | NHCOCH₃ | |
| 28- | 13 | 4-I | H | NHCOCH₃ | |
| 28- | 14 | 2-Cl | 3-Cl | NHCOCH₃ | |
| 28- | 15 | 2-Cl | 4-Cl | NHCOCH₃ | |
| 28- | 16 | 2-Cl | 5-Cl | NHCOCH₃ | |
| 28- | 17 | 2-Cl | 6-Cl | NHCOCH₃ | |
| 28- | 18 | 3-Cl | 4-Cl | NHCOCH₃ | |
| 28- | 19 | 3-Cl | 5-Cl | NHCOCH₃ | |
| 28- | 20 | H | H | N(COCH₃)₂ | |
| 28- | 21 | 2-F | H | N(COCH₃)₂ | |
| 28- | 22 | 3-F | H | N(COCH₃)₂ | |
| 28- | 23 | 4-F | H | N(COCH₃)₂ | |
| 28- | 24 | 2-Cl | H | N(COCH₃)₂ | |
| 28- | 25 | 3-Cl | H | N(COCH₃)₂ | |
| 28- | 26 | 4-Cl | H | N(COCH₃)₂ | |
| 28- | 27 | 2-Br | H | N(COCH₃)₂ | |
| 28- | 28 | 3-Br | H | N(COCH3)₂ | |
| 28- | 29 | 4-Br | H | N(COCH₃)₂ | |
| 28- | 30 | 2-I | H | N(COCH₃)₂ | |
| 28- | 31 | 3-I | H | N(COCH₃)₂ | |
| 28- | 32 | 4-I | H | N(COCH₃)₂ | |
| 28- | 33 | 2-Cl | 3-Cl | N(COCH₃)₂ | |
| 28- | 34 | 2-Cl | 4-Cl | N(COCH₃)₂ | |
| 28- | 35 | 2-Cl | 5-Cl | N(COCH₃)₂ | |
| 28- | 36 | 2-Cl | 6-Cl | N(COCH₃)₂ | |
| 28- | 37 | 3-Cl | 4-Cl | N(COCH₃)₂ | |
| 28- | 38 | 3-Cl | 5-Cl | N(COCH₃)₂ | |
| 28- | 39 | H | H | NHCOOEt | |
| 28- | 40 | 2-F | H | NHCOOEt | |
| 28- | 41 | 3-F | H | NHCOOEt | |
| 28- | 42 | 4-F | H | NHCOOEt | |
| 28- | 43 | 2-Cl | H | NHCOOEt | |
| 28- | 44 | 3-Cl | H | NHCOOEt | |
| 28- | 45 | 4-Cl | H | NHCOOEt | |
| 28- | 46 | 2-Br | H | NHCOOEt | |
| 28- | 47 | 3-Br | H | NHCOOEt | |
| 28- | 48 | 4-Br | H | NHCOOEt | |
| 28- | 49 | 2-I | H | NHCOOEt | |
| 28- | 50 | 3-I | H | NHCOOEt | |
| 28- | 51 | 4-I | H | NHCOOEt | |
| 28- | 52 | 2-Cl | 3-Cl | NHCOOEt | |
| 28- | 53 | 2-Cl | 4-Cl | NHCOOEt | |
| 28- | 54 | 2-Cl | 5-Cl | NHCOOEt | |
| 28- | 55 | 2-Cl | 6-Cl | NHCOOEt | |
| 28- | 56 | 3-Cl | 4-Cl | NHCOOEt | |
| 28- | 57 | 3-Cl | 5-Cl | NHCOOEt | |

**Tabelle 29**

| | | | | | | |
|---|---|---|---|---|---|---|
| 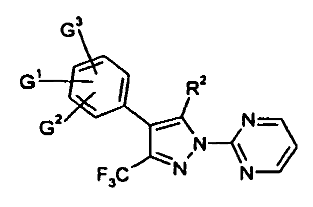 | | | | | | |

| Beispielnr. | | G¹ | G² | G³ | R² | Physikalische Daten |
|---|---|---|---|---|---|---|
| 29- | 01 | H | H | H | NHCOCH₃ | |
| 29- | 02 | H | 2-F | H | NHCOCH₃ | |
| 29- | 03 | H | 3-F | H | NHCOCH₃ | |
| 29- | 04 | H | 4-F | H | NHCOCH₃ | |
| 29- | 05 | H | 2-Cl | H | NHCOCH₃ | |
| 29- | 06 | H | 3-Cl | H | NHCOCH₃ | |
| 29- | 07 | H | 4-Cl | H | NHCOCH₃ | NMR siehe unten |
| 29- | 08 | H | 2-Br | H | NHCOCH₃ | |
| 29- | 09 | H | 3-Br | H | NHCOCH₃ | |
| 29- | 10 | H | 4-Br | H | NHCOCH₃ | |
| 29- | 11 | H | 2-I | H | NHCOCH₃ | |
| 29- | 12 | H | 3-I | H | NHCOCH₃ | NMR siehe unten |
| 29- | 13 | H | 4-I | H | NHCOCH₃ | |
| 29- | 14 | 3-Cl | 2-Cl | H | NHCOCH₃ | |
| 29- | 15 | 4-Cl | 2-Cl | H | NHCOCH₃ | |
| 29- | 16 | 5-Cl | 2-Cl | H | NHCOCH₃ | |
| 29- | 17 | 6-Cl | 2-Cl | H | NHCOCH₃ | |
| 29- | 18 | 4-Cl | 3-Cl | H | NHCOCH₃ | |
| 29- | 19 | 5-Cl | 3-Cl | H | NHCOCH₃ | NMR siehe unten |
| 29- | 20 | H | H | H | N(COCH₃)₂ | |
| 29- | 21 | H | 2-F | H | N(COCH₃)₂ | |
| 29- | 22 | H | 3-F | H | N(COCH₃)₂ | |
| 29- | 23 | H | 4-F | H | N(COCH₃)₂ | |
| 29- | 24 | H | 2-Cl | H | N(COCH₃)₂ | |
| 29- | 25 | H | 3-Cl | H | N(COCH₃)₂ | |
| 29- | 26 | H | 4-Cl | H | N(COCH₃)₂ | NMR siehe unten |
| 29- | 27 | H | 2-Br | H | N(COCH₃)₂ | |
| 29- | 28 | H | 3-Br | H | N(COCH₃)₂ | |
| 29- | 29 | H | 4-Br | H | N(COCH₃)₂ | 1H: 2.25ppm, Diacetyl |
| 29- | 30 | H | 2-I | H | N(COCH₃)₂ | |
| 29- | 31 | H | 3-I | H | N(COCH₃)₂ | |
| 29- | 32 | H | 4-I | H | N(COCH₃)₂ | |
| 29- | 33 | 3-Cl | 2-Cl | H | N(COCH₃)₂ | |
| 29- | 34 | 4-Cl | 2-Cl | H | N(COCH₃)₂ | |
| 29- | 35 | 5-Cl | 2-Cl | H | N(COCH₃)₂ | |
| 29- | 36 | 6-Cl | 2-Cl | H | N(COCH₃)₂ | |
| 29- | 37 | 4-Cl | 3-Cl | H | N(COCH₃)₂ | 1H: 2.27ppm, Diacetyl |
| 29- | 38 | 5-Cl | 3-Cl | H | N(COCH₃)₂ | |
| 29- | 39 | H | H | H | NHCOOMe | |
| 29- | 40 | H | 2-F | H | NHCOOMe | |
| 29- | 41 | H | 3-F | H | NHCOOMe | |
| 29- | 42 | H | 4-F | H | NHCOOMe | |
| 29- | 43 | H | 2-Cl | H | NHCOOMe | |
| 29- | 44 | H | 3-Cl | H | NHCOOMe | |
| 29- | 45 | H | 4-Cl | H | NHCOOMe | |
| 29- | 46 | H | 2-Br | H | NHCOOMe | |
| 29- | 47 | H | 3-Br | H | NHCOOMe | |
| 29- | 48 | H | 4-Br | H | NHCOOMe | |
| 29- | 49 | H | 2-I | H | NHCOOMe | |
| 29- | 50 | H | 3-I | H | NHCOOMe | |
| 29- | 51 | H | 4-I | H | NHCOOMe | |
| 29- | 52 | 3-Cl | 2-Cl | H | NHCOOMe | |
| 29- | 53 | 4-Cl | 2-Cl | H | NHCOOMe | |
| 29- | 54 | 5-Cl | 2-Cl | H | NHCOOMe | |
| 29- | 55 | 6-Cl | 2-Cl | H | NHCOOMe | |
| 29- | 56 | 4-Cl | 3-Cl | H | NHCOOMe | |
| 29- | 57 | 5-Cl | 3-Cl | H | NHCOOMe | |
| 29- | 58 | H | H | H | NHCOOEt | |
| 29- | 59 | H | 2-F | H | NHCOOEt | |
| 29- | 60 | H | 3-F | H | NHCOOEt | |
| 29- | 61 | H | 4-F | H | NHCOOEt | |
| 29- | 62 | H | 2-Cl | H | NHCOOEt | |
| 29- | 63 | H | 3-Cl | H | NHCOOEt | |
| 29- | 64 | H | 4-Cl | H | NHCOOEt | NMR siehe unten |
| 29- | 65 | H | 2-Br | H | NHCOOEt | |
| 29- | 66 | H | 3-Br | H | NHCOOEt | |
| 29- | 67 | H | 4-Br | H | NHCOOEt | |
| 29- | 68 | H | 2-I | H | NHCOOEt | |
| 29- | 69 | H | 3-I | H | NHCOOEt | |
| 29- | 70 | H | 4-I | H | NHCOOEt | |
| 29- | 71 | 3-Cl | 2-Cl | H | NHCOOEt | |
| 29- | 72 | 4-Cl | 2-Cl | H | NHCOOEt | |
| 29- | 73 | 5-Cl | 2-Cl | H | NHCOOEt | |
| 29- | 74 | 6-Cl | 2-Cl | H | NHCOOEt | |
| 29- | 75 | 4-Cl | 3-Cl | H | NHCOOEt | |
| 29- | 76 | 5-Cl | 3-Cl | H | NHCOOEt | |
| 29- | 77 | H | H | H | N(COOMe)₂ | |
| 29- | 78 | H | 2-F | H | N(COOMe)₂ | |
| 29- | 79 | H | 3-F | H | N(COOMe)₂ | |
| 29- | 80 | H | 4-F | H | N(COOMe)₂ | |
| 29- | 81 | H | 2-Cl | H | N(COOMe)₂ | |
| 29- | 82 | H | 3-Cl | H | N(COOMe)₂ | |
| 29- | 83 | H | 4-Cl | H | N(COOMe)₂ | |
| 29- | 84 | H | 2-Br | H | N(COOMe)₂ | |
| 29- | 85 | H | 3-Br | H | N(COOMe)₂ | |
| 29- | 86 | H | 4-Br | H | N(COOMe)₂ | |
| 29- | 87 | H | 2-I | H | N(COOMe)₂ | |
| 29- | 88 | H | 3-I | H | N(COOMe)₂ | |
| 29- | 89 | H | 4-I | H | N(COOMe)₂ | |
| 29- | 90 | 3-Cl | 2-Cl | H | N(COOMe)₂ | |
| 29- | 91 | 4-Cl | 2-Cl | H | N(COOMe)₂ | |
| 29- | 92 | 5-Cl | 2-Cl | H | N(COOMe)₂ | |
| 29- | 93 | 6-Cl | 2-Cl | H | N(COOMe)₂ | |
| 29- | 94 | 4-Cl | 3-Cl | H | N(COOMe)₂ | |
| 29- | 95 | 5-Cl | 3-Cl | H | N(COOMe)₂ | |
| 29- | 96 | H | H | H | N(COOEt)₂ | |
| 29- | 97 | H | 2-F | H | N(COOEt)₂ | |
| 29- | 98 | H | 3-F | H | N(COOEt)₂ | |
| 29- | 99 | H | 4-F | H | N(COOEt)₂ | |
| 29- | 100 | H | 2-Cl | H | N(COOEt)₂ | |
| 29- | 101 | H | 3-Cl | H | N(COOEt)₂ | |
| 29- | 102 | H | 4-Cl | H | N(COOEt)₂ | |
| 29- | 103 | H | 2-Br | H | N(COOEt)₂ | |
| 29- | 104 | H | 3-Br | H | N(COOEt)₂ | |
| 29- | 105 | H | 4-Br | H | N(COOEt)₂ | |
| 29- | 106 | H | 2-I | H | N(COOEt)₂ | |
| 29- | 107 | H | 3-I | H | N(COOEt)₂ | |
| 29- | 108 | H | 4-I | H | N(COOEt)₂ | |
| 29- | 109 | 3-Cl | 2-Cl | H | N(COOEt)₂ | |
| 29- | 110 | 4-Cl | 2-Cl | H | N(COOEt)₂ | |
| 29- | 111 | 5-Cl | 2-Cl | H | N(COOEt)₂ | |
| 29- | 112 | 6-Cl | 2-Cl | H | N(COOEt)₂ | |
| 29- | 113 | 4-Cl | 3-Cl | H | N(COOEt)₂ | |
| 29- | 114 | 5-Cl | 3-Cl | H | N(COOEt)₂ | |
| 29- | 115 | H | H | H | NHCOC₂H₅ | |
| 29- | 116 | H | 2-F | H | NHCOC₂H₅ | |
| 29- | 117 | H | 3-F | H | NHCOC₂H₅ | |
| 29- | 118 | H | 4-F | H | NHCOC₂H₅ | |
| 29- | 119 | H | 2-Cl | H | NHCOC₂H₅ | |
| 29- | 120 | H | 3-Cl | H | NHCOC₂H₅ | |
| 29- | 121 | H | 4-Cl | H | NHCOC₂H₅ | |
| 29- | 122 | H | 2-Br | H | NHCOC₂H₅ | |
| 29- | 123 | H | 3-Br | H | NHCOC₂H₅ | |
| 29- | 124 | H | 4-Br | H | NHCOC₂H₅ | |
| 29- | 125 | H | 2-I | H | NHCOC₂H₅ | |
| 29- | 126 | H | 3-I | H | NHCOC₂H₅ | |
| 29- | 127 | H | 4-I | H | NHCOC₂H₅ | |
| 29- | 128 | 3-Cl | 2-Cl | H | NHCOC₂H₅ | |
| 29- | 129 | 4-Cl | 2-Cl | H | NHCOC₂H₅ | |
| 29- | 130 | 5-Cl | 2-Cl | H | NHCOC₂H₅ | |
| 29- | 131 | 6-Cl | 2-Cl | H | NHCOC₂H₅ | |
| 29- | 132 | 4-Cl | 3-Cl | H | NHCOC₂H₅ | |
| 29- | 133 | 5-Cl | 3-Cl | H | NHCOC₂H₅ | |
| 29- | 134 | H | H | H | N(COC₂H₅)₂ | |
| 29- | 135 | H | 2-F | H | N(COC₂H₅)₂ | |
| 29- | 136 | H | 3-F | H | N(COC₂H₅)₂ | |
| 29- | 137 | H | 4-F | H | N(COC₂H₅)₂ | |
| 29- | 138 | H | 2-Cl | H | N(COC₂H₅)₂ | |
| 29- | 139 | H | 3-Cl | H | N(COC₂H₅)₂ | |
| 29- | 140 | H | 4-Cl | H | N(COC₂H₅)₂ | |
| 29- | 141 | H | 2-Br | H | N(COC₂H₅)₂ | |
| 29- | 142 | H | 3-Br | H | N(COC₂H₅)₂ | |
| 29- | 143 | H | 4-Br | H | N(COC₂H₅)₂ | 1H: 1.02, 2.53ppm, COCH₂CH₃ |
| 29- | 144 | H | 2-I | H | N(COC₇H₅)₂ | |
| 29- | 145 | H | 3-I | H | N(COC₂H₅)₂ | |
| 29- | 146 | H | 4-I | H | N(COC₂H₅)₂ | |
| 29- | 147 | 3-Cl | 2-Cl | H | N(COC₂H₅)₂ | |
| 29- | 148 | 4-Cl | 2-Cl | H | N(COC₂H₅)₂ | |
| 29- | 149 | 5-Cl | 2-Cl | H | N(COC₂H₅)₂ | |
| 29- | 150 | 6-Cl | 2-Cl | H | N(COC₂H₅)₂ | |
| 29- | 151 | 4-Cl | 3-Cl | H | N(COC₂H₅)₂ | |
| 29- | 152 | 5-Cl | 3-Cl | H | N(COC₂H₅)₂ | |
| 29- | 153 | 4-OMe | 3-F | 5-F | NHCOCH₃ | NMR siehe unten |
| 29- | 154 | 4-Me | 3-OMe | H | NHCOCH₃ | NMR siehe unten |
| 29- | 155 | 4-Cl | 3-OCF₃ | H | NHCOCH₃ | NMR siehe unten |
| 29- | 156 | H | 4-OCHF₂ | H | NHCOCH₃ | NMR siehe unten |
| 29- | 157 | 4-F | 3-Cl | H | NHCOCH₃ | NMR siehe unten |
| 29- | 158 | 4-OMe | 3-F | 5-F | N(COCH₃)₂ | NMR siehe unten |
| 29- | 159 | 4-Me | 3-OMe | H | N(COCH₃)₂ | NMR siehe unten |
| 29- | 160 | 4-Cl | 3-OCF₃ | H | N(COCH₃)₂ | NMR siehe unten |
| 29- | 161 | H | 4-OCHF₂ | H | N(COCH₃)₂ | NMR siehe unten |
| 29- | 162 | 4-F | 3-Cl | H | N(COCH₃)₂ | NMR siehe unten |
| 29- | 163 | 3-CF₃ | 5-CF₃ | | NHCOCH₃ | NMR siehe unten |
| 29- | 164 | 3-Cl | 5-CF₃ | | NHCOCH₃ | NMR siehe unten |
| 29- | 165 | 3-Cl | 5-CF₃ | | NHCO₂CH₂CCH | NMR siehe unten |
| 29- | 166 | 3-Cl | 5-CF₃ | | N(CO₂CH₂CCH)₂ | NMR siehe unten |
| 29- | 167 | 3-CF₃ | 5-CF₃ | | N(CO₂CH₂CCH)₂ | NMR siehe unten |

**Tabelle 30**

| | | | | | |
|---|---|---|---|---|---|
| 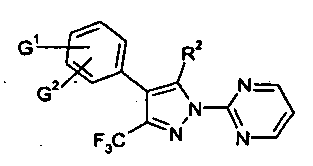 | | | | | |

| Beispielnr. | | G¹ | G² | R² | Physikalische Daten |
|---|---|---|---|---|---|
| 30- | 01 | H | H | NH-CO-COOMe | |
| 30- | 02 | 2-F | H | NH-CO-COOMe | |
| 30- | 03 | 3-F | H | NH-CO-COOMe | |
| 30- | 04 | 4-F | H | NH-CO-COOMe | |
| 30- | 05 | 2-Cl | H | NH-CO-COOMe | |
| 30- | 06 | 3-Cl | H | NH-CO-COOMe | |
| 30- | 07 | 4-Cl | H | NH-CO-COOMe | |
| 30- | 08 | 2-Br | H | NH-CO-COOMe | |
| 30- | 09 | 3-Br | H | NH-CO-COOMe | |
| 30- | 10 | 4-Br | H | NH-CO-COOMe | 1H: 3.90ppm, COOMe |
| 30- | 11 | 2-I | H | NH-CO-COOMe | |
| 30- | 12 | 3-I | H | NH-CO-COOMe | |
| 30- | 13 | 4-I | H | NH-CO-COOMe | |
| 30- | 14 | 2-Cl | 3-Cl | NH-CO-COOMe | |
| 30- | 15 | 2-Cl | 4-Cl | NH-CO-COOMe | |
| 30- | 16 | 2-Cl | 5-Cl | NH-CO-COOMe | |
| 30- | 17 | 2-Cl | 6-Cl | NH-CO-COOMe | |
| 30- | 18 | 3-Cl | 4-Cl | NH-CO-COOMe | |
| 30- | 19 | 3-Cl | 5-Cl | NH-CO-COOMe | |
| 30- | 20 | H | H | NH-CO-COOEt | |
| 30- | 21 | 2-F | H | NH-CO-COOEt | |
| 30- | 22 | 3-F | H | NH-CO-COOEt | |
| 30- | 23 | 4-F | H | NH-CO-COOEt | |
| 30- | 24 | 2-Cl | H | NH-CO-COOEt | |
| 30- | 25 | 3-Cl | H | NH-CO-COOEt | |
| 30- | 26 | 4-Cl | H | NH-CO-COOEt | |
| 30- | 27 | 2-Br | H | NH-CO-COOEt | |
| 30- | 28 | 3-Br | H | NH-CO-COOEt | |
| 30- | 29 | 4-Br | H | NH-CO-COOEt | |
| 30- | 30 | 2-I | H | NH-CO-COOEt | |
| 30- | 31 | 3-I | H | NH-CO-COOEt | |
| 30- | 32 | 4-I | H | NH-CO-COOEt | |
| 30- | 33 | 2-Cl | 3-Cl | NH-CO-COOEt | |
| 30- | 34 | 2-Cl | 4-Cl | NH-CO-COOEt | |
| 30- | 35 | 2-Cl | 5-Cl | NH-CO-COOEt | |
| 30- | 36 | 2-Cl | 6-Cl | NH-CO-COOEt | |
| 30- | 37 | 3-Cl | 4-Cl | NH-CO-COOEt | |
| 30- | 38 | 3-Cl | 5-Cl | NH-CO-COOEt | |

**Tabelle 31**

| | | | | | |
|---|---|---|---|---|---|
| 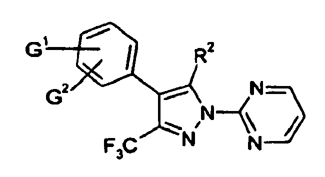 | | | | | |

| Beispielnr. | | G¹ | G² | R² | Physikalische Daten |
|---|---|---|---|---|---|
| 31- | 01 | H | H | NHCO-Ph | |
| 31- | 02 | 2-F | H | NHCO-Ph | |
| 31- | 03 | 3-F | H | NHCO-Ph | |
| 31- | 04 | 4-F | H | NHCO-Ph | |
| 31- | 05 | 2-Cl | H | NHCO-Ph | |
| 31- | 06 | 3-Cl | H | NHCO-Ph | |
| 31- | 07 | 4-Cl | H | NHCO-Ph | |
| 31- | 08 | 2-Br | H | NHCO-Ph | |
| 31- | 09 | 3-Br | H | NHCO-Ph | |
| 31- | 10 | 4-Br | H | NHCO-Ph | |
| 31- | 11 | 2-I | H | NHCO-Ph | |
| 31- | 12 | 3-I | H | NHCO-Ph | |
| 31- | 13 | 4-I | H | NHCO-Ph | |
| 31- | 14 | 2-Cl | 3-Cl | NHCO-Ph | |
| 31- | 15 | 2-Cl | 4-Cl | NHCO-Ph | |
| 31- | 16 | 2-Cl | 5-Cl | NHCO-Ph | |
| 31- | 17 | 2-Cl | 6-Cl | NHCO-Ph | |
| 31- | 18 | 3-Cl | 4-Cl | NHCO-Ph | |
| 31- | 19 | 3-Cl | 5-Cl | NHCO-Ph | |
| 31- | 20 | H | H | NHCO-2-Cl-C₆H₄ | |
| 31- | 21 | 2-F | H | NHCO-2-Cl-C₆H₄ | |
| 31- | 22 | 3-F | H | NHCO-2-Cl-C₆H₄ | |
| 31- | 23 | 4-F | H | NHCO-2-Cl-C₆H₄ | |
| 31- | 24 | 2-Cl | H | NHCO-2-Cl-C₆H₄ | |
| 31- | 25 | 3-Cl | H | NHCO-2-Cl-C₆H₄ | |
| 31- | 26 | 4-Cl | H | NHCO-2-Cl-C₆H₄ | |
| 31- | 27 | 2-Br | H | NHCO-2-Cl-C₆H₄ | |
| 31- | 28 | 3-Br | H | NHCO-2-Cl-C₆H₄ | |
| 31- | 29 | 4-Br | H | NHCO-2-Cl-C₆H₄ | 19F: -61.25ppm, CF₃. |
| 31- | 30 | 2-I | H | NHCO-2-Cl-C₆H₄ | |
| 31- | 31 | 3-I | H | NHCO-2-Cl-C₆H₄ | |
| 31- | 32 | 4-I | H | NHCO-2-Cl-C₆H₄ | |
| 31- | 33 | 2-Cl | 3-Cl | NHCO-2-Cl-C₆H₄ | |
| 31- | 34 | 2-Cl | 4-Cl | NHCO-2-Cl-C₆H₄ | |
| 31- | 35 | 2-Cl | 5-Cl | NHCO-2-Cl-C₆H₄ | |
| 31- | 36 | 2-Cl | 6-Cl | NHCO-2-Cl-C₆H₄ | |
| 31- | 37 | 3-Cl | 4-Cl | NHCO-2-Cl-C₆H₄ | |
| 31- | 38 | 3-Cl | 5-Cl | NHCO-2-Cl-C₆H₄ | |
| 31- | 39 | H | H | NHCO-3-Cl-C₆H₄ | |
| 31- | 40 | 2-F | H | N-HCO-3-Cl-C₆H₄ | |
| 31- | 41 | 3-F | H | NHCO-3-Cl-C₆H₄ | |
| 31- | 42 | 4-F | H | NHCO-3-Cl-C₆H₄ | |
| 31- | 43 | 2-Cl | H | NHCO-3-Cl-C₆H₄ | |
| 31- | 44 | 3-Cl | H | NHCO-3-Cl-C₆H₄ | |
| 31- | 45 | 4-Cl | H | NHCO-3-Cl-C₆H₄ | |
| 31- | 46 | 2-Br | H | NHCO-3-Cl-C₆H₄ | |
| 31- | 47 | 3-Br | H | NHCO-3-Cl-C₆H₄ | |
| 31- | 48 | 4-Br | H | NHCO-3-Cl-C₆H₄ | |
| 31- | 49 | 2-I | H | NHCO-3-Cl-C₆H₄ | |
| 31- | 50 | 3-I | H | NHCO-3-Cl-C₆H₄ | |
| 31- | 51 | 4-I | H | NHCO-3-Cl-C₆H₄ | |
| 31- | 52 | 2-Cl | 3-Cl | NHCO-3-Cl-C₆H₄ | |
| 31- | 53 | 2-Cl | 4-Cl | NHCO-3-Cl-C₆H₄ | |
| 31- | 54 | 2-Cl | 5-Cl | NHCO-3-Cl-C₆H₄ | |
| 31- | 55 | 2-Cl | 6-Cl | NHCO-3-Cl-C₆H₄ | |
| 31- | 56 | 3-Cl | 4-Cl | NHCO-3-Cl-C₆H₄ | |
| 32- | 57 | 3-Cl | 5-Cl | NHCO-3-Cl-C₆H₄ | |
| 31- | 58 | H | H | NHCO-4-Cl-C₆H₄ | |
| 31- | 59 | 2-F | H | NHCO-2-F-C₆H₄ | |
| 31- | 60 | 3-F | H | NHCO-2-F-C₆H₄ | |
| 31- | 61 | 4-F | H | NHCO-2-F-C₆H₄ | |
| 31- | 62 | 2-Cl | H | NHCO-2-F-C₆H₄ | |
| 31- | 63 | 3-Cl | H | NHCO-2-F-C₆H₄ | |
| 31- | 64 | 4-Cl | H | NHCO-2-F-C₆H₄ | |
| 31- | 65 | 2-Br | H | NHCO-2-F-C₆H₄ | |
| 31- | 66 | 3-Br | H | NHCO-2-F-C₆H₄ | |
| 31- | 67 | 4-Br | H | NHCO-2-F-C₆H₄ | |
| 31- | 68 | 2-I | H | NHCO-2-F-C₆H₄ | |
| 31- | 69 | 3-I | H | NHCO-2-F-C₆H₄ | |
| 31- | 70 | 4-I | H | NHCO-2-F-C₆H₄ | |
| 31- | 71 | 2-Cl | 3-Cl | NHCO-2-F-C₆H₄ | |
| 31- | 72 | 2-Cl | 4-Cl | NHCO-2-F-C₆H₄ | |
| 31- | 73 | 2-Cl | 5-Cl | NHCO-2-F-C₆H₄ | |
| 31- | 74 | 2-Cl | 6-Cl | NHCO-2-F-C₆H₄ | |
| 31- | 75 | 3-Cl | 4-Cl | NHCO-2-F-C₆H₄ | |
| 31- | 76 | 3-Cl | 5-Cl | NHCO-2-F-C₆H₄ | |
| 31- | 77 | H | H | NHCO-3-F-C₆H₄ | |
| 31- | 78 | 2-F | H | NHCO-3-F-C₆H₄ | |
| 31- | 79 | 3-F | H | NHCO-3-F-C₆H₄ | |
| 31- | 80 | 4-F | H | NHCO-3-F-C₆H₄ | |
| 31- | 81 | 2-Cl | H | NHCO-3-F-C₆H₄ | |
| 31- | 82 | 3-Cl | H | NHCO-3-F-C₆H₄ | |
| 31- | 83 | 4-Cl | H | NHCO-3-F-C₆H₄ | |
| 31- | 84 | 2-Br | H | NHCO-3-F-C₆H₄ | |
| 31- | 85 | 3-Br | H | NHCO-3-F-C₆H₄ | |
| 31- | 86 | 4-Br | H | NHCO-3-F-C₆H₄ | |
| 31- | 87 | 2-I | H | NHCO-3-F-C₆H₄ | |
| 31- | 88 | 3-I | H | NHCO-3-F-C₆H₄ | |
| 31- | 89 | 4-I | H | NHCO-3-F-C₆H₄ | |
| 31- | 90 | 2-Cl | 3-Cl | NHCO-3-F-C₆H₄ | |
| 31- | 91 | 2-Cl | 4-Cl | NHCO-3-F-C₆H₄ | |
| 31- | 92 | 2-Cl | 5-Cl | NHCO-3-F-C₆H₄ | |
| 31- | 93 | 2-Cl | 6-Cl | NHCO-3-F-C₆H₄ | |
| 31- | 94 | 3-Cl | 4-Cl | NHCO-3-F-C₆H₄ | |
| 31- | 95 | 3-Cl | 5-Cl | NHCO-3-F-C₆H₄ | |
| 31- | 96 | H | H | NHCO-4-F-C₆H₄ | |
| 31- | 97 | 2-F | H | NHCO-4-F-C₆H₄ | |
| 31- | 98 | 3-F | H | NHCO-4-F-C₆H₄ | |
| 31- | 99 | 4-F | H | NHCO-4-F-C₆H₄ | |
| 31- | 100 | 2-Cl | H | NHCO-4-F-C₆H₄ | |
| 31- | 101 | 3-Cl | H | NHCO-4-F-C₆H₄ | |
| 31- | 102 | 4-Cl | H | NHCO-4-F-C₆H₄ | |
| 31- | 103 | 2-Br | H | NHCO-4-F-C₆H₄ | |
| 31- | 104 | 3-Br | H | NHCO-4-F-C₆H₄ | |
| 31- | 105 | 4-Br | H | NHCO-4-F-C₆H₄ | |
| 31- | 106 | 2-I | H | NHCO-4-F-C₆H₄ | |
| 31- | 107 | 3-I | H | NHCO-4-F-C₆H₄ | |
| 31- | 108 | 4-I | H | NHCO-4-F-C₆H₄ | |
| 31- | 109 | 2-Cl | 3-Cl | NHCO-4-F-C₆H₄ | |
| 31- | 110 | 2-Cl | 4-Cl | NHCO-4-F-C₆H₄ | |
| 31- | 111 | 2-Cl | 5-Cl | NHCO-4-F-C₆H₄ | |
| 31- | 112 | 2-Cl | 6-Cl | NHCO-4-F-C₆H₄ | |
| 31- | 113 | 3-Cl | 4-Cl | NHCO-4-F-C₆H₄ | |
| 31- | 114 | 3-Cl | 5-Cl | NHCO-4-F-C₆H₄ | |

**Tabelle 32**

| | | | | |
|---|---|---|---|---|
| 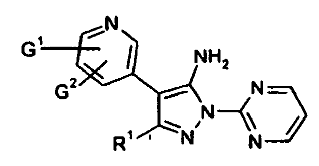 | | | | |

| Beispielnr | | . R¹ | G¹ | Physikalische Daten |
|---|---|---|---|---|
| 32- | 01 | CF₃ | H | |
| 32- | 02 | CF₃ | 2-Cl | |
| 32- | 03 | CF₃ | 6-Cl | NMR siehe unten |
| 32- | 04 | CF₃ | 5-Cl | |
| 32- | 05 | CF₃ | 4-Cl | |
| 32- | 06 | CF₃ | 5-F | |
| 32- | 07 | CF₃ | 5-Br | |
| 32- | 08 | CF₃ | 5-I | |
| 32- | 09 | CF₃ | 2-OMe | |
| 32- | 10 | CF₃ | 4-OMe | |
| 32- | 11 | CF₃ | 5-OMe | |
| 32- | 12 | CF₃ | 6-OMe | NMR siehe unten |
| 32- | 13 | CF₃ | 2-OEt | |
| 32- | 14 | CF₃ | 4-OEt | |
| 32- | 15 | CF₃ | 5-OEt | |
| 32- | 16 | CF₃ | 6-OEt | |
| 32- | 17 | CF₃ | 6-CF₃ | NMR siehe unten |
| 32- | 18 | Me | H | |
| 32- | 19 | Me | 2-Cl | |
| 32- | 20 | Me | 6-Cl | |
| 32- | 21 | Me | 5-Cl | |
| 32- | 22 | Me | 4-Cl | |
| 32- | 23 | Me | 5-F | |
| 32- | 24 | Me | 5-Br | |
| 32- | 25 | Me | 5-I | |
| 32- | 26 | Me | 2-OMe | |
| 32- | 27 | Me | 4-OMe | |
| 32- | 28 | Me | 5-OMe | |
| 32- | 29 | Me | 6-OMe | |
| 32- | 30 | Me | 2-OEt | |
| 32- | 31 | Me | 4-OEt | |
| 32- | 32 | Me | 5-OEt | |
| 32- | 33 | Me | 6-OEt | |
| 32- | 34 | Me | 6-CF₃ | |
| 32- | 35 | Et | H | |
| 32- | 36 | Et | 2-Cl | |
| 32- | 37 | Et | 6-Cl | IgP = 1,76 |
| 32- | 38 | Et | 5-Cl | |
| 32- | 39 | Et | 4-Cl | |
| 32- | 40 | Et | 5-F | |
| 32- | 41 | Et | 5-Br | |
| 32- | 42 | Et | 5-I | |
| 32- | 43 | Et | 2-OMe | |
| 32- | 44 | Et | 4-OMe | |
| 32- | 45 | Et | 5-OMe | |
| 32- | 46 | Et | 6-OMe | |
| 32- | 47 | Et | 2-OEt | |
| 32- | 48 | Et | 4-OEt | |
| 32- | 49 | Et | 5-OEt | |
| 32- | 50 | Et | 6-OEt | |
| 32- | 51 | Et | 6-CF₃ | |
| 32- | 52 | CF₃ | 6-NMe₂ | NMR siehe unten |
| 32- | 53 | CF₃ | 6-OiPr | NMR siehe unten |
| 32- | 54 | CF₃ | G1 = 6-OMe G2 = 5-Cl | NMR siehe unten |

**Tabelle 33**

| | | | | | |
|---|---|---|---|---|---|
| 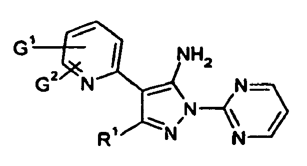 | | | | | |

| Beispielnr. | | R¹ | G¹ | G² | Physikalische Daten |
|---|---|---|---|---|---|
| 33- | 01 | CF₃ | H | H | |
| 33- | 02 | CF₃ | 3-Cl | H | |
| 33- | 03 | CF₃ | 4-Cl | H | |
| 33- | 04 | CF₃ | 5-Cl | H | |
| 33- | 05 | CF₃ | 6-Cl | H | |
| 33- | 06 | CF₃ | 5-F | H | |
| 33- | 07 | CF₃ | 5-Br | H | |
| 33- | 08 | CF₃ | 5-I | H | |
| 33- | 09 | CF₃ | 3-OMe | H | |
| 33- | 10 | CF₃ | 4-OMe | H | |
| 33- | 11 | CF₃ | 5-OMe | H | |
| 33- | 12 | CF₃ | 6-OMe | H | |
| 33- | 13 | CF₃ | 3-OEt | H | |
| 33- | 14 | CF₃ | 4-OEt | H | |
| 33- | 15 | CF₃ | 5-OEt | H | |
| 33- | 16 | CF₃ | 6-OEt | H | |
| 33- | 17 | CF₃ | 3-CF₃ | H | |
| 33- | 18 | CF₃ | 4-CF₃ | H | |
| 33- | 19 | CF₃ | 5-CF₃ | H | |
| 33- | 20 | CF₃ | 6-CF₃ | H | |
| 33- | 21 | CF₃ | 3-Cl | 5-Cl | |
| 33- | 22 | CH₃ | H | H | |
| 33- | 23 | CH₃ | 3-Cl | H | |
| 33- | 24 | CH₃ | 4-Cl | H | |
| 33- | 25 | CH₃ | 5-Cl | H | |
| 33- | 26 | CH₃ | 6-Cl | H | |
| 33- | 27 | CH₃ | 5-F | H | |
| 33- | 28 | CH₃ | 5-Br | H | |
| 33- | 29 | CH₃ | 5-I | H | |
| 33- | 30 | CH₃ | 3-OMe | H | |
| 33- | 31 | CH₃ | 4-OMe | H | |
| 33- | 32 | CH₃ | 5-OMe | H | |
| 33- | 33 | CH₃ | 6-OMe | H | |
| 33- | 34 | CH₃ | 3-OEt | H | |
| 33- | 35 | CH₃ | 4-OEt | H | |
| 33- | 36 | CH₃ | 5-OEt | H | |
| 33- | 37 | CH₃ | 6-OEt | H | |
| 33- | 38 | CH₃ | 3-CF₃ | H | |
| 33- | 39 | CH₃ | 4-CF₃ | H | |
| 33- | 40 | CH₃ | 5-CF₃ | H | |
| 33- | 41 | CH₃ | 6-CF₃ | H | |
| 33- | 42 | CH₃ | 3-Cl | 5-Cl | |
| 33- | 43 | C₂H₅ | H | H | |
| 33- | 44 | C₂H₅ | 3-Cl | H | |
| 33- | 45 | C₂H₅ | 4-Cl | H | |
| 33- | 46 | C₂H₅ | 5-Cl | H | |
| 33- | 47 | C₂H₅ | 6-Cl | H | |
| 33- | 48 | C₂H₅ | 5-F | H | |
| 33- | 49 | C₂H₅ | 5-Br | H | |
| 33- | 50 | C₂H₅ | 5-I | H | |
| 33- | 51 | C₂H₅ | 3-OMe | H | |
| 33- | 52 | C₂H₅ | 4-OMe | H | |
| 33- | 53 | C₂H₅ | 5-OMe | H | |
| 33- | 54 | C₂H₅ | 6-OMe | H | |
| 33- | 55 | C₂H₅ | 3-OEt | H | |
| 33- | 56 | C₂H₅ | 4-OEt | H | |
| 33- | 57 | C₂H₅ | 5-OEt | H | |
| 33- | 58 | C₂H₅ | 6-OEt | H | |
| 33- | 59 | C₂H₅ | 3-CF₃ | H | |
| 33- | 60 | C₂H₅ | 4-CF₃ | H | |
| 33- | 61 | C₂H₅ | 5-CF₃ | H | |
| 33- | 62 | C₂H₅ | 6-CF₃ | H | |
| 33- | 63 | C₂H₅ | 3-Cl | 5-Cl | |

**Tabelle 34**

| | | | |
|---|---|---|---|
| 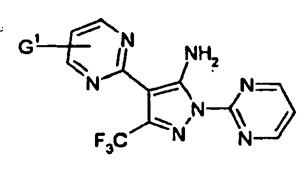 | | | |

| Beispielnr. | | G¹ | Physikalische Daten |
|---|---|---|---|
| 34- | 01 | H | |
| 34- | 02 | 5-F | |
| 34- | 03 | 5-Cl | |
| 34- | 04 | 5-Br | |
| 34- | 05 | 5-I | |
| 34- | 06 | 5-CF₃ | |

**Tabelle 35**

| | | | | | |
|---|---|---|---|---|---|
| 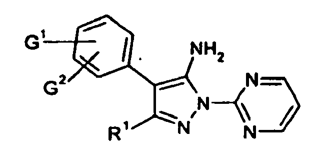 | | | | | |

| Beispielnr. | | G¹ | G² | R¹ | Physikalische Daten |
|---|---|---|---|---|---|
| 35- | 01 | H | H | Cyclopropyl | |
| 35- | 02 | 2-F | H | Cyclopropyl | |
| 35- | 03 | 3-F | H | Cyclopropyl | |
| 35- | 04 | 4-F | H | Cyclopropyl | |
| 35- | 05 | 2-Cl | H | Cyclopropyl | |
| 35- | 06 | 3-Cl | H | Cyclopropyl | |
| 35- | 07 | 4-Cl | H | Cyclopropyl | logo 2,66; ¹H-NMR (CD₃CN): 8.74 (d, 2H), 7.22 (t, 1H) |
| 35- | 08 | 2-Br | H | Cyclopropyl | |
| 35- | 09 | 3-Br | H | Cyclopropyl | |
| 35- | 10 | 4-Br | H | Cyclopropyl | |
| 35- | 11 | 2-I | H | Cyclopropyl | |
| 35- | 12 | 3-I | H | Cyclopropyl | |
| 35- | 13 | 4-I | H | Cyclopropyl | |
| 35- | 14 | 2-Cl | 3-Cl | Cyclopropyl | |
| 35- | 15 | 2-Cl | 4-Cl | Cyclopropyl | |
| 35- | 16 | 2-Cl | 5-Cl | Cyclopropyl | |
| 35- | 17 | 2-Cl | 6-Cl | Cyclopropyl | |
| 35- | 18 | 2-Cl | 4-F | Cyclopropyl | |
| 35- | 19 | 3-Cl | 4-Cl | Cyclopropyl | |
| 35- | 20 | 3-Cl | 5-Cl | Cyclopropyl | NMR siehe unten |
| 35- | 21 | H | H | 1 -Cl-Cyclopropyl | |
| 35- | 22 | 2-F | H | 1-Cl-Cyclopropyl | |
| 35- | 23 | 3-F | H | 1-Cl-Cyclopropyl | |
| 35- | 24 | 4-F | H | 1-Cl-Cyclopropyl | |
| 35- | 25 | 2-Cl | H | 1-Cl-Cyclopropyl | |
| 35- | 26 | 3-Cl | H | 1-Cl-Cyclopropyl | |
| 35- | 27 | 4-Cl | H | 1-Cl-Cyclopropyl | |
| 35- | 28 | 2-Br | H | 1-Cl-Cyclopropyl | |
| 35- | 29 | 3-Br | H | 1-Cl-Cyclopropyl | |
| 35- | 30 | 4-Br | H | 1-Cl-Cyclopropyl | |
| 35- | 31 | 2-I | H | 1-Cl-Cyclopropyl | |
| 35- | 32 | 3-I | H | 1-Cl-Cyclopropyl | |
| 35- | 33 | 4-I | H | 1 -Cl-Cyclopropyl | |
| 35- | 34 | 2-Cl | 3-Cl | 1-Cl-Cyclopropyl | |
| 35- | 35 | 2-Cl | 4-Cl | 1-Cl-Cyclopropyl | |
| 35- | 36 | 2-Cl | 5-Cl | 1-Cl-Cyclopropyl | |
| 35- | 37 | 2-Cl | 6-Cl | 1-Cl-Cyclopropyl | |
| 35- | 38 | 3-Cl | 4-Cl | 1-Cl-Cyclopropyl | |
| 35- | 39 | 3-Cl | 5-Cl | 1-Cl-Cyclopropyl | NMR siehe unten |
| 35- | 40 | H | H | 1-CF₃-Cyclopropyl | |
| 35- | 41 | 2-F | H | 1-CF₃-Cyclopropyl | |
| 35- | 42 | 3-F | H | 1-CF₃-Cyclopropyl | |
| 35- | 43 | 4-F | H | 1-CF₃-Cyclopropyl | |
| 35- | 44 | 2-Cl | H | 1-CF₃-Cyclopropyl | |
| 35- | 45 | 3-Cl | H | 1-CF₃-Cyclopropyl | |
| 35- | 46 | 4-Cl | H | 1-CF₃-Cyclopropyl | |
| 35- | 47 | 2-Br | H | 1-CF₃-Cyclopropyl | |
| 35- | 48 | 3-Br | H | 1-CF₃-Cyclopropyl | |
| 35- | 49 | 4-Br | H | 1-CF₃-Cyclopropyl | |
| 35- | 50 | 2-I | H | 1-CF₃-Cyclopropyl | |
| 35- | 51 | 3-I | H | 1-CF₃-Cyclopropyl | |
| 35- | 52 | 4-I | H | 1-CF₃-Cyclopropyl | |
| 35- | 53 | 2-Cl | 3-Cl | 1-CF₃-Cyclopropyl | |
| 35- | 54 | 2-Cl | 4-Cl | 1-CF₃-Cyclopropyl | |
| 35- | 55 | 2-Cl | 5-Cl | 1-CF₃-Cyclopropyl | |
| 35- | 56 | 2-Cl | 6-Cl | 1-CF₃-Cyclopropyl | |
| 35- | 57 | 3-Cl | 4-Cl | 1-CF₃-Cyclopropyl | |
| 35- | 58 | 3-Cl | 5-Cl | 1-CF₃-Cyclopropyl | |
| 35- | 59 | H | H | Cyclobutyl | |
| 35- | 60 | 2-F | H | Cyclobutyl | |
| 35- | 61 | 3-F | H | Cyclobutyl | |
| 35- | 62 | 4-F | H | Cyclobutyl | |
| 35- | 63 | 2-Cl | H | Cyclobutyl | |
| 35- | 64 | 3-Cl | H | Cyclobutyl | |
| 35- | 65 | 4-Cl | H | Cyclobutyl | |
| 35- | 66 | 2-Br | H | Cyclobutyl | |
| 35- | 67 | 3-Br | H | Cyclobutyl | |
| 35- | 68 | 4-Br | H | Cyclobutyl | |
| 35- | 69 | 2-I | H | Cyclobutyl | |
| 35- | 70 | 3-I | H | Cyclobutyl | |
| 35- | 71 | 4-I | H | Cyclobutyl | |
| 35- | 72 | 2-Cl | 3-Cl | Cyclobutyl | |
| 35- | 73 | 2-Cl | 4-Cl | Cyclobutyl | |
| 35- | 74 | 2-Cl | 5-Cl | Cyclobutyl | |
| 35- | 75 | 2-Cl | 6-Cl | Cyclobutyl | |
| 35- | 76 | 3-Cl | 4-Cl | Cyclobutyl | |
| 35- | 77 | 3-Cl | 5-Cl | Cyclobutyl | NMR siehe unten |
| 35- | 78 | H | H | Cyclopentyl | |
| 35- | 79 | 2-F | H | Cyclopentyl | |
| 35- | 80 | 3-F | H | Cyclopentyl | |
| 35- | 81 | 4-F | H | Cyclopentyl | |
| 35- | 82 | 2-Cl | H | Cyclopentyl | |
| 35- | 83 | 3-Cl | H | Cyclopentyl | |
| 35- | 84 | 4-Cl | H | Cyclopentyl | |
| 35- | 85 | 2-Br | H | Cyclopentyl | |
| 35- | 86 | 3-Br | H | Cyclopentyl | |
| 35- | 87 | 4-Br | H | Cyclopentyl | logP 3.34 |
| 35- | 88 | 2-I | H | Cyclopentyl | |
| 35- | 89 | 3-I | H | Cyclopentyl | |
| 35- | 90 | 4-I | H | Cyclopentyl | |
| 35- | 91 | 2-Cl | 3-Cl | Cyclopentyl | |
| 35- | 92 | 2-Cl | 4-Cl | Cyclopentyl | |
| 35- | 93 | 2-Cl | 5-Cl | Cyclopentyl | |
| 35- | 94 | 2-Cl | 6-Cl | Cyclopentyl | |
| 35- | 95 | 3-Cl | 4-Cl | Cyclopentyl | |
| 35- | 96 | 3-Cl | 5-Cl | Cyclopentyl | |
| 35- | 97 | H | H | Cyclohexyl | |
| 35- | 98 | 2-F | H | Cyclohexyl | |
| 35- | 99 | 3-F | H | Cyclohexyl | |
| 35- | 100 | 4-F | H | Cyclohexyl | |
| 35- | 101 | 2-Cl | H | Cyclohexyl | |
| 35- | 102 | 3-Cl | H | Cyclohexyl | |
| 35- | 103 | 4-Cl | H | Cyclohexyl | |
| 35- | 104 | 2-Br | H | Cyclohexyl | |
| 35- | 105 | 3-Br | H | Cyclohexyl | |
| 35- | 106 | 4-Br | H | Cyclohexyl | |
| 35- | 107 | 2-I | H | Cyclohexyl | |
| 35- | 108 | 3-I | H | Cyclohexyl | |
| 35- | 109 | 4-I | H | Cyclohexyl | |
| 35- | 110 | 2-Cl | 3-Cl | Cyclohexyl | |
| 35- | 111 | 2-Cl | 4-Cl | Cyclohexyl | |
| 35- | 112 | 2-Cl | 5-Cl | Cyclohexyl | |
| 35- | 113 | 2-Cl | 6-Cl | Cyclohexyl | |
| 35- | 114 | 3-Cl | 4-Cl | Cyclohexyl | |
| 35- | 115 | 3-Cl | 5-Cl | Cyclohexyl | |
| 35- | 116 | H | H | 1-F-Cyclopropyl | |
| 35- | 117 | 2-F | H | 1-F-Cyclopropyl | |
| 35- | 118 | 3-F | H | 1-F-Cyclopropyl | |
| 35- | 119 | 4-F | H | 1-F-Cyclopropyl | |
| 35- | 120 | 2-Cl | H | 1-F-Cyclopropyl | |
| 35- | 121 | 3-Cl | H | 1-F-Cyclopropyl | |
| 35- | 122 | 4-Cl | H | 1-F-Cyclopropyl | |
| 35- | 123 | 2-Br | H | 1-F-Cyclopropyl | |
| 35- | 124 | 3-Br | H | 1-F-Cyclopropyl | |
| 35- | 125 | 4-Br | H | 1-F-Cyclopropyl | 19F: - 175.31ppm, 1-FcPr |
| 35- | 126 | 2-I | H | 1-F-Cyclopropyl | |
| 35- | 127 | 3-I | H | 1-F-Cyclopropyl | |
| 35- | 128 | 4-I | H | 1-F-Cyclopropyl | |
| 35- | 129 | 2-Cl | 3-Cl | 1-F-Cyclopropyl | |
| 35- | 130 | 2-Cl | 4-Cl | 1-F-Cyclopropyl | |
| 35- | 131 | 2-Cl | 5-Cl | 1-F-Cyclopropyl | |
| 35- | 132 | 2-Cl | 6-Cl | 1-F-Cyclopropyl | |
| 35- | 133 | 3-Cl | 4-Cl | 1-F-Cyclopropyl | |
| 35- | 134 | 3-Cl | 5-Cl | 1-F-Cyclopropyl | NMR siehe unten |
| 35- | 135 | H | H | CH₂-Cyclopropyl | |
| 35- | 136 | 2-F | H | CH₂-Cyclopropyl | |
| 35- | 137 | 3-F | H | CH₂-Cyclopropyl | |
| 35- | 138 | 4-F | H | CH₂-Cyclopropyl | |
| 35- | 139 | 2-Cl | H | CH₂-Cyclopropyl | |
| 35- | 140 | 3-Cl | H | CH₂-Cyclopropyl | |
| 35- | 141 | 4-Cl | H | CH₂-Cyclopropyl | |
| 35- | 142 | 2-Br | H | CH₂-Cyclopropyl | |
| 35- | 143 | 3-Br | H | CH₂-Cyclopropyl | |
| 35- | 144 | 4-Br | H | CH₂-Cyclopropyl | 1H(d6DMSO): 0.84, 1H; 0.30, 2H, 0.00, 2H; Cyclopropyl |
| 35- | 145 | 2-I | H | CH₂-Cyclopropyl | |
| 35- | 146 | 3-I | H | CH₂-Cyclopropyl | |
| 35- | 147 | 4-I | H | CH₂-Cyclopropyl | |
| 35- | 148 | 2-Cl | 3-Cl | CH₂-Cyclopropyl | |
| 35- | 149 | 2-Cl | 4-Cl | CH₂-Cyclopropyl | |
| 35- | 150 | 2-Cl | 5-Cl | CH₂-Cyclopropyl | |
| 35- | 151 | 2-Cl | 6-Cl | CH₂-Cyclopropyl | |
| 35- | 152 | 3-Cl | 4-Cl | CH₂-Cyclopropyl | |
| 35- | 153 | 3-Cl | 5-Cl | CH₂-Cyclopropyl | |
| 35- | 154 | H | H | CH₂Cyclohexyl | |
| 35- | 155 | 2-F | H | CH₂Cyclohexyl | |
| 35- | 156 | 3-F | H | CH₂Cyclohexyl | |
| 35- | 157 | 4-F | H | CH₂Cyclohexyl | |
| 35- | 158 | 2-Cl | H | CH₂Cyclohexyl | |
| 35- | 159 | 3-Cl | H | CH₂Cyclohexyl | |
| 35- | 160 | 4-Cl | H | CH₂Cyclohexyl | |
| 35- | 161 | 2-Br | H | CH₂Cyclohexyl | |
| 35- | 162 | 3-Br | H | CH₂Cyclohexyl | |
| 35- | 163 | 4-Br | H | CH₂Cyclohexyl | 1H(d6DMSO):2.42ppm, CH₂ |
| 35- | 164 | 2-I | H | CH₂Cyclohexyl | |
| 35- | 165 | 3-I | H | CH₂Cyclohexyl | |
| 35- | 166 | 4-I | H | CH₂Cyclohexyl | |
| 35- | 167 | 2-Cl | 3-Cl | CH₂Cyctohexyl | |
| 35- | 168 | 2-Cl | 4-Cl | CH₂Cyclohexyl | |
| 35- | 169 | 2-Cl | 5-Cl | CH₂Cyclohexyl | |
| 35- | 170 | 2-Cl | 6-Cl | CH₂Cyclohexyl | |
| 35- | 171 | 3-Cl | 4-Cl | CH₂Cyclohexyl | |
| 35- | 172 | 3-Cl | 5-Cl | CH₂Cyclohexyl | |
| 35- | 173 | H | H | CH₂CH₂Cyclohexyl | |
| 35- | 174 | 2-F | H | CH₂CH₂Cyclohexyl | |
| 35- | 175 | 3-F | H | CH₂CH₂Cyclohexyl | |
| 35- | 176 | 4-F | H | CH₂CH₂Cyclohexyl | |
| 35- | 177 | 2-Cl | H | CH₂CH₂Cyclohexyl | |
| 35- | 178 | 3-Cl | H | CH₂CH₁Cyclohexyl | |
| 35- | 179 | 4-Cl | H | CH₂CH₂Cyclohexyl | |
| 35- | 180 | 2-Br | H | CH₂CH₂Cyclohexyl | |
| 35- | 181 | 3-Br | H | CH₂CH₂Cyclohexyl | |
| 35- | 182 | 4-Br | H | CH₂CH₂Cyclohexyl | 1H(d6DMSO): 0.82, 1.14, 1.59, Cyclohexyl |
| 35- | 183 | 2-I | H | CH₂CH₂Cyclohexyl | |
| 35- | 184 | 3-I | H | CH₂CH₂Cyclohexyl | |
| 35- | 185 | 4-I | H | CH₂CH₂Cyclohexyl | |
| 35- | 186 | 2-Cl | 3-Cl | CH₂CH₂Cyclohexyl | |
| 35- | 187 | 2-Cl | 4-Cl | CH₂CH₂Cyclohexyl | |
| 35- | 188 | 2-Cl | 5-Cl | CH₂CH₂Cyclohexyl | |
| 35- | 189 | 2-Cl | 6-Cl | CH₂CH₂Cyclohexyl | |
| 35- | 190 | 3-Cl | 4-Cl | CH₂CH₂Cyclohexyl | |
| 35- | 191 | 3-Cl | 5-Cl | CH₂CH2Cyclohexyl | |
| 35- | 192 | H | H | CH₂Cyclopentyl | |
| 35- | 193 | 2-F | H | CH₂Cyclopentyl | |
| 35- | 194 | 3-F | H | CH₂Cyclopentyl | |
| 35- | 195 | 4-F | H | CH₂Cyclopentyl | |
| 35- | 196 | 2-Cl | H | CH₂Cyclopentyl | |
| 35- | 197 | 3-Cl | H | CH₂Cyclopentyl | |
| 35- | 198 | 4-Cl | H | CH₂Cyclopentyl | |
| 35- | 199 | 2-Br | H | CH₂Cyclopentyl | |
| 35- | 200 | 3-Br | H | CH₂Cyclopentyl | |
| 35- | 201 | 4-Br | H | CH₂Cyclopentyl | |
| 35- | 202 | 2-I | H | CH₂Cyclopentyl | |
| 35- | 203 | 3-I | H | CH₂Cyclopentyl | |
| 35- | 204 | 4-I | H | CH₂Cyclopentyl | |
| 35- | 205 | 2-Cl | 3-Cl | CH₂Cyclopentyl | |
| 35- | 206 | 2-Cl | 4-Cl | CH₂Cyclopentyl | |
| 35- | 207 | 2-Cl | 5-Cl | CH₂Cyclopentyl | |
| 35- | 208 | 2-Cl | 6-Cl | CH₂Cyclopentyl | |
| 35- | 209 | 3-Cl | 4-Cl | CH₂Cyclopentyl | |
| 35- | 210 | 3-Cl | 5-Cl | CH₂Cyclopentyl | |

**Tabelle 36**

| | | | | | |
|---|---|---|---|---|---|
| 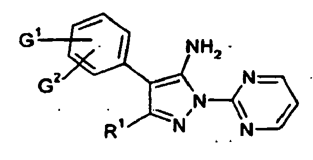 | | | | | |

| Beispielnr. | | G¹ | G² | R¹ | |
|---|---|---|---|---|---|
| 36- | 01 | H | H | CH(OMe)₂ | |
| 36- | 02 | 2-F | H | CH(OMe)₂ | |
| 36- | 03 | 3-F | H | CH(OMe)₂ | |
| 36- | 04 | 4-F | H | CH(OMe)₂ | |
| 36- | 05 | 2-Cl | H | CH(OMe)₂ | |
| 36- | 06 | 3-Cl | H | CH(OMe)₂ | |
| 36- | 07 | 4-Cl | H | CH(OMe)₂ | |
| 36- | 08 | 2-Br | H | CH(OMe)₂ | |
| 36- | 09 | 3-Br | H | CH(OMe)₂ | |
| 36- | 10 | 4-Br | H | CH(OMe)₂ | 1H: 5.22ppm, CH(OMe)₂ |
| 36- | 11 | 2-I | H | CH(OMe)₂ | |
| 36- | 12 | 3-I | H | CH(OMe)₂ | |
| 36- | 13 | 4-I | H | CH(OMe)₂ | |
| 36- | 14 | 2-Cl | 3-Cl | CH(OMe)₂ | |
| 36- | 15 | 2-Cl | 4-Cl | CH(OMe)₂ | |
| 36- | 16 | 2-Cl | 5-Cl | CH(OMe)₂ | |
| 36- | 17 | 2-Cl | 6-Cl | CH(OMe)₂ | |
| 36- | 18 | 3-Cl | 4-Cl | CH(OMe)₂ | |
| 36- | 19 | 3-Cl | 5-Cl | CH(OMe)₂ | |
| 36- | 20 | H | H | CH(OEt)₂ | |
| 36- | 21 | 2-F | H | CH(OEt)₂ | |
| 36- | 22 | 3-F | H | CH(OEt)₂ | |
| 36- | 23 | 4-F | H | CH(OEt)₂ | |
| 36- | 24 | 2-Cl | H | CH(OEt)₂ | |
| 36- | 25 | 3-Cl | H | CH(OEt)₂ | |
| 36- | 26 | 4-Cl | H | CH(OEt)₂ | |
| 36- | 27 | 2-Br | H | CH(OEt)₂ | |
| 36- | 28 | 3-Br | H | CH(OEt)₂ | |
| 36- | 29 | 4-Br | H | CH(OEt)₂ | |
| 36- | 30 | 2-I | H | CH(OEt)₂ | |
| 36- | 31 | 3-I | H | CH(OEt)₂ | |
| 36- | 32 | 4-I | H | CH(OEt)₂ | |
| 36- | 33 | 2-Cl | 3-Cl | CH(OEt)₂ | |
| 36- | 34 | 2-Cl | 4-Cl | CH(OEt)₂ | |
| 36- | 35 | 2-Cl | 5-Cl | CH(OEt)₂ | |
| 36- | 36 | 2-Cl | 6-Cl | CH(OEt)₂ | |
| 36- | 37 | 3-Cl | 4-Cl | CH(OEt)₂ | |
| 36- | 38 | 3-Cl | 5-Cl | CH(OEt)₂ | |
| 36- | 39 | H | H | CH(OnPr)₂ | |
| 36- | 40 | 2-F | H | CH(OnPr)₂ | |
| 36- | 41 | 3-F | H | CH(OnPr)₂ | |
| 36- | 42 | 4-F | H | CH(OnPr)₂ | |
| 36- | 43 | 2-Cl | H | CH(OnPr)₂ | |
| 36- | 44 | 3-Cl | H | CH(OnPr)₂ | |
| 36- | 45 | 4-Cl | H | CH(OnPr)₂ | |
| 36- | 46 | 2-Br | H | CH(OnPr)₂ | |
| 36- | 47 | 3-Br | H | CH(OnPr)₂ | |
| 36- | 48 | 4-Br | H | CH(OnPr)₂ | |
| 36- | 49 | 2-I | H | CH(OnPr)₂ | |
| 36- | 50 | 3-I | H | CH(OnPr)₂ | |
| 36- | 51 | 4-I | H | CH(OnPr)₂ | |
| 36- | 52 | 2-Cl | 3-Cl | CH(OnPr)₂ | |
| 36- | 53 | 2-Cl | 4-Cl | CH(OnPr)₂ | |
| 36- | 54 | 2-Cl | 5-Cl | CH(OnPr)₂ | |
| 36- | 55 | 2-Cl | 6-Cl | CH(OnPr)₂ | |
| 36- | 56 | 3-Cl | 4-Cl | CH(OnPr)₂ | |
| 36- | 57 | 3-Cl | 5-Cl | CH(OnPr)₂ | |
| 36- | 58 | H | H | CH(OnBu)₂ | |
| 36- | 59 | 2-F | H | CH(OnBu)₂ | |
| 36- | 60 | 3-F | H | CH(OnBu)₂ | |
| 36- | 61 | 4-F | H | CH(OnBu)₂ | |
| 36- | 62 | 2-Cl | H | CH(OnBu)₂ | |
| 36- | 63 | 3-Cl | H | CH(OnBu)₂ | |
| 36- | 64 | 4-Cl | H | CH(OnBu)₂ | |
| 36- | 65 | 2-Br | H | CH(OnBu)₂ | |
| 36- | 66 | 3-Br | H | CH(OnBu)₂ | |
| 36- | 67 | 4-Br | H | CH(OnBu)₂ | |
| 36- | 68 | 2-I | H | CH(OnBu)₂ | |
| 36- | 69 | 3-I | H | CH(OnBu)₂ | |
| 36- | 70 | 4-I | H | CH(OnBu)₂ | |
| 36- | 71 | 2-Cl | 3-Cl | CH(OnBu)₂ | |
| 36- | 72 | 2-Cl | 4-Cl | CH(OnBu)₂ | |
| 36- | 73 | 2-Cl | 5-Cl | CH(OnBu)₂ | |
| 36- | 74 | 2-Cl | 6-Cl | CH(OnBu)₂ | |
| 36- | 75 | 3-Cl | 4-Cl | CH(OnBu)₂ | |
| 36- | 76 | 3-Cl | 5-Cl | CH(OnBu)₂ | |
| 36- | 77 | 4-Cl | 3-CF₃ | CH(OMe)₂ | ¹H-NMR (d₆-DMSO): 8.87, (d, 2H), 7.42 (t, 1H), 5.23 ppm (s, 1H) |
| 36- | 78 | 3-Cl | 5-CF₃ | CH(OMe)₂ | |
| 36- | 79 | 4-Cl | 3-CF₃ | CH(OEt)₂ | |
| 36- | 80 | 3-Cl | 5-CF₃ | CH(OEt)₂ | |

**Tabelle 37**

| | | | | | |
|---|---|---|---|---|---|
| 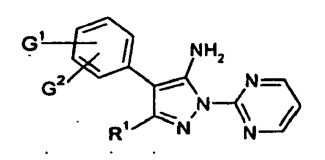 | | | | | |

| Beispielnr. | | G¹ | G² | R¹ | Physikalische Daten |
|---|---|---|---|---|---|
| 37- | 01 | H | H | CN | |
| 37- | 02 | 2-F | H | CN | |
| 37- | 03 | 3-F | H | CN | |
| 37- | 04 | 4-F | H | CN | |
| 37- | 05 | 2-Cl | H | CN | |
| 37- | 06 | 3-Cl | H | CN | |
| 37- | 07 | 4-Cl | H | CN | |
| 37- | 08 | 2-Br | H | CN | |
| 37- | 09 | 3-Br | H | CN | |
| 37- | 10 | 4-Br | H | CN | 1H(d6DMSO): 7.48, 7.71 ppm, 4-Br-Ph. |
| 37- | 11 | 2-I | H | CN | |
| 37- | 12 | 3-I | H | CN | |
| 37- | 13 | 4-I | H | CN | |
| 37- | 14 | 2-Cl | 3-Cl | CN | |
| 37- | 15 | 2-Cl | 4-Cl | CN | |
| 37- | 16 | 2-Cl | 5-Cl | CN | |
| 37- | 17 | 2-Cl | 6-Cl | CN | |
| 37- | 18 | 3-Cl | 4-Cl | CN | |
| 37- | 19 | 3-Cl | 5-Cl | CN | |
| 37- | 20 | H | H | CHO | |
| 37- | 21 | 2-F | H | CHO | |
| 37- | 22 | 3-F | H | CHO | |
| 37- | 23 | 4-F | H | CHO | |
| 37- | 24 | 2-Cl | H | CHO | |
| 37- | 25 | 3-Cl | H | CHO | |
| 37- | 26 | 4-Cl | H | CHO | |
| 37- | 27 | 2-Br | H | CHO | |
| 37- | 28 | 3-Br | H | CHO | |
| 37- | 29 | 4-Br | H | CHO | |
| 37- | 30 | 2-I | H | CHO | |
| 37- | 31 | 3-I | H | CHO | |
| 37- | 32 | 4-I | H | CHO | |
| 37- | 33 | 2-Cl | 3-Cl | CHO | |
| 37- | 34 | 2-Cl | 4-Cl | CHO | |
| 37- | 35 | 2-Cl | 5-Cl | CHO | |
| 37- | 36 | 2-Cl | 6-Cl | CHO | |
| 37- | 37 | 3-Cl | 4-Cl | CHO | |
| 37- | 38 | 3-Cl | 5-Cl | CHO | |

**Tabelle 38**

| | | | | | |
|---|---|---|---|---|---|
| 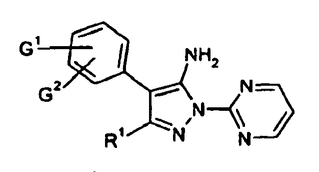 | | | | | |

| Beispielnr. | | G¹ | G² | R¹ | Physikalische Daten |
|---|---|---|---|---|---|
| 38- | 01 | H | H | CH=NOH | |
| 38- | 02 | 2-F | H | CH=NOH | |
| 38- | 03 | 3-F | H | CH=NOH | |
| 38- | 04 | 4-F | H | CH=NOH | |
| 38- | 05 | 2-Cl | H | CH=NOH | |
| 38- | 06 | 3-Cl | H | CH=NOH | |
| 38- | 07 | 4-Cl | H | CH=NOH | |
| 38- | 08 | 2-Br | H | CH=NOH | |
| 38- | 09 | 3-Br | H | CH=NOH | |
| 38- | 10 | 4-Br | H | CH=NOH | 1H(d6DMSO):7.96, 1H, s; 11.13ppm, 1H, s; CH=NOH |
| 38- | 11 | 2-I | H | CH=NOH | |
| 38- | 12 | 3-I | H | CH=NOH | |
| 38- | 13 | 4-I | H | CH=NOH | |
| 38- | 14 | 2-Cl | 3-Cl | CH=NOH | |
| 38- | 15 | 2-Cl | 4-Cl | CH=NOH | |
| 38- | 16 | 2-Cl | 5-Cl | CH=NOH | |
| 38- | 17 | 2-Cl | 6-Cl | CH=NOH | |
| 38- | 18 | 3-Cl | 4-Cl | CH=NOH | |
| 38- | 19 | 3-Cl | 5-Cl | CH=NOH | |
| 38- | 20 | H | H | CH=NOMe | |
| 38- | 21 | 2-F | H | CH=NOMe | |
| 38- | 22 | 3-F | H | CH=NOMe | |
| 38- | 23 | 4-F | H | CH=NOMe | |
| 38- | 24 | 2-Cl | H | CH=NOMe | |
| 38- | 25 | 3-Cl | H | CH=NOMe | |
| 38- | 26 | 4-Cl | H | CH=NOMe | |
| 38- | 27 | 2-Br | H | CH=NOMe | |
| 38- | 28 | 3-Br | H | CH=NOMe | |
| 38- | 29 | 4-Br | H | CH=NOMe | 1H(d6DMSO): 3.76ppm; OMe. |
| 38- | 30 | 2-I | H | CH=NOMe | |
| 38- | 31 | 3-I | H | CH=NOMe | |
| 38- | 32 | 4-I | H | CH=NOMe | |
| 38- | 33 | 2-Cl | 3-Cl | CH=NOMe | |
| 38- | 34 | 2-Cl | 4-Cl | CH=NOMe | |
| 38- | 35 | 2-Cl | 5-Cl | CH=NOMe | |
| 38- | 36 | 2-Cl | 6-Cl | CH=NOMe | |
| 38- | 37 | 3-Cl | 4-Cl | CH=NOMe | |
| 38- | 38 | 3-Cl | 5-Cl | CH=NOMe | |
| 38- | 39 | H | H | CH=NOEt | |
| 38- | 40 | 2-F | H | CH=NOEt | |
| 38- | 41 | 3-F | H | CH=NOEt | |
| 38- | 42 | 4-F | H | CH=NOEt | |
| 38- | 43 | 2-Cl | H | CH=NOEt | |
| 38- | 44 | 3-Cl | H | CH=NOEt | |
| 38- | 45 | 4-Cl | H | CH=NOEt | |
| 38- | 46 | 2-Br | H | CH=NOEt | |
| 38- | 47 | 3-Br | H | CH=NOEt | |
| 38- | 48 | 4-Br | H | CH=NOEt | 1H(d6DMSO):3.98,1.14ppm; OEt |
| 38- | 49 | 2-I | H | CH=NOEt | |
| 38- | 50 | 3-1 | H | CH=NOEt | |
| 38- | 51 | 4-I | H | CH=NOEt | |
| 38- | 52 | 2-Cl | 3-Cl | CH=NOEt | |
| 38- | 53 | 2-Cl | 4-Cl | CH=NOEt | |
| 38- | 54 | 2-Cl | 5-Cl | CH=NOEt | |
| 38- | 55 | 2-Cl | 6-Cl | CH=NOEt | |
| 38- | 56 | 3-Cl | 4-Cl | CH=NOEt | |
| 38- | 57 | 3-Cl | 5-Cl | CH=NOEt | |
| 38- | 58 | H | H | CH=NOnPr | |
| 38- | 59 | 2-F | H | CH=NOnPr | |
| 38- | 60 | 3-F | H | CH=NOnPr | |
| 38- | 61 | 4-F | H | CH=NOnPr | |
| 38- | 62 | 2-Cl | H | CH=NOnPr | |
| 38- | 63 | 3-Cl | H | CH=NOnPr | |
| 38- | 64 | 4-Cl | H | CH=NOnPr | |
| 38- | 65 | 2-Br | H | CH=NOnPr | |
| 38- | 66 | 3-Br | H | CH=NOnPr | |
| 38- | 67 | 4-Br | H | CH=NOnPr | |
| 38- | 68 | 2-I | H | CH=NOnPr | |
| 38- | 69 | 3-I | H | CH=NOnPr | |
| 38- | 70 | 4-I | H | CH=NOnPr | |
| 38- | 71 | 2-Cl | 3-Cl | CH=NOnPr | |
| 38- | 72 | 2-Cl | 4-Cl | CH=NOnPr | |
| 38- | 73 | 2-Cl | 5-Cl | CH=NOnPr | |
| 38- | 74 | 2-Cl | 6-Cl | CH=NOnPr | |
| 38- | 75 | 3-Cl | 4-Cl | CH=NOnPr | |
| 38- | 76 | 3-Cl | 5-Cl | CH=NOnPr | |
| 38- | 77 | H | H | CH=NOiPr | |
| 38- | 78 | 2-F | H | CH=NOiPr | |
| 38- | 79 | 3-F | H | CH=NOiPr | |
| 38- | 80 | 4-F | H | CH=NOiPr | |
| 38- | 81 | 2-Cl | H | CH=NOiPr | |
| 38- | 82 | 3-Cl | H | CH=NOiPr | |
| 38- | 83 | 4-Cl | H | CH=NOiPr | |
| 38- | 84 | 2-Br | H | CH=NOiPr | |
| 38- | 85 | 3-Br | H | CH=NOiPr | |
| 38- | 86 | 4-Br | H | CH=NOiPr | |
| 38- | 87 | 2-I | H | CH=NOiPr | |
| 38- | 88 | 3-I | H | CH=NOiPr | |
| 38- | 89 | 4-I | H | CH=NOiPr | |
| 38- | 90 | 2-Cl | 3-Cl | CH=NOiPr | |
| 38- | 91 | 2-Cl | 4-Cl | CH=NOiPr | |
| 38- | 92 | 2-Cl | 5-Cl | CH=NOiPr | |
| 38- | 93 | 2-Cl | 6-Cl | CH=NOiPr | |
| 38- | 94 | 3-Cl | 4-Cl | CH=NOiPr | |
| 38- | 95 | 3-Cl | 5-Cl | CH=NOiPr | |

**Tabelle 39**

| | | | | | |
|---|---|---|---|---|---|
| 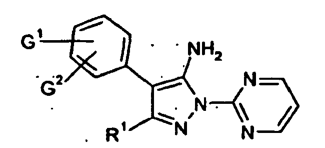 | | | | | |

| Beispielnr. | | G¹ | G² | R¹ | Physikalische Daten |
|---|---|---|---|---|---|
| 39- | 01 | H | H | C₆H₅-CH₂ | |
| 39- | 02 | 2-F | H | C₆H₅-CH₂ | |
| 39- | 03 | 3-F | H | C₆H₅-CN₂ | |
| 39- | 04 | 4-F | H | C₆H₅-CH₂ | |
| 39- | 05 | 2-Cl | H | C₆H₅-CH₂ | |
| 39- | 06 | 3-Cl | H | C₆H₅-CH₂ | |
| 39- | 07 | 4-Cl | H | C₆H₅-CH₂ | |
| 39- | 08 | 2-Br | H | C₆H₅-CH₂ | |
| 39- | 09 | 3-Br | H | C₆H₅-CH₂ | |
| 39- | 10 | 4-Br | H | C₆H5-CH2 | 1H: 4.03ppm, CH₂ |
| 39- | 11 | 2-I | H | C₆H₅-CH₂ | |
| 39- | 12 | 3-I | H | C₆H₅-CH₂ | |
| 39- | 13 | 4-I | H | C₆H₅-CH₂ | |
| 39- | 14 | 2-Cl | 3-Cl | C₆H₅-CH₂ | |
| 39- | 15 | 2-Cl | 4-Cl | C₆H₅-CH₂ | |
| 39- | 16 | 2-Cl | 5-Cl | C₆H₅-CH₂ | |
| 39- | 17 | 2-Cl | 6-Cl | C₆H₅-CH₂ | |
| 39- | 18 | 3-Cl | 4-Cl | C₆H₅-CH₂ | |
| 39- | 19 | 3-Cl | 5-Cl | C₆H₅-CH₂ | |
| 39- | 20 | H | H | 2-Cl-C₆H₄-CH₂ | |
| 39- | 21 | 2-F | H | 2-Cl-C₆H₄-CH₂ | |
| 39- | 22 | 3-F | H | 2-Cl-C₆H₄-CH₂ | |
| 39- | 23 | 4-F | H | 2-Cl-C₆H₄-CH₂ | |
| 39- | 24 | 2-Cl | H | 2-Cl-C₆H₄-CH₂ | |
| 39- | 25 | 3-Cl | H | 2-Cl-C₆H₄-CH₂ | |
| 39- | 26 | 4-Cl | H | 2-Cl-C₆H₄-CH₂ | |
| 39- | 27 | 2-Br | H | 2-Cl-C₆H₄-CH₂ | |
| 39- | 28 | 3-Br | H | 2-Cl-C₆H₄-CH₂ | |
| 39- | 29 | 4-Br | H | 2-Cl-C₆H₄-CH₂ | |
| 39- | 30 | 2-I | H | 2-Cl-C₆H₄-CH₂ | |
| 39- | 31 | 3-I | H | 2-Cl-C₆H₄-CH₂ | |
| 39- | 32 | 4-I | H | 2-Cl-C₆H₄-CH₂ | |
| 39- | 33 | 2-Cl | 3-Cl | 2-Cl-C₆H₄-CH₂ | |
| 39- | 34 | 2-Cl | 4-Cl | 2-Cl-C₆H₄-CH₂ | |
| 39- | 35 | 2-Cl | 5-Cl | 2-Cl-C₆H₄-CH₂ | |
| 39- | 36 | 2-Cl | 6-Cl | 2-Cl-C₆H₄-CH₂ | |
| 39- | 37 | 3-Cl | 4-Cl | 2-Cl-C₆H₄-CH₂ | |
| 39- | 38 | 3-Cl | 5-Cl | 2-Cl-C₆H₄-CH₂ | |
| 39- | 39 | H | H | 3-Cl-C₆H₄-CH₂ | |
| 39- | 40 | 2-F | H | 3-Cl-C₆H₄-CH₂ | |
| 39- | 41 | 3-F | H | 3-Cl-C₆H₄-CH₂ | |
| 39- | 42 | 4-F | H | 3-Cl-C₆H₄-CH₂ | |
| 39- | 43 | 2-Cl | H | 3-Cl-C₆H₄-CH₂ | |
| 39- | 44 | 3-Cl | H | 3-Cl-C₆H₄-CH₂ | |
| 39- | 45 | 4-Cl | H | 3-Cl-C₆H₄-CH₂ | |
| 39- | 46 | 2-Br | H | 3-Cl-C₆H₄-CH₂ | |
| 39- | 47 | 3-Br | H | 3-Cl-C₆H₄-CH₂ | |
| 39- | 48 | 4-Br | H | 3-Cl-C₆H₄-CH₂ | 1H: 4.00ppm, CH₂ |
| 39- | 49 | 2-I | H | 3-Cl-C₆H₄-CH₂ | |
| 39- | 50 | 3-I | H | 3-Cl-C₆H₄-CH₂ | |
| 39- | 51 | 4-I | H | 3-Cl-C₆H₄-CH₂ | |
| 39- | 52 | 2-Cl | 3-Cl | 3-OC₆H₄-CH₂ | |
| 39- | 53 | 2-Cl | 4-Cl | 3-Cl-C₆H₄-CH₂ | |
| 39- | 54 | 2-Cl | 5-Cl | 3-Cl-C₆H₄-CH₂ | |
| 39- | 55 | 2-Cl | 6-Cl | 3-Cl-C₆H₄-CH₂ | |
| 39- | 56 | 3-Cl | 4-Cl | 3-Cl-C₆H₄-CH₂ | |
| 39- | 57 | 3-Cl | 5-Cl | 3-Cl-C₆H₄-CH₂ | |
| 39- | 58 | H | H | 4-Cl-C₆H₄-CH₂ | |
| 39- | 59 | 2-F | H | 4-Cl-C₆H₄-CH₂ | |
| 39- | 60 | 3-F | H | 4-Cl-C₆H₄-CH₂ | |
| 39- | 61 | 4-F | H | 4-Cl-C₆H₄-CH₂ | |
| 39- | 62 | 2-Cl | H | 4-Cl-C₆H₄-CH₂ | |
| 39- | 63 | 3-Cl | H | 4-Cl-C₆H₄-CH₂ | |
| 39- | 64 | 4-Cl | H | 4-Cl-C₆H₄-CH₂ | |
| 39- | 65 | 2-Br | H | 4-Cl-C₆H₄-CH₂ | |
| 39- | 66 | 3-Br | H | 4-Cl-C₆H₄-CH₂ | |
| 39- | 67 | 4-Br | H | 4-Cl-C₆H₄-CH₂ | 1H: 3.98ppm, CH₂ |
| 39- | 68 | 2-I | H | 4-Cl-C₆H₄-CH₂ | |
| 39- | 69 | 3-I | H | 4-Cl-C₆H₄-CH₂ | |
| 39- | 70 | 4-I | H | 4-Cl-C₆H₄-CH₂ | |
| 39- | 71 | 2-Cl | 3-Cl | 4-Cl-C₆H₄-CH₂ | |
| 39- | 72 | 2-Cl | 4-Cl | 4-Cl-C₆H₄-CH₂ | |
| 39- | 73 | 2-Cl | 5-Cl | 4-Cl-C₆H₄-CH₂ | |
| 39- | 74 | 2-Cl | 6-Cl | 4-Cl-C₆H₄-CH₂ | |
| 39- | 75 | 3-Cl | 4-Cl | 4-Cl-C₆H₄-CH₂ | |
| 39- | 76 | 3-Cl | 5-Cl | 4-Cl-C₆H₄-CH₂ | |
| 39- | 77 | H | H | 2-F-C₆H₄-CH₂ | |
| 39- | 78 | 2-F | H | 2-F-C₆H₄-CH₂ | |
| 39- | 79 | 3-F | H | 2-F-C₆H₄-CH₂ | |
| 39- | 80 | 4-F | H | 2-F-C₆H₄-CH₂ | |
| 39- | 81 | 2-Cl | H | 2-F-C₆H₄-CH₂ | |
| 39- | 82 | 3-Cl | H | 2-F-C₆H₄-CH₂ | |
| 39- | 83 | 4-Cl | H | 2-F-C₆H₄-CH₂ | |
| 39- | 84 | 2-Br | H | 2-F-C₆H₄-CH₂ | |
| 39- | 85 | 3-Br | H | 2-F-C₆H₄-CH₂ | |
| 39- | 86 | 4-Br | H | 2-F-C₆H₄-CH₂ | 19F: -118.45ppm. PhF |
| 39- | 87 | 2-I | H | 2-F-C₆H₄-CH₂ | |
| 39- | 88 | 3-I | H | 2-F-C₆H₄-CH₂ | |
| 39- | 89 | 4-I | H | 2-F-C₆H₄-CH₂ | |
| 39- | 90 | 2-Cl | 3-Cl | 2-F-C₆H₄-CH₂ | |
| 39- | 91 | 2-Cl | 4-Cl | 2-F-C₆H₄-CH₂ | |
| 39- | 92 | 2-Cl | 5-Cl | 2-F-C₆H₄-CH₂ | |
| 39- | 93 | 2-Cl | 6-Cl | 2-F-C₆H₄-CH₂ | |
| 39- | 94 | 3-Cl | 4-Cl | 2-F-C₆H₄-CH₂ | |
| 39- | 95 | 3-Cl | 5-Cl | 2-F-C₆H₄-CH₂ | |
| 39- | 96 | H | H | 3-F-C₆H₄-CH₂ | |
| 39- | 97 | 2-F | H | 3-F-C₆H₄-CH₂ | |
| 39- | 98 | 3-F | H | 3-F-C₆H₄-CH₂ | |
| 39- | 99 | 4-F | H | 3-F-C₆H₄-CH₂ | |
| 39- | 100 | 2-Cl | H | 3-F-C₆H₄-CH₂ | |
| 39- | 101 | 3-Cl | H | 3-F-C₆H₄-CH₂ | |
| 39- | 102 | 4-Cl | H | 3-F-C₆H₄-CH₂ | |
| 39- | 103 | 2-Br | H | 3-F-C₆H₄-CH₂ | |
| 39- | 104 | 3-Br | H | 3-F-C₆H₄-CH₂ | |
| 39- | 105 | 4-Br | H | 3-F-C₆H₄-CH₂ | 1H(d6DMSO): 6.90, 6.96, 7.26ppm, F-PhenylH |
| 39- | 106 | 2-I | H | 3-F-C₆H₄-CH₂ | |
| 39- | 107 | 3-I | H | 3-F-C₆H₄-CH₂ | |
| 39- | 108 | 4-I | H | 3-F-C₆H₄-CH₂ | |
| 39- | 109 | 2-Cl | 3-Cl | 3-F-C₆H₄-CH₂ | |
| 39- | 110 | 2-Cl | 4-Cl | 3-F-C₆H₄-CH₂ | |
| 39- | 111 | 2-Cl | 5-Cl | 3-F-C₆H₄-CH₂ | |
| 39- | 112 | 2-Cl | 6-Cl | 3-F-C₆H₄-CH₂ | |
| 39- | 113 | 3-Cl | 4-Cl | 3-F-C₆H₄-CH₂ | |
| 39- | 114 | 3-Cl | 5-Cl | 3-F-C₆H₄-CH₂ | |
| 39- | 115 | H | H | 4-F-C₆H₄-CH₂ | |
| 39- | 116 | 2-F | H | 4-F-C₆H₄-CH₂ | |
| 39- | 117 | 3-F | H | 4-F-C₆H₄-CH₂ | |
| 39- | 118 | 4-F | H | 4-F-C₆H₄-CH₂ | |
| 39- | 119 | 2-Cl | H | 4-F-C₆H₄-CH₂ | |
| 39- | 120 | 3-Cl | H | 4-F-C₆H₄-CH₂ | |
| 39- | 121 | 4-Cl | H | 4-F-C₆H₄-CH₂ | |
| 39- | 122 | 2-Br | H | 4-F-C₆H₄-CH₂ | |
| 39- | 123 | 3-Br | H | 4-F-C₆H₄-CH₂ | |
| 39- | 124 | 4-Br | H | 4-F-C₆H₄-CH₂ | 1H(d6DMSO): 7.04, 7.14, F-PhenylH |
| 39- | 125 | 2-I | H | 4-F-C₆H₄-CH₂ | |
| 39- | 126 | 3-I | H | 4-F-C₆H₄-CH₂ | |
| 39- | 127 | 4-I | H | 4-F-C₆H₄-CH₂ | |
| 39- | 128 | 2-Cl | 3-Cl | 4-F-C₆H₄-CH₂ | |
| 39- | 129 | 2-Cl | 4-Cl | 4-F-C₆H₄-CH₂ | |
| 39- | 130 | 2-Cl | 5-Cl | 4-F-C₆H₄-CH₂ | |
| 39- | 131 | 2-Cl | 6-Cl | 4-F-C₆H₄-CH₂ | |
| 39- | 132 | 3-Cl | 4-Cl | 4-F-C₆H₄-CH₂ | |
| 39- | 133 | 3-Cl | 5-Cl | 4-F-C₆H₄-CH₂ | |
| 39- | 134 | H | H | 2-CF₃-C₆H₄-CH₂ | |
| 39- | 135 | 2-F | H | 2-CF₃-C₆H₄-CH₂ | |
| 39- | 136 | 3-F | H | 2-CF₃-C₆H₄-CH₂ | |
| 39- | 137 | 4-F | H | 2-CF₃-C₆H₄-CH₂ | |
| 39- | 138 | 2-Cl | H | 2-CF₃-C₆H₄-CH₂ | |
| 39- | 139 | 3-Cl | H | 2-CF₃-C₆H₄-CH₂ | |
| 39- | 140 | 4-Cl | H | 2-CF₃-C₆H₄-CH₂ | |
| 39- | 141 | 2-Br | H | 2-CF₃-C₆H₄-CH₂ | |
| 39- | 142 | 3-Br | H | 2-CF₃-C₆H₄-CH₂ | |
| 39- | 143 | 4-Br | H | 2-CF₃-C₆H₄-CH₂ | |
| 39- | 144 | 2-I | H | 2-CF₃-C₆H₄-CH₂ | |
| 39- | 145 | 3-I | H | 2-CF₃-C₆H₄-CH₂ | |
| 39- | 146 | 4-I | H | 2-CF₃-C₆H₄-CH₂ | |
| 39- | 147 | 2-Cl | 3-Cl | 2-CF₃-C₆H₄-CH₂ | |
| 39- | 148 | 2-Cl | 4-Cl | 2-CF₃-C₆H₄-CH₂ | |
| 39- | 149 | 2-Cl | 5-Cl | 2-CF₃-C₆H₄-CH₂ | |
| 39- | 150 | 2-Cl | 6-Cl | 2-CF₃-C₆H₄-CH₂ | |
| 39- | 151 | 3-Cl | 4-Cl | 2-CF₃-C₆H₄-CH₂ | |
| 39- | 152 | 3-Cl | 5-Cl | 2-CF₃-C₆H₄-CH₂ | |
| 39- | 153 | H | H | 3-CF₃-C₆H₄-CH₂ | |
| 39- | 154 | 2-F | H | 3-CF₃-C₆H₄-CH₂ | |
| 39- | 155 | 3-F | H | 3-CF₃-C₆H₄-CH₂ | |
| 39- | 156 | 4-F | H | 3-CF₃-C₆H₄-CH₂ | |
| 39- | 157 | 2-Cl | H | 3-CF₃-C₆H₄-CH₂ | |
| 39- | 158 | 3-Cl | H | 3-CF₃-C₆H₄-CH₂ | |
| 39- | 159 | 4-Cl | H | 3-CF₃-C₆H₄-CH₂ | |
| 39- | 160 | 2-Br | H | 3-CF₃-C₆H₄-CH₂ | |
| 39- | 161 | 3-Br | H | 3-CF₃-C₆H₄-CH₂ | |
| 39- | 162 | 4-Br | H | 3-CF₃-C₆H₄-CH₂ | 1H: 4.08ppm, CH₂ |
| 39- | 163 | 2-I | H | 3-CF₃-C₆H₄-CH₂ | |
| 39- | 164 | 3-I | H | 3-CF₃-C₆H₄-CH₂ | |
| 39- | 165 | 4-I | H | 3-CF₃-C₆H₄-CH₂ | |
| 39- | 166 | 2-Cl | 3-Cl | 3-CF₃-C₆H₄-CH₂ | |
| 39- | 167 | 2-Cl | 4-Cl | 3-CF₃-C₆H₄-CH₂ | |
| 39- | 168 | 2-Cl | 5-Cl | 3-CF₃-C₆H₄-CH₂ | |
| 39- | 169 | 2-Cl | 6-Cl | 3-CF₃-C₆H₄-CH₂ | |
| 39- | 170 | 3-Cl | 4-Cl | 3-CF₃-C₆H₄-CH₂ | |
| 39- | 171 | 3-Cl | 5-Cl | 3-CF₃-C₆H₄-CH₂ | |
| 39- | 172 | H | H | 4-CF₃-C₆H₄-CH₂ | |
| 39- | 173 | 2-F | H | 4-CF₃-C₆H₄-CH₂ | |
| 39- | 174 | 3-F | H | 4-CF₃-C₆H₄-CH₂ | |
| 39- | 175 | 4-F | H | 4-CF₃-C₆H₄-CH₂ | |
| 39- | 176 | 2-Cl | H | 4-CF₃-C₆H₄-CH₂ | |
| 39- | 177 | 3-Cl | H | 4-CF₃-C₆H₄-CH₂ | |
| 39- | 178 | 4-Cl | H | 4-CF₃-C₆H₄-CH₂ | |
| 39- | 179 | 2-Br | H | 4-CF₃-C₆H₄-CH₂ | |
| 39- | 180 | 3-Br | H | 4-CF₃-C₆H₄-CH₂ | |
| 39- | 181 | 4-Br | H | 4-CF₃-C₆H₄-CH₂ | |
| 39- | 182 | 2-I | H | 4-CF₃-C₆H₄-CH₂ | |
| 39- | 183 | 3-I | H | 4-CF₃-C₆H₄-CH₂ | |
| 39- | 184 | 4-I | H | 4-CF₃-C₆H₄-CH₂ | |
| 39- | 185 | 2-CL | 3-CL | 4-CF₃-C₆H₄-CH₂ | |
| 39- | 186 | 2-Cl | 4-Cl | 4-CF₃-C₆H₄-CH₂ | |
| 39- | 187 | 2-Cl | 5-Cl | 4-CF₃-C₆H₄-CH₂ | |
| 39- | 188 | 2-Cl | 6-Cl | 4-CF₃-C₆H₄-CH₂ | |
| 39- | 189 | 3-Cl | 4-Cl | 4-CF₃-C₆H₄-CH₇ | |
| 39- | 190 | 3-Cl | 5-Cl | 4-CF₃-C₆H₄-CH₂ | |
| 39- | 191 | H | H | 2,4-Cl₂-C₆H₃-CH₂ | |
| 39- | 192 | 2-F | H | 2,4-Cl₂-C₆H₃-CH₂ | |
| 39- | 193 | 3-F | H | 2,4-Cl₂-C₆H₃-CH₂ | |
| 39- | 194 | 4-F | H | 2,4-Cl₂-C₆H₃-CH₂ | |
| 39- | 195 | 2-Cl | H | 2,4-Cl₂-C₆H₃-CH₂ | |
| 39- | 196 | 3-Cl | H | 2,4-Cl₂-C₆H₃-CH₂ | |
| 39- | 197 | 4-Cl | H | 2,4-Cl₂-C₆H₃-CH₂ | |
| 39- | 198 | 2-Br | H | 2,4-Cl₂-C₆H₃-CH₂ | |
| 39- | 199 | 3-Br | H | 2,4-Cl₂-C₆H₃-CH₂ | |
| 39- | 200 | 4-Br | H | 2,4-Cl₂-C₆H₃-CH₂ | 1H(d6DMSO): 7.30, 7.38, 7.62, PhH. |
| 39- | 201 | 2-I | H | 2,4-Cl₂-C₆H₃-CH₂ | |
| 39- | 202 | 3-I | H | 2,4-Cl₂-C₆H₃-CH₂ | |
| 39- | 203 | 4-I | H | 2,4-Cl₂-C₆H₃-CH₂ | |
| 39- | 204 | 2-Cl | 3-Cl | 2,4-Cl₂-C₆H₃-CH₂ | |
| 39- | 205 | 2-Cl | 4-Cl | 2,4-Cl₂-C₆H₃-CH₂ | |
| 39- | 206 | 2-Cl | 5-Cl | 2,4-Cl₂-C₆H₃-CH₂ | |
| 39- | 207 | 2-Cl | 6-Cl | 2,4-Cl₂-C₆H₃-CH₂ | |
| 39- | 208 | 3-Cl | 4-Cl | 2,4-Cl₂-C₆H₃-CH₂ | |
| 39- | 209 | 3-Cl | 5-Cl | 2,4-Cl₂-C₆H₃-CH₂ | |
| 39- | 210 | H | H | 2-Br-C₆H₄-CH₂ | |
| 39- | 211 | 2-F | H | 2-Br-C₆H₄-CH₂ | |
| 39- | 212 | 3-F | H | 2-Br-C₆H₄-CH₂ | |
| 39- | 213 | 4-F | H | 2-Br-C₆H₄-CH₂ | |
| 39- | 214 | 2-Cl | H | 2-Br-C₆N₄-CH₂ | |
| 39- | 215 | 3-Cl | H | 2-Br-C₆H₄-CH₂ | |
| 39- | 216 | 4-Cl | H | 2-Br-C₆H₄-CH₂ | |
| 39- | 217 | 2-Br | H | 2-Br-C₆H₄-CH₂ | |
| 39- | 218 | 3-Br | H | 2-Br-C₆H₄-CH₂ | |
| 39- | 219 | 4-Br | H | 2-Br-C₆H₄-CH₂ | 1H(d6DMSO): 3.97ppm, CH₂ |
| 39- | 220 | 2-I | H | 2-Br-C₆H₄-CH₂ | |
| 39- | 221 | 3-I | H | 2-Br-C₆H₄-CH₂ | |
| 39- | 222 | 4-I | H | 2-Br-C₆H₄-CH₂ | |
| 39- | 223 | 2-Cl | 3-Cl | 2-Br-C₆H₄-CH₂ | |
| 39- | 224 | 2-Cl | 4-Cl | 2-Br-C₆H₄-CH₂ | |
| 39- | 225 | 2-Cl | 5-Cl | 2-Br-C₆H₄-CH₂ | |
| 39- | 226 | 2-Cl | 6-Cl | 2-Br-C₆H₄-CH₂ | |
| 39- | 227 | 3-Cl | 4-Cl | 2-Br-C₆H₄-CH₂ | |
| 39- | 228 | 3-Cl | 5-Cl | 2-Br-C₆H₄-CH₂ | |
| 39- | 229 | H | H | 3-Br-C₆H₄-CH₂ | |
| 39- | 230 | 2-F | H | 3-Br-C₆H₄-CH₂ | |
| 39- | 231 | 3-F | H | 3-Br-C₆H₄-CH₂ | |
| 39- | 232 | 4-F | H | 3-Br-C₆H₄-CH₂ | |
| 39- | 233 | 2-Cl | H | 3-Br-C₆H₄-CH₂ | |
| 39- | 234 | 3-Cl | H | 3-Br-C₆H₄-CH₂ | |
| 39- | 235 | 4-Cl | H | 3-Br-C₆H₄-CH₂ | |
| 39- | 236 | 2-Br | H | 3-Br-C₆H₄-CH₂ | |
| 39- | 237 | 3-Br | H | 3-Br-C₆H₄-CH₂ | |
| 39- | 238 | 4-Br | H | 3-Br-C₆H₄-CH₂ | |
| 39- | 239 | 2-I | H | 3-Br-C₆H₄-CH₂ | |
| 39- | 240 | 3-I | H | 3-Br-C₆H₄-CH₂ | |
| 39- | 241 | 4-I | H | 3-Br-C₆H₄-CH₂ | |
| 39- | 242 | 2-Cl | 3-Cl | 3-Br-C₆H₄-CH₂ | |
| 39- | 243 | 2-Cl | 4-Cl | 3-Br-C₆H₄-CH₂ | |
| 39- | 244 | 2-Cl | 5-Cl | 3-Br-C₆H₄-CH₂ | |
| 39- | 245 | 2-Cl | 6-Cl | 3-Br-C₆H₄-CH₂ | |
| 39- | 246 | 3-Cl | 4-Cl | 3-Br-C₆H₄-CH₂ | |
| 39- | 247 | 3-Cl | 5-Cl | 3-Br-C₆H₄-CH₂ | |
| 39- | 248 | H | H | 4-Br-C₆H₄-CH₂ | |
| 39- | 249 | 2-F | H | 4-Br-C₆H₄-CH₂ | |
| 39- | 250 | 3-F | H | 4-Br-C₆H₄-CH₂ | |
| 39- | 251 | 4-F | H | 4-Br-C₆H₄-CH₂ | |
| 39- | 252 | 2-Cl | H | 4-Br-C₆H₄-CH₂ | |
| 39- | 253 | 3-Cl | H | 4-Br-C₆H₄-CH₂ | |
| 39- | 254 | 4-Cl | H | 4-Br-C₆H₄-CH₂ | |
| 39- | 255 | 2-Br | H | 4-Br-C₆H₄-CH₂ | |
| 39- | 256 | 3-Br | H | 4-Br-C₆H₄-CH₂ | |
| 39- | 257 | 4-Br | H | 4-Br-C₆H₄-CH₂ | 1H: 3.97ppm, CH₂ |
| 39- | 258 | 2-I | H | 4-Br-C₆H₄-CH2 | |
| 39- | 259 | 3-I | H | 4-Br-C₆H₄-CH₂ | |
| 39- | 260 | 4-I | H | 4-Br-C₆H₄-CH₂ | |
| 39- | 261 | 2-Cl | 3-Cl | 4-Br-C₆H₄-CH₂ | |
| 39- | 262 | 2-Cl | 4-Cl | 4-Br-C₆H₄-CH₂ | |
| 39- | 263 | 2-Cl | 5-Cl | 4-Br-C₆H₄-CH₂ | |
| 39- | 264 | 2-Cl | 6-Cl | 4-Br-C₆H₄-CH₂ | |
| 39- | 265 | 3-Cl | 4-Cl | 4-Br-C₆H₄-CH₂ | |
| 39- | 266 | 3-Cl | 5-Cl | 4-Br-C₆H₄-CH₂ | |

**Tabelle 40**

| | | | | | |
|---|---|---|---|---|---|
| 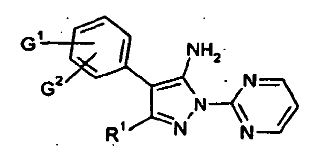 | | | | | |

| Beispielnr. | | G¹ | G² | R¹ | Physikalische Daten |
|---|---|---|---|---|---|
| 40- | 01 | H | H | 2-CH₃-C₆H₄-CH₂ | |
| 40- | 02 | 2-F | H | 2-CH₃-C₆H₄-CH₂ | |
| 40- | 03 | 3-F | H | 2-CH₃-C₆H₄-CH₂ | |
| 40- | 04 | 4-F | H | 2-CH₃-C₆H₄-CH₂ | |
| 40- | 05 | 2-Cl | H | 2-CH₃-C₆H₄-CH₂ | |
| 40- | 06 | 3-Cl | H | 2-CH₃-C₆H₄-CH₂ | |
| 40- | 07 | 4-Cl | H | 2-CH₃-C₆H₄-CH₂ | |
| 40- | 08 | 2-Br | H | 2-CH₃-C₆H₄-CH₂ | |
| 40- | 09 | 3-Br | H | 2-CH₃-C₆H₄-CH₂ | |
| 40- | 10 | 4-Br | H | 2-CH₃-C₆H₄-CH₂ | 1H(d6DMSO): 2.20ppm, Ph-CH₃ |
| 40- | 11 | 2-I | H | 2-CH₃-C₆H₄-CH₂ | |
| 40- | 12 | 3-I | H | 2-CH₃-C₆H₄-CH₂ | |
| 40- | 13 | 4-I | H | 2-CH₃-C₆H₄-CH₂ | |
| 40- | 14 | 2-Cl | 3-Cl | 2-CH₃-C₆H₄-CH₂ | |
| 40- | 15 | 2-Cl | 4-Cl | 2-CH₃-C₆H₄-CH₂ | |
| 40- | 16 | 2-Cl | 5-Cl | 2-CH₃-C₆H₄-CH₂ | |
| 40- | 17 | 2-Cl | 6-Cl | 2-CH₃-C₆H₄-CH₂ | |
| 40- | 18 | 3-Cl | 4-Cl | 2-CH₃-C₆H₄-CH₂ | |
| 40- | 19 | 3-Cl | 5-Cl | 2-CH₃-C₆H₄-CH₂ | |
| 40- | 20 | H | H | 3-CH₃-C₆H₄-CH₂ | |
| 40- | 21 | 2-F | H | 3-CH₃-C₆H₄-CH₂ | |
| 40- | 22 | 3-F | H | 3-CH₃-C₆H₄-CH₂ | |
| 40- | 23 | 4-F | H | 3-CH₃-C₆H₄-CH_{2.} | |
| 40- | 24 | 2-Cl | H | 3-CH₃-C₆H₄-CH₂. | |
| 40- | 25 | 3-Cl | H | 3-CH₃-C₆H₄-CH₂ | |
| 40- | 26 | 4-Cl | H | 3-CH₃-C₆H₄-CH₂ | |
| 40- | 27 | 2-Br | H | 3-CH₃-C₆H₄-CH₂ | |
| 40- | 28 | 3-Br | H | 3-CH₃-C₆H₄-CH₂ | |
| 40- | 29 | 4-Br | H | 3-CH₃-C₆H₄-CH₂ | 1H(d6DMSO): 2.20ppm, Ph-CH₃ |
| 40- | 30 | 2-I | H | 3-CH₃-C₆H₄-CH₂ | |
| 40- | 31 | 3-I | H | 3-CH₃-C₆H₄-CH₂ | |
| 40- | 32 | 4-I | H | 3-CH₃-C₆H₄-CH₂ | |
| 40- | 33 | 2-Cl | 3-Cl | 3-CH₃-C₆H₄-CH₂ | |
| 40- | 34 | 2-Cl | 4-Cl | 3-CH₃-C₆H₄-CH₂ | |
| 40- | 35 | 2-Cl | 5-Cl | 3-CH₃-C₆H₄-CH₂ | |
| 40- | 36 | 2-Cl | 6-Cl | 3-CH₃-C₆H₄-CH₂ | |
| 40- | 37 | 3-Cl | 4-Cl | 3-CH₃-C₆H₄-CH₂ | |
| 40- | 38 | 3-Cl | 5-Cl | 3-CH₃-C₆H₄-CH₂ | |
| 40- | 39 | H | H | 4-CH₃-C₆H₄-CH₂ | |
| 40- | 40 | 2-F | H | 4-CH₃-C₆H₄-CH₂ | |
| 40- | 41 | 3-F | H | 4-CH₃-C₆H₄-CH₂ | |
| 40- | 42 | 4-F | H | 4-CH₃-C₆H₄-CH₂ | |
| 40- | 43 | 2-Cl | H | 4-CH₃-C₆H₄-CH₂ | |
| 40- | 44 | 3-Cl | H | 4-CH₃-C₆H₄-CH₂ | |
| 40- | 45 | 4-Cl | H | 4-CH₃-C₆H₄-CH₂ | |
| 40- | 46 | 2-Br | H | 4-CH₃-C₆H₄-CH₂ | |
| 40- | 47 | 3-Br | H | 4-CH₃-C₆H₄-CH₂ | |
| 40- | 48 | 4-Br | H | 4-CH₃-C₆H₄-CH₂ | 1H(d6DMSO): 2.22ppm, Ph-CH₃ |
| 40- | 49 | 2-I | H | 4-CH₃-C₆H₄-CH₂ | |
| 40- | 50 | 3-I | H | 4-CH₃-C₆H₄-CH₂ | |
| 40- | 51 | 4-I | H | 4-CH₃-C₆H₄-CH₂ | |
| 40- | 52 | 2-Cl | 3-Cl | 4-CH₃-C₆H₄-CH₂ | |
| 40- | 53 | 2-Cl | 4-Cl | 4-CH₃-C₆H₄-CH₂ | |
| 40- | 54 | 2-Cl | 5-Cl | 4-CH₃-C₆H₄-CH₂ | |
| 40- | 55 | 2-Cl | 6-Cl | 4-CH₃-C₆H₄-CH₂ | |
| 40- | 56 | 3-Cl | 4-Cl | 4-CH₃-C₆H₄-CH₂ | |
| 40- | 57 | 3-Cl | 5-Cl | 4-CH₃-C₆H₄-CH₂ | |
| 40- | 58 | H | H | 2,4,6-Me₃-C₆H₂-CH₂ | |
| 40- | 59 | 2-F | H | 2,4,6-Me₃-C₆H₂-CH₂ | |
| 40- | 60 | 3-F | H | 2,4,6-Me₃-C₆H₂-CH₂ | |
| 40- | 61 | 4-F | H | 2,4,6-Me₃-C₆H₂-CH₂ | |
| 40- | 62 | 2-Cl | H | 2,4,6-Me₃-C₆H₂-CH₂ | |
| 40- | 63 | 3-Cl | H | 2,4,6-Me₃-C₆H₂-CH₂ | |
| 40- | 64 | 4-Cl | H | 2,4,6-Me₃-C₆H₂-CH₂ | |
| 40- | 65 | 2-Br | H | 2,4,6-Me₃-C₆H₂-CH₂ | |
| 40- | 66 | 3-Br | H | 2,4,6-Me₃-C₆H₂-CH₂ | |
| 40- | 67 | 4-Br | H | 2,4,6-Me₃-C₆H₂-CH₂ | |
| 40- | 68 | 2-I | H | 2,4,6-Me₃-C₆H₂-CH₂ | |
| 40- | 69 | 3-I | H | 2,4,6-Me₃-C₆H₂-CH₂ | |
| 40- | 70 | 4-I | H | 2,4,6-Me₃-C₆H₂-CH₂ | |
| 40- | 71 | 2-Cl | 3-Cl | 2,4,6-Me₃-C₆H₂-CH2 | |
| 40- | 72 | 2-Cl | 4-Cl | 2,4,6-Me₃-C₆H₂-CH₂ | |
| 40- | 73 | 2-Cl | 5-Cl | 2,4,6-Me₃-C₆H₂-CH₂ | |
| 40- | 74 | 2-Cl | 6-Cl | 2,4,6-Me₃-C₆H₂-CH₂ | |
| 40- | 75 | 3-Cl | 4-Cl | 2,4,6-Me₃-C₆H₂-CH₂ | |
| 40- | 76 | 3-Cl | 5-Cl | 2,4,6-Me₃-C₆H₂-CH₂ | |
| 40- | 77 | H | H | 2-MeO-C₆H₄-CH₂ | |
| 40- | 78 | 2-F | H | 2-MeO-C₆H₄-CH₂ | |
| 40- | 79 | 3-F | H | 2-MeO-C₆H₄-CH₂ | |
| 40- | 80 | 4-F | H | 2-MeO-C₆H₄-CH₂ | |
| 40- | 81 | 2-Cl | H | 2-MeO-C₆H₄-CH₂ | |
| 40- | 82 | 3-Cl | H | 2-MeO-C₆H₄-CH₂ | |
| 40- | 83 | 4-Cl | H | 2-MeO-C₆H₄-CH₂ | |
| 40- | 84 | 2-Br | H | 2-MeO-C₆H₄-CH₂ | |
| 40- | 85 | 3-Br | H | 2-MeO-C₆H₄-CH₂ | |
| 40- | 86 | 4-Br | H | 2-MeO-C₆H₄-CH₂ | 1H(d6DMSO): 3.71, OMe |
| 40- | 87 | 2-I | H | 2-MeO-C₆H₄-CH₂ | |
| 40- | 88 | 3-I | H | 2-MeO-C₆H₄-CH₂ | |
| 40- | 89 | 4-I | H | 2-MeO-C₆H₄-CH₂ | |
| 40- | 90 | 2-Cl | 3-Cl | 2-MeO-C₆H₄-CH₂ | |
| 40- | 91 | 2-Cl | 4-Cl | 2-MeO-C₆H₄-CH₂ | |
| 40- | 92 | 2-Cl | 5-Cl | 2-MeO-C₆H₄-CH₂ | |
| 40- | 93 | 2-Cl | 6-Cl | 2-MeO-C₆H₄-CH₂ | |
| 40- | 94 | 3-Cl | 4-Cl | 2-MeO-C₆H₄-CH₂ | |
| 40- | 95 | 3-Cl | 5-Cl | 2-MeO-C₆H₄-CH₂ | |
| 40- | 96 | H | H | 3-MeO-C₆H₄-CH₂ | |
| 40- | 97 | 2-F | H | 3-MeO-C₆H₄-CH₂ | |
| 40- | 98 | 3-F | H | 3-MeO-C₆H₄-CH₂ | |
| 40- | 99 | 4-F | H | 3-MeO-C₆H₄-CH₂ | |
| 40- | 100 | 2-Cl | H | 3-MeO-C₆H₄-CH₂ | |
| 40- | 101 | 3-Cl | H | 3-MeO-C₆H₄-CH₂ | |
| 40- | 102 | 4-Cl | H | 3-MeO-C₆H₄-CH₂ | |
| 40- | 103 | 2-Br | H | 3-MeO-C₆H₄-CH₂ | |
| 40- | 104 | 3-Br | H | 3-MeO-C₆H₄-CH₂ | |
| 40- | 105 | 4-Br | H | 3-MeO-C₆H₄-CH₂ | |
| 40- | 106 | 2-I | H | 3-MeO-C₆H₄-CH₂ | |
| 40- | 107 | 3-I | H | 3-MeO-C₆H₄-CH₂ | |
| 40- | 108 | 4-I | H | 3-MeO-C₆H₄-CH₂ | |
| 40- | 109 | 2-Cl | 3-Cl | 3-MeO-C₆H₄-CH₂ | |
| 40- | 110 | 2-Cl | 4-Cl | 3-MeO-C₆H₄-CH₂ | |
| 40- | 111 | 2-Cl | 5-Cl | 3-MeO-C₆H₄-CH₂ | |
| 40- | 112 | 2-Cl | 6-Cl | 3-MeO-C₆H₄-CH₂ | |
| 40- | 113 | 3-Cl | 4-Cl | 3-MeO-C₆H₄-CH₂ | |
| 40- | 114 | 3-Cl | 5-Cl | 3-MeO-C₆H₄-CH₂ | |
| 40- | 115 | H | H | 4-MeO-C₆H₄-CH₂ | |
| 40- | 116 | 2-F | H | 4-MeO-C₆H₄-CH₂ | |
| 40- | 117 | 3-F | H | 4-MeO-C₆H₄-CH₂ | |
| 40- | 118 | 4-F | H | 4-MeO-C₆H₄-CH₂ | |
| 40- | 119 | 2-Cl | H | 4-MeO-C₆H₄-CH₂ | |
| 40- | 120 | 3-Cl | H | 4-MeO-C₆H₄-CH₂ | |
| 40- | 121 | 4-Cl | H | 4-MeO-C₆H₄-CH₂ | |
| 40- | 122 | 2-Br | H | 4-MeO-C₆H₄-CH₂ | |
| 40- | 123 | 3-Br | H | 4-MeO-C₆H₄-CH₂ | |
| 40- | 124 | 4-Br | H | 4-MeO-C₆H₄-CH₂ | ¹H(d₆-DMSO): 6.79, 7.05, MeOPhenyl |
| 40- | 125 | 2-I | H | 4-MeO-C₆H₄-CH₂ | |
| 40- | 126 | 3-I | H | 4-MeO-C₆H₄-CH₂ | |
| 40- | 127 | 4-I | H | 4-MeO-C₆H₄-CH₂ | |
| 40- | 128 | 2-Cl | 3-Cl | 4-MeO-C₆H₄-CH₂ | |
| 40- | 129 | 2-Cl | 4-Cl | 4-MeO-C₆H₄-CH₂ | |
| 40- | 130 | 2-Cl | 5-Cl | 4-MeO-C₆H₄-CH₂ | |
| 40- | 131 | 2-Cl | 6-Cl | 4-MeO-C₆H₄-CH₂ | |
| 40- | 132 | 3-Cl | 4-Cl | 4-MeO-C₆H₄-CH₂ | |
| 40- | 133 | 3-Cl | 5-Cl | 4-MeO-C₆H₄-CH₂ | |
| 40- | 134 | 4-Br | H | 3,4-(MeO)₂-C₆H₃-CH₂ | ¹H(d₆-DMSO): 3.59 (s, 3H, OMe), 3.66 ppm (s, 3H, OMe) |

**Tabelle 41**

| | | | | | |
|---|---|---|---|---|---|
| 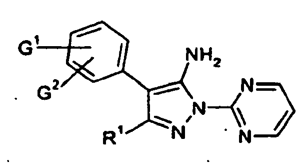 | | | | | |

| Beispielnr. | | G¹ | G² | R¹ | Physikalische Daten |
|---|---|---|---|---|---|
| 41- | 01 | H | H | C₆H₅-CH₂CH₂ | |
| 41- | 02 | 2-F | H | C₆H₅-CH₂CH₂ | |
| 41- | 03 | 3-F | H | C₆H₅₋CH₂CH₂ | |
| 41- | 04 | 4-F | H | C₆H₅-CH₂CH₂ | |
| 41- | 05 | 2-Cl | H | C₆H₅-CH₂CH₂ | |
| 41- | 06 | 3-Cl | H | C₆H₅-CH₂CH₂ | |
| 41- | 07 | 4-Cl | H | C₆H₅-CH₂CH₂ | |
| 41- | 08 | 2-Br | H | C₆H₅-CH₂CH₂ | |
| 41- | 09 | 3-Br | H | C₆H₅-CH₂CH₂ | |
| 41- | 10 | 4-Br | H | C₆H₅-CH₂CH₂ | 1H(6DMSO): 2.86, C₂H₄ |
| 41- | 11 | 2-I | H | C₆H₅-CH₂CH₂ | |
| 41- | 12 | 3-I | H | C₆H₅-CH₂CH₂ | |
| 41- | 13 | 4-I | H | C₆H₅-CH₂CH₂ | |
| 41- | 14 | 2-Cl | 3-Cl | C₆H₅-CH₂CH₂ | |
| 41- | 15 | 2-Cl | 4-Cl | C₆H₅-CH₂CH₂ | |
| 41- | 16 | 2-Cl | 5-Cl | C₆H₅-CH₂CH₂ | |
| 41- | 17 | 2-Cl | 6-Cl | C₆H₅-CH₂CH₂ | |
| 41- | 18 | 3-Cl | 4-Cl | C₆H₅-CH₂CH₂ | |
| 41- | 19 | 3-Cl | 5-Cl | C₆H₅-CH₂CH₂ | |
| 41- | 20 | H | H | 2-Cl-C₆H₄-CH₂CH₂ | |
| 41- | 21 | 2-F | H | 2-Cl-C₆H₄-CH₂CH₂ | |
| 41- | 22 | 3-F | H | 2-Cl-C₆H₄-CH₂CH₂ | |
| 41- | 23 | 4-F | H | 2-Cl-C₆H₄-CH₂CH₂ | |
| 41- | 24 | 2-Cl | H | 2-Cl-C₆H₄-CH₂CH₂ | |
| 41- | 25 | 3-Cl | H | 2-Cl-C₆H₄-CH₂CH₂ | |
| 41- | 26 | 4-Cl | H | 2-Cl-C₆H₄-CH₂CH₂ | |
| 41- | 27 | 2-Br | H | 2-Cl-C₆H₄-CH₂CH₂ | |
| 41- | 28 | 3-Br | H | 2-Cl-C₆H₄-CH₂CH₂ | |
| 41- | 29 | 4-Br | H | 2-Cl-C₆H₄-CH₂CH₂ | |
| 41- | 30 | 2-I | H | 2-Cl-C₆H₄-CH₂CH₂ | |
| 41- | 31 | 3-I | H | 2-Cl-C₆H₄-CH₂CH₂ | |
| 41- | 32 | 4-I | H | 2-Cl-C₆H₄-CH₂CH₂ | |
| 41- | 33 | 2-Cl | 3-Cl | 2-Cl-C₆H₄-CH₂CH₂ | |
| 41- | 34 | 2-Cl | 4-Cl | 2-Cl-C₆H₄-CH₂CH₂ | |
| 41- | 35 | 2-Cl | 5-Cl | 2-Cl-C₆H₄-CH₂CH₂ | |
| 41- | 36 | 2-Cl | 6-Cl | 2-Cl-C₆H₄-CH₂CH₂ | |
| 41- | 37 | 3-Cl | 4-Cl | 2-Cl-C₆H₄-CH₂CH₂ | |
| 41- | 38 | 3-Cl | 5-Cl | 2-Cl-C₆H₄-CH₂CH₂ | |
| 41- | 39 | H | H | 3-Cl-C₆H₄-CH₂CH₂ | |
| 41- | 40 | 2-F | H | 3-ClC₆H₄-CH₂CH₂ | |
| 41- | 41 | 3-F | H | 3-Cl-C₆H₄-CH₂CH₂ | |
| 41- | 42 | 4-F | H | 3-ClC₆H₄-CH₂CH₂ | |
| 41- | 43 | 2-Cl | H | 3-ClC₆H₄-CH₂CH₂ | |
| 41- | 44 | 3-Cl | H | 3-ClC₆H₄-CH₂CH₂ | |
| 41- | 45 | 4-Cl | H | 3-Cl-C₆H₄-CH₂CH₂ | |
| 41- | 46 | 2-Br | H | 3-ClC₆H₄-CH₂CH₂ | |
| 41- | 47 | 3-Br | H | 3-Cl-C₆H₄-CH₂CH₂ | |
| 41- | 48 | 4-Br | H | 3-Cl-C₆H₄-CH₂CH₂ | |
| 41- | 49 | 2-I | H | 3-Cl-C₆H₄-CH₂CH₂ | |
| 41- | 50 | 3-I | H | 3-Cl-C₆H₄-CH₂CH₂ | |
| 41- | 51 | 4-I | H | 3-Cl-C₆H₄-CH₂CH₂ | |
| 41- | 52 | 2-Cl | 3-Cl | 3-Cl-C₆H₄-CH₂CH₂ | |
| 41- | 53 | 2-Cl | 4-Cl | 3-ClC₆H₄-CH₂CH₂ | |
| 41- | 54 | 2-Cl | 5-Cl | 3-Cl-C₆H₄-CH₂CH₂ | |
| 41- | 55 | 2-Cl | 6-Cl | 3-Cl-C₆H₄-CH₂CH₂ | |
| 41- | 56 | 3-Cl | 4-Cl | 3-Cl-C₆H₄-CH₂CH₂ | |
| 41- | 57 | 3-Cl | 5-Cl | 3-Cl-C₆H₄-CH₂CH₂ | |
| 41- | 58 | H | H | 4-Cl-C₆H₄-CH₂CH₂ | |
| 41- | 59 | 2-F | H | 4-Cl-C6H₄-CH₂CH₂ | |
| 41- | 60 | 3-F | H | 4-Cl-C₆H₄-CH₂CH₂ | |
| 41- | 61 | 4-F | H | 4-Cl-C₆H₄-CH₂CH₂ | |
| 41- | 62 | 2-Cl | H | 4-Cl-C₆H₄-CH₂CH₂ | |
| 41- | 63 | 3-Cl | H | 4-Cl-C₆H₄-CH₂CH₂ | |
| 41- | 64 | 4-Cl | H | 4-Cl-C₆H₄-CH₂CH₂ | |
| 41- | 65 | 2-Br | H | 4-Cl-C₆H₄-CH₂CH₂ | |
| 41- | 66 | 3-Br | H | 4-Cl-C₆H₄-CH₂CH₂ | |
| 41- | 67 | 4-Br | H | 4-Cl-C₆H₄-CH₂CH₂ | |
| 41- | 68 | 2-I | H | 4-Cl-C₆H₄-CH₂CH₂ | |
| 41- | 69 | 3-I | H | 4-Cl-C₆H₄-CH₂CH₂ | |
| 41- | 70 | 4-I | H | 4-Cl-C₆H₄-CH₂CH₂ | |
| 41- | 71 | 2-Cl | 3-Cl | 4-Cl-C₆H₄-CH₂CH₂ | |
| 41- | 72 | 2-Cl | 4-Cl | 4-Cl-C₆H₄-CH₂CH₂ | |
| 41- | 73 | 2-Cl | 5-Cl | 4-Cl-C₆H₄-CH₂CH₂ | |
| 41- | 74 | 2-Cl | 6-Cl | 4-Cl-C₆H₄-CH₂CH₂ | |
| 41- | 75 | 3-Cl | 4-Cl | 4-Cl-C₆H₄-CH₂CH₂ | |
| 41- | 76 | 3-Cl | 5-Cl | 4-Cl-C₆H₄-CH₂CH₂ | |
| 41- | 77 | H | H | C₆H₅-CH₂CH₂CH₂ | |
| 41- | 78 | 2-F | H | C₆H₅-CH₂CH₂CH₂ | |
| 41- | 79 | 3-F | H | C₆H₅-CH₂CH₂CH₂ | |
| 41- | 80 | 4-F | H | C₆H₅-CH₂CH₂CH₂ | |
| 41- | 81 | 2-Cl | H | C₆H₅-CH₂CH₂CH₂ | |
| 41- | 82 | 3-Cl | H | C₆H₅-CH₂CH₂CH₂ | |
| 41- | 83 | 4-Cl | H | C₆H₅-CH₂CH₂ | |
| 41- | 84 | 2-Br | H | C₆H₅-CH₂CH₂CH₂ | |
| 41- | 85 | 3-Br | H | C₆H₅-CH₂CH₂CH₂ | |
| 41- | 86 | 4-Br | H | C₆H₅-CH₂CH₂CH₂ | 1H(d6DMSO): 1.79, 2.55ppm C₃H₆ |
| 41- | 87 | 2-I | H | C₆H₅-CH₂CH₂CH₂ | |
| 41- | 88 | 3-I | H | C₆H₅-CH₂CH₂CH₂ | |
| 41- | 89 | 4-I | H | C₆H₅-CH₂CH₂CH₂ | |
| 41- | 90 | 2-Cl | 3-Cl | C₆H₅-CH₂CH₂CH₂ | |
| 41- | 91 | 2-Cl | 4-Cl | C₆H₅-CH₂CH₂CH₂ | |
| 41- | 92 | 2-Cl | 5-Cl | C₆H₅-CH₂CH₂CH₂ | |
| 41- | 93 | 2-Cl | 6-Cl | C₆H₅-CH₂CH₂CH₂ | |
| 41- | 94 | 3-Cl | 4-Cl | C₆H₅-CH₂CH₂CH₂ | |
| 41- | 95 | 3-Cl | 5-Cl | C₆H₅-CH₂CH₂CH₂ | |

**Tabelle 42**

| | | | | | | |
|---|---|---|---|---|---|---|
| 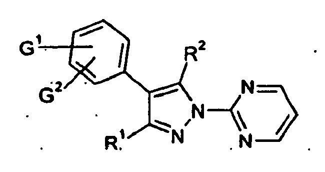 | | | | | | |

| Beispielnr. | | G¹ | G² | R¹ | R² | Physikalische Daten |
|---|---|---|---|---|---|---|
| 42- | 01 | H | H | CH₂F | NHEt | |
| 42- | 02 | 2-F | H | CH₂F | NHEt | |
| 42- | 03 | 3-F | H | CH₂F | NHEt | |
| 42- | 04 | 4-F | H | CH₂F | NHEt | |
| 42- | 05 | 2-Cl | H | CH₂F | NHEt | |
| 42- | 06 | 3-Cl | H | CH₂F | NHEt | |
| 42- | 07 | 4-Cl | H | CH₂F | NHEt | |
| 42- | 08 | 2-Br | H | CH₂F | NHEt | |
| 42- | 09 | 3-Br | H | CH₂F | NHEt | |
| 42- | 10 | 4-Br | H | CH₂F | NHEt | |
| 42- | 11 | 2-I | H | CH₂F | NHEt | |
| 42- | 12 | 3-I | H | CH₂F | NHEt | |
| 42- | 13 | 4-I | H | CH₂F | NHEt | |
| 42- | 14 | 2-Cl | 3-Cl | CH₂F | NHEt | |
| 42- | 15 | 2-Cl | 4-Cl | CH₂F | NHEt | |
| 42- | 16 | 2-Cl | 5-Cl | CH₂F | NHEt | |
| 42- | 17 | 2-Cl | 6-Cl | CH₂F | NHEt | |
| 42- | 18 | 2-Cl | 4-F | CH₂F | NHEt | |
| 42- | 19 | 3-Cl | 4-Cl | CH₂F | NHEt | |
| 42- | 20 | 3-Cl | 5-Cl | CH₂F | NHEt | NMR siehe unten |
| 42- | 21 | H | H | CH₂F | NHCH₂Ph | |
| 42- | 22 | 2-F | H | CH₂F | NHCH₂Ph | |
| 42- | 23 | 3-F | H | CH₂F | NHCH₂Ph | |
| 42- | 24 | 4-F | H | CH₂F | NHCH₂Ph | |
| 42- | 25 | 2-Cl | H | CH₂F | NHCH₂Ph | |
| 42- | 26 | 3-Cl | H | CH₂F | NHCH₂Ph | |
| 42- | 27 | 4-Cl | H | CH₂F | NHCH₂Ph | |
| 42- | 28 | 2-Br | H | CH₂F | NHCH₂Ph | |
| 42- | 29 | 3-Br | H | CH₂F | NHCH₂Ph | |
| 42- | 30 | 4-Br | H | CH₂F | NHCH₂Ph | |
| 42- | 31 | 2-I | H | CH₂F | NHCH₂Ph | |
| 42- | 32 | 3-I | H | CH₂F | NHCH₂Ph | |
| 42- | 33 | 4-I | H | CH₂F | NHCH₂Ph | |
| 42- | 34 | 2-Cl | 3-Cl | CH₂F | NHCH₂Ph | |
| 42- | 35 | 2-Cl | 4-Cl | CH₂F | NHCH₂Ph | |
| 42- | 36 | 2-Cl | 5-Cl | CH₂F | NHCH₂Ph | |
| 42- | 37 | 2-Cl | 6-Cl | CH₂F | NHCH₂Ph | |
| 42- | 38 | 3-Cl | 4-Cl | CH₂F | NHCH₂Ph | |
| 42- | 39 | 3-Cl | 5-Cl | CH₂F | NHCH₂Ph | NMR siehe unten |
| 42- | 40 | H | H | CH₂F | N(CH₂Ph)₂ | |
| 42- | 41 | 2-F | H | CH₂F | N(CH₂Ph)₂ | |
| 42- | 42 | 3-F | H | CH₂F | N(CH₂Ph)₂ | |
| 42- | 43 | 4-F | H | CH₂F | N(CH₂Ph)₂ | |
| 42- | 44 | 2-Cl | H | CH₂F | N(CH₂Ph)₂ | |
| 42- | 45 | 3-Cl | H | CH₂F | N(CH₂Ph)₂ | |
| 42- | 46 | 4-Cl | H | CH₂F | N(CH₂Ph)₂ | |
| 42- | 47 | 2-Br | H | CH₂F | N(CH₂Ph)₂ | |
| 42- | 48 | 3-Br | H | CH₂F | N(CH₂Ph)₂ | |
| 42- | 49 | 4-Br | H | CH₂F | N(CH₂Ph)₂ | |
| 42- | 50 | 2-I | H | CH₂F | N(CH₂Ph)₂ | |
| 42- | 51 | 3-I | H | CH₂F | N(CH₂Ph)₂ | |
| 42- | 52 | 4-I | H | CH₂F | N(CH₂Ph)₂ | |
| 42- | 53 | 2-Cl | 3-Cl | CH₂F | N(CH₂Ph)₂ | |
| 42- | 54 | 2-Cl | 4-Cl | CH₂F | N(CH₂Ph)₂ | |
| 42- | 55 | 2-Cl | 5-Cl | CH₂F | N(CH₂Ph)₂ | |
| 42- | 56 | 2-Cl | 6-Cl | CH₂F | N(CH₂Ph)₂ | |
| 42- | 57 | 3-Cl | 4-Cl | CH₂F | N(CH₂Ph)₂ | |
| 42- | 58 | 3-Cl | 5-Cl | CH₂F | N(CH₂Ph)₂ | NMR siehe unten |
| 42- | 59 | H | H | CHF₂ | NEt₂ | |
| 42- | 60 | 2-F | H | CHF₂ | NEt₂ | |
| 42- | 61 | 3-F | H | CHF₂ | NEt₂ | |
| 42- | 62 | 4-F | H | CHF₂ | NEt₂ | |
| 42- | 63 | 2-Cl | H | CHF₂ | NEt₂ | |
| 42- | 64 | 3-Cl | H | CHF₂ | NEt₂ | |
| 42- | 65 | 4-Cl | H | CHF₂ | NEt₂ | |
| 42- | 66 | 2-Br | H | CHF₂ | NEt₂ | |
| 42- | 67 | 3-Br | H | CHF₂ | NEt₂ | |
| 42- | 68 | 4-Br | H | CHF₂ | NEt₂ | |
| 42- | 69 | 2-I | H | CHF₂ | NEt₂ | |
| 42- | 70 | 3-I | H | CHF₂ | NEt₂ | |
| 42- | 71 | 4-I | H | CHF₂ | NEt₂ | |
| 42- | 72 | 2-Cl | 3-Cl | CHF₂ | NEt₂ | |
| 42- | 73 | 2-Cl | 4-Cl | CHF₂ | NEt₂ | |
| 42- | 74 | 2-Cl | 5-Cl | CHF₂ | NEt₂ | |
| 42- | 75 | 2-Cl | 6-Cl | CHF₂ | NEt₂ | |
| 42- | 76 | 3-Cl | 4-Cl | CHF₂ | NEt₂ | |
| 42- | 77 | 3-Cl | 5-Cl | CHF₂ | NEt₂ | NMR siehe unten |
| 42- | 78 | H | H | CHF₂ | NHCH₂Ph | |
| 42- | 79 | 2-F | H | CHF₂ | NHCH₂Ph | |
| 42- | 80 | 3-F | H | CHF₂ | NHCH₂Ph | |
| 42- | 81 | 4-F | H | CHF₂ | NHCH₂Ph | |
| 42- | 82 | 2-Cl | H | CHF₂ | NHCH₂Ph | |
| 42- | 83 | 3-Cl | H | CHF₂ | NHCH₂Ph | |
| 42- | 84 | 4-Cl | H | CHF₂ | NHCH₂Ph | |
| 42- | 85 | 2-Br | H | CHF₂ | NHCH₂Ph | |
| 42- | 86 | 3-Br | H | CHF₂ | NHCH₂Ph | |
| 42- | 87 | 4-Br | H | CHF₂ | NHCH₂Ph | |
| 42- | 88 | 2-I | H | CHF₂ | NHCH₂Ph | |
| 42- | 89 | 3-I | H | CHF₂ | NHCH₂Ph | |
| 42- | 90 | 4-I | H | CHF₂ | NHCH₂Ph | |
| 42- | 91 | 2-Cl | 3-Cl | CHF₂ | NHCH₂Ph | |
| 42- | 92 | 2-Cl | 4-Cl | CHF₂ | NHCH₂Ph | |
| 42- | 93 | 2-Cl | 5-Cl | CHF₂ | NHCH₂Ph | |
| 42- | 94 | 2-Cl | 6-Cl | CHF₂ | NHCH₂Ph | |
| 42- | 95 | 3-Cl | 4-Cl | CHF₂ | NHCH₂Ph | |
| 42- | 96 | 3-Cl | 5-Cl | CHF₂ | NHCH₂Ph | NMR siehe unten |
| 42- | 97 | H | H | CHF₂ | N(CH₂Ph)₂ | |
| 42- | 98 | 2-F | H | CHF₂ | N(CH₂Ph)₂ | |
| 42- | 99 | 3-F | H | CHF₂ | N(CH₂Ph)₂ | |
| 42- | 100 | 4-F | H | CHF₂ | N(CH₂Ph)₂ | |
| 42- | 101 | 2-Cl | H | CHF₂ | N(CH₂Ph)₂ | |
| 42- | 102 | 3-Cl | H | CHF₂ | N(CH₂Ph)₂ | |
| 42- | 103 | 4-Cl | H | CHF₂ | N(CH₂Ph)₂ | |
| 42- | 104 | 2-Br | H | CHF₂ | N(CH₂Ph)₂ | |
| 42- | 105 | 3-Br | H | CHF₂ | N(CH₂Ph)₂ | |
| 42- | 106 | 4-Br | H | CHF₂ | N(CH₂Ph)₂ | |
| 42- | 107 | 2-I | H | CHF₂ | N(CH₂Ph)₂ | |
| 42- | 108 | 3-I | H | CHF₂ | N(CH₂Ph)₂ | |
| 42- | 109 | 4-I | H | CHF₂ | N(CH₂Ph)₂ | |
| 42- | 110 | 2-Cl | 3-Cl | CHF₂ | N(CH₂Ph)₂ | |
| 42- | 111 | 2-Cl | 4-Cl | CHF₂ | N(CH₂Ph)₂ | |
| 42- | 112 | 2-Cl | 5-Cl | CHF₂ | N(CH₂Ph)₂ | |
| 42- | 113 | 2-Cl | 6-Cl | CHF₂ | N(CH₂Ph)₂ | |
| 42- | 114 | 3-Cl | 4-Cl | CHF₂ | N(CH₂Ph)₂ | |
| 42- | 115 | 3-Cl | 5-Cl | CHF₂ | N(CH₂Ph)₂ | NMR siehe unten |
| 42- | 116 | H | H | CHF₂ | NHCOCH₃ | |
| 42- | 117 | 2-F | H | CHF₂ | NHCOCH₃ | |
| 42- | 118 | 3-F | H | CHF₂ | NHCOCH₃ | |
| 42- | 119 | 4-F | H | CHF₂ | NHCOCH₃ | |
| 42- | 120 | 2-Cl | H | CHF₂ | NHCOCH₃ | |
| 42- | 121 | 3-Cl | H | CHF₂ | NHCOCH₃ | |
| 42- | 122 | 4-Cl | H | CHF₂ | NHCOCH₃ | |
| 42- | 123 | 2-Br | H | CH₂ | NHCOCH₃ | |
| 42- | 124 | 3-Br | H | CHF₂ | NHCOCH₃ | |
| 42- | 125 | 4-Br | H | CHF₂ | NHCOCH₃ | |
| 42- | 126 | 2-I | H | CHF₂ | NHCOCH₃ | |
| 42- | 127 | 3-I | H | CHF₂ | NHCOCH₃ | |
| 42- | 128 | 4-I | H | CHF₂ | NHCOCH₃ | |
| 42- | 129 | 2-Cl | 3-Cl | CHF₂ | NHCOCH₃ | |
| 42- | 130 | 2-Cl | 4-Cl | CHF₂ | NHCOCH₃ | |
| 42- | 131 | 2-Cl | 5-Cl | CHF₂ | NHCOCH₃ | |
| 42- | 132 | 2-Cl | 6-Cl | CHF₂ | NHCOCH₃ | |
| 42- | 133 | 2-Cl | 4-F | CHF₂ | NHCOCH₃ | |
| 42- | 134 | 3-Cl | 4-Cl | CHF₂ | NHCOCH₃ | |
| 42- | 135 | 3-Cl | 5-Cl | CHF₂ | NHCOCH₃ | NMR siehe unten |

**Tabelle 43**

| | | | | | |
|---|---|---|---|---|---|
| 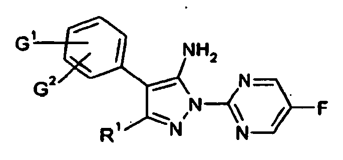 | | | | | |

| Beispielnr. | | G¹ | G² | R¹ | Physikalische Daten |
|---|---|---|---|---|---|
| 43- | 01 | 3-CF₃ | 5-Cl | (R)-CH(CH₃)F | ¹H-NMR (CD₃CN): 8.71 ppm (s, 1H) |
| 43- | 02 | 3-CF₃ | 4-Cl | CF₃ | NMR siehe unten |
| 43- | 03 | 3-CF₃ | 5-Cl | CF₂Br | NMR siehe unten |
| 43- | 04 | 3-CF₃ | 5-Cl | CF₂Cl | NMR siehe unten |
| 43- | 05 | 3-CF₃ | 5-Cl | CF₃ | NMR siehe unten |
| 43- | 06 | 3-CF₃ | 5-Cl | CF₂CH₃ | NMR siehe unten |
| 43- | 07 | 3-Cl | 5-F | CF₃ | lgP = 3,24 |
| 43- | 08 | 3-F | 5-F | CF₃ | lgP = 2,93 |
| 43- | 09 | 2-F | 4-Br | CF₃ | lgP = 3,15 |
| 43- | 10 | 3-F | 4-F | CF₃ | lgP = 2,84 |
| 43- | 11 | 3-Br | 5-Br | CF₃ | lgP = 3,84 |
| 43- | 12 | 3-CH₃ | 3-CH₃ | CF₃ | lgP = 3,28 |
| 43- | 13 | 3-CF₃ | 5-Cl | CF₂CF₃ | |
| 43- | 14 | 3-CF₃ | 5-Cl | CCl₂F | |
| 43- | 15 | 3-CF₃ | 4-Cl | CH(CH₃)F | |
| 43- | 16 | 3-CF₃ | 4-Cl | CF₂Br | |
| 43- | 17 | 3-CF₃ | 4-Cl | CF₂Cl | |
| 43- | 18 | 3-CF₃ | 4-Cl | CCl₂F | |
| 43- | 19 | 3-CF₃ | 4-Cl | CF₂CH₃ | |
| 43- | 20 | 3-CF₃ | 4-Cl | CF₂CF3 | |
| 43- | 21 | 3-Cl | 5-Cl | CF₂Br | |
| 43- | 22 | 3-Cl | 5-Cl | CF₂Cl | |
| 43- | 23 | 3-Cl | 5-Cl | CCl₂F | |
| 43- | 24 | 3-Cl | 5-Cl | CH(CH₃)F | |
| 43- | 25 | 3-Cl | 5-Cl | CF₂CH₃ | |
| 43- | 26 | 3-Cl | 5-Cl | CF₂CF₃ | |
| 43- | 27 | 3-CF₃ | 5-CF₃ | CF₃ | |
| 43- | 28 | 3-CF₃ | 5-CF₃ | CF₂Br | |
| 43- | 29 | 3-CF₃ | 5-CF₃ | CF₂Cl | |
| 43- | 30 | 3-CF₃ | 5-CF₃ | CCl₂F | |
| 43- | 31 | 3-CF₃ | 5-CF₃ | CH(CH₃)F | |
| 43- | 32 | 3-CF₃ | 5-CF₃ | CF₂CH₃ | |
| 43- | 33 | 3-CF₃ | 5-CF₃ | CF₂CF₃ | |
| 43- | 34 | 4-I | H | CF₂Br | |
| 43- | 35 | 4-I | H | CF₂Cl | |
| 43- | 36 | 4-I | H | CCl₂F | |
| 43- | 37 | 4-I | H | CH(CH₃)F | |
| 43- | 38 | 4-I | H | CF₂CH₃ | |
| 43- | 39 | 4-I | H | CF₂CF₃ | |
| 43- | 40 | 3-Cl | 4-Cl | CF₂Br | |
| 43- | 41 | 3-Cl | 4-Cl | CF₂Cl | |
| 43- | 42 | 3-Cl | 4-Cl | CCl₂F | |
| 43- | 43 | 3-Cl | 4-Cl | CH(CH₃)F | |
| 43- | 44 | 3-Cl | 4-Cl | CF₂CH₃ | |
| 43- | 45 | 3-Cl | 4-Cl | CF₂CF₃ | |

Die Bestimmung der in den Tabellen angegebenen logP-Werte erfolgte gemäß EEC-Directive 79/831 Annex V.A8 durch HPLC (Gradientenmethode, Acetonitril / 0,1 %ige wässrige Phosphorsäure oder Ameisensäure).

### NMR-Daten zu den Synthesebeispielen

### Bsp.Nr. 1-01

¹H-NMR (400 MHz, CDCl₃), δ 8.83 (d, 2H), 7.37 (m, 2H), 7.21 (m, 2H), 7.16 (m, 1H), 6.03 (br. s, 2H, NH).

### Bsp.Nr.1-02

¹H-NMR (400 MHz, d₆-DMSO, δ, ppm): 8.93 (d, 2H), 7.53-7.46 (m, 2H), 7.21-7.14 (m, 3H), 6.83 (breites s, 2H, NH₂).

### Bsp.Nr. 1-03

¹H-NMR (400 MHz, CD₃CN, δ, ppm): 8.83 (d, 2H), 7.43-7.35 (m, 3H), 7.22-7.16 (m, 2H), 6.17 (breites s, 2H, NH₂).

### Bsp.Nr. 1-06

¹H-NMR (400 MHz, CD₃CN, δ, ppm): 8.83 (d, 2H), 7.48 (d, 2H); 7.39-7.35 (m, 3H), 6.23 (breites s, 2H, NH₂).

### Bsp.Nr. 1-07

¹H-NMR (400 MHz, CDCl₃), δ 8.84 (d, 2H), 7.37 (m, 2H), 7.28 (t, 1H), 7.22 (m, 1H), 7.12 (m, 1H), 6.01 (br. s, 2H, NH).

### Bsp.Nr:1-08

¹H-NMR (400 MHz, d₆-DMSO), δ 8.96 (d, 2H), 7.55 (m, 3H), 7.42 (t, 1H), 7.36 (d, 1H), 7.04 (s, 2H, NH).

### Bsp.Nr. 1-09

¹H-NMR (400 MHz, CD₃CN, δ, ppm): 8.83 (d, 2H), 7.61 (d, 2H), 7.37 (t, 1H), 7.33 (d, 2H), 6.23 (breites s, 2H, NH₂).

### Bsp.Nr. 1-10

¹H-NMR (400 MHz, CDCl₃), δ 8.82 (d, 2H), 7.96 (d, 1H), 7.42 (d, 1H), 7.37 (dd, 1H), 7.25 (t, 1H), 7.08 (dd, 1H), 5.84 (s, 2H, NH).

### Bsp.Nr. 1-11

¹H-NMR (400 MHz, CDCl₃), δ 8.83 (d, 2H), 7.78 (s, 1H), 7.69 (d, 1H), 7.39 (d, 1H), 7.28 (t, 1H), 7.18 (t, 1H), 6.05 (s, 2H, NH).

### Bsp.Nr. 1-12

¹H-NMR (400 MHz, CDCl₃), δ 8.82 (d, 2H), 7.78 (d, 2H), 7.27 (t, 1H), 7.14 (d, 2H), 6.02 (s, 2H, NH).

### Bsp.Nr. 1-13

¹H-NMR (400 MNz, d₆-DMSO), δ 8.96 (d, 2H), 7.56 (t, 1H), 7.44 (m, 1H), 7.26 (m, 1H), 7.16 (m, 1H), 7.11 (s, 2H, NH).

### Bsp.Nr. 1-14

¹H-NMR (400 MHz, CDCl₃), δ 8.84 (d, 2H), 7.36 (m, 1H), 7.28 (t, 1H), 6.92 (m, 2H), 5.99 (s, 2H, NH).

### Bsp.Nr. 1-15

¹H-NMR (400 MHz, CDCl₃), δ 8.83 (d, 2H), 7.25 (t, 1H), 7.04-7.17 (m, 3H), 6.09 (s, 2H, NH).

### Bsp.Nr. 1-17

¹H-NMR (400 MHz, CDCl₃), δ 8.84 (d, 2H), 7.42 (dt, 1H), 7.28 (m, 2H), 7.17 (t, 1H), 6.06 (s, 2H, NH).

### Bsp.Nr. 1-18

¹H-NMR (400 MHz, CDCl₃), δ 8.84 (d, 2H), 7.29-7.36 (m, 3H), 7.19 (m, 1H), 6.03 (s, 2H, NH).

### Bsp.Nr. 1-20

¹H-NMR (400 MHz, CDCl₃), δ 8.85 (d, 2H), 7.57 (m, 1H), 7.34 (m, 2H), 7.12 (dt, 1H), 6.52 (br. s, 2H, NH).

### Bsp.Nr. 1-22

¹H-NMR (400 MHz, CDCl₃), δ 8.82 (d, 2H), 7.39 (m, 2H), 7.32 (t, 1H), 7.25 (m, 1H), 6.41 (s, 2H, NH).

### Bsp.Nr. 1-23

¹H-NMR (400 MHz, CDCl₃), δ 8.85 (d, 2H), 7.49 (m, 2H), 7.35 (dd, 1H), 7.09 (t, 1H), 6.37 (s, 2H, NH).

### Bsp.Nr. 1-30

¹H-NMR (400 MHz, CDCl₃), δ 8.84 (d, 2H), 7.36 (dd, 1H), 7.29 (m, 1H), 7.27 (t, 1H), 7.06 (dt, 1H), 5.92 (br. s, 2H, NH).

### Bsp.Nr. 1-32

¹H-NMR (400 MHz, d₆-DMSO), δ 8.94 (d, 2H), 7.69 (dd, 1H), 7.55 (t, 1H), 7.42 (dd, 1H), 7.37 (dd, 1H), 7.03 (s, 2H, NH).

### Bsp.Nr. 1-33

¹H-NMR (400 MHz, CD₃CN, δ, ppm): 8.83 (d, 2H), 7.63 (s, 1H), 7.43-7.36 (m, 3H), 6.18 (breites s, 2H, NH₂).

### Bsp.Nr. 1 -34

¹H-NMR (400 MHz, d₆-DMSO), δ 8.96 (d, 2H), 7.62 (d, 1H), 7.50 (m, 2H), 7.47 (d, 1H), 7.05 (s, 2H, NH).

### Bsp.Nr. 1-35

¹H-NMR (400 MHz, CDCl₃), δ 8.85 (d, 2H), 7.58 (m, 1H), 7.41 (m, 1H), 7.35 (t, 1H), 7.13 (m, 1H), 6.54 (br. s, 2H, NH).

### Bsp.Nr. 1-38

¹H-NMR (400 MHz, CDCl₃), δ 8.83 (d, 2H), 7.49 (dd, 1H), 7.46 (d, 1H), 7.38 (d, 1H), 7.28 (t, 1H), 5.97 (br. s, 2H, NH).

### Bsp.Nr. 1-71

¹H-NMR (400 MHz, CDCl₃), δ 8.84 (d, 2H), 7.22-7.31 (m, 3H), 7.16 (m, 1H), 6.04 (s, 2H, NH).

### Bsp.Nr. 1-73

¹H-NMR (400 MHz, d₆-DMSO), δ 8.94 (d, 2H), 7.64 (t, 1H), 7.57 (t, 1H), 7.37 (dd, 1H), 7.19 (dd, 1H), 7.09 (s, 2H, NH).

### Bsp.Nr. 1-74

¹H-NMR (400 MHz, CDCl₃), δ 8.82 (d, 2H), 7.28 (t, 1H), 7.20 (s, 1H), 7.03 (m, 2H), 6.18 (s, 2H, NH).

### Bsp.Nr. 1-75

¹H-NMR (400 MHz, CDCl₃), δ 8.84 (d, 2H), 7.61 (t, 1H), 7.28 (t, 1H), 7.19 (dd, 1H), 7.09 (d, 1H), 6.09 (br. s, 2H, NH).

### Bsp.Nr. 1-79

¹H-NMR (400 MHz, CDCl₃), δ 8.82 (d, 2H), 7.46 (dd, 1H), 7.21-7.30 (m, 3H), 6.03 (s, 2H, NH).

### Bsp.Nr. 1-80

¹H-NMR (400 MHz, CD₃CN, δ, ppm): 8.84 (d, 2H), 7.60-7.55 (d+s, 2H), 7.37 (t, 1H), 7.33 (d, 1H), 6.33 (breites s, 2H, NH₂).

### Bsp.Nr. 1-81

¹H-NMR (400 MHz, CD₃CN, δ, ppm): 8.84 (d, 2H), 7.46 (s, 1H), 7.39-7.36 (m, 3H), 6.38 (breites s, 2H, NH₂).

### Bsp.Nr. 1-82

¹H-NMR (400 MHz, d₆-DMSO), δ 8.95 (d, 2H), 7.84 (d, 1H), 7.57 (d, 1H), 7.54 (t, 1H), 7.23 (dd, 1H), 7.12 (s, 2H, NH).

### Bsp.Nr. 1-83

¹H-NMR (400 MHz, CDCl₃), δ 8.84 (d, 2H), 7.52 (m, 1H), 7.46 (m, 1H), 7.36 (m, 1H), 7.29 (t, 1H), 6.17 (br. s, 2H, NH).

### Bsp.Nr. 1-87

¹H-NMR (400 MHz, d₆-DMSO), δ 8.97 (d, 2H), 7.72 (d, 1H), 7.69 (d, 1H), 7.56 (t, 1H), 7.37 (dd, 1H), 7.14 (br. s, 2H, NH).

### Bsp.Nr. 1-88

¹H-NMR (400 MHz, CDCl₃), δ 8.83 (d, 2H), 7.70 (m, 1H), 7.68 (m, 1H), 7.28 (t, 1H), 7.20 (dd, 1H), 6.09 (s, 2H, NH).

### Bsp.Nr. 1-89

¹H-NMR (400 MHz, CDCl₃), δ 8.84 (d, 2H), 7.67 (m, 1H), 7.52 (m, 2H), 7.30 (t, 1H), 6.12 (s, 2H, NH).

### Bsp.Nr. 2-02

¹H-NMR (400 MHz, CDCl₃), δ 8.84 (d, 2H), 7.29 (t, 1H), 6.88-7.04 (m, 2H), 6.13 (s, 2H, NH).

### Bsp.Nr. 2-04

¹H-NMR (400 MHz, d₆-DMSO), δ 8.96 (d, 2H), 7.64 (m, 1H), 7.56 (t, 1H), 7.50 (m, 1H), 7.13 (s, 2H, NH).

### Bsp.Nr. 2-05

¹H-NMR (400 MHz, CDCl₃), δ 8.81 (d, 2H), 7.28 (t, 1H), 6.78 (d, 1H), 6.75 (d, 1H), 6.08 (s, 2H, NH).

### Bsp.Nr. 2-06

¹H-NMR (400 MHz, d₆-DMSO, δ, ppm): 8.93 (d, 2H), 7.53 (t, 1H), 7.26-7.22 (dd, 2H), 6.96 (breites s, 2H, NH₂).

### Bsp.Nr. 2-12

¹H-NMR (400 MHz, CDCl₃), δ 8.84 (d, 2H), 7.65 (s, 1H), 7.49 (s, 1H), 7.27 (t, 1H), 5.98 (s, 2H).

### Bsp.Nr. 2-13

¹H-NMR (400 MHz, CDCl₃), δ 8.82 (d, 2H), 7.25 (t, 1H), 7.04 (d, 2H), 6.04 (s, 2H, NH).

### Bsp.Nr. 2-14

¹H-NMR (400 MHz, CDCl₃), δ 8.84 (d, 2H), 7.58 (m, 1H), 7.28 (t, 1H), 7.20 (m, 1H), 5.98 (s, 2H, NH).

### Bsp.Nr. 2-15

¹H-NMR (400 MHz, CDCl₃), δ 8.85 (d, 2H), 7.38 (m, 1H), 7.33 (t, 1H), 7.22 (m, 1H), 6.32 (br. s, 2H, NH).

### Bsp.Nr. 2-16

¹H-NMR (400 MHz, CDCl₃), δ 8.84 (d, 2H), 7.32 (t, 1H), 7.14 (m, 1H), 6.09 (br. d, 2H, NH).

### Bsp.Nr. 2-17

¹H-NMR (400 MHz, CDCl₃), δ 8.84 (d, 2H), 7.33 (t, 1H), 7.24 (m, 1H), 7.06 (m, 1H), 6.03 (s, 2H, NH).

### Bsp.Nr. 3-02

¹H-NMR (400 MHz, d₆-DMSO, δ, ppm): 8.94 (d, 2H), 7.71-7.64 (m, 3H), 7.53 (t, 1H), 6.90 (breites s, 2H, NH₂).

### Bsp.Nr. 3-03

¹H-NMR (400 MHz, CD₃CN, δ, ppm): 8.94 (d, 2H), 7.79 (d, 2H), 7.60 (d, 2H), 7.53 (t, 1H), 6.92 (breites s, 2H, NH₂).

### Bsp.Nr. 3-11

¹H-NMR (400 MHz, CDCl₃), δ 8.84 (d, 2H), 7.96 (d, 1H), 7.82 (d, 1H), 7.68 (s, 1H), 7.29 (t, 1H), 5.84 (br. s, 2H, NH).

### Bsp.Nr. 3-14

¹H-NMR (400 MHz, CDCl₃), δ 8.85 (d, 2H), 7.88 (m, 3H), 7.31 (t, 1H), 6.18 (s, 2H, NH).

### Bsp.Nr. 3-15

¹H-NMR (400 MHz, CDCl₃), δ 8.83 (d, 2H), 7.52 (m, 2H), 7.45 (d, 1H), 7.29 (t, 1H), 6.12 (s, 2H, NH).

### Bsp.Nr. 3-20

¹H-NMR (400 MHz, CDCl₃), δ 8.85 (d, 2H), 7.78 (d, 1H), 7.60 (s, 1H), 7.44 (d, 1H), 7.32 (t, 1H), 6.52 (s, 2H, NH).

### Bsp.Nr. 3-23

¹H-NMR (400 MHz, CDCl₃), δ 8.83 (d, 2H), 7.63 (m, 2H), 7.31 (d, 1H), 7.22 (t, 1H), 6.05 (br. s, 2H, NH).

### Bsp.Nr. 3-24

¹H-NMR (400 MHz, d₆-DMSO), δ 8.94 (d, 2H), 7.82 (d, 1H), 7.77 (d, 1H), 7.66 (dd, 1H), 7.55 (t, 1H), 7.19 (s, 2H, NH).

### Bsp.Nr. 3-26

¹H-NMR (400 MHz, CDCl₃), δ 8.84 (d, 2H), 7.62 (m, 3H), 7.29 (t, 1H), 5.99 (br. s, 2H, NH).

### Bsp.Nr. 3-31

¹H-NMR (400 MHz, CDCl₃), δ 8.84 (d, 2H), 7.66 (d, 1H), 7.57 (m, 1H), 7.29 (m, 2H), 6.04 (s, 2H, NH).

### Bsp.Nr. 3-32

¹H-NMR (400 MHz, CDCl₃), δ 8.83 (d, 2H), 7.49 (s, 1H), 7.30 (m, 2H), 7.28 (t, 1H), 6.18 (s, 2H, NH).

### Bsp.Nr. 3-33

¹H-NMR (400 MHz, CDCl₃), δ 8.82 (d, 2H), 7.60 (m, 3H), 7.31 (t, 1H), 6.02 (br. s, 2H, NH).

### Bsp.Nr. 3-34

¹H-NMR (400 MHz, CDCl₃), δ 8.84 (d, 2H), 7.60 (m, 2H), 7.32 (m, 1H), 7.28 (t, 1H), 6.09 (br. s, 2H, NH).

### Bsp.Nr. 4-02

¹H-NMR (400 MHz, CDCl₃), δ 8.83 (d, 2H), 7.44 (t, 1H), 7.28 (m, 2H), 7.19 (s, 1H), 7.12 (dd, 1H), 6.38-6.75 (t, 1H), 6.07 (s, 2H, NH).

### Bsp.Nr. 4-03

¹H-NMR (400 MHz, CDCl₃), δ 8.83 (d, 2H), 7.42 (d, 2H), 7.27 (t, 1H), 7.20 (d, 2H), 6.38-6.76 (t, 1H), 6.02 (s, 2H, NH).

### Bsp.Nr. 4-05

¹H-NMR (400 MHz, CDCl₃), δ 8.82 (d, 2H), 7.47 (t, 1H), 7.35 (d, 1H), 7.19-7.32 (m, 3H), 6.09 (s, 2H, NH).

### Bsp.Nr. 4-06

¹H-NMR (400 MHz, CD₃CN, δ, ppm): 8.84 (d, 2H), 7.50 (d, 2H), 7.38-7.36 (m, 3H), 6.24 (breites s, 2H, NH₂).

### Bsp.Nr. 4-07

¹H-NMR (400 MHz, CDCl₃), δ 8.84 (d, 2H), 7.46 (d, 2H), 7.32 (d, 2H), 7.28 (t, 1H), 6.05 (br. s, 2H, NH).

### Bsp.Nr. 4-10

¹H-NMR (400 MHz, d₆-DMSO), δ 8.96 (d, 2H), 7.56 (t, 1H), 7.45 (d, 2H), 7.36 (d, 2H), 7.26-7.44 (dt, 1H), 7.01 (br. s, 2H, NH).

### Bsp.Nr. 4-16

¹H-NMR (400 MHz, CDCl₃), δ 8.84 (d, 2H), 7.46 (t, 1H), 7.37 (d, 1H), 7.27 (t, 1H), 7.24 (m, 1H), 7.19 (m, 1H), 6.09 (br. s, 2H, NH), 4.93-5.16 (doppelt. sext., 1H).

### Bsp.Nr. 4-18

¹H-NMR (400 MHz, CDCl₃), δ 8.84 (d, 2H), 7.28-7.41 (m, 4H), 6.06 (s, 2H, NH).

### Bsp.Nr. 4-19

¹H-NMR (400 MHz, d₆-DMSO), δ 8.95 (d, 2H), 7.64 (dt, 1H), 7.56 (t, 1H), 7.45 (dd, 1H), 7.29 (d, 1H), 7.17 (s, 2H, NH).

### Bsp.Nr. 4-21

¹H-NMR (400 MHz, d₆-DMSO), δ 8.96 (d, 2H), 7.78 (d, 1H), 7.55 (t, 1H), 7.43 (m, 2H), 7.16 (s, 2H, NH).

### Bsp.Nr. 4-23

¹H-NMR (400 MHz, d₆-DMSO), δ 8.95 (d, 2H), 7.58 (m, 1H), 7.56 (t, 1H), 7.47 (m, 1H), 7.28 (s, 1H), 7.21 (s, 2H, NH).

### Bsp.Nr. 4-34

¹H-NMR (400 MHz, CDCl₃), δ 8.84 (d, 2H), 7.46 (m, 2H), 7.39 (m, 1H), 7.30 (t, 1H), 6.16 (s, 2H, NH).

### Bsp.Nr. 4-43

¹H-NMR (400 MHz, d₆-DMSO), δ 8.95 (d, 2H), 7.53 (t, 1H), 7.44 (m, 2H), 7.37 (dd, 1H), 7.16 (s, 2H, NH).

### Bsp.Nr. 4-55

¹H-NMR (400 MHz, d₆-DMSO), δ 8.97 (d, 2H), 7.60 (dd, 1H), 7.56 (t, 1H), 7.42 (d, 1H), 7.36 (dd, 1H), 7.29 (s, 1H), 7.08 (s, 2H, NH).

### Bsp.Nr. 5-01

¹H-NMR (400 MHz, CD₃CN, δ, ppm): 8.84 (d, 2H), 7.49-7.35 (m, 6H), 6.17 (breites s, 2H, NH₂).

### Bsp.Nr. 5-02

¹H-NMR (400 MHz, CD₃CN, δ, ppm): 8.83 (d, 2H), 7.35 (t, 1H), 7.28 (s, 4H), 6.14 (breites s, 2H, NH₂), 2.38 (s, 3H).

### Bsp.Nr. 5-22

¹H-NMR (400 MHz, CD₃CN, δ, ppm): 8.83 (d, 2H), 7.35 (t, 1H), 7.30 (d, 2H), 7.02 (d, 2H), 6.10 (breites s, 2H, NH₂), 3.83 (s, 3H).

### Bsp.Nr. 5-58

¹H-NMR (400 MHz, d₆-DMSO), δ 8.95 (d, 2H), 7.56 (t, 1H), 7.35 (d, 2H), 7.27 (d, 2H), 6.91 (s, 2H, NH), 2.48 (s, 3H).

### Bsp.Nr.5-59

¹H-NMR (400 MHz, CDCl₃), δ 8.94 (d, 2H), 7.72 (d, 2H), 7.58 (d, 2H), 7.30 (t, 1H), 6.22 (s, 2H, NH), 2.80 (s, 3H).

### Bsp.Nr. 5-60

¹H-NMR (400 MHz, CDCl₃), δ 8.84 (d, 2H), 7.99 (d, 2H), 7.62 (d, 2H), 7.32 (t, 1H), 6.23 (s, 2H, NH), 3.11 (s, 3H).

### Bsp.Nr. 5-62

¹H-NMR (400 MHz, CD₃CN, δ, ppm): 8.84 (d, 2H), 7.78 (d, 2H), 7.54 (d, 2H), 7.38 (t, 1H), 6.33 (breites s, 2H, NH₂).

### Bsp.Nr. 5-71

¹H-NMR (400 MHz, CD₃CN, δ, ppm): 8.84 (d, 2H), 7.80 (d, 2H), 7.58 (d, 2H), 7.38 (t, 1H), 6.37 (breites s, 2H, NH₂).

### Bsp.Nr. 5-89

¹H-NMR (400 MHz, CDCl₃), δ 8.80 (d, 2H), 7.28 (d, 2H), 7.23 (t, 1H), 6.79 (d, 2H), 5.92 (br. s, 2H, NH), 3.00 (s, 6H).

### Bsp.Nr. 6-01

¹H-NMR (400 MHz, CDCl₃), δ 8.82 (d, 2H), 7.32 (t, 1H), 7.25 (t, 1H), 7.17 (m, 3H), 6.03 (br. s, 2H, NH), 2.41 (s, 3H).

### Bsp.Nr. 6-61

¹H-NMR (400 MHz, CDCl₃), δ 8.83 (d, 2H), 7.75 (s, 1H), 7.66 (d, 1H), 7.50 (m, 2H), 7.28 (t, 1H), 6.08 (s, 2H, NH).

### Bsp.Nr. 6-70

¹H-NMR (400 MHz, CDCl₃), δ 8.84 (d, 2H), 7.71 (s, 1H), 7.63 (m, 2H), 7.57 (m, 1H), 7.29 (t, 1H), 6.20 (br. s, 2H, NH).

### Bsp.Nr. 6-72

¹H-NMR (400 MHz, CDCl₃), δ 8.83 (d, 2H), 8.15 (d, 2H), 7.49 (d, 2H), 7.28 (t, 1H), 6.14 (br.s, 2H, NH), 3.96 (s, 3H).

### Bsp.Nr. 6-78

¹H-NMR (400 MHz, CDCl₃), δ 8.84 (d, 2H), 8.32 (m, 1H), 8.22 (dd, 1H), 7.76 (d, 1H), 7.64 (dd, 1H), 7.33 (t, 1H), 6.19 (br. s, 2H, NH).

### Bsp.Nr. 6-77

¹H-NMR (400 MHz, CDCl₃), δ 8.82 (d, 2H), 7.32 (dd, 1H), 7.25 (t, 1H), 6.72 (m, 3H), 6.03 (br. s, 2H, NH), 2.98 (s, 6H).

### Bsp.Nr. 7-01

¹H-NMR (400 MHz, d₆-DMSO, δ, ppm): 8.92 (d, 2H), 7.50 (t, 1H), 7.33-7.09 (m, 4H), 6.51 (breites s, 2H, NH₂), 2.17 (s, 3H).

### Bsp.Nr. 8-05

¹H-NMR (400 MHz, d₆-DMSO), δ 8.95 (d, 2H), 7.53 (t, 1H), 7.22 (d, 1H), 7.14 (d, 1H), 7.07 (dd, 1H), 6.80 (s, 2H, NH), 2.26 (s, 6H).

### Bsp.Nr. 8-06

¹H-NMR (400 MHz, d₆-DMSO, δ, ppm): 8.93 (d, 2H), 7.51 (t**,** 1H), 6.99 (s, 1H), 6.96 (s, 2H), 6.62 (breites s, 2H, NH₂), 2.31 (s, 6H).

### Bsp.Nr. 8-25

¹H-NMR (400 MHz, d₆-DMSO), 8 8.95 (d, 2H), 7.56 (t, 1H), 7.25 (dd, 1H), 7.17 (dd, 1H), 7.10 (d, 1H), 6.93 (s, 2H, NH), 3.87 (s, 3H).

### Bsp.Nr. 8-26

¹H-NMR (400 MHz, CDCl₃), δ 8.83 (d, 2H), 7.42 (m, 1H), 7.27 (d, 1H), 7.25 (t, 1H), 7.01 (d, 1H), 5.98 (br. s, 2H, NH), 3.95 (s, 3H).

### Bsp.Nr. 8-27

¹H-NMR (400 MHz, CDCl₃), δ 8.82 (d, 2H), 7.40 (d, 1H), 7.32 (dd, 1H), 7.27 (t, 1H), 6.97 (d, 1H), 6.02 (br. s, 2H, NH), 3.95 (s, 3H).

### Bsp.Nr. 8-33

¹H-NMR (400 MHz, d₆-DMSO), δ 8.94 (d, 2H), 7.56 (t, 1H), 7.08 (m, 4H, Ar-H + NH), 3.97 (s, 3H).

### Bsp.Nr. 8-41

¹H-NMR (400 MHz, CDCl₃), δ 8.81 (d, 2H), 7.36 (m, 2H), 7.25 (m, 2H), 7.09 (m, 3H), 7.02 (d, 2H), 6.02 (s, 2H, NH).

### Bsp.Nr. 8-42

¹H-NMR (400 MHz, d₆-DMSO), δ 8.96 (d, 2H), 7.55 (t, 1H), 7.04 (d, 1H), 7.01 (m, 3H, NH + Ar-H), 6.95 (d, 1H), 2.38 (s, 3H).

### Bsp.Nr. 8-43

¹H-NMR (400 MHz, d₆-DMSO), δ 8.94 (d, 2H), 7.54 (t, 1H), 7.36 (t, 1H), 7.08 (m, 2H), 6.97 (s, 2H, NH), 2.36 (s, 3H).

### Bsp.Nr. 8-44

¹H-NMR (400 MHz, CDCl₃), δ 8.81 (d, 2H), 7.43 (m, 2H), 7.25 (t, 1H), 6.86 (d, 1H), 6.02, (s, 2H, NH), 3.81 (s, 3H).

### Bsp.Nr. 8-45

¹H-NMR (400 MHz, d₆-DMSO), δ 8.96 (d, 2H), 7.55 (t, 1H), 7.21 (d, 1H), 6.89 (m, 3H, NH + Ar-H), 6.83 (d, 1H), 3.82 (s, 3 H), 2.19 (s, 3H).

### Bsp.Nr. 8-48

¹H-NMR (400 MHz, CDCl₃), δ 8.79 (d, 2H), 7.29 (m, 2H), 7.25 (t, 1H), 6.91 (d, 1H), 6.03 (s, 2H, NH), 3.81 (s, 3H).

### Bsp.Nr. 8-54

¹H-NMR (400 MHz, CDCl₃), δ 8.82 (d, 2H), 7.39 (s, 1H), 7.36 (s, 1H), 7.28 (t, 1H), 5.93 (s, 2H, NH), 2.41 (s, 3H).

### Bsp.Nr. 8-62

¹H-NMR (400 M:Hz, CDCl₃), δ 8.82 (d, 2H), 7.25 (t, 1H), 7.09 (d, 1H), 6.89 (d, 1H), 5.88 (br. s, 2H, NH), 3.94 (s, 3H), 3.93 (s, 3H).

### Bsp.Nr. 8-65

¹H-NMR (400 MHz, d₆-DMSO), δ 8.94 (d, 2H), 7.56 (t, 1H), 7.14 (d, 1H), 7.11 (d, 1H), 7.04 (s, 2H, NH), 3.93 (s, 3H).

### Bsp.Nr. 8-66

¹H-NMR (400 MHz, d₆-DMSO), δ 8.97 (d, 2H), 7.57 (t, 1H), 7.25 (d, 1H), 7.10 (d, 1H), 7.02 (s, 2H, N H), 6.92 (dd, 1H), 6.86-7.37 (t, 1H), 3.85 (s, 3H).

### Bsp.Nr. 8-88

¹H-NMR (400 MHz, CDCl₃), δ 8.82 (d, 2H), 7.40 (d, 1H), 7.28 (t, 1H), 7.24 (dd, 1H), 6.98 (d, 1H), 5.99 (br. s, 2H, NH), 4.03 (t, 2H), 1.86 (sext, 2H), 1.09 (t, 3H).

### Bsp.Nr. 8-96

¹H-NMR (400 MHz, CDCl₃), δ 8.82 (d, 2H), 7.41 (d, 1H), 7.27 (t, 1H), 7.21 (dd, 1H), 6.97 (d, 1H), 5.98 (br. s, 2H, NH), 4.07 (t, 2H), 1.83 (m, 2H), 1.52 (m, 2H), 1.01 (t, 3H).

### Bsp.Nr. 8-107

¹H-NMR (400 MHz, CDCl₃), δ 8.83 (d, 2H), 7.27 (t, 1H), 6.89 (d, 1H), 6.78 (d, 1H), 6.09 (s, 2H), 6.02 (br. s, 2H, NH).

### Bsp.Nr. 8-108

¹H-NMR (400 MHz, CDCl₃), δ 8.82 (d, 2H), 7.27 (t, 1H), 7.20 (m, 2H), 6.97 (6, 1H), 6.05 (br. s, 2H, NH), 3.85 (s, 3H).

### Bsp.Nr. 8-111

¹H-NMR (400 MHz, CDCl₃), δ 8:85 (d, 2H), 7.30 (t, 1H), 7.27 (m, 1H), 7.13 (m, 2H), 6.11 (br. s, 2H, NH), 3.88 (s, 3H).

### Bsp.Nr. 8-112

¹H-NMR (400 MHz, CDCl₃), δ 8.84 (d, 2H), 7.29 (t, 1H), 7.24 (m, 1H), 7.11 (m, 2H), 6.09 (br. s, 2H, NH), 4.08 (q, 2H), 1.43 (t, 3H).

### Bsp.Nr. 8-113

¹H-NMR (400 MHz, CDCl₃), δ 8.73 (d, 2H), 7.27 (t, 1H), 6.74 (m, 1H), 6.70 (m, 1H), 6.59 (m, 1H), 6.09 (br. s, 2H, NH), 4.05 (q, 2H), 1.44 (t, 3H).

### Bsp.Nr. 9-06

¹H-NMR (400 MHz, CDCl₃), δ 8.82 (d, 2H), 7.25 (t, 1H), 6.88 (m, 1H), 6.85 (m, 2H), 6.02 (s, 2H), 5.82 (br. s, 2H, NH).

### Bsp.Nr. 9-09

¹H-NMR (400 MHz, CDCl₃), δ 8.82 (d, 2H), 7.24 (t, 1H), 6.94 (m, 2H), 6.86 (m, 1H), 5.94 (br. s, 2H, NH), 4.29 (s, 4H).

### Bsp.Nr. 9-14

¹H-NMR (400 MHz, CDCl₃), δ 8.83 (d, 2H), 7.28 (t, 1H), 7.12 (m, 3H), 6.03 (br. s, 2H, NH).

### Bsp.Nr. 10-13

¹H-NMR (400 MHz, CDCl₃), δ 8.95 (d, 2H), 7.58 (d, 2H), 7.41 (t, 1H), 7.22 (m, 6H), 7.02 (m, 4H), 6.55 (d, 2H), 3.98 (s, 4H).

### Bsp.Nr. 10-21

¹H-NMR (400 MHz, CDCl₃), δ 8.96 (d, 2H), 7.45 (m, 1H), 7.36 (d, 1H), 7.22 (m, 6H), 7.19 (m, 1H), 6.98 (m, 4H), 6.78 (dd, 1H), 4.02 (s, 4H).

### Bsp.Nr. 10-22

¹H-NMR (400 MHz, CDCl₃), δ 8.97 (d, 2H), 7.43 (t, 1H), 7.22 (m, 6H), 7.04 (m, 4H), 6.24 (m, 1H), 6.21 (m, 1H), 4.04 (s, 3H), 4.00 (s, 4H).

### Bsp.Nr. 10-23

¹H-NMR (400 MHz, CDCl₃), δ 8.95 (d, 2H), 7.42 (t, 1H), 7.22 (m, 7H), 7.11 (dd, 1H), 7.01 (m, 4 H), 6.78 (d, 1H), 6.63 (s, 1H), 6.17-6.66 (t, 1H), 4.01 (s, 4H).

### Bsp.N r. 10-24

¹H-NMR (400 MHz, CDCl₃), δ 8.94 (d, 2H), 7.41 (t, 1H), 7.21 (m, 8H), 7.04 (m, 4H), 6.93 (m, 1H), 6.63 (m, 1H), 6.44 (dd, 1H), 4.00 (s, 4H), 3.94 (s, 3H).

### Bsp.Nr. 10-25

¹H-NMR (400 MHz, CDCl₃), δ 8.94 (d, 2H), 7.39 (t, 1H), 7.19 (m, 6H), 7.15 (d, 2H), 7.03 (m, 4H), 6.80 (d, 2H), 4.00 (s, 4H), 2.52 (s, 3H).

### Bsp.Nr. 10-26

¹H-NMR (400 MHz, CDCl₃), δ 8.96 (d, 2H), 7.44 (t, 1H), 7.35 (d, 1H), 7.22 (m, 6H), 7.02 (m, 4H), 6.86 (d, 1H), 6.65 (dd, 1H), 4.01 (s, 4H).

### Bsp.Nr. 10-27

¹H-NMR (400 MHz, CDCl₃), δ 8.93 (d, 2H), 7.40 (m, 1H), 7.38 (t, 1H), 7.17 (m, 6H), 6.99 (m, 4H), 6.94 (d, 1H), 6.48 (m, 1H), 4.03 (s, 4H).

### Bsp.Nr. 10-28

¹H-NMR (400 MHz, CDCl₃), δ 8.95 (d, 2H), 7.41 (t, 1H), 7.20 (m, 6H), 7.00 (m, 6H), 6.81 (m, 2H), 6.31-6.80 (t, 1H), 4.00 (s, 4H).

### Bsp.Nr. 10-29

¹H-NMR (400 MHz, CDCl₃), δ 8.98 (d, 2H), 7.43 (t, 1H), 7.22 (m, 6H), 7.02 (m, 4H), 6.98 (m, 1H), 6.63 (m, 2H), 4.01 (s, 4H).

### Bsp.Nr. 11-07

¹H-NMR (400 MHz, CDCl₃), δ 8.79 (d, 2H), 7.42 (br. S, 1H, NH), 7.35 (s, 4H), 7.25 (t, 1H), 2.59 (s, 3 H).

### Bsp.Nr. 11-82

¹H-NMR (400 MHz, CDCl₃), δ 8.82 (d, 2H), 7.44 (br. s, 1H**,** NH), 7.37 (m, 1H), 7.32 (m, 2H), 7.25 (t, 1H), 2.62 (s, 3H).

### Bsp.Nr. 12-07

¹H-NMR (400 MHz, CDCl₃), δ 8.81 (d, 2H), 7.53 (br. s, NH), 7.36 (s, 4H), 7.24 (t, 1H), 2.82 (q, 2H), 1.05 (t, 3H).

### Bsp.Nr. 12-12

¹H-NMR (400 MHz, CDCl₃), δ 8.81 (d, 2H), 8.03 (br. s, 1H, NH), 7.79 (m, 1H), 7.68 (m, 1H), 7.38 (m, 1H), 7.25 (t, 1H), 7.09 (m, 1H), 2.82 (q, 2H), 1.04 (t, 3H).

### Bsp.Nr. 12-18

¹H-NMR (400 MHz, CDCl₃), δ 8.82 (d, 2H), 7.92 (br. s, 1H, NH), 7.4 (m, 1H), 7.24 (m, 2H), 7.10 (dt, 1H), 2.84 (q, 2H), 1.06 (t, 3H).

### Bsp.Nr. 12-80

¹H-NMR (400 MHz, CDCl₃), δ 8.82 (d, 2H), 8.07 (br. s, 1H, NH), 7.46 (dd, 1H), 7.25 (m, 2H), 7.14 (dd, 1H), 2.83 (q, 2H), 1.03 (t, 3 H).

### Bsp.Nr. 12-82

¹H-NMR (400 MHz, CDCl₃), δ 8.82 (d, 2H), 7.62 (br. s, NH), 7.37 (m, 1H), 7.30 (m, 2H), 7.27 (t, 1H), 2.84 (q, 2H), 1.08 (t, 3H).

### Bsp.Nr. 12-98

¹H-NMR (400 MHz, CDCl₃), δ 8.82 (d, 2H), 8.05 (br. s, 1H, NH), 7.25 (t, 1H), 6.97 (s, 1H), 6.94 (s, 1H), 4.06 (s, 3H), 2.87 (q, 2H), 1.07 (t, 3H).

### Bsp.Nr. 12-99

¹H-NMR (400 MHz, CDCl₃), 8 8.84 (d, 2H), 8.11 (br. s, 1H, NH), 7.76 (m, 1H), 7.53 (m**,** 2H), 7.26 (t, 1H), 2.79 (q, 2H), 1.05 (t, 3H).

### Bsp.Nr. 12-100

¹H-NMR (400 MHz, CDCl₃), δ 8.82 (d, 2H), 8.04 (br. s, 1H, NH), 7.32 (d, 2H), 7.23 (m, 3H), 2.84 (q, 2H), 2.52 (s, 3H), 1.03 (t, 3H).

### Bsp.Nr. 12-101

¹H-NMR (400 MHz, CDCl₃), δ 8.81 (d, 2H), 8.01 (br. s, 1H, NH), 7.27 (t, 1H), 7.10 (m, 2H), 6.93 (m, 1H), 3.94 (s, 3H), 2.83 (q, 2H), 1.04 (t, 3H).

### Bsp.Nr. 12-102

¹H-NMR (400 MHz, CDCl₃), δ 8.83 (d, 2H), 7.92 (br. s, 1H, NH), 7.36 (dd, 1H), 7.24 (m, 2H), 7.18 (m, 1H), 7.09 (m, 1H), 6.27-6.79 (t, 1H), 2.82 (q, 2H), 1.02 (t, 3H).

### Bsp.Nr. 12-103

¹H-NMR (400 MHz, CDCl₃), δ 8.81 (d, 2H), 8.14 (br. s, 1H, NH), 7.40 (d, 2H), 7.25 (t, 1H), 7.11 (d, 2H), 6.34-6.82 (t, 1H), 2.81 (q, 2H), 1.02 (t, 3H).

### Bsp Nr. 12-104

¹H-NMR (400 MHz, CDCl₃), δ 8.79 (d, 2H), 7.28 (s, 1H, NH), 7.25 (t, 1H), 7.11 (d, 1H), 6.87 (dd, 1H), 6.84 (d, 1H), 3.82 (s, 3H), 2.88 (q, 2H), 2.25 (s, 3H), 1.03 (t, 3H).

### Bsp.Nr. 13-19

¹H-NMR (400 MHz, CDCl₃), δ 8.91 (d, 2H), 7.38 (m, 2H), 7.28 (m, 2H), 2.65 (s, 6H).

### Bsp.Nr. 13-31

¹H-NMR (400 MHz, CDCl₃), δ 8.88 (d, 2H), 7.78 (m, 1H), 7.49 (m, 1H), 7.38 (t, 1H), 7.37 (m. 1H), 7.14 (t, 1H), 2.88 (q, 4H), 0.91 (t, 6H).

### Bsp.Nr.13-32

¹H-NMR (400 MHz, CDCl₃), δ 8.87 (d, 2H), 7.76 (d, 2H), 7.37 (t, 1H), 7.13 (d, 2H), 2.86 (q, 4H), 0.89 (t, 6H).

### Bsp.Nr. 13-115

¹H-NMR (400 MHz, CDCl₃), δ 8.88 (d, 2H), 7.45 (dd, 1H), 7.38 (t, 1H), 7.24 (m, 1H), 7.17 (m, 1H), 2.88 (q, 4H), 0.90 (t, 6H).

### Bsp.Nr. 13-116

¹H-NMR (400 MHz, CDCl₃), δ 8.88 (d,2H), 7.52 (d, 1H), 7.41 (m, 1H), 7.38 (m, 1H), 7.30 (s, 1H), 7.27 (t, 1H), 2.88 (q, 4H), 0.92 (t, 6H).

### Bsp.Nr.13-117

¹H-NMR (400 MHz, CDCl₃), δ 8.87 (d, 2H), 7.38 (t, 1H). 6.97 (m, 1H), 6.93 (m, 1H), 4.06 (s, 3H), 2.91 (q, 4H), 0.94 (t, 6H).

### Bsp.Nr. 13-118

¹H-NMR (400 MHz, CDCl₃), δ 8.80 (d, 2H), 7.39 (m, 2H), 7.31 (m, 2H), 3.08 (m, 2H), 2.74 (m, 2H), 1.47 (m, 2H), 1.33 (m, 1H), 1.12 (m, 2H), 0.88 (t, 3H).

### Bsp.Nr. 14-18

¹H-NMR (400 MHz, CD₃CN, δ, ppm): 8.95 (d, 2H), 7.66-7.63 (s+d, 2H), 7.59 (t, 1H), 7.41 (d,1H).

### Bsp.Nr. 14-37

¹H-NMR (400 MHz, CD₃CN, δ, ppm): 8.96 (d, 2H), 7.66-7.59 (m, 3H), 7.39 (dd, 1H).

### Bsp.Nr. 15-07

¹H-NMR (400 MHz, CD₃CN, δ**,** ppm): 8.82 (d, 2H), 7.45 (m, 4 H), 7.42 (t, 1H), 6.83-6.56 (t, ³J = 54 Hz, CHF₂), 6.24 (breites s, 2H, NH₂).

### Bsp.Nr. 15-19

¹H-NMR (400 MHz, CDCl₃), δ 8.80 (d, 2H), 7.40 (m, 2H), 7.32 (m, 1H), 7.22 (t, 1H), 6.18 (s, 2H, NH), 5.26 (s, 2H).

### Bsp.Nr. 15-38

¹H-NMR (400 MHz, d₆-DMSO), δ 8.94 (d, 2H), 7.54 (m, 1H), 7.50 (t, 1H), 7.42 (m, 2H), 7.18 (s, 2H, NH), 6.86-7.15 (t, 1H).

### Bsp.Nr. 15-45

¹H-NMR (400 MHz, CDCl₃), δ 8.84 (d, 2H), 7.43 (d, 2H), 7.37 (d, 2H), 7.28 (t, 1H), 5.98 (br. s, 2H, NH).

### Bsp.Nr. 15-48

¹H-NMR (400 MHz, CDCl₃), δ 8.82 (d, 2H), 7.58 (d, 2H), 7.30 (d, 2H), 7.24 (t, 1H), 5.97 (br. s, 2H, NH).

### Bsp.Nr. 15-51

¹H-NMR (400 MHz, CDCl₃), δ 8.82 (d, 2H), 7.78 (d, 2H), 7.24 (t, 1H), 7.17 (d, 2H), 5.96 (br. s, 2H, NH).

### Bsp.Nr.15-57

¹H-NMR (400 MHz, CDCl₃), δ 8.84 (d, 2H), 7.38 (m, 1H), 7.35 (m, 2H), 7.28 (t, 1H), 6.05 (s, 2H, NH).

### Bsp.Nr. 15-64

¹H-NMR (400 MHz, CD₃CN, δ, ppm): 8.84 (d, 2H), 7.45 (d, 2H), 7.39-7.34 (m, 3H), 6.15 (breites s, 2H, NH₂).

### Bsp.Nr. 15-95

¹H-NMR (400 MHz, d₆-DMSO), δ 8.92 (d, 2H), 7.56 (m, 1H), 7.49 (t, 1H), 7.48-7.62 (t, 1H), 7.44 (m, 2H), 7.14 (s, 2H, NH).

### Bsp.Nr. 15-105

¹H-NMR (400 MHz, CDCl₃), δ 8.82 (d, 2H), 7.59 (d, 2H), 7.32 (d, 2H), 7.25 (t, 1H), 5.96 (br. s, 2H, NH).

### Bsp.Nr. 15-108

¹H-NMR (400 MHz, CDCl₃), δ 8.82 (d, 2H), 7.59 (d, 2H), 7.27 (t, 1H), 7.18 (d, 2H), 5.98 (br. s, 2H, NH).

### Bsp.Nr. 15-114

¹H-NMR (400 MHz, CDCl₃), δ 8.84 (d, 2H), 7.38 (m, 1H), 7.36 (m, 2H), 7.27 (t, 1H), 6.35 (s, 2H, NH).

### Bsp.Nr. 15-153

¹H-NMR (400 MHz, CDCl₃), δ 8.82 (d, 2H), 7.56 (d, 1H), 7.43 (d, 1H), 7.31 (dd, 1H), 7.28 (t, 1 H), 6.05 (br. s, 2H, NH).

### Bsp.Nr. 15-154

¹H-NMR (400 MHz, CDCl₃), δ 8.84 (d, 2H), 7.57 (d, 1H), 7.45 (d, 1H), 7.32 (dd, 1H), 7.27 (t, 1H), 6.03 (br. s, 2H, NH).

### Bsp.Nr. 15-155

¹H-NMR (400 MHz, CDCl₃), δ 8.84 (d, 2H), 7.77 (m, 1H), 7.56 (m, 2H), 7.27 (t, 1H), 6.04 (br. s, 2H, NH).

### Bsp.Nr. 15-156

¹H-NMR (400 MHz, CDCl₃), δ 8.82 (d, 2H), 7.79 (m, 1H), 7.58 (m, 2H), 7.28 (t, 1H), 6.02 (br. s, 2H, NH).

### Bsp.Nr. 15-157

¹H-NMR (400 MHz, CDCl₃), δ 8.83 (d, 2H), 7.67 (m, 1H), 7.54 (m, 2H), 7.27 (t, 1H), 6.07 (br. s, 2H, NH).

### Bsp.Nr. 15-158

¹H-NMR (400 MHz, CDCl₃), δ 8.84 (d, 2H), 7.68 (m, 1H), 7.57 (m, 2H), 7.28 (t, 1H), 6.06 (br. s, 2H, NH).

### Bsp.Nr. 15-159

¹H-NMR (400 MHz, CDCl₃), δ 8.83 (d, 2H), 7.39 (m, 1H), 7.29 (t, 1H), 7.24 (m, 2H), 6.10 (s, 2H, NH).

### Bsp.Nr. 15-160

¹H-NMR (400 MHz, CDCl₃), δ 8.83 (d, 2H), 7.69 (d, 1H), 7.53 (d, 1H), 7.27 (t, 1H), 7.19 (dd, 1H), 6.05 (br. s, 2H, NH).

### Bsp.Nr. 15-161

¹H-NMR (400 MHz, CDCl₃), δ 8.84 (d, 2H), 7.63 (m, 2H), 7.61 (m, 1H), 7.29 (t, 1H), 6.09 (br. s, 2H, NH).

### Bsp.Nr. 15-162

¹H-NMR (400 MHz, CDCl₃), δ 8.85 (d, 2H), 7.64 (m, 1H), 7.62 (m, 2H), 7.29 (t, 1H), 6.09 (br. s, 2H, NH).

### Bsp.Nr. 15-163

¹H-NMR (400 MHz, CDCl₃), δ 8.83 (d, 2H), 7.92 (m, 2H), 7.89 (m, 1H), 7.31 (t, 1H), 6.11 (br. s, 2H, NH).

### Bsp.Nr. 15-164

¹H-NMR (400 MHz, CDCl₃), δ 8.85 (d, 2H), 7.94 (m, 2H), 7.88 (m, 1H), 7.32 (t, 1H), 6.10 (br. s, 2H, NH).

### Bsp.Nr. 15-165

¹H-NMR (400 MHz, CDCl₃), δ 8.83 (d, 2H), 7.53 (m, 1H), 7.33 (m, 2H), 7.29 (t, 1H), 6.11 (br. s, 2H, NH).

### Bsp.Nr. 15-166

¹H-NMR (400 MHz, CDCl₃), δ 8.84 (d, 2H), 7.55 (m, 1H), 7.38 (m, 1H), 7.33 (m, 1H), 7.28 (t, 1H), 6.09 (br. s, 2H, NH).

### Bsp.Nr. 15-167

¹H-NMR (400 MHz, CDCl₃), δ 8.81 (d, 2H), 7.67 (m, 2H), 7.56 (m, 1H), 7.22 (t, 1H), 6.19 (br. s, 2H, NH), 5.46 (m, 1H), 5.33 (m, 1H).

### Bsp.Nr. 15-168

¹H-NMR (400 MHz, CDCl₃), δ 8.83 (d, 2H), 7.69 (d, 1H), 7.56 (d, 1H), 7.25 (t, 1H), 7.19 (dd, 1H), 6.02 (br. s, 2H, NH).

### Bsp.Nr. 15-169

¹H-NMR (400 MHz, CDCl₃), δ 8.83 (d, 2H), 7.41 (m, 1H), 7.20-7.31 (m, 3H), 6.10 (br. s, 2H, NH).

### Bsp.Nr. 16-04

¹H-NMR (400 MHz, CD₃CN, δ, ppm): 8.74 (d, 2H), 7.42-7.38 (m, 2H), 7.23-7.16 (m, 3H), 6.15 (breites s, 2H, NH₂) - das Pyrazol-3-methyl-Protonensignal liegt unter dem Lösungsmittelpeak.

### Bsp.Nr. 16-05

¹H-NMR (400 MHz, CD₃CN, δ, ppm): 8.74 (d, 2H), 7.54 (d, 1H), 7.39-7.33 (m, 3H), 7.22 (t, 1H), 6.01 (breites s, 2H, NH₂), 2.10 (s, 3H).

### Bsp.Nr. 16-06

¹H-NMR (400 MHz, CD₃CN, δ, ppm): 8.74 (d, 2H), 7.43-7.26 (m, 4H), 7.21 (t, 1H), 6.15 (breites s, 2H, NH₂), 2.20 (s, 3H).

### Bsp.Nr. 16-07

¹H-NMR (400 MHz, CD₃CN, δ, ppm): 8.75 (d, 2H), 7.43 (d, 2H), 7.40 (d, 2H), 7.22 (t, 1H), 6.21 (breites s, 2H, NH₂) - das Pyrazol-3-methyl-Protonensignal liegt unter dem Lösungsmittelpeak.

### Bsp.Nr. 16-15

¹H-NMR (400 MHz, d₆-DMSO, δ, ppm): 8.81 (d, 2H), 7.65 (s, 1H), 7.45-7.36 (dd, 2H), 7.32 (t, 1H), 6.45 (breites s, 2H, NH₂), 1.99 (s, 3H).

### Bsp.Nr.16-39

¹H-NMR (400 MHz, CDCl₃), δ 8.76 (d, 2H), 7.27 (m, 3H), 7.15 (t, 1H), 6.03 (br. s, 2H, NH), 2.69 (q, 2H), 1.18 (t, 3H).

### Bsp.Nr. 16-134

¹H-NMR (400 MHz, CDCl₃), δ 8.77 (d, 2H), 7.27 (m, 1H), 7.25 (m, 2H), 7.16 (t, 1H), 6.01 (br. s, 2H, NH), 2.59 (d, 2H), 1.86 (nonett, 1H), 0.95 (d, 6H).

### Bsp.Nr. 18-07

¹H-NMR (400 MHz, d₆-DMSO), δ 8.91 (d, 2H), 7.49 (d, 2H), 7.46 (t, 1H), 7.42 (d, 2H), 6.82 (s, 2H, NH), 5.56-5.71 (dq, 1H), 1.56 (dd, 3H).

### Bsp.Nr. 18-19

¹H-NMR (400 MHz, d₆-DMSO), δ 8.92 (d, 2H), 7.52 (m, 1H), 7.47 (t, 1H), 7.43 (m, 2H), 7.05 (s, 2H, NH), 5.59-5.76 (dq, 1H), 1.58 (dd, 3H).

### Bsp.Nr. 18-26

¹H-NMR (400 MHz, d₆-DMSO), δ 8.93 (d, 2H), 7.48 (t, 1H), 7.45 (d, 2H), 7.39 (d, 2H), 6.90 (s, 2H, NH), 1.91-2.05 (t, 3H).

### Bsp.Nr. 18-32

¹H-NMR (400 MHz, d₆-DMSO), δ 8.93 (d, 2H), 7.77 (d, 2H), 7.48 (t, 1H), 7.19 (d, 2H), 6.81 (s, 2H, NH), 1.92-2.04 (t, 3H).

### Bsp.Nr. 18-34

¹H-NMR (400 MHz, CDCl₃), δ 8.82 (d, 2H), 7.52 (d, 1H), 7.38 (m, 1H), 7.31 (m, 1H), 7.22 (t, 1H), 5.79 (br. s, 2H, NH), 1.96 (t, 3H).

### Bsp.Nr. 18-38

¹H-NMR (400 MHz, CDCl₃), δ 8.84 (d, 2H), 7.38 (m, 1H), 7.36 (m, 2H), 7.27 (t, 1H), 6.35 (s, 2H, NH).

### Bsp.Nr. 18-48

¹H-NMR (400 MHz, CDCl₃), δ 8.84 (d, 2H), 7.57 (d, 2H), 7.25 (t, 1H), 7.22 (d, 2H), 6.01 (br. s, 2H, NH).

### Bsp.Nr. 18-51

¹H-NMR (400 MHz, CDCl₃), δ 8.82 (d, 2H), 7.76 (d, 2H), 7.27 (t, 1H), 7.10 (d, 2H), 5.94 (br. s, 2H, NH).

### Bsp.Nr. 18-57

¹H-NMR (400 MHz, CDCl₃), δ 8.82 (d, 2H), 7.37 (m, 1H), 7.29 (t, 1H), 7.25 (m, 2H), 6.03 (s, 2H, NH).

### Bsp.Nr. 18-190

¹H-NMR (400 MHz, CDCl₃), δ 8.80 (d, 2H), 7.39 (s, 2H), 7.32 (s, 1H), 7.19 (t, 1H), 6.08 (br. s, 2H, NH), 5.39-5.54 (ddd, 1H), 2.00 (m, 1H), 1.74 (m, 1H), 0.93 (t, 3H).

### Bsp.Nr. 18-209

¹H-NMR (400 MHz, CDCl₃), δ 8.79 (d, 2H), 7.37 (s, 2H), 7.31 (s, 1H), 7.20 (t, 1H), 6.06 (br. s, 2H, NH), 5.46-5.62 (ddd, 1H), 2.01 (m, 1H), 1.68 (m, 1H), 1.47 (m, 1H), 1.34 (m, 1H), 0.89 (t, 3H).

### Bsp.Nr. 18-229

¹H-NMR (400 MHz, CDCl₃), δ 8.81 (d, 2H), 7.36 (m, 1H), 7.26 (m, 2H), 7.23 (t, 1H), 5.95 (br. s, 2H, NH), 3.59 (s, 3H).

### Bsp.Nr. 18-230

¹H-NMR (400 MHz, CDCl₃), δ 8.83 (d, 2H), 7.64 (m, 1H), 7.57 (m, 2H), 7.23 (t, 1H), 6.04 (br. s, 2H, NH), 2.03 (t, 3H).

### Bsp.Nr. 18-231

¹H-NMR (400 MHz, CDCl₃), δ 8.82 (d, 2H), 7.99 (s, 2H), 7.82 (s, 1H), 7.24 (t, 1H), 6.09 (br. s, 2H, NH), 5.63-5.85 (dq, 1H), 1.58 (dd, 3H).

### Bsp.Nr. 18-232

¹H-NMR (400 MHz, CDCl₃), δ 8.80 (d, 2H), 7.69 (s, 1H), 7.67 (s, 1H), 7.56 (s, 1H), 7.21 (t, 1H), 6.08 (br.s, 2H, NH), 5.65-5.81 (dq, 1H), 1.60 (dd, 3H).

### Bsp.Nr. 18-233

¹H-NMR (400 MHz, CDCl₃), δ 8.82 (d, 2H), 7.95 (m, 2H), 7.83 (m, 1H), 7.27 (t, 1H), 6.07 (br. s, 2H, NH), 2.09 (t, 3H).

### Bsp.Nr. 18-234

¹H-NMR (400 MHz, CDCl₃), δ 8.78 (d, 2H), 7.90 (m, 1H), 7.85 (m, 2H), 7.33 (t, 1H), 6.06 (br. s, 2H, NH).

### Bsp.Nr. 18-235

¹H-NMR (400 MHz, CDCl₃), δ 8.83 (d, 2H), 7.64 (m, 2H), 7.57 (m, 1H), 7.24 (t, 1H), 6.05 (br. s, 2H, NH), 2.04 (t, 3H).

### Bsp.Nr. 18-236

¹H-NMR (400 MHz, CDCl₃), δ 8.86 (d, 2H), 7.62 (m, 1H), 7.54 (m, 2H), 7.30 (t, 1H), 6.03 (br. s, 2H, NH).

### Bsp.Nr. 18-237

¹H-NMR (400 MHz, CDCl₃), δ 8.85 (d, 2H), 7.44 (m, 1H), 7.29 (t, 1H), 7.27-7.36 (m, 2H), 6.04 (br. s, 2H, NH).

### Bsp.Nr. 18-240

¹H-NMR (400 MHz, CDCl₃), δ 8.83 (d, 2H), 7.56 (s, 1H), 7.40 (m, 1H), 7.27 (m, 1H), 7.24 (t, 1H), 6.06 (br. s, 2H, NH), 2.08 (t, 3H).

### Bsp.Nr. 18-241

¹H-NMR (400 MHz, CDCl₃), δ 8.84 (d, 2H), 7.32 (m, 1H), 7.29 (t, 1H), 7.25 (m, 1H), 7.17 (m, 1H), 6.05 (br. s, 2H, NH).

### Bsp.Nr. 18-242

¹H-NMR (400 MHz, CDCl₃), δ 8.84 (d, 2H), 7.68 (m, 1H), 7.45 (m, 2H), 7.28 (t, 1H), 6.04 (br. s, 2H, NH).

### Bsp.Nr. 18-243

¹H-NMR (400 MHz, CDCl₃), δ 8.79 (d, 2H), 7.62 (m, 1H), 7.59 (m, 2H), 7.20 (t, 1H), 6.06 (br. s, 2H, NH), 5.62 (dq, 1H), 1.60 (dd, 3H).

### Bsp.Nr. 18-244

¹H-NMR (400 MHz, CDCl₃), δ 8.83 (d, 2H), 7.70 (s, 1H), 7.58 (d, 1H), 7.46 (d, 1H), 7.29 (t, 1H), 5.98 (br.s, 2H, NH).

### Bsp.Nr. 18-245

¹H-NMR (400 MHz, CDCl₃), δ 8.79 (d, 2H), 7.83 (d, 1H), 7.61 (d, 1H), 7.57 (s, 1H), 7.20 (t, 1H), 6.02 (br. s, 2H, NH), 5.62 (dq, 1H), 1.56 (dd, 3 H).

### Bsp.Nr. 18-246

¹H-NMR (400 MHz, CDCl₃), δ 8.84 (d, 2H), 7.67 (d, 1H), 7.56 (d, 1H), 7.28 (t, 1H), 7.12 (dd, 1H), 6.00 (br.s, 2H, NH).

### Bsp.Nr. 19-26

¹H-NMR (400 MHz, CD₃CN, δ, ppm): 8.66 (s, 2H), 7.45 (d, 2H), 7.39 (d, 2H), 6.16 (breites s, 2H, NH₂), 2.34 (s, 3H).

### Bsp.Nr. 19-37

¹H-NMR (400 MHz, CD₃CN, δ, ppm): 8.66 (s, 2H), 7.58 (d, 1H), 7.55 (s, 1H), 7.33 (d, 1H), 6.25 (breites s, 2H, NH₂), 2.35 (s, 3H).

### Bsp.Nr. 19-45

¹H-NMR (400 MHz, CD₃CN, δ, ppm): 8.77 (s, 2H), 7.47 (d, 2H), 7.40 (d, 2H), 6.22 (breites s, 2H, NH₂).

### Bsp.Nr. 19-56

¹H-NMR (400 MHz, CD₃CN, δ, ppm): 8.75 (s, 2H), 7.61 (d, 1H), 7.56 (s, 1H), 7.33 (d, 1H), 6.17 (breites s, 2H, NH₂).

### Bsp.Nr. 19-64

¹H-NMR (400 MHz, CD₃CN, δ, ppm): 8.81 (s, 2H), 7.46 (d, 2H), 7.39 (d, 2H), 6.13 (breites s, 2H, NH₂).

### Bsp.Nr. 19-75

¹H-NMR (400 MHz, CD₃CN, δ, ppm): 8.82 (s, 2H), 7.59 (d, 1H), 7.55 (s, 1H), 7.33 (d, 1H), 6.24 (breites s, 2H, NH₂).

### Bsp.Nr. 19-121

¹H-NMR (400 MHz, CD₃CN, δ, ppm): 9.10 (d, 1H), 7.74 (d, 1H), 7.46 (d, 2H), 7.39 (d, 1H), 6.18 (breites s, 2H, NH₂).

### Bsp.Nr. 19-195

¹H-NMR (400 MHz, CDCl₃), δ 9.18 (d, 1H), 7.68 (d, 1H), 7.61 (d, 1H), 7.55 (d, 1H), 7.28 (dd, 1H), 5.97 (br. s, 2H, NH).

### Bsp.Nr. 19-196

¹H-NMR (400 MHz, CDCl₃), δ 9.17 (d, 1H), 7.71 (d, 1H), 7.60 (d, 1H), 7.51 (d, 1H), 7.16 (dd, 1H), 5.96 (br. s, 2H, NH).

### Bsp.Nr. 19-197

¹H-NMR (400 MHz, CDCl₃), δ 9.19 (d, 1H), 7.88 (m, 3H), 7.62 (d, 1H), 6.04 (br. s, 2H, NH).

### Bsp.Nr. 24-06

¹H-NMR (400 MHz, CD₃CN, δ, ppm): 8.72 (d, 2H), 7.64 (breites NH), 7.39-7.28 (m, 4H), 7.19 (t, 1H), 2.59 (d, 3H), 2.08 (s, 3H).

### Bsp.Nr. 25-07

¹H-NMR (400 MHz, CD₃CN, δ, ppm): 8.74 (d, 2H), 7.70 (breites d, 1H, NH), 7.42-7.35 (2d, 4H), 7.22 (t, 1H), 2.88 (m, 2H), 0.97 (t, 3H) - das Pyrazol-3-methyl-Protonensignal liegt unter dem Lösungsmittelpeak.

### Bsp.Nr. 25-34

¹H-NMR (400 MHz, CD₃CN, δ, ppm): 8.71 (d, 2H), 7.76 (breites NH), 7.57 (s, 1H), 7.40-7.34 (d+d, 2H), 7.19 (t, 1H), 2.84-2.79 (m, 2H), das Pyrazol-3-methyl-Protonensignal liegt unter dem Lösungsmittelpeak, 0.98 (t, 3H).

### Bsp.Nr. 26-23

¹H-NMR (400 MHz, CD₃CN, δ, ppm): 8.75 (d, 2H), 7.51 (breites d, 1H, NH), 7.43 (m, 2H), 7.22 (t, 1H), 7.15 (m, 2H), 3.25-3.17 (m, 1H), 2.09 (s, 3H), 0.96 (d, 6H).

### Bsp.Nr. 26-24

¹H-NMR (400 MHz, CD₃CN, δ, ppm): 8.81 (d, 2H), 7.61-7.35 (m, 6H), 3.21 (m, 1H), 2.08 (s, 3H), 1.06-0.98 (d+d, 6H).

### Bsp.Nr. 26-26

¹H-NMR (400 MHz, CD₃CN, δ, ppm): 8.75 (d, 2H), 7.53 (breites s, 1H, NH), 7.40 (m, 4H), 7.23 (t, 1H), 3.25 (m, 1H), 0.97 (d, 6H) - das Pyrazol-3-methyl-Protonensignal liegt unter dem Lösungsmittelpeak.

### Bsp.Nr. 26-46

¹H-NMR (400 MHz, CD₃CN, δ, ppm): 8.71 (d, 2H), 8.14 (breites s, 1H, NH), 7.35 (d, 2H), 7.29 (d, 2H), 7.21-7.16 (m, 3H), 6.99-6.97 (m, 2H), 4.07 (d, 2H), 2.05 (s, 3H).

### Bsp.Nr. 26-65

¹H-NMR (400 MHz, CD₃CN, δ, ppm): 8.73 (d, 2H), 7.71 (breites s, 1H, NH), 7.39-7.33 (d+d, 4H), 7.20 (t, 1H), 2.69 (breites dd, 2H), 2.08 (s, 3H), 0.84 (m, 1H), 0.34 (m, 2H), 0.00 (m, 2H).

### Bsp.Nr.27-19

¹H-NMR (400 MHz, CDCl₃), δ 8.83 (d, 2H), 8.00 (br. t, 1H, NH), 7.38 (m, 1H), 7.32 (m, 2H), 7.25 (t, 1H), 2.78 (m, 2H), 1.42 (sext, 2H), 0.78 (t, 3H).

### Bsp.Nr. 27-26

¹H-NMR (400 MHz, CDCl₃), δ 8.82 (d, 2H), 7.64 (br. S, NH), 7.36 (s, 4H), 7.24 (t, 1H), 3.17 (sept, 1H), 0.99 (d, 6H).

### Bsp.Nr. 27-38

¹H-NMR (400 MHz, CDCl₃), δ 8.82 (d, 2H), 7.72 (br. d, 1H, NH), 7.34 (m, 2H), 7.27 (t, 1H), 7.24 (m, 1H), 3.18 (m, 1H), 1.03 (d, 6H).

### Bsp.Nr. 27-45

¹H-NMR (400 MHz, CDCl₃), δ 8.79 (d, 2H), 8.37 (br. S, 1H, NH), 7.21 (m, 8H), 6.98 (m, 2H), 4.07 (s, 2H).

### Bsp.Nr. 27-50

¹H-NMR (400 MHz, CDCl₃), δ 8.80 (d, 2H), 8.06 (br. t, 1H, NH), 7.63 (m, 2H), 7.23 (m, 5H), 7.03 (d, 1H), 6.98 (m, 2H), 4.06 (s, 2H).

### Bsp.Nr.27-51

¹H-NMR (400 MHz, CDCl₃), δ 8.79 (d, 2H), 8.11 (br. t, 1H, NH), 7.62 (d, 2H), 7.22 (m, 5H), 6.98 (m, 3H), 4.05 (s, 2H).

### Bsp.Nr. 27-76

¹H-NMR (400 MHz, CDCl₃), δ 8.82 (d, 2H), 8.02 (br. s, 1H, NH), 7.35 (m, 1H), 7.30 (m, 2H), 7.26 (t, 1H), 2.68 (d, 2H), 0.89 (m, 1H), 0.45 (m, 2H), 0.22 (m, 2H).

### Bsp.Nr. 27-114

¹H-NMR (400 MHz, CDCl₃), δ 8.83 (d, 2H), 8.12 (br. t, 1H, NH), 7.38 (m, 1H), 7.30 (m, 2H), 7.25 (t, 1H), 5.63 (m, 1H), 5.06 (m, 2H), 3.48 (m, 2H).

### Bsp.Nr. 27-133

¹H-NMR (400 MHz, CDCl₃), δ 8.82 (d, 2H), 8.13 (br. t, 1H, NH), 7.37 (m, 1H), 7.29 (m, 2H), 7.24 (t, 1H), 2.63 (t, 2H), 1.64 (nonett, 1H), 0.78 (d, 6H).

### Bsp.Nr. 27-140

¹H-NMR (400 MHz, CDCl₃), δ 8.81 (d, 2H), 8.39 (br. S, 1H, NH), 7.26 (m, 5H), 7.18 (d, 2H), 6.88 (d, 2H), 4.07 (s, 2H).

### Bsp.Nr. 27-153

¹H-NMR (400 MHz, CDCl₃), δ 8.87 (d, 2H), 8.62 (br. t, 1H, NH), 7.51 (m, 1H), 7.33 (m, 2H), 7.08 (m, 2H), 7.02 (m, 2H), 4.14 (d, 2H).

### Bsp.Nr. 27-154

¹H-NMR (400 MHz, CDCl₃), δ 8.80 (d, 2H), 7.99 (br. d, 1H, NH), 7.35 (m, 1H), 7.31 (m, 2H), 7.27 (t, 1H), 3.42 (m, 1H), 1.55 (m, 4H), 1.42 (m, 4H).

### Bsp.Nr. 27-155

¹H-NMR (400 MHz, CDCl₃), δ 8.81 (d, 2H), 8.22 (br. t, 1H, NH), 7.33 (m. 1H), 7.19 (m, 4H), 7.12 (m, 1H), 7.02 (m, 1H), 6.98 (m, 2H), 4.09 (s, 2H).

### Bsp.Nr. 27-156

¹H-NMR (400 MHz, CDCl₃), δ 8.79 (d, 2H), 8.23 (br. t, 1H, NH), 7.23 (m, 6H), 7.01 (d, 2H), 6.97 (m, 2H), 6.28-6.79 (t, 1H), 4.05 (s, 2H).

### Bsp.Nr.27-157

¹H-NMR (400 MHz, CDCl₃), δ 8.81 (d, 2H), 8.04 (br. t, 1H, NH), 7.37 (m, 1H), 7.22 (m, 5H), 7.08 (m, 1H), 6.98 (m, 2H), 4.12 (s, 2H).

### Bsp.Nr. 27-158

¹H-NMR (300 MHz, CDCl₃), δ 8.84 (d, 2H), 8.05 (br. t, 1H, NH), 7.51 (m, 1H), 7.19-7.40 (m, 6H), 6.92 (m, 2H), 4.08 (s, 2H).

### Bsp.Nr. 27-159

¹H-NMR (400 MHz, CDCl₃), δ 8.79 (d, 2H), 8.42 (br. t, 1H, NH), 7.21 (m, 4H), 7.08 (d, 1H), 7.02 (m, 2H), 6.85 (dd, 1H), 6.68 (d, 1H), 4.11 (s, 2H), 3.69 (s, 3H), 2.24 (s, 3H).

### Bsp.Nr. 27-160

¹H-NMR (400 MHz, CDCl₃), δ 8.80 (d, 2H), 8.18 (br. t, 1H, NH), 7.36 (d, 1H), 7.20 (m, 5H), 7.14 (dd, 1H), 6.95 (m, 2H), 4.09 (s, 2H).

### Bsp.Nr. 27-161

¹H-NMR (400 MHz, CDCl₃), δ 8.79 (d, 2H), 8.12 (br. t, 1H, NH), 7.19 (m, 8H), 7.01 (m, 2H), 4.07 (s, 2H), 2.52 (s, 3H).

### Bsp.Nr. 27-162

¹H-NMR (400 MHz, CDCl₃), δ 8.80 (d, 2H), 8.09 (br. t, 1H, NH), 7.17-7.34 (m, 5H), 7.05 (d, 1H), 6.97 (m, 4H), 6.14-6.63 (t, 1H), 4.08 (s, 2H).

### Bsp.Nr. 27-163

¹H-NMR (400 MHz, CDCl₃), δ 8.79 (d, 2H), 8.17 (br. t, 1H, NH), 7.22 (m, 4H), 7.01 (m, 2H), 6.88 (m, 2H), 4.14 (s, 2H), 4.03 (s, 3H).

### Bsp.Nr. 28-07

¹H-NMR (400 MHz, CD₃CN, δ, ppm): 8.79 (d, 2H), 7.41-7.38 (2d, 4H), 7.31 (t, 1H), 2.29 (s, 3H) - das Pyrazol-3-methyl-Protonensignal liegt unter dem Lösungsmittelpeak.

### Bsp.Nr. 29-07

¹H-NMR (400 MHz, CD₃CN, δ, ppm): 8.87 (d, 2H), 8.68 (breites NH), 7.49-7.30 (m, 5H).

### Bsp.Nr. 29-12

¹H-NMR (400 MHz, CDCl₃), δ 9.38 (br. s, 1H, NH), 8.84 (d, 2H), 7.77 (m, 1H), 7.68 (d, 1H), 7.38 (d, 1H), 7.36 (t, 1H), 7.17 (m, 1H), 2.08 (s, 3H).

### Bsp.Nr. 29-19

¹H-NMR (400 MHz, CDCl₃), δ 9.62 (br. s, 1H, NH), 8.91 (d, 2H), 7.42 (t, 1H), 7.37 (m, 1H), 7.29 (m, 2H), 2.08 (s, 3H).

### Bsp.Nr. 29-26

¹H-NMR (400 MHz, CD₃CN, δ, ppm): 8.84 (d, 2H), 7.50-7.47 (m, 3H), 7.32 (d, 2H), 2.19 (s, 6H).

### Bsp.Nr. 29-153

¹H-NMR (400 MHz, CDCl₃), δ 9.41 (br. s, 1H, NH), 8.89 (d, 2H), 7.40 (t, 1H), 6.99 (m, 1H), 6.95 (m, 1H), 4.04 (s, 3H), 2.09 (s, 3H).

### Bsp.Nr. 29-154

¹H-NMR (400 MHz, CDCl₃), δ 8.87 (d, 2H), 8.79 (br. s, 1H, NH), 7.38 (t, 1H), 7.16 (d, 1H), 6.88 (m, 2H), 3.82 (s, 3H), 2.27 (s, 3H), 1.98 (s, 3H).

### Bsp.Nr.29-155

¹H-NMR (400 MHz, CDCl₃), δ 9.58 (br. s, 1H, NH), 8.92 (d, 2H), 7.54 (d, 1H), 7.41 (t, 1H), 7.35 (m, 2H), 2.07 (s, 3H).

### Bsp.Nr. 29-156

¹H-NMR (400 MHz, CDCl₃), δ 9.18 (br. s, 1H, NH), 8.91 (d, 2H), 7.39 (m, 3H), 7.16 (d, 2H), 6.34-6.82 (t, 1H), 2.01 (s, 3H).

### Bsp.Nr. 29-157

¹H-NMR (400 MHz, CDCl₃), δ 9.32 (br. s, 1H, NH), 8.89 (d, 2H), 7.40 (m, 2H), 7.28 (m, 1H), 7.17 (t, 1H), 2.05 (s, 3 H).

### Bsp.Nr. 29-158

¹H-NMR (400 MHz, CDCl₃), δ 8.83 (d, 2H), 7.39 (t, 1H), 6.87 (m, 1H), 6.84 (m, 1H), 4.08 (s, 3H), 2.27 (s, 6H).

### Bsp.Nr.29-159

¹H-NMR (400 MHz, CDCl₃), δ 8.82 (d, 2H), 7.36 (t, 1H), 7.18 (d, 1H), 6.79 (dd, 1H), 6.63 (d, 1H), 3.78 (s, 3H), 2.25 (s, 6H), 2.24 (s, 3H).

### Bsp.Nr. 29-160

¹H-NMR (400 MHz, CDCl₃), δ 8.83 (d, 2H), 7.39 (t, 1H), 7.35 (d, 1H), 6.92 (dd, 1H), 6.77 (d, 1H), 2.27 (s, 6H).

### Bsp.Nr. 29-161

¹H-NMR (400 MHz, CDCl₃), δ 8.82 (d, 2H), 7.38 (t, 1H), 7.32 (d, 2H), 7.18 (d, 2H), 6.35-6.82 (t, 1H), 2.24 (s, 6H).

### Bsp.Nr. 29-162

¹H-NMR (400 MHz, CDCl₃), δ 8.83 (d, 2H), 7.36 (m, 2H), 7.18 (m, 2H), 2.25 (s, 6H).

### Bsp.Nr. 29-163

¹H-NMR (400 MHz, CDCl₃), δ 9.87 (br. s, 1H, NH), 8.94 (d, 2H), 7.65 (m, 3H), 7.44 (t, 1H), 2.07 (s, 3 H).

### Bsp.Nr. 29-164

¹H-NMR (400 MHz, CDCl₃), δ 9.78 (br. s, 1H, NH), 8.92 (d, 2H), 7.60 (m, 2H), 7.55 (m, 1H), 7.43 (t, 1H), 2.08 (s, 3H).

### Bsp.Nr. 29-165

¹H-NMR (400 MHz, CDCl₃), δ 8.84 (d, 2H), 7.68 (m, 1H), 7.61 (m, 2H), 7.58 (br. s, 1H, NH), 7.38 (t, 1H), 4.17 (m, 1H), 3.99 (m, 1H), 2.05 (s, 1H).

### Bsp.Nr.29-166

¹H-NMR (400 MHz, CDCl₃), δ 8.80 (d, 2H), 7.63 (m, 1H), 7.56 (m, 2H), 7.31 (t, 1H), 4.78 (dd, 2H), 4.59 (dd, 2H), 2.42 (m, 2H), 2.11 (t, 3H).

### Bsp.Nr. 29-167

¹H-NMR (400 MHz, CDCl₃), δ 8.84 (d, 2H), 8.00 (m, 2H), 7.78 (m, 1H), 7.32 (t, 1H), 4.96 (s, 2H), 4.83 (s, 2H), 2.82 (s, 2H).

### Bsp.Nr. 32-03

¹ H-NMR (400 MHz, CDCl₃), δ 8.85 (d, 2H), 8.44 (d, 1H), 7.73 (dd, 1H). 7.44 (d, 1H), 7.31 (t, 1H), 6.51 (br. s, 2H, NH).

### Bsp.Nr. 32-12

¹H-NMR (400 MHz, CDCl₃), δ 8.82 (d, 2H), 8.21 (d, 1H), 7.61 (dd, 1H). 7.29 (t, 1H), 6.85 (d, 1H), 6.02 (br. s, 2H, NH), 3.99 (s, 3 H).

### Bsp.Nr. 32-17

¹H-NMR (400 M.Hz, CDCl₃), δ 8.86 (d, 2H), 8.81 (d, 1H), 7.94 (dd, 1H), 7.77 (d, 1H), 7.34 (t, 1H), 6.25 (br. s, 2H, NH).

### Bsp.Nr. 32-20

¹H-NMR (400 M.Hz, d₆-DMSO, δ, ppm): 8.82 (d, 2H), 8,41 (d, 1H), 7.83-7.81 (dd, 1H), 7.52 (d, 1H), 7.35 (t, 1H), 6.81 (breites s, 2H, NH₂), 2.16 (s, 3H).

### Bsp.Nr. 32-37

¹H-NMR (400 MHz, d₆-DMSO, δ, ppm): 8.83 (d, 2H), 8.39 (d, 1H), 7.82-7.79 (dd, 1H), 7.52 (d, 1H), 7.35 (t, 1H), 6.75 (breites s, 2H, NH₂), 2.59-2.53 (q, 2H), 1.13-1.09 (t, 3H).

### Bsp-Nr. 32-52

¹H-NMR (400 MHz, COCl₃)), δ 8.82 (d, 2H), 8.20 (d, 1H), 7.46 (dd, 1H), 7.25 (t, 1H), 6.61 (d, 1H), 5.94 (br. s, 2H, NH), 3.14 (s, 6H).

### Bsp.Nr. 32-53

¹H-NMR (400 MHz, CDCl₃), 8 8.83 (d, 2H), 8.20 (d, 1H), 7.62 (dd, 1H), 7.27 (t, 1H), 6.79 (d, 1H), 6.04 (br. s, 2H, NH), 5.35 (sept, 1H), 1.39 (d, 6H).

### Bsp.Nr. 32-54

¹H-NMR (400 MHz, CDCl₃), δ 8.84 (d, 2H), 8.11 (d, 1H), 7.68 (d, 1H). 7.29 (t, 1H), 6.05 (br. s, 2H, NH), 4.07 (s, 3H).

### Bsp.Nr. 33-63

¹H-NMR (400 MHz, d₆-DMSO, δ, ppm): 8.82 (d, 2H), 8.63 (s, 1H), 8.18 (s, 1H), 7.34 (t, 1H), 6.69 (breites s, 2H, NH₂), unter dem Lösungsmittelpeak (q, 2H), 1.04 (t, 3H).

### Bsp.Nr. 35-20

¹H-NMR (400 MHz, CDCl₃), δ 8.74 (d, 2H), 7.39 (m, 2H), 7.24 (m, 1H), 7.11 (t, 1H), 6.07 (br. s, 2H, NH), 1.78 (m,1H), 1.12 (m, 2H), 0.91 (m, 2H).

### Bsp.Nr. 35-39

¹H-NMR (400 MHz, CDCl₃), δ 8.79 (d, 2H), 7.52 (m, 2H), 7.30 (m, 1H), 7.19 (t, 1H), 6.11 (br. s, 2H, NH), 1.39 (m, 4H).

### Bsp.Nr. 35-77

¹H-NMR (400 MHz, CDCl₃), δ 8.88 (d, 2H), 7.25 (m, *1*H), 7.22 (m, 2H), 7.17 (t, 1H), 6.05 (br. s, 2H, NH), 3.48 (quint, 1H), 2.46 (m, 2H), 2.21 (m, 2H), 1.98 (m, 1H), 1.82 (m, 1H).

### Bsp.Nr. 35-134

¹H-NMR (400 MHz, CDCl₃), δ 8.89 (d, 2H), 7.46 (m, 2H), 7.28 (m, 1H), 7.21 (t, 1H). 6.07 (br. s, 2H, NH), 1.31 (m, 2H), 1.19 (m, 2H).

### Bsp.Nr. 42-20

¹H-NMR (400 MHz, CDCl₃), δ 8.79 (d, 2H), 7.96 (br. s, 1H, NH), 7.37 (m, 2H), 7.31 (m, 1H), 7.22 (t, 1H), 5.19-5.37 (d, 2H), 2.93 (q, 2H), 1.08 (t, 3H).

### Bsp.Nr. 42-39

¹H-NMR (400 MHz, CDCl₃), 8 8.77 (d, 2H), 7.92 (br. s, 1H, NH), 7.29 (m, 1H), 7.21 (m, 6H), 7.03 (m, 2H), 5.17-5.35 (d, 2H), 4.18 (s, 2H).

### Bsp.Nr. 42-58

¹H-NMR (400 MHz, CDCl₃), δ 8.95 (d, 2H), 7.37 (t, 1H), 7.21 (m, 7H), 7.07 (m, 4H), 6.67 (d, 2H), 5.14-5.29 (d, 2H), 4.07 (s, 4H).

### Bsp.Nr. 42-77

¹H-NMR (400 MHz, CDCl₃), δ 8.88 (d, 2H), 7.42 (m, 2H), 7.35 (m, 2H), 6.55-6.90 (t, 1H), 2.95 (q, 4H), 0.96 (t, 6H).

### Bsp.Nr. 42-96

¹H-NMR (400 MHz, CDCl₃), δ 8.79 (d, 2H), 7.22 (m, 8H). 7.00 (m, 2H), 6.44-6.82 (t, 1H), 4.12 (s, 2H).

### Bsp.Nr. 42-115

¹H-NMR (400 MHz, CDCl₃), δ 8.96 (d, 2H), 7.42 (t, 1H), 7.31 (m, 7H), 7.06 (m, 4H), 6.67 (m, 2H), 6.43-6.78 (t, 1H), 4.04 (s, 4H).

### Bsp.Nr. 42-135

¹H-NMR (400 MHz, CDCl₃)_{;} δ 9.66 (br. s, 1H, NH), 8.87 (d, 2H), 7.36 (m, 3H), 7.31 (m, 1H), 6.61 -6.97 (t, 1H), 2.09 (s, 3 H).

### Bsp.Nr. 43-02

¹H-NMR (400 MHz, CDCl₃), δ 38.70 (s, 2H), 7.74 (d, 1H), 7.69 (d, 1H), 7.50 (dd, 1H), 5.92 (br. s, 2H, NH).

### Bsp.Nr. 43-03

¹H-NMR (400 MHz, CDCl₃), δ 8.70 (s, 2H), 7.63 (m, 2H), 7.61 (s, 1H), 5.90 (br. s, 2H, NH).

### Bsp.Nr. 43-04

¹H-NMR (400 MHz, CDCl₃), δ 8.71 (s, 2H), 7.62 (m, 2H), 7.59 (s, 1H). 5.91 (br. s, 2H, NH).

### Bsp.Nr. 43-05

¹H-NMR (400 MHz, CDCl₃), δ 8.72 (s, 2H), 7.60 (m, 2H), 7.57 (s, 1H), 5.96 (br. s, 2H, NH).

### Bsp.Nr. 43-06

¹H-NMR (400 MHz, CDCl₃), δ 8.69 (s, 2H), 7.63 (m, 2H), 7.58 (s, 1H), 5.85 (br. s, 2H, NH), 2.02 (t, 3H).

### Detaillierte Synthesebeispiele

### Beispiel 1-79

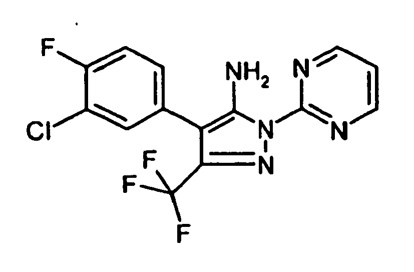

*Schritt 1:* 1,43 g Natrium (62 mmol) wurden in 15 ml Ethanol unter Rückfluss aufgelöst. Bei 40 °C wurde eine Lösung aus 10 g (59 mmol) 3-Chlor-4-fluorbenzylnitril und 8,8 g (62 mmol) Trifluoressigsäureethylester in 10 ml Ethanol zugetropft; danach wurde das Reaktionsgemisch 12 Stunden unter Rückfluss gerührt. Nach dem Abkühlen wurde das Reaktionsgemisch am Rotationsverdampfer eingeengt, in Essigsäureethylester gelöst und zweimal mit 100 ml IN Salzsäure und einmal mit Wasser gewaschen. Die organische Phase wurde abgetrennt, über Natriumsulfat getrocknet und filtriert. Das Lösungsmittel wurde vom Filtrat am Rotationsverdampfer sorgfältig unter vermindertem Druck abdestilliert. Der Rückstand wurde in einem Lösungsmittelgemisch aus Petrolether/Dichlormethan (Vol.: 1:1) verrührt. Der sich abscheidende farblose Feststoff wurde über eine Glasfritte abgesaugt und getrocknet. Es wurden 12 g (72,8 % der Theorie) 2-(3-Chlor-4-fluorphenyl)-4,4,4-trifluor-3-oxobutan-nitril als Tautomerengemisch erhalten.

¹H-NMR (400 MHz, d₆-DMSO, δ, ppm): Tautomerengemisch im Verhältnis ca. 3:2, 8.22 (A-1, dd, 1H), 7.69 (A-1, m, 1H), 7.62 (B-1, dd, 1H), 7.47 (B-1, m, 1H), 7.42 (B-1, t, 1H), 7.17 (A-1, t, 1H), 4.61 (B-1, s, 1H).

*Schritt 2*: 0,5 g (1,86 mmol) 2-(3-Chlor-4-fluorphenyl)-4,4,4-trifluor-3-oxobutan-nitril und 0,2 g (1,86 mmol) 2-Pyrimidinyl-hydrazin wurden in 5 ml Toluol gelöst. Nach Zugabe katalytischer Mengen para-Toluolsulfonsäure wurde das Reaktionsgemisch 12 Minuten bei 170 °C (5 bar) in einem Mikrowellengerät (Hersteller: CEM) gerührt. Nach Abkühlen wurde das Reaktionsgemisch eingeengt und der Rückstand säulenchromatografisch aufgearbeitet (Kieselgel, Cyclohexan/Essisäurcethylester, Vol.: 7:1 bis 3:1). Es wurden 0,16 g (24 % der Theorie) 4-(3-Chlor-4-fluorphenyl)-1-pyrimidin-2-yl-3-(trifluormethyl)-1*H*-pyrazol-5-amin als farbloser Feststoff erhalten.

¹H-NMR (400 Mhz, d₆-DMSO, δ, ppm): 8.93 (d, 2H), 7.51 (m, 2H), 7.47 (t, 1H), 7.35 (m, 1H), 6.87 (breites s, 2H, NH₂).

### Beispiel 1-87

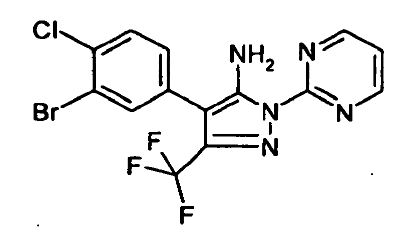

*Schritt 1*: 3-Brom-4-Chlortoluol (1,0 equiv, 3,0 g, 14,6 mmol) und N-Bromsuccinimid (1,2 equiv., 3,1 g, 17,5 mmol) wurden in CCl₄ gelöst, mit AIBN (0,01 equiv., 23,9 mg, 0,15 mmol) versetzt und 4 h lang bei Rückfluß gerührt. Nach dem Abkühlen auf Raumtemperatur wurde mit ges. NaHCO₃-Lösung und mit Wasser gewaschen, die organische Phase eingeengt und der Rückstand säulenchromatografisch mit (EtOAc/Heptan-Gradient) gereinigt. Es wurden 2.0 g (47 % der Theorie) 2-Brom-4-brommethyl-1-chlorbenzol erhalten.

*Schritt 2*: 2-Brom-4-brommethyl-1-chlorbenzol (1,0 equiv, 2,0 g, 7,0 mmol) und Kaliumcyanid (1,3 equiv., 0,59 g, 9,1 mmol) wurden bei Raumtemperatur in einem Gemisch aus Ethanol und Wasser (2:1 Volumenanteile) gelöst und 6 h lang bei Raumtemperatur gerührt. Danach wurde die Reaktionsmischung eingeengt und mit Ethylacetat versetzt. Die organische Phase wurde mit Wasser gewaschen und die wässrige Phase mit Ethylacetat extrahiert. Die organische. Phase wurde getrocknet, eingeengt und der Rückstand säulenchromatographisch (EtOAc/Heptan-Gradient) gereinigt. Es wurden 1.3 g (80 % der Theorie) (3-Brom-4-chlorphenyl)-acetonitril erhalten.

*Schritt 3*: Natriummethylat (1,2 equiv., 1,46 g, 6,8 mmol) wurde zu einer Lösung von (3-Brom-4-chlorphenyl)-acetonitril (1,0 equiv, 1,3 g, 5,6 mmol) in abs. Tetrahydrofuran gegeben, und die resultierende Reaktionslösung wurde 1 Stunde lang bei 50 °C gerührt. Dann wurde Ethyltrifluoracetat (1,2 equiv, 0,9 g, 6,8 mmol) in 2 ml Tetrahydrofuran zugetropft; danach wurde das Reaktionsgemisch 6 Stunden unter Rückfluss gerührt. Nach dem Abkühlen wurde das Reaktionsgemisch unter vermindertem Druck eingeengt, in Essigsäureethylester gelöst und zweimal mit 100 ml 1N Salzsäure und einmal mit Wasser gewaschen. Die organische Phase wurde abgetrennt, über Natriumsulfat getrocknet und filtriert. Das Lösungsmittel wurde vom Filtrat sorgfältig unter vermindertem Druck abdestilliert und der Rückstand in einem Lösungsmittelgemisch aus Petrolether/Dichlormethan (Vol.: 1:1) verrührt. Der sich abscheidende farblose Feststoff wurde über eine Glasfritte abgesaugt und getrocknet. Es wurden 1,7 g (92 % der Theorie) 2-(3-Brom-4-chlorphenyl)-4,4,4-trifluor-3-oxo-butyronitril als Tautomerengemisch erhalten.

*Schritt 4:* 2-(3-Brom-4-chlorphenyl)-4,4,4-trifluor-3-oxo-butyronitril (1,0 equiv, 1,7 g, 5,2 mmol) und 2-Pyrimidinyl-hydrazin (1,0 equiv, 0,57 g, 5,2 mmol) wurden in 10 ml Toluol gelöst. Nach Zugabe katalytischer Mengen von Essigsäure wurde das Reaktionsgemisch 6 Stunden lang unter Rückfluß gerührt. Nach dem Abkühlen auf Raumtemperatur wurde das Reaktionsgemisch eingeengt und der Rückstand säulenchromatografisch gereinigt (EtOAc/Heptan-Gradient). Es wurden 0,19 g (9% der Theorie) 4-(3-Hrom-4-chlorphenyl-2-pyrimidin.2-yl-5(trifluormethyl)-2*H*-pyrazol-3-ylamin als farbloser Feststoff erhalten.

¹H-NMR (400 MHz, d₆-DMSO, δ, ppm): 8.97 (d, 2H), 7.72 (d, 1H), 7.69 (d, 1H), 7.56 (t, 1H), 7.38 (dd, 1H), 7.13 (s, 2H, NH₂).

### Bsp.Nr. 3-25

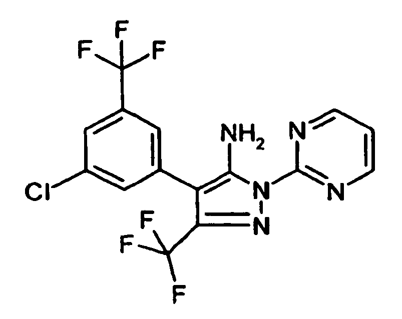

*Schritt 1:* 838 mg (2,655 mmol) 2-(3-Chloro-5-trifluoromethyl-phenyl)-4,4,4-trifluoro-3-oxo-butyronitril wurden in 2,036 g (13,276 mmol) Phosphorylchlorid vorgelegt und mit 0,37 ml (2,655 mmol) Triethylamin versetzt. Das Reakionsgemisch wurde 3 h bei 80-100 °C gerührt und vorsichtig in warmes Wasser eingerührt. Nach dem Extrahieren mit Essigsäurethylester wurde die organische Phase mit Magnesiumsulfat getrocknet, filtriert und im Vakuum eingeengt. Dabei verblieben 758 mg (85 %) 3-Chloro-2-(3-chloro-5-tritluoromethyl-phenyl)-4,4,4-trifluoro-but-2-enenitril.

*Schritt 2*: 750 mg (2,245 mmol) 3-Chloro-2-(3-chloro-5-trifluoromethyl-phenyl)-4,4,4-trifluoro-but-2-enenitril wurden in 10 ml Ethanol vorgelegt und mit 247 mg (2,245 mmol) Pyrimidin-2-yl-hydrazin sowie 227 mg (2,245 mmol) Triethylamin versetzt. Das Gemisch wurde für 10 h unter Rückfluß erhitzt. Nach dem Abkühlen wurden 75 ml Wasser zugegeben und der entstandene Feststoff abgesaugt. Nach säulenchromatografischer Reinigung des Feststoffes verblieben 500 mg (54 %) 4-(3-Chloro-5-trifluoromethyl-phenyl)-2-pyrimidin-2-yl-5-trifluoromethyl-2*H*-pyrazol-3-ylamin.

¹H-NMR (400 MHz, CDCl₃), δ 8.84 (d, 2H), 7.61 (s, 2H), 7.58 (s, 1H), 7.30 (t, 1H), 6.18 (s, 2H, NH).

### Bsp.Nr. 8-92

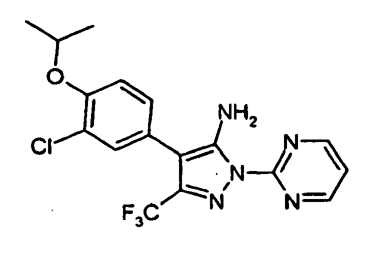

*Schritt 1*: Aminocrotonitril (7150 mg, 52.5 mmol, 1 equiv) wurde in wässriger HCl vorgelegt und nach 5 min Rühren mit 2-Hydrazinopyrimidin (5786 mg, 52.54 mmol, 1 equiv) versetzt. Die Reaktionslösung wurde 4h lang unter Rückfluß gerührt und nach dem Abkühlen auf Raumtemperatur in 2N NaOH gegossen. Anschließend wurde die wässrige Phase mehrfach mit Essigester extrahiert. Die vereinigten organischen Phasen wurden über Natriumsulfat getrocknet, abfiltriert und eingeengt. Der Rückstand wurde aus Essigester umkristallisiert und lieferte 2-Pyrimidin-2-yl-5-trifluoromethyl-2*H*-pyrazol-3-ylamin (5002 mg, 42% der theor. Ausbeute).

*Schritt 2:* 2-Pyrimidin-2-yl-5-trifluoromethyl-2*H*-pyrazol-3-ylamin (1000 mg, 4.36 mmol, 1 equiv) wurde in Essigsäure gegeben und nach 5 min Rühren bei Raumtemperatur mit einer Lösung von Brom (0.22 ml, 4.36 mmol, 1 equiv) in Essigsäure tropfenweise versetzt. Das Reaktionsgemisch wurde 4h lang bei Raumtemperatur gerührt, danach mit Wasser versetzt und mehrfach mit Dichlormethan extrahiert. Die vereinigten organischen Phasen wurden über Natriumsulfat getrocknet und eingeengt Der resultierende Rückstand wurde säulenchromatografisch gereinigt, und es wurden 1120 mg 4-Brom-2-pyrimidin-2-yl-5-trifluoromethyl-2*H*-pyrazol-3-ylamin (83 % der theor. Ausbeute) erhalten.

*Schritt 3:* 4-Bromo-2-pyrimidin-2-yl-5-trifluoromethyl-2*H*-pyrazol-3-ylamin (200 mg, 0.65 mmol, 1.0 equiv) und 3-Chlor-4-isopropoxyphenylboronsäure (181 mg, 0.84 mmol, 1.3 equiv) wurden unter Argon in einem ausgeheizten Rundkolben mit DMF versetzt (4.5 ml) und 5 min lang gerührt. Danach erfolgte die Zugabe von Tetrakis(triphenylphosphin)palladium(0) (60 mg, 0.05 mmol, 0.08 equiv) und Natriumcarbonatlösung (2M, 0.5 ml). Das resultierende Reaktionsgemisch wurde 4 h lang bei 80 °C gerührt. Nach dem Abkühlen auf Raumtemperatur erfolgte die Zugabe von Wasser und Dichlormethan. Nach mehrfachem Extrahieren der wässrigen phase mit Dichlormethan wurden die vereinigten organischen Phasen über Natriumsulfat getrocknet und eingeengt. Die Reinigung des resultierenden Rückstands über präp. HPLC lieferte 4-(3-Chloro-4-isopropoxy-phenyl)-2-pyrimidin-2-yl-5-trifluoromethyl-1-2*H-*pyrazol-3-ylamin (20 mg, 7%).

¹H-NMR (400 MHz, CDCl₃), δ 8.83 (d, 2H), 7.41 (d, 1H), 7.27 (t, 1H). 7.21 (dd, 1H), 7.00 (d, 1H), 5.98 (br. s, 2H, NH), 4.58 (sept, 1H), 1.42 (d, 6H).

### Beispiel 11-10

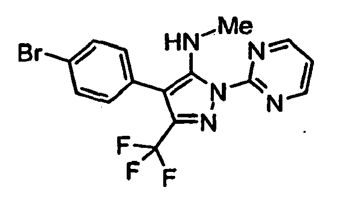

0.30 g (0,78 mmol) 4-(4-Bromphenyl)-1-pyrimidin-2-yl-3-(trifluormethyl)-1*H*-pyrazol-5-amin (Beispiel 01-09) wurden in 6 ml DMF vorgelegt und mit Stickstoff überlagert. 0,04 g (1 mmol) Natriumhydrid 0.041 g (60%) und 0,12 g (0,86 mmol) Methyliodid wurden zugegeben und die Mischung 5 Stunden lang auf 40°C erhitzt. Nach extraktiver Aufarbeitung mit Heptan-Ethylacetat (Vol. 1:1) und Wasser wurde das Produkt mittels einer präparativen HPLC-Trennung isoliert. Es wurden 0,060 g 4-(4-Bromphenyl)-N-methyl-1-pyrimidin-2-yl-3-(trifluorrnethyl)-1*H*-pyrazol-5-amin (11-10) als farbloser Feststoff erhalten.

¹H-NMR (300 MHz, CDCl₃, δ, ppm): 8.81, 2H, d; 7.50, 2H, d; 7.29, 2H, d; 7.26, 1H, tr; 2.60, 3H, s.

### Beispiel 12-07

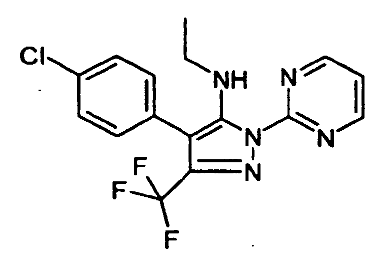

0,3 g (0,9 mmol) 4-(4-Chlorphenyl)-1-pyrimidin-2-yl-3-(trifluormethyl)-1*H*-pyrazol-5-amin (Tabelle 1, Substanz 1-06) wurden in 1,5 ml DMF gelöst und mit 0,07 g (1,8 mmol, 60 %ige Dispersion in Mineralöl) Natriumhydrid bei Raumtemperatur versetzt. 0,28 g (1,8 mmol) Ethyliodid wurden in 0,5 ml DMF gelöst und zugetropft. Das Reaktionsgemisch rührte 12 Stunden bei 55 °C und wurde anschließend auf zehn Prozent seines Volumens eingeengt. Der Rückstand wurde mit IN Salzsäure und Dichlormethan intensiv verrührt, die Dichlormethanphase wurde in der Phasentrenn-Kartusche (Hersteller: Whatman) separiert und das Lösungsmittel vom Filtrat am Rotationsverdampfer sorgfältig abdestilliert. Der Rückstand wurde mittels präparativer HPLC chromatografiert.

Es wurden 0,092 g (24,2 % der Theorie) 4-(4-Chlorphenyl)-*N*-ethyl-1-pyrimidin-2-yl-3-(trifluor-methyl)-1*H*-pyrazol-5-amin als amorpher Rückstand erhalten.

¹H-NMR (400 MHz, CD₃CN, δ, ppm): 8.83 (d, 2H), 7.77 (breites s, 1H, NH), 7.41 (s, 4H), 7.37 (t, 1H), 2.84 (breites m, 2H), 0.96 (t, 3H).

### Beispiel 14-07

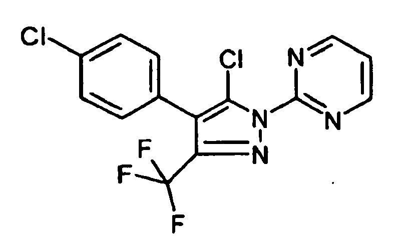

1,5 g (4,4 mmol) 4-(4-Chlorphenyl)-1-pyrimidin-2-yl-3-(trifluoromethyl)-1*H*-pyrazol-5-amin (Tabelle 1, Substanz 1-06) wurden in 10 ml konzentrierter Salzsäure gelöst. Nach einer Stunde Rühren bei Raumtemperatur entstand ein schwer löslicher Niederschlag. Es erfolgte die Zugabe von 0,72 g (5,2 mmol) Kupfer-(II)-chlorid und weiteren 10 ml konzentrierter Salzsäure. Anschließend wurden 0,54 g (7,9 mmol) Natriumnitrit portionsweise zugegeben (Gasentwicklung). Es wurde 12 Stunden bei Raumtemperatur nachgerührt, dann wurde das Reaktionsgemisch auf Eis gegeben und anschließend mit Essigsäureethylester extrahiert. Die organische Phase wurde abgetrennt, über Natriumsulfat getrocknet und filtriert, das Lösungsmittel wurde am Rotationsverdampfer sorgfältig abdestilliert. Der Rückstand wurde säulenchromatografisch aufgearbeitet (Kieselgel, Dichlormethan/Acetonitril, Vol.: 9: 1).

Es wurden 0,28 g (11,3 % der Theorie) 2-[5-Chlor-4-(4-chlorphenyl)-3-(trifluormethyl)-1*H-*pyrazol-1-yl]pyrimidin als gelb-orangener Feststoff erhalten.

¹H-NMR (400 MHz, d₆-DMSO, δ, ppm): 8.95 (d, 2H), 7.58 (t, 1H), 7.51 (d, 2H), 7.46 (d, 2H).

### Beispiel 14-29

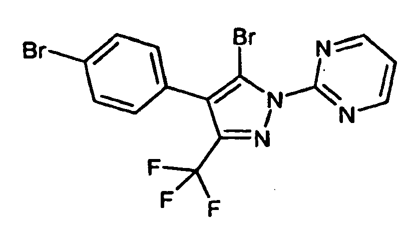

1,50 g (3,90 mmol) 4-(4-Bromphenyl)-1-pyrimidin-2-yl-3-(trifluormethyl)-1*H*-pyrazol-5-amin (Beispiel 01-09) wurden in 8 ml (23,15 g, 91,6 mmol) Bromoform vorgelegt. 0,60 g (5,86 mmol) t-Butylnitrit wurden bei 0-10 °C zugegeben und die Mischung für 2 h auf 70 °C erhitzte Nach Einengen am Rotationsverdampfer wurde das Produkt säulenchromatografisch isoliert. Es wurden 0,82 g 5-Brom-4-(4-Bromphenyl)-N-methyl-1-pyrimidin-2-yl-3-(trifluormethyl)-1H-pyrazol (Beispiel 14-29) als Feststoff erhalten.

¹H-NMR (400 MHz, CDCl₃, 8, ppm): 8.94, 2H, d; 7.61, 2H, d; 7.48, 1H, tr; 7.30, 2H, d; 19F-NMR (CDCl₃): -61.23 ppm.

### Beispiel 29-37

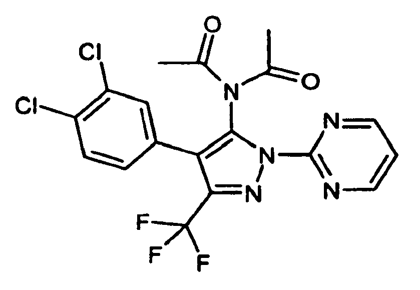

0,50 g (1,34 mmol) 4-(3,4-Dichlorphenyl)-1-pyrimidin-2-yl-3-(trifluoromethyl)-1*H*-pyrazol-5-amin (Beispiel 01-80) wurden in 3 ml Dioxan vorgelegt. 0,27 g (2,67 mmol) Acetanhydrid und 0,016 g (0,13 mmol) DMAP wurden zugegeben und die Mischung für 6 h auf 100 °C erhitzt. Nach extraktiver Aufarbeitung mit Heptan-Ethylacetat (Vol. 1:1) und Wasser wurde das Produkt säulenchromatografisch isoliert. Es wurden 0,190 g N,N-Diacetyl-4-(3,4-dichlorphenyl)-1-pyrimidin-2-yl-3-(trifluormethyl)-1*H*-pyrazol-5-amin (Beispiel 29-37) als farbloser Feststoff erhalten.

¹H-NMR (300 MHz, CDCl₃, δ, ppm): 8.84, 2H, d; 7.54, 1H, d; 7.39, 3H, d + tr; 7.16, 1H, dd; 2.27, 6H, s; ¹⁹F-NMR (300 MHz, CDCl₃): -61.19 ppm.

### Beispiel 31-29

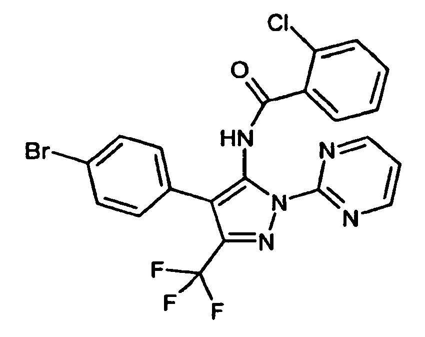

0,10 g (0,26 mmol) 4-(4-Bromphenyl)-1-pyrimidin-2-yl-3-(tritluomethyl)-1*H*-pyrazol-5-amin (Beispiel 01-09) wurden in 3 ml Dioxan vorgelegt. 0,050 g (0,286 mmol) 2-Chlorbenzoylchlorid, 0,050 g (0,39 mmol) Diisopropylethylamin und 0,006 g (0,05 mmol) DMAP wurden zugegeben und die Mischung für 6 h auf 100 °C erhitzt. Nach extraktiver Aufarbeitung mit Heptan-Ethylacetat (Vol. 1:1) und Wasser wurde das Produkt äulenchromatoigrafisch isoliert. Es wurden 0,100 g N-(2-Chlorbenzoyl)-4-(3,4-dichlorphenyl)-1-pyrimidin-2-yl-3-(trifluorinethyl)-1*H*-pyrazol-5-amin (Beispiel 31-29) als farbloser Feststoff erhalten.

¹H-NMR (300 MHz, CDCl₃, δ, ppm): 10.16, 1H, s; 8.86, 2H, d; 7.55, 2H, d; 7.37, 1H, tr; 7.48-7.32, 6H, m; ¹⁹F-NMR (300 MHz, CDCl₃): -61.25 ppm.

### Beispiel 36-10

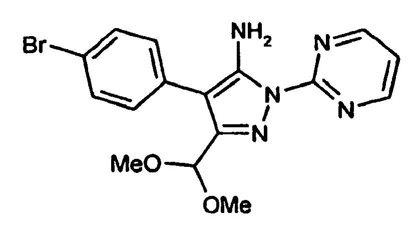

0,40 g (1,34 mmol) 2-(4-Bromphenyl)-4,4-dimethoxy-3-oxo-butyronitril wurden in 6 ml Methanol vorgelegt. 0,148 g (1,34 mmol) 2-Pyrimidinyl-hydrazin und 0,32 g (5,36 mmol) Essigsäure wurden zugegeben und die Mischung für 2 h auf 65 °C erhitzt. Nach dem Einengen am Rotationsverdampfer wurde der Rückstand mit Heptan-Ethylacetat (Vol. 4:1) ausgerührt. Es wurden 0,29 g 4-(4-Bromphenyl)-3-(dimethoxymethyl)-1-pyrimidin-2-yl-1*H*-pyrazol-5-amin (Beispiel 36-10) als farbloser Feststoff erhalten.

¹H-NMR (400 MHz, d6DMSO, δ, ppm): 8.87, 2H, d; 7.55, 2H, d; 7.44, 3H, d + tr; 6.77, 2H, s, br; 5.22, 1H, s; 3.27, 6H, s.

### Beispiel 38-10

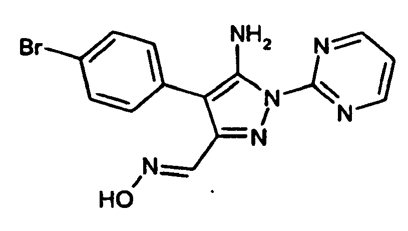

0,15 g (0,38 mmol) 4-(4-Bromphenyl)-3-(dimethoxymethyl)-1-pyrimidin-2-yl-1*H*-pyrazol-5-amin (36-10) wurden in 4 ml Dioxan und 1 ml Wasser vorgelegt. 0,053 g (0,769 mmol) Hydroxylammonium-chlorid und 0,38 ml (0,769 mmol) 2N Salzsäure wurden zugegeben und die Mischung für 8 h auf 100 °C erhitzt. Beim Verrühren mit 40 ml Wasser fiel das Oxim-Produkt aus. Es wurden 0,089 g 4-(4-Bromphenyl)-1-pyrimidin-2-yl-1*H*-pyrazol-5-amin-3-carbaldehyd-oxim (Beispiel 38-10) als farbloser Feststoff erhalten.

¹H-NMR (400 MHz, d6DMSO, δ, ppm): 11.13, 1H, s; 8.88, 2H, d; 7.96, 1H, s; 7.57, 2H, d; 7.46, 1H, tr; 7.32, 2H, d; 6.74, 2H, s, br.

### Beispiel 37-10

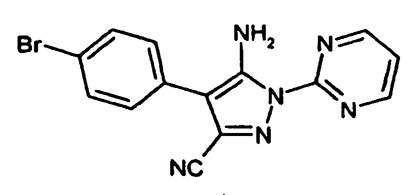

0,089 g (0,248 mmol) 4-(4-Bromphenyl)-1-pyrirnidin-2-yl-1*H*-pyrazol-5-amin-3-carbaldehydoxim (Beispiel 38-10) wurden in 2 ml 1,2-Dichlorethan vorgelegt. 0,114 g (0,743 mmol) Phosphoroxychlorid wurden zugegeben und die Mischung für 20 h bei 25 °C gerührt. Nach dem Einengen am Rotationsverdampfer und dem Verrühren des Rückstandes mit Wasser wurde der verbleibende Feststoff abgesaugt. Es wurden 0,033 g 4-(4-Bromphenyl)-3-cyano-1-pyrimidin-2-yl-1*H*-pyrazol-5-amin (Beispiel 37-10) als farbloser Feststoff erhalten.

¹H-NMR (400 MHz, d6DMSO, δ, ppm): 8.95, 2H, d; 7.71, 2H, d; 7.59, 1H, tr; 7.48, 2H, d; 7.28, 2H, s, br.

### Biologische Beispiele

### Beispiel A

### Phaedon-Test (PHAECO Spritzbehandlung)

| | | |
|---|---|---|
| Lösungsmittel | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* wurden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wurde die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele bei einer Aufwandmenge von 500 g/ha nach 7 d eine Wirkung von mindestens 80 %:
Bsp. Nr. 1-3, 1-5, 1-6, 1-8, 1-9, 1-11, 1-12, 1-14, 1-17, 1-18, 1-20, 1-22, 1-23, 1-30, 1-32, 1-33, 1-34, 1-71, 1-73, 1-74, 1-75, 1-79, 1-80, 1-81, 1-82, 1-83, 1-87, 1-88, t-89, 2-4, 2-5, 2-6, 2-12, 2-13, 2-15, 2-16, 2-17, 3-2, 3-3, 3-11, 3-14, 3-15, 3-20, 3-23, 3-24, 3-25, 3-26, 3-31, 3-32, 3-33, 3-34,4-2, 4-3, 4-5, 4-6,4-7, 4-18, 4-19, 4-21, 4-34, 4-36, 5-2, 5-5, 5-9, 5-22, 5-23, 5-25, 5-33, 5-50, 5-58, 5-59, 5-60, 5-62, 5-80, 5-89, 6-1, 6-61, 6-70, 6-78, 8-5, 8-6, 8-11, 8-24, 8-25, 8-26, 8-27, 8-30, 8-31, 8-42, 8-43, 8-54, 8-62, 8-65, 8-66, 8-68, 8-69, 8-73, 8-88, 8-89, 8-92, 8-107, 8-108, 8-109, 8-1 0, 8-111, 8-1 12, 9-4, 9-06, 9-09, 9-14, 11-7, 11-10, 11-82, 12-7, 12-12, 12-80, 12-103, 12-104, 13-19, 13-31, 13-32, 13-115, 13-116, 14-7, 14-18, 15-7, 15-19, 15-38, 15-45, 15-48, 15-51, 15-57, 15-95, 15-105, 15-108, 15-114, 15-154, 15-155, 15-156, 15-157, 15-158, 15-159, 15-160, 15-161, 15-162, 15-163, 15-164, 15-165, 15-166, 15-167, 15-168, 15-169, 16-4, 16-6, 16-7, 16-15, 16-18, 16-19, 16-27, 16-30, 16-38, 16-39, 16-49, 16-87, 16-134, 16-162, 17-37, 17-48, 17-67, 17-124, 17-143, 18-7, 18-10, 18-19, 18-26, 18-32, 18-34, 18.3 8, 18-45, 18-48, 18-51, 18-57, 18-124, 18-143, 18-181, 18-190, 18-200, 18-209, 18-219, 18-229, 18-230, 18-231, 18-232, 18-233, 18-234, 18-235, 18-236, 18-237, 18-238, 18-240, 18-241, 18-242, 18-243, 18-244, 18-245, 18-246, 19-56, 19-57, 19-121, 25-31, 25-07, 26-7, 26-37, 26-46, 26-62, 26-65, 26-84, 26-102, 26-103, 26-116, 26-125, 26-139, 26-140, 27-19, 27-26, 27-38, 27-45, 27-50, 27-51, 27-76, 27-114, 27-140, 27-153, 27-155, 27-156, 27-157, 27-158, 27-159, 27-160, 27-163, 28-7, 29-26, 29-37, 29-64, 29-156, 29-158, 29-159, 29-161, 29-162, 29-163, 29-164, 29-165, 30-10, 32-52, 32-54, 35-07, 39-10, 39-48, 39-86, 39-105, 39-124, 39-162, 39-219, 39-200, 40-10, 40-29, 40-124, 41-86, 42-39, 42-96, 43-02, 43-03, 43-04, 43-05, 43-06, 43-09, 43-10, 43-1

### Beispiel B

### Tetranychus-Test, OP-resistent (TETRUR Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, wurden mit einer Wirkstoffzubereitung der gewünschten Konzentration gespritzt.

Nach der gewünschten Zeit wurde die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele bei einer Aufwandmenge von 100 g/ha nach 5d eine Wirkung von mindestens 80 %:
Bsp. Nr. 15-108, 15-154, 19-57, 27-114, 18-242

Bei diesem Test zeigen z. B. die folgenden Verbindungen der Herstellungsbeispiele bei einer Aufwandmenge von 500 g/ha nach 5 d eine Wirkung von mindestens 80 %:
Bsp. Nr. 1-9, 1-22, 1-24, 1-71, 1-73, 1-81, 1-83, 2-4, 2-12, 2-6, 2-15, 3-11, 3-14, 3-23, 3-24, 3-25, 3-32, 3-33, 8-107, 8-108, 13-116, 12-82, 15-57, 15-105, 15-108, 15-154, 15-155, 15-156, 15-158, 15-159, 15-160, 15-162,15-163, 15-164, 15-165, 15-166, 15-168, 15-169, 16-38, 16-134, 18-10, 18-38, 18-57, 18-232, 18-233, 18-234, 18-235, 18-236, 18-237, 18-238, 18-241, 18-244, 16-134, 25-7, 25-31, 26-37, 26-62, 27-153, 27-158, 27-160, 29-37, 29-165, 43-02

### Beispiel C

### Spodoptera frugiperda-Test ( SPODFR Spritzbehandlung)

| | |
|---|---|
| Lösungsmittel | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben *(Zea mays)* wurden mit einer Wirkstoffzubereitung der gewünschten Konzentration gespritzt und nach dem Abtrocknen mit Raupen des Heerwurms *(Spodoptera frugiperda)* besetzt.

Nach der gewünschten Zeit wurde die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele bei einer Aufwandmenge von 500 g/ha nach 7 d eine Wirkung von ≥ 80 %:
Bsp. Nr. 1-5, 1-6, 1-9, 1-12, 1-13, 1-17, 1-18, 1-22, 1-23, 1-32, 1-33, 1-71, 1-73, 1-74, 1-75, 1-79, 1-80, 1-81, 1-82, 1-83, 1-87, 1-88, 1-89, 1-32, 1-89, 2-2, 2-6, 2-12, 2-13, 2-16, 2-17, 3-11, 3-14, 3-20, 3-24, 3-25, 3-26, 3-31, 3-32, 3-34, 4-3, 4-21, 4-23, 4-34, 4-36, 5-33, 6-70, 6-78, 8-5, 8-11, 8-24, 8-26, 8-27, 8-31, 8-44, 8-48, 8-66, 8-68, 8-69, 8-73, 8-107, 9-06, 9-09, 11-82, 12-7, 12-80, 12-82, 12-99, 12-103, 13-19, 13-31, 13-32, 13-115, 13-116, 14-7, 14-18, 15-19, 15-38, 15-45, 15-48, 15-51, 15-57, 15-95, 15-108, 15-114, 15-154, 15-155, 15-156, 15-157, 15-158, 15-159, 15-160, 15-161, 15-162, 15-163, 15-164, 15-165, 15-166, 15-167, 15-168, 15-169, 16-38, 16-39, 16-134, 18-10, 18-19, 18-26, 18-32, 18-38, 18-45, 18-48, 18-51, 18-57, 18-124, 18-181, 18-190, 18-200, 18-209, 18-229, 18-230, 18-231, 18-232, 18-233, 18-234, 18-235, 18-236, 18-237, 18-238, 18-239, 18-240, 18-241, 18-242, 18-243, 18-244,18-245,18-246, 19-56, 19-57, 27-19, 27-26, 27-38, 27-76, 27-114, 27-153, 27-155, 27-158, 27-160, 29-19, 29-29, 29-37, 29-143, 29-156, 29-157, 29-161, 29-162, 29-163, 29-164, 29-165, 30-10, 42-77, 42-96, 42-135, 43-01, 43-02, 43-03, 43-04, 43-05, 43-06, 43-11.

### Beispiel D

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*)*,* die von allen Stadien der Grünen Pfirsichblattlaus (*Myzus persicae*) befallen sind, wurden mit einer Wirkstoffzubereitung der gewünschten Konzentration gespritzt.

Nach der gewünschten Zeit wurde die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele bei einer Aufwandmenge von 500 g/ha nach 5 d einen Abtötungsgrad von mindestens 80 %:
Bsp. Nr. 1-6, 1-9, 1-11, 1-14, 1-17, 1-22, 1-71, 1-74, 1-79, 1-81, 1-89, 3-14, 3-20, 3-25, 3-26, 3-32, 4-5, 6-61, 8-6, 8-27, 8-44, 8-48, 8-68, 8-69, 8-107, 12-103, 12.104, 13-19, 13-32, 13-116, 15-19, 15-38, 15-45, 15-57, 15-95, 15-105, 15-108, 15-114, 15-154, 15-155, 15-156, 15-158, 15-159, 15-161, 15-162, 15-165, 15-169, 16-15, 16-19, 16-27, 16-39, 16-65, 16-134, 17-37, 18-19, 18-26, 18-32, 18-34, 18-38, 18-57, 18-229, 18-232, 18-235, 18-236, 18-239, 18-242, 19-38, 19-45, 19-48, 19-56, 19-57, 19-76, 19-152, 19-191, 25-07, 26-6, 26-23, 26-124, 26-139, 27-19, 27-26, 27-38, 27-45, 27-76, 27-114, 27-140, 27-153, 27-159, 27-160, 27-163, 35-07, 29-165, 42-96, 43-09, 43-10, 43-11.

### Beispiel E

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, wurden mit *Lucilia cuprina* Larven besetzt.

Nach der gewünschten Zeit wurde die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele bei einer Aufwandmenge von 100 ppm nach 2 d eine Wirkung von ≥ 80 %:
Bsp. Nr. 1-3, 1-5, 1-6, 1-9, 1-11, 1-12, 1-18, 1-22, 1-33, 1-71, 1-73, 1-79, 1-80, 1-81, 1-82, 1-83, 1-89, 2-13, 2-4, 2-6, 2-12, 2-15, 3-14, 3-20, 3-24, 3-25, 3-31, 3-32, 4-3, 4-6, 4-19, 4-21, 4-23, 4-36, 5-2, 5-22, 5-33, 5-62, 8-24, 8-31, 8-68, 8-108, 12-7, 12-82, 13-116, 14-7, 14-18, 15-38, 15-45, 15-51, 15-57, 15-95, 15-108, 15-157, 15-158, 15-159, 15-160, 15-161, 15-162, 15-163, 15-165, 15-166, 15-168, 16-6, 16-7, 16-19, 16-134, 18-7, 18-10, 18-19, 18-38, 18-45, 18-230, 18-233, 18-234, 18-232, 18-235, 18-236, 18-237, 18-244, 19-56, 26-46, 26-65, 26-84, 26-139, 27-114, 27-160.

### Beispiel F

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die einen Schwamm enthalten, der mit der Wirkstoffzubereitung der gewünschten Konzentration behandelt wurde, wurden mit *Musca domestica Adulten* besetzt.

Nach der gewünschten Zeit wurde die Abtötung in % bestimmt. Dabei bedeutet 100 %, dass alle Fliegen abgetötet wurden; 0 % bedeutet, dass keine Fliegen abgetötet wurden.

Bei diesem Test zeigen z. B. die folgenden Verbindungen der Herstellungsbeispiele bei einer Aufwandmenge von 20 ppm nach 2 d eine Wirkung von ≥ 80 %:
Bsp. Nr. 1-6, 1-9, 1-12, 1-18, 1-22, 1-33, 1-73, 1-77, 1-79, 1-80, 1-81, 2-13, 3-24, 4-3, 4-21, 14-18, 15-45, 15-51,15-159, 15-160, 15-168, 18-234, 18-244, 18-236, 18-237

### Beispiel G

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Die Wirkstoffllösung wurde in das Abdomen (*Boophilus microp*/*us*) injiziert, die Tiere wurden in Schalen überführt und in einem klimatisierten Raum aufbewahrt.

Nach der gewünschten Zeit wurde die Wirkung in % bestimmt. Dabei bedeutet 100%, dass keine Zecke fertile Eier gelegt hat.

Bei diesem Test zeigen z. B. die folgenden Verbindungen der Herstellungsbeispiele bei einer Aufwandmenge von 20 µg/Tier nach 7 d eine Wirkung von ≥ 80 %:
Bsp. Nr. 1-5, 1-6, 1-9, 1-11, 1-12, 1-18, 1-22, 1-71, 1-73, 1-79, 1-80, 1-81, 1-82, 1-83,1-89, 2-12, 2-13, 3-2, 3-14, 3-20, 3-24, 3-25, 3-31, 3-32, 4-3, 4-21, 4-36, 5-2, 5-22, 5-33, 5-62, 8-31, 15-57, 2-13, 3-20, 3-31, 8-11, 8-24, 8-54, 8-68, 8-69, 15-45, 15-51, 15-108, 15-157, 15-1 58, 15-160, 15-161, 15-162,15-163, 15-164, 15-165, 15-166, 15-168,16-19, 16-30, 16-38,16-134, 17-37, 18-7, 18-10, 18-19, 18-38, 18-45,18-230, 18-232, 18-233, 18-234, 18-235, 18-236, 18-237, 18-244, 26-84, 27-114

### Beispiel H

### Ctenocephalides felis; oral (CTECFE)

| | |
|---|---|
| Lösungsmittel | Dimethylsulfoxid |

Zwecks Herstellung einer zweckmäßigen Wirkstoffzubereitung vermischt man 1 Gewichtsteil Wirkstoff mit der angegebenen Menge Wasser. Ein Teil des Konzentrats wurde mit citiriertem Rinderblut verdünnt und die gewünschte Konzentration hergestellt.

20 nüchterne adulte Flöhe (*Ctenocephalides felis*) wurden in eine Kammer eingesetzt, die oben und unten mit Gaze verschlossen ist. Auf die Kammer wurde ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen wurden kann. Während das Blut auf 37° C erwärmt wurde, ist im Bereich der Flohkammern Raumtemperatur.

Nach der gewünschten Zeit wurde die Abtötung in % bestimmt. Dabei bedeutet 100 %, dass alle Flöhe abgetötet wurden; 0 % bedeutet, dass kein Floh abgetötet wurde.

Bei diesem Test zeigen z. B. die folgenden Verbindungen der Herstellungsbeispiele bei einer Aufwandmenge von 20 ppm nach 2 d eine Wirkung von ≥ 80 %:
Bsp. Nr. 1-5, 1-9, 1-12, 1-18, 1-22, 1-80, 1-81, 1-83, 1-89, 3-14, 3-20, 3-25, 3-31, 4-3, 8-24, 8-68, 1-81,12-82, 15-57, 15-95, 15-157, 15-158, 15-161, 15-162, 15-163, 18-19, 18-38, 18-230, 18-232, 18-234, 18-235, 18-236, 18-237

## Patentansprüche

1. Verbindungen der Formel (I) 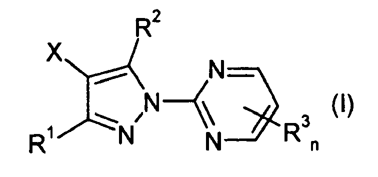 in welcher
X für Phenyl, 2-Pyridyl, 3-Pyridyl oder 2-Pyrimidinyl steht, jeweils substituiert durch einen oder mehreren Substituenten ausgewählt aus Halogen, Alkyl, Haloalkyl, Alkoxy, Alkoxyalkyl, Alkoxyalkoxy, Cycloalkyl, Alkenoxy, Alkinoxy, Benzyloxy, Cycloalkylalkoxy, Haloalkoxy, Haloalkoxyalkyl, Alkylsulfanyl, Haloalkylsulfanyl, Alkylsulfinyl, Haloalkylsulfinyl, Alkylsulfonyl, Haloalkylsulfonyl, Cyano, Alkylcarbonyl, Alkoxycarbonyl, Alkoxycarbonylalkyl, Carboxyl, Carboxamid, Dialkylcarboxamid, Trialkylsilyl, Nitro, Amino, Alkylamino, Dialkylamino, Alkylsulfonylamino, Dialkylsulfonylamino, -CH=NO-H, -CH=NO-Alkyl, -CH=NO-Halolkyl, -C(CH₃)=NO-H, -C(CH₃)=NO-Alkyl, -C(CH₃)=NO-Haloalkyl, Formyl, Benzyl (gegebenfalls wiederum substituiert mit einem oder mehreren Halogenatomen), Phenoxy oder Pyrid-2-yloxy (die beiden letzteren gegebenenfalls substituiert mit einem oder mehreren Substituenten ausgewählt aus Alkyl, Haloge und/oder Haloalkyl) und Phenyl (gegebenenfalls substituiert mit einem oder mehreren Halogenatomen), wobei vicinale Alkyl-, Haloalkyl, Alkoxy- und/oder Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkylanteil gegebenenfalls mit einem oder mehreren Fluoratomen substituiert sein kann,
R¹ für Alkyl (gegebenenfalls ein- oder zweifach substituiert mit Substituenten unabhängig voneinander ausgewählt aus Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, Hydroxy, Cycloalkyl, Phenyl (wiederum gegebenenfalls substituiert mit einem oder mehreren Halogenatomen) und Heterocyclyl), Cycloalkyl (gegebenenfalls substituiert mit einem oder mehreren Haloalkylgruppen und/oder Halogenatomen), Haloalkyl (gegebenenfalls substituiert mit Alkoxy), Cyano, Formyl, -CH=NO-H, -CH=NO-Alkyl, -CH=NO- Haloalkyl, -C(CH₃)=NO-H, -C(CH₃)=NO-Alkyl, -C(CH₃)=NO-Halolkyl oder Benzyl (gegebenenfalls einfach oder mehrfach substituiert mit einem oder mehreren Substituenten unabhängig voneinander ausgewählt aus Halogen, Alkyl, Haloalkyl und Alkoxy) steht,
R² für Amino steht und
R² weiterhin für Amino steht, welches einfach substituiert ist mit Alkyl, Alkylcarbonyl, Cycloalkyl, Cycloalkylalkyl, Alkenyl, Alkoxycarbonyl, Alkenoxycarbonyl, Alkinoxycarbonyl, Alkoxycarbonylalkyl, Alkoxycarbonylcarbonyl, Benzyl oder Phenylcarbonyl (wobei der Phenylring im Benzyl und Phenylcarbonyl wiederum gegebenenfalls substituiert ist mit einem oder mehreren Halogenatomen) und
R² weiterhin für Amino steht welches disubstituiert ist mit zwei Substituenten unabhängig voneinander ausgewählt aus Benzyl, Alkyl, Alkoxycarbonyl, Alkenoxycarbonyl, Alkinoxycarbonyl und Alkylcarbonyl,
R² weiterhin für Piperidin, Pyrrolidin oder Morpholin steht, welche gegebenenfalls einfach oder zweifach substituiert sind mit Alkyl und
R² weiterhin für Halogen steht,
R³ für Halogen, Alkyl, Haloalkyl, Alkoxy oder Dialkylamino steht und
n für 0 oder 1 steht,
die Verbindungen der allgemeinen Formel (I) außerdem N-Oxide und Salze umfassen.

2. Verbindungen der Formel (I) gemäß Anspruch 1, in welcher
X für Phenyl, 2-Pyridyl, 3-Pyridyl oder 2-Pyrimidinyl steht, jeweils substituiert durch einen oder mehreren Substituenten ausgewählt aus Fluor, Chlor, Brom, Jod, C₁-C₆- Alkyl, C₁-C₆-Haloalkyl, C₁-C₆-Alkoxy-C₁-C₆-alkyl, C₁-C₆-Alkoxy, C₁-C₆- Haloalkoxy, C₁-C₆-Haloalkoxy-C₁-C₆-alkyl, C₁-C₆-Alkylsulfanyl, Halo-C₁-C₆- alkylsulfanyl, C₁-C₆-Alkylsulfinyl, Halo-C₁-C₆-alkylsulfinyl, C₁-C₆- Alkylsulfonyl, Halo-C₁-C₆-alkylsulfonyl, Cyano, C₁-C₆-Alkylcarbonyl, C₁-C₆- Alkoxycarbonyl, C₁-C₆-Alkoxycarbonyl-C₁-C₆-alkyl, Nitro, Amino, C₁-C₆- Alkylamino, Di(C₁-C₆-alkyl)amino, C₁-C₆-Alkylsulfonylamino, Di(C₁-C₆- alkyl)sulfonylamino, -CH=NO-H, -CH=NO-C₁-C₆-Alkyl, -CH=NO-Halo-C₁-C₆-Alkyl, -C(CH₃)=NO-H, -C(CH₃)=NO-C₁-C₆-Alkyl, -C(CH₃)=NO-Halo-C₁-C₆-alkyl, Formyl, Benzyl (gegebenfalls wiederum substituiert mit einem oder mehreren Halogenatomen), Phenoxy oder Pyrid-2-yloxy (die beiden letzteren gegebenenfalls substituiert mit einem oder mehreren Halogenatomen und/oder Halo-C₁-C₆-alkylgruppen) und Phenyl (gegebenenfalls substituiert mit einem oder mehreren Halogenatomen), wobei vicinale Alkyl-, Haloalkyl, Alkoxy- und/oder Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkylanteil gegebenenfalls mit einem oder mehreren Fluoratomen substituiert sein kann,
R¹ für C₁-C₆-Alkyl (gegebenenfalls ein- oder zweifach substituiert mit Substituenten unabhängig voneinander ausgewählt aus C₁-C₆-Alkoxy, Halo-C₁-C₆-alkoxy, C₁- C₆-Alkylsulfanyl, Halo-C₁-C₆-alkylsulfanyl, C₁-C₆-Alkylsulfinyl, Halo-C₁-C₆- alkylsulfinyl, C₁-C₆-Alkylsulfonyl, Halo-C₁-C₆-alkylsulfonyl, C₁-C₆- Alkylcarbonyl, C₁-C₆-Alkoxycarbonyl, Hydroxy, C₃-C₆-Cycloalkyl, Phenyl und Heterocyclyl), C₃-C₆-Cycloalkyl (gegebenenfalls substituiert mit einem oder mehreren Halogenatomen), Halo-C₁-C₆-alkyl (gegebenenfalls substituiert mit C₁- C₆-Alkoxy), Cyano, Formyl, -CH=NO-H, -CH=NO-C₁-C₆-Alkyl, -CH=NO-Halo- C₁-C₆-Alkyl, -C(CH₃)=NO-H, -C(CH₃)=NO-C₁-C₆-Alkyl, -C(CH₃)=NO-Halo- C₁-C₆-Alkyl oder Benzyl (gegebenenfalls einfach oder mehrfach substituiert mit einem oder mehreren Substituenten unabhängig voneinander ausgewählt aus Halogen, C₁-C₆-Alkyl und C₁-C₆-Alkoxy) steht,
R² für Amino steht, gegebenenfalls einfach substituiert mit C₁-C₆-Alkyl, C₁-C₆- Alkylcarbonyl, C₁-C₆-Alkoxycarbonyl-C₁-C₆-alkyl, C₃-C₅-Cycloalkyl, C₃-C₅- Cycloalkyl-C₁-C₃-alkyl, C₁-C₆-Alkenyl, C₁-C₆-Alkoxycarbonyl, C₃-C₆- Alkenoxycarbonyl, C₃-C₆-Alkinoxycarbonyl, C₁-C₆-Alkoxycarbonylcarbonyl, Benzyl oder Phenylcarbonyl (wobei der Phenylring in Benzyl und Phenylcarbonyl wiederum gegebenenfalls substituiert ist mit einem oder mehreren Halogenatomen),
R² weiterhin für Amino steht, welches disubstituiert ist mit zwei Substituenten unabhängig voneinander ausgewählt aus Benzyl, C₁-C₆-Alkyl und C₁-C₆- Alkylcarbonyl, C₁-C₆-Alkoxycarbonyl, C₃-C₆-Alkenoxycarbonyl und C₃-C₆- Alkinoxycarbonyl,
R² weiterhin für Piperidin, Pyrrolidin oder Morpholin steht, welche gegebenenfalls substituiert sind mit ein oder zwei C₁-C₆-Alkyl,
R² weiterhin für Fluor, Chlor, Brom oder Jod steht,
R³ für Fluor, Chlor, Brom, C₁-C₆-Alkyl, Halo-C₁-C₆-alkyl, C₁-C₆-Alkoxy oder Di- C₁-C₆-alkylamino steht und
n für 0 oder 1 steht.

3. Verbindungen der Formel (I) gemäß Anspruch 1 oder 2, **dadurch gekennzeichnet, dass** wenn X für Phenyl steht, substituiert durch C₁-C₅-Alkyl, Cl, F, Br, CF₃, C₂-C₃-Alkenyl, C₂-C₃-Alkinyl, C₁-C₄ Alkoxy, Alkoxyalkyl, Phenyl, Benzyl, O-Phenyl oder O-Benzyl, und R² für NH₂ steht und R¹ für CF₃, C₁-C₃-Alkyl oder C₁-C₃-Alkoxy-C₁-C₃-Alkyl steht und n für 0 steht, dann ist X mindestens zweifach substituiert und für den Fall dass X mit zwei identischen Substituenten substituiert ist, stehen diese zwei Substituenten weder in Position 2 und 4 noch in Position 3 und 4.

4. Verfahren zur Synthese von Verbindungen der Formel (IA-1) 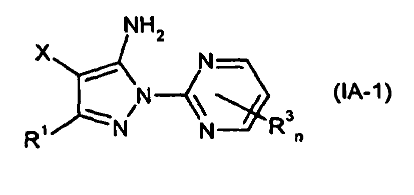 **dadurch gekennzeichnet, dass** eine Verbindung der Formel (V) 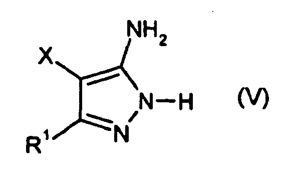 mit einer Verbindung der Formel (VI) 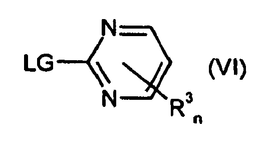 wobei LG für Halogen oder Alkylsulfonyl steht, umgesetzt wird.

5. Verfahren zur Herstellung von Verbindungen der Formel (IA-3) 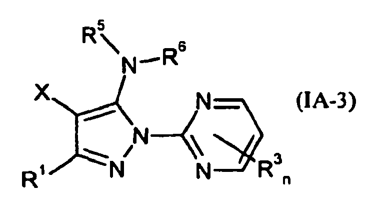 wobei X, R¹, R³ und n die in Anspruch 1 gegebenen Bedeutungen haben können und
R⁵ für Wasserstoff, Alkyl, Alkylcarbonyl, Cycloalkyl, Cycloalkylalkyl, Alkenyl, Alkoxycarbonyl, Alkenoxycarbonyl, Alkinoxycarbonyl, Alkoxycarbonylcarbonyl, Benzyl oder Phenylcarbonyl (wobei der Phenylring in Benzyl und Phenylcarbonyl wiederum gegebenenfalls substituiert ist mit einem oder mehreren Fluor, Brom- und/oder Chloratomen) steht,
R⁶ für Wasserstoff steht,
R6 weiterhin für den Fall, dass R⁵ Benzyl, Alkyl, Alkoxycarbonyl, Alkenoxycarbonyl, Alkinoxycarbonyl oder Alkylcarbonyl ist, ebenfalls Benzyl, Alkyl, Alkoxycarbonyl, Alkenoxycarbonyl, Alkinoxycarbonyl oder Alkylcarbonyl sein kann,
**dadurch gekennzeichnet, dass** Verbindungen der Formel (IA-1) 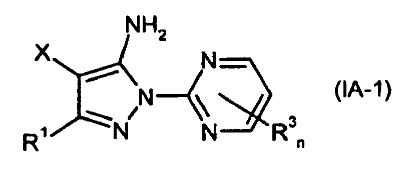 mit einem oder zwei Halogenierungsmitteln R⁵-LG und/oder R⁶-LG, wobei LG für Halogen oder Alkylsulfonyl steht, umgesetzt wird.

6. Verfahren zur Herstellung von Verbindungen der Formel (IB) 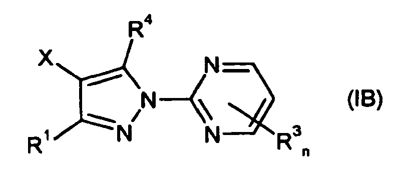 wobei X, R¹, R², R³ und n die in Anspruch 1 angegebenen Bedeutungen haben können, und R⁴ für ein Halogen steht, **dadurch gekennzeichnet, dass** eine Verbindung der Formel (IA-1) 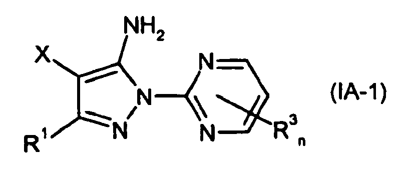 in Anwesenheit von Nitrosylionen mit einem Halogenid umgesetzt wird.

7. Verfahren zur Synthese von Verbindungen der Formel (IA-1-2) 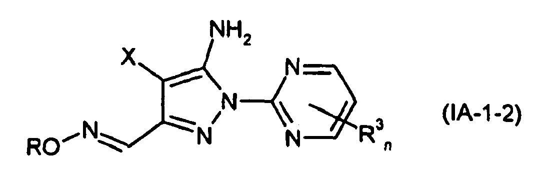 wobei X, R³ und n die in Anspruch 1 gegebenen Bedeutungen haben und R für Alkyl steht, **dadurch gekennzeichnet, dass** eine Verbindung der Formel (IA-1-1) 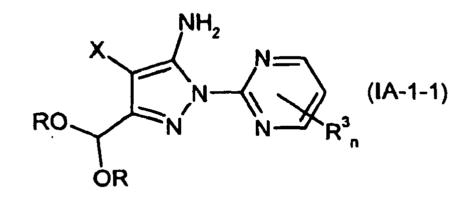 in Gegenwart einer Säure mit einem O-Alkyl-Hydroxylamin R-O-NH₂ umgesetzt wird.

8. Verfahren zur Synthese von Verbindungen der Formel (IA- 1-3) 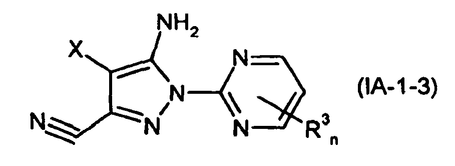 wobei X, R³ und n die in Anspruch 1 angegebenen Bedeutungen haben, **dadurch gekennzeichnet, dass** eine Verbindung der Formel (IA-1-1) 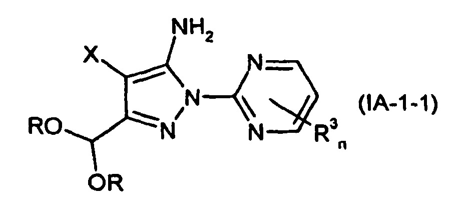 in Gegenwart einer Säure mit Hydroxylamin zu einer Verbindung der Formel (IA-1-4) 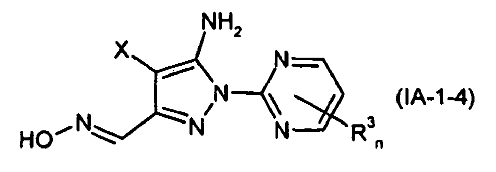 umgesetzt und diese anschliessend zur Verbindung der Formel (IA-1-3) dehydratisiert wird.

9. Verfahren zur Synthese von Verbindungen der Formel IA-1 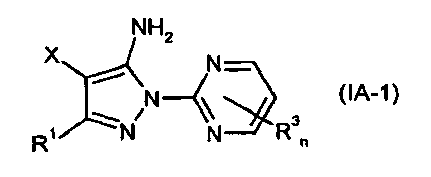 wobei X, R¹ R³ und n die in Anspruch 1 angegebenen Bedeutungen haben, **dadurch gekennzeichnet, dass** eine Verbindung der Formel (IIA) 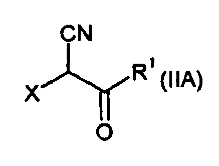 oder eine Verbindung der Formel (IIB) 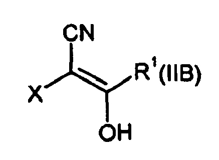 oder eine Verbindung der Formel (IIC) 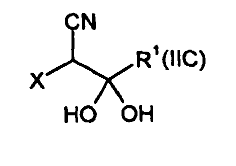 mit einem Chlorierungsmittel zu einer Verbindung der Formel (VII) 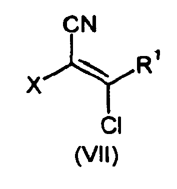 umgesetzt wird und anschließend eine Kondensation mit einer Verbindung der Formel (III) 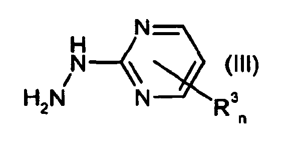 in einem geeigneten organischen Lösungsmittel in Gegenwart von basischen Hilfsreagenzien zur Verbindung der Formel IA erfolgt.

10. Verfahren zur Synthese von Verbindungen der Formel IA-1 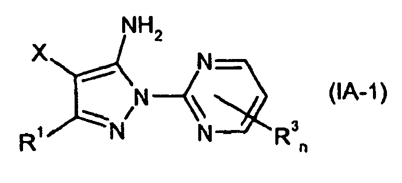 wobei X, R¹ R³ und n die in Anspruch 1 angegebenen Bedeutungen haben, **dadurch gekennzeichnet, dass** eine Verbindung der Formel (IA-1-5) 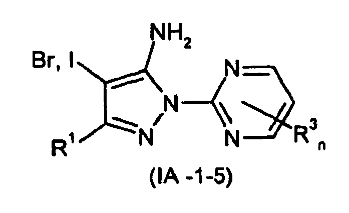 mit einer Verbindung der Formel (VIII)
X-B(OR)₂ (VIII)
in Gegenwart von geeigneten Palladium-Katalysatoren und Basen zu einer Verbindung der Formel (IA-1) umgesetzt wird.

11. Verwendung von Verbindungen der Formel (I) gemäß einem der Ansprüche 1 bis 3 zur Bekämpfung von tierischen Schädlingen ausgewählt aus der Gruppe bestehend aus Insekten, Spinnentieren, Helminthen, Nematoden und Mollusken, die in der Landwirtschaft, im Gartenbau, in Forsten, in Gärten und Freizeiteinrichtungen und im Vorrats- und Materialschutz vorkommen.

12. Verfahren zur Bekämpfung von tierischen Schädlingen ausgewählt aus der Gruppe bestehend aus Insekten, Spinnentieren, Helminthen, Nematoden und Mollusken, die in der Landwirtschaft, im Gartenbau, in Forsten, in Gärten und Freizeiteinrichtungen und im Vorrats- und Materialschutz vorkommen, **dadurch gekennzeichnet, dass** man Verbindungen der Formel (I) gemäß einem der Ansprüche 1 bis 3 auf tierische Schädlinge und/oder deren Lebensraum und/oder Saatgut einwirken lässt.

13. Verfahren zur Herstellung agrochemischer Zusammensetzungen, **dadurch gekennzeichnet, dass** man Verbindungen der Formel (I) gemäß einem der Ansprüche 1 bis 3 mit Streckmitteln und/oder oberflächenaktiven Stoffen vermischt.

## Claims

1. Compounds of the formula (I) 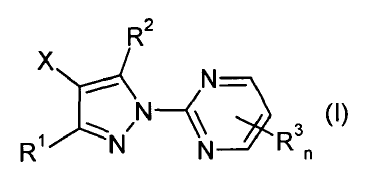 in which
X represents phenyl, 2-pyridyl, 3-pyridyl or 2-pyrimidinyl, in each case substituted by one or more substituents selected from the group consisting of halogen, alkyl, haloalkyl, alkoxy, alkoxyalkyl, alkoxyalkoxy, cycloalkyl, alkenoxy, alkynoxy, benzyloxy, cycloalkylalkoxy, haloalkoxy, haloalkoxyalkyl, alkylsulphanyl, haloalkylsulphanyl, alkylsulphinyl, haloalkylsulphinyl, alkylsulphonyl, haloalkylsulphonyl, cyano, alkylcarbonyl, alkoxycarbonyl, alkoxycarbonylalkyl, carboxyl, carboxamide, dialkylcarboxamide, trialkylsilyl, nitro, amino, alkylamino, dialkylamino, alkylsulphonylamino, dialkylsulphonylamino, -CH=NO-H, -CH=NO- alkyl, -CH=NO-halolkyl, -C(CH₃)=NO-H, -C(CH₃)=NO-alkyl, -C(CH₃)=NO- haloalkyl, formyl, benzyl (for its part optionally substituted by one or more halogen atoms), phenoxy or pyrid-2-yloxy (the latter two optionally substituted by one or more substituents selected from the group consisting of alkyl, halogen and haloalkyl) and phenyl (optionally substituted by one or more halogen atoms), where vicinal alkyl, haloalkyl, alkoxy and/or haloalkoxy groups together with the carbon atoms to which they are attached may form a five- to six-membered cyclic system which contains 0 to 2 oxygen atoms and whose alkyl moiety may optionally be substituted by one or more fluorine atoms,
R¹ represents alkyl (optionally mono- or disubstituted by substituents independently of one another selected from the group consisting of alkoxy, haloalkoxy, alkylsulphanyl, haloalkylsulphanyl, alkylsulphinyl, haloalkylsulphinyl, alkylsulphonyl, haloalkylsulphonyl, alkylcarbonyl, alkoxycarbonyl, hydroxyl, cycloalkyl, phenyl (for its part optionally substituted by one or more halogen atoms) and heterocyclyl), cycloalkyl (optionally substituted by one or more haloalkyl groups and/or halogen atoms), haloalkyl (optionally substituted by alkoxy), cyano, formyl, -CH=NO-H, -CH=NO-alkyl, -CH=NO-haloalkyl, -C(CH₃)=NO-H, -C(CH₃)=NO-alkyl, -C(CH₃)=NO-haloalkyl or benzyl (optionally mono- or polysubstituted by one or more substituents independently of one another selected from the group consisting of halogen, alkyl, haloalkyl and alkoxy),
R² represents amino and
R² furthermore represents amino which is monosubstituted by alkyl, alkylcarbonyl, cycloalkyl, cycloalkylalkyl, alkenyl, alkoxycarbonyl, alkenoxycarbonyl, alkynoxycarbonyl, alkoxycarbonylalkyl, alkoxycarbonylcarbonyl, benzyl or phenylcarbonyl (where the phenyl ring in benzyl and phenylcarbonyl for its part is optionally substituted by one or more halogen atoms) and
R² furthermore represents amino which is disubstituted by two substituents independently of one another selected from the group consisting of benzyl, alkyl, alkoxycarbonyl, alkenoxycarbonyl, alkynoxycarbonyl and alkylcarbonyl,
R² furthermore represents piperidine, pyrrolidine or morpholine which are optionally mono- or disubstituted by alkyl and
R² furthermore represents halogen,
R³ represents halogen, alkyl, haloalkyl, alkoxy or dialkylamino and
n represents 0 or 1,
where the compounds of the general formula (I) also include N-oxides and salts.

2. Compounds of the formula (I) according to Claim 1 in which
X represents phenyl, 2-pyridyl, 3-pyridyl or 2-pyrimidinyl steht, in each case substituted by one or more substituents selected from the group consisting of fluorine, chlorine, bromine, iodine, C₁-C₆-alkyl, C₁-C₆-haloalkyl, C₁-C₆-alkoxy- C₁-C₆-alkyl, C₁-C₆-alkoxy, C₁-C₆-haloalkoxy, C₁-C₆-haloalkoxy-C₁-C₆-alkyl, C₁-C₆-alkylsulphanyl, halo-C₁-C₆-alkylsulphanyl, C₁-C₆-alkylsulphinyl, halo-C₁- C₆-alkylsulphinyl, C₁-C₆-alkylsulphonyl, halo-C₁-C₆-alkylsulphonyl, cyano, C₁- C₆-alkylcarbonyl, C₁-C₆-alkoxycarbonyl, C₁-C₆-alkoxycarbonyl-C₁-C₆-alkyl, nitro, amino, C₁-C₆-alkylamino, di(C₁-C₆-alkyl)amino, C₁-C₆- alkylsulphonylamino, di(C₁-C₆-alkyl)sulphonylamino, -CH=NO-H, -CH=NO-C₁-C₆-alkyl, -CH=NO-halo-C₁-C₆-alkyl, -C(CH₃)=NO-H, -C(CH₃)=NO-C₁-C₆-alkyl, -C(CH₃)=NO-halo-C₁-C₆-alkyl, formyl, benzyl (for its part optionally substituted by one or more halogen atoms), phenoxy or pyrid-2- yloxy (the latter two optionally substituted by one or more halogen atoms and/or halo-C₁-C₆-alkyl groups) and phenyl (optionally substituted by one or more halogen atoms), where vicinal alkyl, haloalkyl, alkoxy and/or haloalkoxy groups together with the carbon atoms to which they are attached may form a five- to six- membered cyclic system which contains 0 to 2 oxygen atoms and whose alkyl moiety may optionally be substituted by one or more fluorine atoms,
R¹ represents C₁-C₆-alkyl (optionally mono- or disubstituted by substituents independently of one another selected from the group consisting of C₁-C₆-alkoxy, halo-C₁-C₆-alkoxy, C₁-C₆-alkylsulphanyl, halo-C₁-C₆-alkylsulphanyl, C₁-C₆- alkylsulphinyl, halo-C₁-C₆-alkylsulphinyl, C₁-C₆-alkylsulphonyl, halo-C₁-C₆- alkylsulphonyl, C₁-C₆-alkylcarbonyl, C₁-C₆-alkoxycarbonyl, hydroxyl, C₃-C₆- cycloalkyl, phenyl and heterocyclyl), C₃-C₆-cycloalkyl (optionally substituted by one or more halogen atoms), halo-C₁-C₆-alkyl (optionally substituted by C₁-C₆- alkoxy), cyano, formyl, -CH=NO-H, -CH=NO-C₁-C₆-alkyl, -CH=NO-halo-C₁-C₆- alkyl, -C(CH₃)=NO-H, -C(CH₃)=NO-C₁-C₆-alkyl, -C(CH₃)=NO-halo-C₁-C₆- alkyl or benzyl (optionally mono- or polysubstituted by one or more substituents independently of one another selected from the group consisting of halogen, C₁- C₆-alkyl and C₁-C6-alkoxy),
R² represents amino which is optionally monosubstituted by C₁-C₆-alkyl, C₁-C₆- alkylcarbonyl, C₁-C₆-alkoxycarbonyl-C₁-C₆-alkyl, C₃-C₅-cycloalkyl, C₃-C₅- cycloalkyl-C₁-C₃-alkyl, C₁-C₆-alkenyl, C₁-C₆-alkoxycarbonyl, C₃-C₆- alkenoxycarbonyl, C₃-C₆-alkynoxycarbonyl, C₁ -C₆-alkoxycarbonylcarbonyl, benzyl or phenylcarbonyl (where the phenyl ring in benzyl and phenylcarbonyl for its part is optionally substituted by one or more halogen atoms),
R² furthermore represents amino which is disubstituted by two substituents independently of one another selected from the group consisting of benzyl, C₁-C₆- alkyl and C₁-C₆-alkylcarbonyl, C₁-C₆-alkoxycarbonyl, C₃-C₆-alkenoxycarbonyl and C₃-C₆-alkynoxycarbonyl,
R² furthermore represents piperidine, pyrrolidine or morpholine which are optionally substituted by one or two C₁-C₆-alkyl,
R² furthermore represents fluorine, chlorine, bromine or iodine,
R³ represents fluorine, chlorine, bromine, C₁-C₆-alkyl, halo-C₁-C₆-alkyl, C₁-C₆- alkoxy or di-C₁-C₆-alkylamino and
n represents 0 or 1.

3. Compounds of the formula (I) according to Claim 1 or 2, **characterized in that**, if X represents phenyl substituted by C₁-C₅-alkyl, Cl, F, Br, CF₃, C₂-C₃-alkenyl, C₂-C₃-alkynyl, C₁-C₄ alkoxy, alkoxyalkyl, phenyl, benzyl, O-phenyl or O-benzyl, and R² represents NH₂ and R¹ represents CF₃, C₁-C₃-alkyl or C₁-C₃-alkoxy-C₁-C₃-alkyl and n represents 0, then X is at least disubstituted, and if X is substituted by two identical substituents, these two substituents are located neither in positions 2 and 4 nor in positions 3 and 4.

4. Process for the synthesis of compounds of the formula (IA-1) 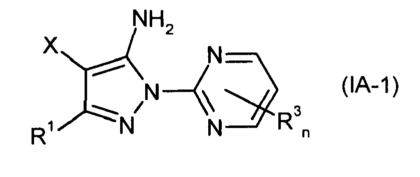 **characterized in that** a compound of the formula (V) 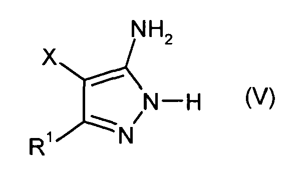 is reacted with a compound of the formula (VI) 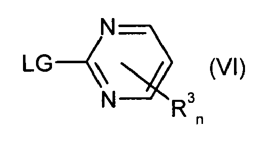 where LG represents halogen or alkylsulphonyl.

5. Process for preparing compounds of the formula (IA-3) 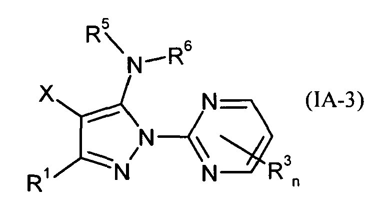 where X, R¹, R³ and n may have the meanings given in Claim 1 and
R⁵ represents hydrogen, alkyl, alkylcarbonyl, cycloalkyl, cycloalkylalkyl, alkenyl, alkoxycarbonyl, alkenoxycarbonyl, alkynoxycarbonyl, alkoxycarbonylcarbonyl, benzyl or phenylcarbonyl (where the phenyl ring in benzyl and phenylcarbonyl for its turn is optionally substituted by one or more fluorine, bromine and/or chlorine atoms),
R⁶ represents hydrogen,
R⁶ furthermore, if R⁵ represents benzyl, alkyl, alkoxycarbonyl, alkenoxycarbonyl, alkynoxycarbonyl or alkylcarbonyl, may also represent benzyl, alkyl, alkoxycarbonyl, alkenoxycarbonyl, alkynoxycarbonyl or alkylcarbonyl,
**characterized in that** a compound of the formula (IA-1) 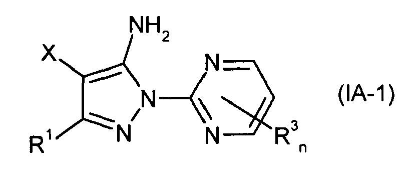 is reacted with one or two halogenating agents R⁵-LG and/or R⁶-LG, where LG represents halogen or alkylsulphonyl.

6. Process for preparing compounds of the formula (IB) 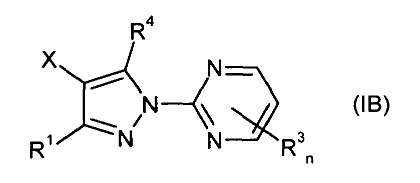 where X, R¹, R², R³ and n may have the meanings given in Claim 1 and R⁴ represents a halogen, **characterized in that** a compound of the formula (IA-1) 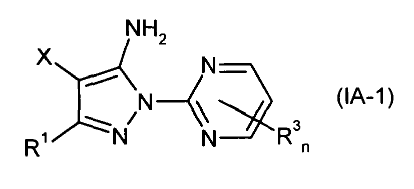 is reacted in the presence of nitrosyl ions with a halide.

7. Process for the synthesis of compounds of the formula (IA-1-2) 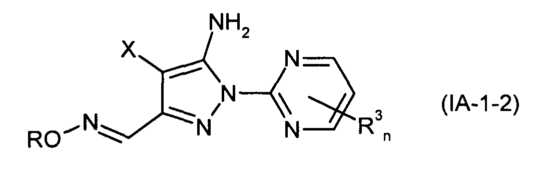 where X, R³ and n have the meanings given in Claim 1 and R represents alkyl, **characterized in that** a compound of the formula (IA-1-1) 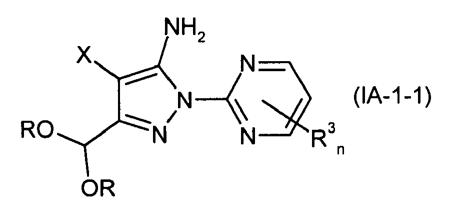 is reacted in the presence of an acid with an O-alkyl hydroxylamine R-O-NH₂.

8. Process for the synthesis of compounds of the formula (IA-1-3) 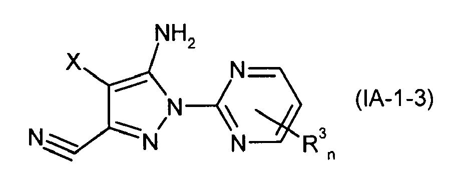 where X, R³ and n have the meanings given in Claim 1, **characterized in that** a compound of the formula (IA-1-1) 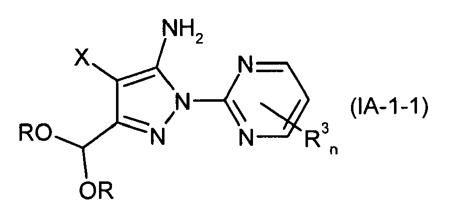 is reacted in the presence of an acid with hydroxylamine to give a compound of the formula (IA-1-4) 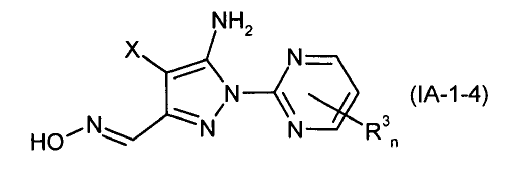 and this is then dehydrated to give the compound of the formula (IA-1-3).

9. Process for the synthesis of compounds of the formula IA-1 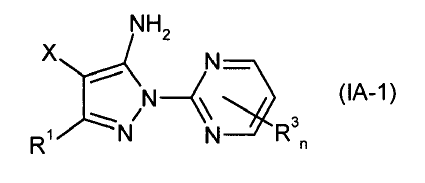 where X, R¹, R³ and n have the meanings given in Claim 1, **characterized in that** a compound of the formula (IIA) 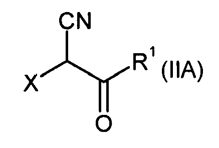 or a compound of the formula (IIB) 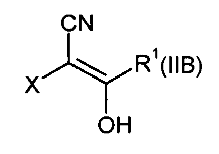 or a compound of the formula (IIC) 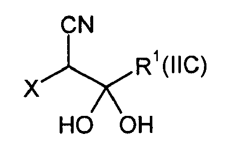 is reacted with a chlorinating agent to give a compound of the formula (VII) 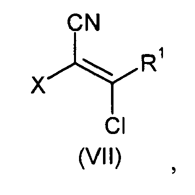 and subsequently a condensation with a compound of the formula (III) 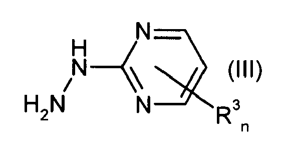 in a suitable organic solvent in the presence of basic auxiliary reagents is carried out to give the compound of the formula IA-1.

10. Process for the synthesis of compounds of the formula IA-1 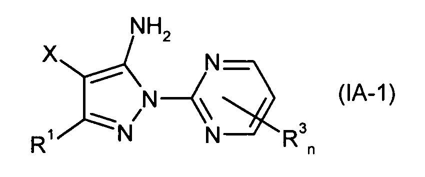 where X, R¹, R³ and n have the meanings given in Claim 1, **characterized in that** a compound of the formula (IA-1-5) 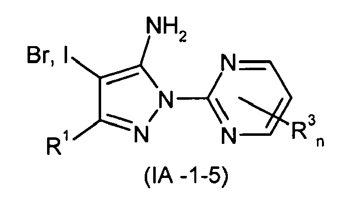 is reacted with a compound of the formula (VIII)
X-B(OR)₂ (VIII)
in the presence of suitable palladium catalysts and bases to give a compound of the formula (IA-1).

11. Use of compounds of the formula (I) according to any of Claims 1 to 3 for controlling animal pests selected from the group consisting of insects, arachnids, helminths, nematodes and molluscs, which are encountered in agriculture, in horticulture, in forests, in gardens and leisure facilities, and in the protection of stored products and of materials.

12. Method for controlling animal pests selected from the group consisting of insects, arachnids, helminths, nematodes and molluscs, which are encountered in agriculture, in horticulture, in forests, in gardens and leisure facilities, and in the protection of stored products and of materials, **characterized in that** compounds of the formula (I) according to any of Claims 1 to 3 are allowed to act on animal pests and/or their habitat and/or seed.

13. Process for preparing agrochemical compositions, **characterized in that** compounds of the formula (I) according to any of Claims 1 to 3 are mixed with extenders and/or surfactants.

## Revendications

1. Composés de formule (I) 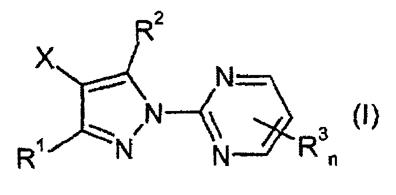 dans laquelle
X représente un groupe phényle, 2-pyridyle, 3-pyridyle ou 2-pyrimidinyle, substitués chacun par un ou plusieurs substituants choisis parmi des atomes d'halogène, des groupes alkyle, halogénoalkyle, alcoxy, alcoxyalkyle, alcoxyalcoxy, cycloalkyle, alcénoxy, alcynoxy, benzyloxy, cycloalkylalcoxy, halogénoalcoxy, halogénoalcoxyalkyle, alkylsulfanyle, halogénoalkylsulfanyle, alkylsulfinyle, halogénoalkylsulfinyle, alkylsulfonyle, halogénoalkylsulfonyle, cyano, alkylcarbonyle, alcoxycarbonyle, alcoxycarbonylalkyle, carboxy, carboxamido, dialkylcarboxamido, trialkylsilyle, nitro, amino, alkylamino, dialkylamino, alkylsulfonylamino, dialkylsulfonylamino, -CH=NO-H, -CH=NO-alkyle, -CH=NO-halogénoalkyle, -C(CH₃)=NO-H, -C(CH₃)=NO-alkyle, -C(CH₃)=NO-halogénoalkyle, formyle, benzyle (éventuellement substitué à son tour par un ou plusieurs atomes d'halogène) phénoxy ou pyrid-2-yloxy (les deux derniers éventuellement substitués par un ou plusieurs substituants choisis parmi des groupes alkyle, halogéno et/ou halogénoalkyle) et phényle (éventuellement substitué par un ou plusieurs atomes d'halogène), des groupes alkyle, halogénoalkyle, alcoxy et/ou halogénoalcoxy vicinaux pouvant former ensemble, avec les atomes de carbone auxquels ils sont liés, un système cyclique à cinq ou six chaînons, qui contient de 0 à 2 atomes d'oxygène, et dont le fragment alkyle peut éventuellement être substitué par un ou plusieurs atomes de fluor,
R¹ représente un groupe alkyle (éventuellement une ou deux fois substitué par des substituants choisis, indépendamment l'un de l'autre, parmi des groupes alcoxy, halogénoalcoxy, alkylsulfanyle, halogénoalkylsulfanyle, alkylsulfinyle, halogénoalkylsulfinyle, alkylsulfonyle, halogénoalkylsulfonyle, alkylcarbonyle, alcoxycarbonyle, hydroxy, cycloalkyle, phényle (à son tour éventuellement substitué par un ou plusieurs atomes d'halogène) et hétérocyclyle), cycloalkyle (éventuellement substitués par un ou plusieurs groupes halogénoalkyle et/ou atomes d'halogène), halogénoalkyle (éventuellement substitués par alcoxy), cyano, formyle, -CH=NO-H, -CH=NO-alkyle, -CH=NO-halogénoalkyle, -C(CH₃)=NO-H, -C(CH₃)=NO-alkyle, -C(CH₃)=NO-halogénoalkyle ou benzyle (éventuellement une ou plusieurs fois substitué par un ou plusieurs substituants choisis, indépendamment les uns de autres, parmi des atomes d'halogène, des groupes alkyle, halogénoalkyle et alcoxy),
R² représente un groupe amino et
R² représente en outre un groupe amino qui est monosubstitué par un groupe alkyle, alkylcarbonyle, cycloalkyle, cycloalkylalkyle, alcényle, alcoxycarbonyle, alcénoxycarbonyle, alcynoxycarbonyle, alcoxycarbonylalkyle, alcoxycarbonylcarbonyle, benzyle ou phénylcarbonyle (le cycle phényle dans les groupes benzyle et phénylcarbonyle étant à son tour éventuellement substitué par un ou plusieurs atomes d'halogène) et
R² représente en outre un groupe amino qui est disubstitué par deux substituants choisis, indépendamment l'un de l'autre, parmi des groupes benzyle, alkyle, alcoxycarbonyle, alcényloxycarbonyle, alcynoxycarbonyle et alkylcarbonyle,
R² représente en outre un cycle pipéridine, pyrrolidine ou morpholine, cycles qui sont éventuellement une ou deux fois substitués par alkyle et
R² représente en outre un atome d'halogène,
R³ représente un atome d'halogène, un groupe alkyle, halogénoalkyle, alcoxy ou dialkylamino et
n représente 0 ou 1,
les composés de formule générale (I) comprennent en outre des N-oxydes et des sels.

2. Composés de formule (I) selon la revendication 1, dans lesquels
X représente un groupe phényle, 2-pyridyle, 3-pyridyle ou 2-pyrimidinyle, substitués chacun par un ou plusieurs substituants choisis parmi les atomes de fluor, chlore, brome, iode, des groupes alkyle en C₁-C₆, halogénoalkyle en C₁-C₆, alcoxy(C₁-C₆)-alkyle(C₁-C₆), alcoxy en C₁-C₆, halogénoalcoxy en C₁-C₆, halogénoalcoxy(C₁-C₆) -alkyle (C₁-C₆), alkyl(C₁-C₆) sulfanyle, halogénoalkyl(C₁-C₆)sulfanyle, alkyl(C₁-C₆)-sulfinyle, halogénoalkyl(C₁-C₆)sulfinyle, alkyl(C₁-C₆) sulfonyle, halogénoalkyl(C₁-C₆)-sulfonyle, cyano, alkyl(C₁-C₆) carbonyle, alcoxy(C₁-C₆)carbonyle, alcoxy(C₁-C₆)carbonyl-alkyle(C₁-C₆), nitro, amino, alkyl(C₁-C₆)amino, di [alkyl(C₁-C₆)]amino, alkyl(C₁-C₆) sulfonylamino, di[alkyl(C₁-C₆)]sulfonylamino, -CH=NO-H, -CH=NO-alkyle (C₁-C₆), -CH=NO-halogénoalkyle (C₁-C₆), -C(CH₃) =NO-H, -C(CH₃) =NO-alkyle(C₁-C₆), -C(CH₃) =NO-halogénoalkyle (C₁-C₆), formyle, benzyle (éventuellement substitué à son tour par un ou plusieurs atomes d'halogène), phénoxy ou pyrid-2-yloxy (les deux derniers éventuellement substitués par un ou plusieurs atomes d'halogène et/ou groupes halogénoalkyle en C₁-C₆) et phényle (éventuellement substitué par un ou plusieurs atomes d'halogène), des groupes alkyle, halogénoalkyle, alcoxy et/ou halogénoalcoxy vicinaux pouvant former ensemble, avec les atomes de carbone auxquels ils sont liés, un système cyclique à cinq ou six chaînons, qui contient de 0 à 2 atomes d'oxygène, et dont le fragment alkyle peut éventuellement être substitué par un ou plusieurs atomes de fluor,
R¹ représente un groupe alkyle en C₁-C₆ (éventuellement une ou deux fois substitué par des substituants choisis, indépendamment l'un de l'autre, parmi des groupes alcoxy en C₁-C₆, halogénoalcoxy en C₁-C₆, alkyl (C₁-C₆) sulfanyle, halogénoalkyl (C₁-C₆) sulfanyle, alkyl (C₁-C₆) sulfinyle, halogénoalkyl (C₁-C₆) sulfinyle, alkyl (C₁-C₆) sulfonyle, halogénoalkyl (C₁-C₆) sulfonyle, alkyl (C₁-C₆) carbonyle, alcoxy (C₁-C₆) carbonyle, hydroxy, cycloalkyle en C₃-C₆, phényle et hétérocyclyle), cycloalkyle en C₃-C₆ (éventuellement substitués par un ou plusieurs atomes d'halogène), halogénoalkyle (C₁-C₆) (éventuellement substitué par alcoxy en C₁-C₆), cyano, formyle, -CH=NO-H, -CH=NO-alkyle (C₁-C₆) , -CH=NO-halogénoalkyle (C₁-C₆), -C(CH₃) =NO-H, -C(CH₃) =NO-alkyle (C₁-C₆), -C(CH₃) =NO-halogénoalkyle (C₁-C₆) ou benzyle (éventuellement un ou plusieurs fois substitué par un ou plusieurs substituants choisis, indépendamment les uns de autres, parmi des atomes d'halogène, des groupes alkyle en C₁-C₆ et alcoxy en C₁-C₆) ,
R² représente un groupe amino, éventuellement monosubstitué par un groupe alkyle en C₁-C₆, alkyl(C₁-C₆)carbonyle, alcoxy(C₁-C₆)carbonyl-alkyle(C₁-C₆), cycloalkyle en C₃-C₅, cycloalkyl(C₃-C₅)alkyle(C₁-C₃) , alcényle en C₁-C₆, alcoxy(C₁- C₆) carbonyle, alcénoxy(C₃-C₆)carbonyle, alcynoxy(C₃-C₆)carbonyle, alcoxy(C₁-C₆)carbonyl-carbonyle, benzyle ou phénylcarbonyle (le cycle phényle dans les groupes benzyle et phénylcarbonyle étant à son tour éventuellement substitué par un ou plusieurs atomes d'halogène),
R² représente en outre un groupe amino qui est disubstitué par deux substituants choisis, indépendamment l'un de l'autre, parmi des groupes benzyle, alkyle en C₁-C₆ et alkyl(C₁-C₆) carbonyle, alcoxy(C₁-C₆) carbonyle, alcénoxy(C₃-C₆)carbonyle et alcynoxy(C₃-C₆)carbonyle,
R² représente en outre un cycle pipéridine, pyrrolidine ou morpholine, cycles qui sont éventuellement substitués par un ou deux groupes alkyle en C₁-C₆,
R² représente en outre un atome de fluor, chlore, brome ou iode,
R³ représente un atome de fluor, chlore, brome, un groupe alkyle en C₁-C₆, halogénoalkyle en C₁-C₆, alcoxy en C₁-C₆ ou di [alkyl(C₁-C₆)]amino et
n représente 0 ou 1.

3. Composés de formule (I) selon la revendication 1 ou 2, **caractérisés en ce que** lorsque X représente un groupe phényle substitué par alkyle en C₁-C₅, Cl, F, Br, CF₃, alcényle en C₂-C₃, alcynyle en C₂-C₃, alcoxy en C₁-C₄, alcoxyalkyle, phényle, benzyle, O-phényle ou O-benzyle, et R² représente NH₂ et R¹ représente CF₃, un groupe alkyle en C₁-C₃ ou alcoxy(C₁-C₃)-alkyle(C₁-C₃) et n représente 0, alors X est au moins deux fois substitué et dans le cas où X est substitué par deux substituants identiques, ces deux substituants ne sont ni en position 2 et 4 ni en position 3 et 4.

4. Procédé pour la synthèse de composés de formule (IA-1) 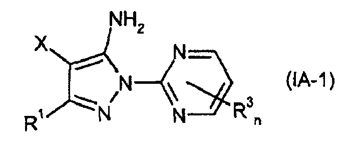 **caractérisé en ce qu'**on fait réagir un composé de formule (V) 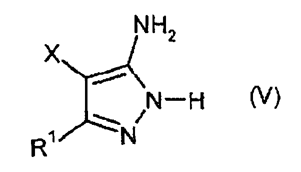 avec un composé de formule (VI) 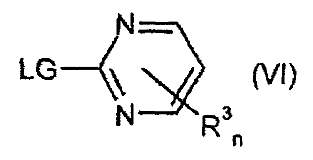 dans laquelle LG représente un atome d'halogène ou un groupe alkylsulfonyle.

5. Procédé pour la préparation de composés de formule (IA-3) 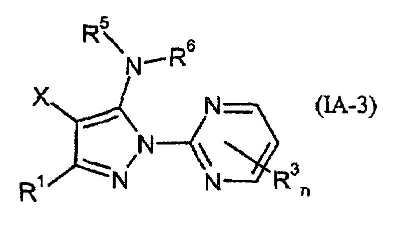 dans laquelle X, R¹, R³ et n peuvent avoir les significations données dans la revendication 1 et
R⁵ représente un atome d'hydrogène, un groupe alkyle, alkylcarbonyle, cycloalkyle, cycloalkylalkyle, alcényle, alcoxycarbonyle, alcénoxycarbonyle, alcynoxycarbonyle, alcoxycarbonylcarbonyle, benzyle ou phénylcarbonyle (le cycle phényle dans les groupes benzyle et phénylcarbonyle étant à son tour éventuellement substitué par un ou plusieurs atomes de fluor, brome et/ou chlore),
R⁶ représente un atome d'hydrogène,
R⁶ peut en outre, dans le cas où R⁵ est un groupe benzyle, alkyle, alcoxycarbonyle, alcénoxycarbonyle, alcynoxycarbonyle ou alkylcarbonyle, être également un groupe benzyle, alkyle, alcoxycarbonyle, alcénoxycarbonyle, alcynoxycarbonyle ou alkylcarbonyle,
**caractérisé en ce qu'**on fait réagir des composés de formule (IA-1) 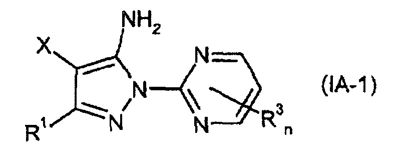 avec un ou deux agents d'halogénation R⁵-LG et/ou R⁶-LG, LG représentant un atome d'halogène ou un groupe alkylsulfonyle.

6. Procédé pour la préparation de composés de formule (IB) 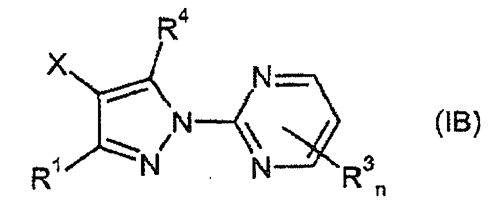 dans laquelle X, R¹, R², R³ et n peuvent avoir les significations indiquées dans la revendication 1, et R⁴ représente un atome d'halogène, **caractérisé en ce qu'**on fait réagir un composé de formule (IA-1) 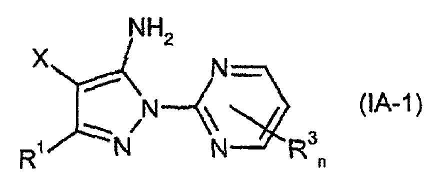 avec un halogénure en présence d'ions nitrosyle.

7. Procédé pour la synthèse de composés de formule (IA-1-2) 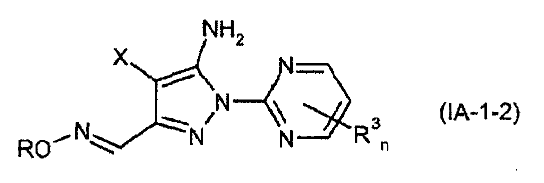 dans laquelle X, R³ et n ont les significations données dans la revendication 1 et R représente un groupe alkyle, **caractérisé en ce qu'**on fait réagir un composé de formule (IA-1-1) 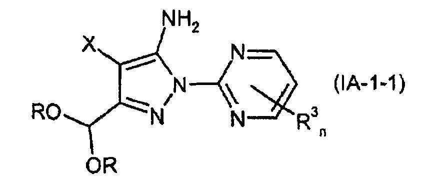 avec une O-alkyl-hydroxylamine R-O-NH₂ en présence d'un acide.

8. Procédé pour la synthèse de composés de formule (IA-1-3) 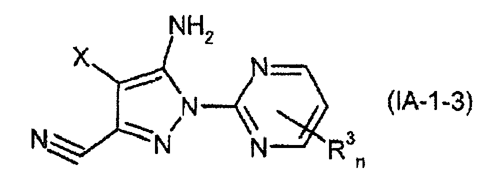 dans laquelle X, R³ et n ont les significations indiquées dans la revendication 1, **caractérisé en ce qu'**on fait réagir un composé de formule (IA-1-1) 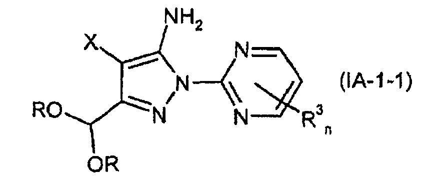 avec de l'hydroxylamine en présence d'un acide, pour aboutir à un composé de formule (IA-1-4) 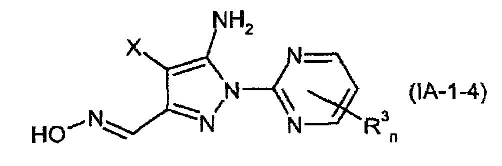 et on soumet ensuite ce dernier à une déshydratation pour aboutir au composé (IA-1-3).

9. Procédé pour la synthèse de composés de formule IA-1 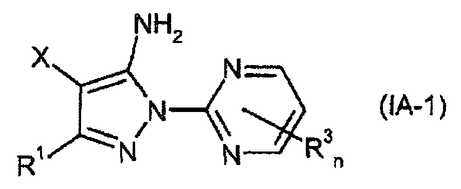 dans laquelle X, R¹, R³ et n ont les significations données dans la revendication 1, **caractérisé en ce qu'**on fait réagir un composé de formule (IIA) 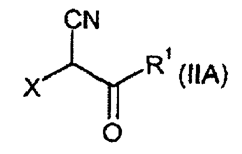 ou un composé de formule (IIB) 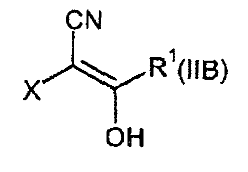 ou un composé de formule (IIC) 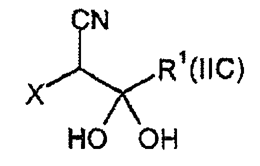 avec un agent de chloration, pour aboutir à un composé de formule (VII) 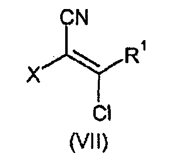 et on effectue ensuite une condensation avec un composé de formule (III) 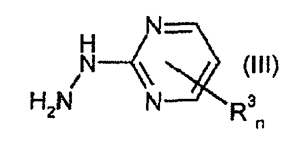 dans un solvant organique approprié, en présence de réactifs auxiliaires basiques, pour aboutir au composé de formule IA-1.

10. Procédé pour la synthèse de composés de formule (IA-1) 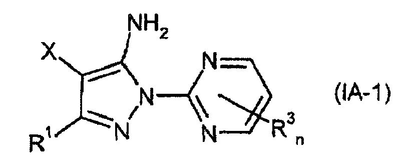 dans laquelle X, R¹, R³ et n ont les significations indiquées dans la revendication 1, **caractérisé en ce qu'**on fait réagir un composé de formule (IA-1-5) 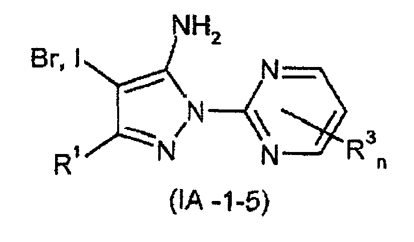 avec un composé de formule (VIII)
X-B(OR)₂ (VIII)
En présence de catalyseurs au palladium appropriés et de bases, pour aboutir à un composé de formule (IA-1).

11. Utilisation de composés de formule (I) selon l'une quelconque des revendications 1 à 3, pour la lutte contre des ravageurs animaux choisis dans le groupe constitués par les insectes, les arachnides, les helminthes, les nématodes et les mollusques, qui apparaissent en agriculture, en horticulture, dans les forêts, dans les jardins et dans les aménagements de loisir et dans la protection de stocks et de matériaux.

12. Procédé pour la lutte contre des ravageurs animaux choisis dans le groupe constitués par les insectes, les arachnides, les helminthes, les nématodes et les mollusques, qui apparaissent en agriculture, en horticulture, dans les forêts, dans les jardins et dans les aménagements de loisir et dans la protection de stocks et de matériaux, **caractérisé** et ce qu'on fait agir des composés de formule (I) selon l'une quelconque des revendications 1 à 3 sur les ravageurs animaux et/ou leur habitat et/ou des semences.

13. Procédé pour la préparation de compositions agronomiques, **caractérisé en ce qu'**on mélange des composés de formule (I) selon l'une quelconque des revendications 1 à 3 avec des diluants et/ou des substances tensioactives.
